# EUROPEAN PATENT SPECIFICATION

(11) **EP 3 429 609 B1**
(45) Date of publication and mention of the grant of the patent: **01.03.2023**
(21) Application number: 17767569.1
(22) Date of filing: 16.03.2017
(51) Int. Cl.: A61K 38/00, A61K 38/17, A61K 38/39

(54) **COMPOSITIONS AND METHODS FOR TREATMENT OF TYPE VII COLLAGEN DEFICIENCIES**
ZUSAMMENSETZUNGEN UND VERFAHREN ZUR BEHANDLUNG VON MÄNGELN VON KOLLAGEN TYP VII
COMPOSITIONS ET PROCÉDÉS POUR LE TRAITEMENT DE DÉFICITS EN COLLAGÈNE DE TYPE VII

(30) Priority: 18.03.2016 US 201662310623 P
(43) Date of publication of application: 23.01.2019
(73) Proprietor: Precigen, Inc., Germantown, Maryland 20876 (US)
(72) Inventor: DAILEY, Vernon, Walkersville, MD 21793 (US); CHAKIATH, Marion, Darnestown, MD 20874 (US); ZHANG, Shyuan, Boyds, MD 20841 (US); MASLOWSKI, John, Exton, PA 19341 (US); MALYALA, Anna, Exton, PA 19341 (US)
(74) Representative: Pohlman, Sandra M.
(86) International application number: PCT/US2017/022800
(87) International publication number: WO 2017/161180

(56) References cited:
- EP-B1- 0 378 576
- US-A1- 2006 258 006
- US-A1- 2009 162 411
- US-A1- 2011 028 694
- US-A1- 2013 237 485
- M P Marinkovich ET AL: "Pre-Clinical Development of a Genetically-Modified Human Dermal Fibroblast (FCX-007) for the Treatmentof Recessive Dystrophic Epidermolysis Bullosa", Poster presented at the American Society of Human Genetics Annual Meeting 2015 Baltimore, 1 January 2015 (2015-01-01), XP055617051, Retrieved from the Internet: URL:http://fibrocell.com/wp-content/upload s/2015/12/ASHG-2015-poster_RDEB.pdf [retrieved on 2019-08-30]
- GEORGIADIS ET AL.: 'Lentiviral Engineered Fibroblasts Expressing Codon-Optimized COL7A1 Restore Anchoring Fibrils in RDEB' JOURNAL OF INVESTIGATIVE DERMATOLOGY vol. 136, 22 September 2015, pages 284 - 292, XP055351934
- SCHAMBACH ET AL.: 'Woodchuck hepatitis virus post-transcriptional regulatory element deleted from X protein and promoter sequences enhances retroviral vector titer and expression' GENE THERAPY vol. 13, 15 December 2005, pages 641 - 645, XP002374147
- SUSPENE ET AL.: 'Twin gradients in APOBEC3 edited HIV-1 DNA reflect the dynamics of lentiviral replication' NUCLEIC ACIDS RESEARCH vol. 34, 08 September 2006, pages 4677 - 4684, XP055422965
- LOIS ET AL.: 'Germline Transmission and Tissue-Specific Expression of Transgenes Delivered by . Lentiviral Vectors' SCIENCE vol. 295, 01 February 2002, pages 868 - 872, XP002288582
- FINE ET AL.: 'Pseudosyndactyly and musculoskeletal contractures in inherited epidermolysis bullosa: experience of the National Epidermolysis Bullosa Registry , 1986-2002' J HAND SURG BR vol. 30, 01 February 2005, pages 14 - 22, XP004705033
- SERRANO ET AL.: 'A Comparison of Targeting Performance of Oncoretroviral Versus Lentiviral . Vectors on Human Keratinocytes' HUMAN GENE THERAPY vol. 14, 01 November 2003, pages 1579 - 1585, XP055422977

## Description

### SEQUENCE LISTING

The instant application contains a Sequence Listing which has been submitted electronically in ASCII format. Said ASCII copy, created on March 9, 2017, is named 0100-0016PR1_SL.txt and is 92,924 bytes in size.

### TECHNICAL FIELD OF THE INVENTION

The present invention relates to the treatment of Type VII collagen deficiencies such as dystrophic epidermolysis bullosa comprising the administration of autologous genetically modified cells comprising a nucleotide sequence encoding Type VII collagen (C7) or a functional variant thereof.

### BACKGROUND OF THE INVENTION

Type VII collagen (C7) is a protein important for anchoring fibril formation at the dermal-epidermal junction (DEJ), which holds together the layers of skin (Burgeson 1993; Leigh *et al.* 1988). Dystrophic epidermolysis bullosa (DEB) is an inherited disease characterized by a deficiency in C7 resulting in an impairment of anchoring fibrils to adhere between the epidermis and the underlying dermis affecting the skin and organs. Mutations of the COL7A1 gene encoding C7 causes the C7 deficiency. Patients suffering from DEB are highly susceptible to severe blistering. Dominant dystrophic epidermolysis bullosa (DDEB) is characterized by generalized blistering. Transient bullous dermatosis is a form of DDEB, which corrects itself during infancy. Recessive dystrophic epidermolysis bullosa (RDEB) is a debilitating skinblistering disorder. RDEB inversa is another form of RDEB, where blisters form on areas where skin rubs on skin. Without these fibrils, skin layers separate causing severe blistering, open wounds and scarring in response to any kind of friction, including normal daily activities like rubbing or scratching.

There are currently no curative treatments and patients largely rely on palliative wound care. Preclinical studies employing a number of strategies show encouraging results suggesting that the effects of the disease can be corrected by restoring C7 function in RDEB skin. Initial approaches relied on grafting fragile engineered epidermal tissue and require removal of the skin tissue to be replaced, resulting in scarring (Mavilio *et al.* 2006; Chen, Nat. Genet. 2002; Siprashvili 2010). Other methods have included delivery of the corrective C7 by direct virusbased expression and systemic delivery of recombinant protein (Remington 2009; Woodley *et al.* 2003). All of these potential treatments have a number of drawbacks including cost and biosafety concerns.

The use of replication-defective, self-inactivating (SIN) lentiviral vectors (LV) offers several advantages for gene therapy: 1) ability to transduce both dividing and non-dividing cells, 2) high transduction efficiency, 3) long-term sustained transgene expression, 4) the ability to dispense sequences encoding viral proteins that might trigger an immune response, 5) an increased safety profile due to transcriptionally inactive SIN long terminal repeat (LTR), and 6) an ability to package large transgenes. Safety studies from clinical trials using LV vectors for gene therapy have showed no preferential integration in or near proto-oncogenes or tumor suppressor genes. Patients who have been dosed at 10 billion LV vector-transduced T cells per subject and followed for a median of 4 years, showed no leukemia or other adverse events. Trials with the indications of metachromatic leukodystrophy, Wiskott-Aldrich Syndrome (WAS) and X- adrenoleukodystrophy (ALD), revealed no aberrant clonal expansion seen up to 21 months, 32 months, and 36 months of follow up, respectively (Biffi *et al.* 2013; Aiuti *et al.* 2013).

Treatment with autologous genetically-modified human dermal fibroblasts (GM-HDF) offers a promising alternative based on correcting the hereditary defect ex vivo in the patients' own fibroblasts (Ortiz-Urda *et al.* 2003; Woodley *et al.* 2003). Georgiadis et al. (J.Investigative Dermatology 136:284-92 (2015)) described transduction of RDEB fibroplasts with a self-inactivating-lentiviral vector encoding codon-optimized COL7a1. It has been reported that hCOL7A1 gene-corrected fibroblasts are better than keratinocytes at supplying C7 to the basement membrane zone (BMZ) in mice with RDEB skin grafts (Goto *et al.* 2006). In addition, injection of fibroblasts can be more easily completed than grafting of keratinocytes.

### SUMMARY OF THE INVENTION

The present invention is directed to autologous genetically modified cells for use in a method of treating a patient suffering from a Type VII collagen (C7) deficiency, wherein said method comprises: (i) contacting cells, obtained from the dermis or epidermis, preferably fibroblasts or keratinocytes, of the C7-deficient patient, with a transducing lentiviral vector comprising a nucleotide sequence encoding *COL7A1* or a functional variant thereof to form the autologous genetically modified cells having a vector copy number wherein said lentiviral vector has a transducing vector copy number in the range of 0.1 to 5.0 copies per cell; (ii) culturing said autologous genetically modified cells; and administering an effective amount of the genetically modified cells to the C7-deficient patient. Wherein the transducing lentiviral vector further comprises (a) a modif 5' long terminal repeat (LTR), wherein the promoter of the modified 5' LTR is is a cytomegalovirus promoter, (b) at least one lentiviral central polypurine tract element, and (c) a modified 3' LTR, wherein the modified 3' LTR comprises a deletion relative to the wild-type 3' LTR, wherein a hepatitis virus post-transcriptional regulatory element (PRE) has been deleted administration may be done by injection, preferably intradermal injection. In one aspect of the invention, the C7 deficiency is dystrophic epidermolysis bullosa (DEB), such as recessive dystrophic epidermolysis bullosa (RDEB) or dominant dystrophic epidermolysis bullosa (DDEB). Subtypes of RDEB are also envisioned to be treated according to the present invention including Hallopeau-Siemens, non-Hallopeau-Siemens RDEB, RDEB inversa, pretibial RDEB, acral RDEB, and RDEB centripetalis. In one embodiment of the invention, the transducing lentiviral vector is in the form of a lentiviral vector particle.

In one embodiment of the invention, the transducing lentiviral vector particle is constructed from a transfer lentiviral vector comprising (a) a modified 5' long terminal repeat in LTR, wherein the promoter of the modified 5' LTR is a cytomegalovirus promoter, (b) the *COL7A1* gene or a functional variant thereof, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted, wherein the *COL7A1* gene or functional variant is incorporated into the cells to form genetically modified cells having a functional *COL7A1* gene or functional variant thereof.

In an embodiment, there are at least two lentiviral central polypurine tract elements. In another embodiment, the PRE is woodchuck hepatitis virus post-transcriptional regulatory element (WPRE). Preferably, the transfer lentiviral vector used to construct the transducing vector is selected from the group consisting of pSMPUW and pFUGW. Preferably, the transducing lentiviral vector particle is INXN-2004 or INXN-2002.

The INXN-2004 lentiviral vector particle comprises a transfer vector comprising the amino acid sequence set forth in SEQ ID NO: 34.

The INXN-2002 lentiviral vector particle is constructed from a pSMPUW lentiviral expression plasmid comprising (a) a modified 5' long terminal repeat in LTR, wherein the promoter of the modified 5' LTR is a cytomegalovirus promoter, (b) a functional COL7A 1 gene, (c) at least one lentiviral central polypurine tract, and (d) a modified 3' LTR, wherein the modified 3' LTR comprises a deletion relative to the wild-type 3' LTR, wherein a hepatitis virus posttranscriptional regulatory element (PRE) has been deleted.

Administration of the genetically modified fibroblasts autologous to the C7-deficient patient may be done in any suitable manner, including by injection, topically, orally, or embedded in a biocompatible matrix.

Another aspect of the invention is directed to autologous genetically modified fibroblasts from a C7-deficient patient, such as RDEB or DDEB patient, transduced with a a lentiviral vector particle comprising a functional *COL7A1* gene or a functional variant thereof, and expressing type VII collagen, wherein the lentiviral vector particle has a transducing vector copy number in the range of 0.1 to 5.0 copies per cell, wherein said transducing lentiviral vector particle is constructed from a transfer lentiviral vector comprising (a) a modified 5' long terminal repeat in LTR, wherein the promoter of the modified 5' LTR is a cytomegalovirus promoter, (b) a functional COL7A 1 gene, (c) at least one lentiviral central polypurine tract, and (d) a modified 3' LTR, wherein the modified 3' LTR comprises a deletion relative to the wild-type 3' LTR, wherein a hepatitis virus posttranscriptional regulatory element (PRE) has been deleted, wherein the *COL7A1* gene or the functional variant thereof is incorporated into the cells to form genetically modified cells having a functional *COL7A1* gene.

Another embodiment is directed to a self-inactivating lentiviral vector formed from a transfer vector comprising (a) a modified 5' long terminal in LTR, wherein the promoter of the modified 5' LTR is a cytomegalovirus promoter, (b) the *COL7A1* gene or a functional variant thereof, (c) at least one lentiviral central polypurine tract element, and (d) a modified 3' LTR, wherein the modified 3' LTR comprises a deletion relative to the wild-type 3' LTR, and wherein a hepatitis virus post-transcriptional regulatory element (PRE) has been deleted, wherein the deleted PRE is a woodchuck post-transcriptional regulatory element PRE(WPRE). In one embodiment, the vector comprises at least two lentiviral central polypurine tract elements. The vector of the invention includes the vector designated INXN-2004 and comprising the sequence of IGE308 plasmid (SEQ) ID NO: 34); or the vector designated INXN-2002 herein.

Another embodiment of the invention is directed to pharmaceutical compositions comprising a fibroblast obtained from a patient suffering from RDEB or DDEB transduced with a lentiviral vector according to the claims, such as the lentiviral vector designated INXN-2004 comprising the sequence of IGE308 plasmid; or the lentiviral vector designated INXN-2002 herein.

In one embodiment, the present invention is directed to cells transduced in vitro or ex vivo using the vectors according to the claims, such as INXN-2002 and INXN-2004.

In one embodiment, the present invention is directed to transduced autologous fibroblasts using vectors INXN-2002 or INXN-2004, which may be obtained and propagated according to methods described in U.S. Patent No. 8,728,819 and International Patent Application/

WO2008/027984 entitled "Methods for culturing minimally-passaged fibroblasts and uses thereof".

The present invention also relates to patient an autologous population of cells for use in treating a suffering from pseudosyndactyly, wherein said autologous population of cells is to be obtained from said patient and transduced with a lentiviral vector particle comprising a functional *COL7A1* gene or a functional variant thereof, and expressing type VII collagen, wherein said lentiviral vector particle has a transducing vector copy number in the range of 0.1 to 5.0 copies per cell, wherein said transducing lentiviral vector particle is constructed from a transfer lentiviral vector comprising (a) a modified 5' long terminal repeat in LTR, wherein the promoter of the modified 5' LTR is a cytomegalovirus promoter, (b) a functional COL7A 1 gene, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted, wherein the *COL7A1* gene or the functional variant thereof is incorporated into the cells to form genetically modified cells having a functional *COL7A1* gene.

The invention further relates to a medical use for treating, inhibiting or reducing the blistering of a dystrophic epidermolysis bullosa (DEB) or for treating lesions in patients suffering from RDEB comprising administering the autologous genetically modified cells of the present invention.

Another aspect of the invention relates to isolated population of genetically modified fibroblasts autologous to a C7-deficient patient, such as DDEB or RDEB patient, transduced with a vector particle comprising a functional *COL7A1* gene or functional variant thereof and expressing type VII collagen, as well as methods of making these isolated populations, as defined by the claims.

The invention also relates to a method of increasing the integrated transgene copy number per cell in genetically modified human dermal fibroblasts or keratinocytes comprising (a) contacting a transducing lentiviral vector comprising a nucleotide sequence encoding a *COL7A1* gene or a functional variant thereof with a human dermal fibroblast or keratinocyte obtained from a C7-deficient patient to form a transduction composition; (b) subjecting the first transduction composition to spinoculation to form transduced human dermal fibroblast or keratinocyte; (c) contacting the transduced human dermal fibroblasts or keratinocytes of step (b) with a second transducing lentiviral vector to form na second transduction composition; and (d) subjecting said first transduction composition to spinoculation to form transduced human dermal fibroblast or keratinocyte, wherein the integrated transgene copy number of the transduced human dermal fibroblasts or keratinocytes is at least 5, 10, 15, 20, 25, 27, 28, 29, 30, 35, 40, 45, or 50- fold higher relative to transduced human dermal fibroblasts or keratinocytes not subjected to spinoculation or a second transduction step, and wherein the transducing lentiviral vector further comprises (a) a modified 5' long terminal repeat (LTR), wherein the promoter of the modified 5' LTR is a cytomegalovirus promoter, (b) at least one lentiviral central polypurine tract element, and (c) a modified 3' LTR, wherein the modified 3' LTR comprises a deletion relative to the wild-type 3' LTR, wherein a hepatitis virus post- transcriptional regulatory element (PRE) has been deleted. In one aspect, the transducing lentiviral vector is INXN-2002 or INXN-2004, preferably INXN-2004. It has been found that the the transduced human dermal fibroblast or keratinocytes have at least or about 1, 2, 5, 10, 20, 25, 27, 28, 29, 30, 35, 40, 45, or 50-fold greater integrated transgene copy number per cell relative to a transduction composition not subjected to spinoculation or super-transduction. In another embodiment, the invention relates to having an integrated transgene copy number per cell of at least 0.05, 0.09, 0.41, or 0.74 or a range of between about 0.1 to about 5, 0.1 to about 1, 0.4 to about 1, or 0.4 to about 0.75. Expression has been found to be increased using the transduction methods described herein. For example, it has been found that expression of C7 has increased by 10, 25, 50, 100, 150, or 200-fold relative to transduced human dermal fibroblasts or keratinocytes not subjected to spinoculation or a second transduction.

### BRIEF DESCRIPTION OF THE DRAWINGS

A more complete understanding of the present invention may be obtained by reference to the accompanying drawings, when considered in conjunction with the subsequent detailed description. The embodiments illustrated in the drawings are intended only to exemplify the invention and should not be construed as limiting the invention to the illustrated embodiments.
Figure 1A depicts type VII collagen (C7) trimers, which form anchoring fibrils. The *COL7A1* gene encodes a 290-kDa alpha chain and three of the chains form a triple helix (trimer). Image from Bruckner- Tuderman L. Molecular Therapy (2008) 17:6-7.
Figure 1B depicts the general structure of normal and RDEB skin. C7 anchoring fibrils bind to other collagens, extracellular matrix proteins, and Lam332 to mediate attachment of the dermis to the epidermis. Absence of anchoring fibrils can lead to blister formation.

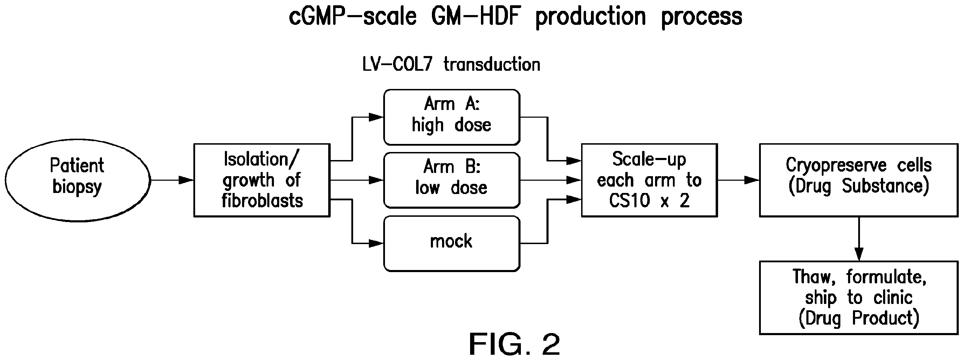

Figure 2 describes a cGMP-scale GM-HDF production process according to one aspect of the present invention. A C7 expression cassette was cloned into a self-inactivating lentivirus backbone. A pilot-scale production of lentiviral vector (INXN-2002) comprising *COL7A1* gene (LV-COL7) with a titer of ∼9 × 10⁶ IU/mL was generated for use in the cGMP-scale production process. Fibroblasts were isolated from RDEB patient biopsies, grown, then split into three arms for mock, high-, and low-dose LV-COL7 transduction. Each arm of fibroblasts was grown to 2 × CS10 scale then cryopreserved (Drug Substance). For patient treatment, Drug Substance vials are thawed and formulated (Drug Product), then shipped back to the clinic for wound-site injection of the originating patient.

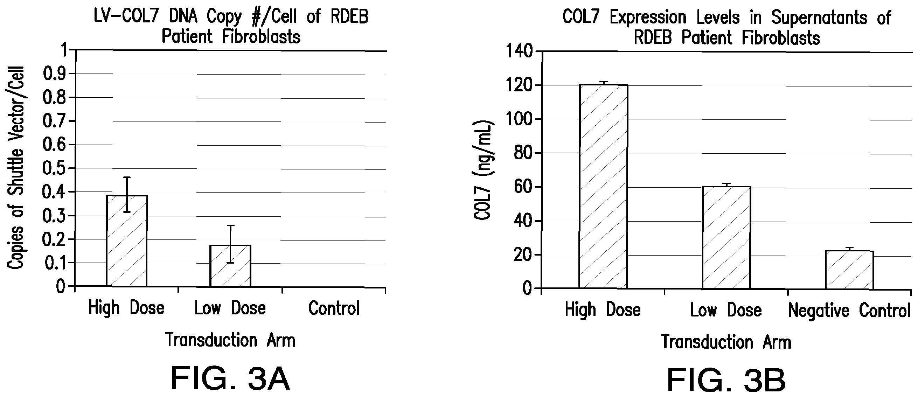

Figure 3A provides the LV-COL7 (for INXN-2002) copy number per cell. Drug Substance vials were thawed and assayed for LV-COL7 DNA copies per cell using qPCR. Primers were specific for the LV shuttle vector. Results demonstrate dose-dependent levels of copies per cell with an average of 0.38 and 0.18 copies from the High and Low Dose arms, respectively.
Figure 3B provides the C7 expression levels produced by RDEB patient fibroblasts transduced with LV-COL7: Drug Substance vials were thawed and cultured for 3 days. Conditioned cell culture supernatants were collected and assayed for C7 levels by ELISA. Results show virus dose-dependent protein expression that ranges from 60 to 120 ng/mL C7 in LV-COL7-transduced cells.

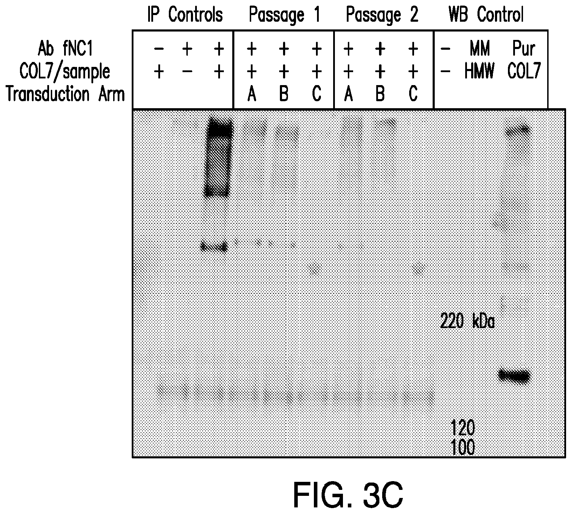

Figure 3C displays the trimeric form of C7 produced by RDEB patient fibroblasts transduced with LV- COL7: Conditioned cell culture supernatants were also used in an immunoprecipitation assay. Immunoprecipitated C7 was separated on non-denaturing SDS-PAGE and visualized by western blot. The C7 produced by RDEB fibroblasts was predominantly trimeric with LV-COL7-transduced cells expressing more COL7 than mock-transduced cells (Arm C). Some lower molecular weight species showing immunoreactivity, were also observed.

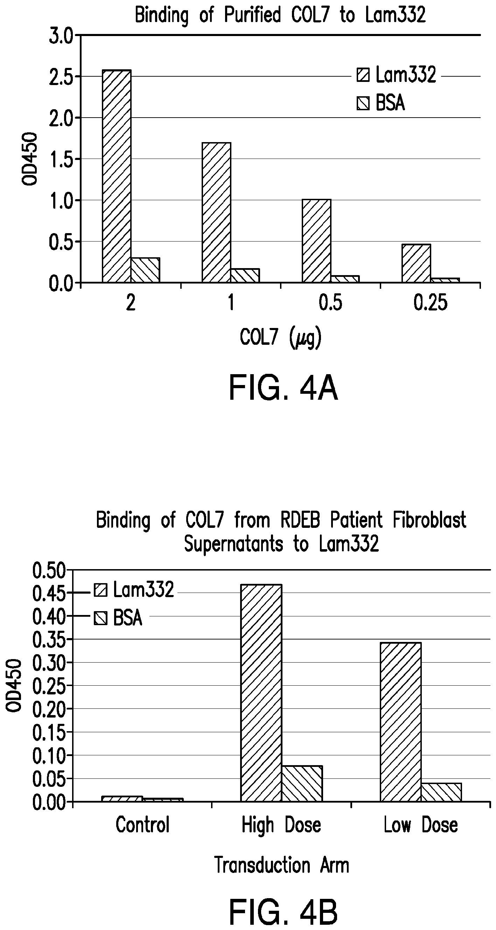

Figure 4A depicts binding of purified COL7 to Lam332. COL7 was expressed in CHO-DG44 cells, was purified by size exclusion chromatography, and was assayed for preferential binding to Lam332 as compared to BSA to establish the assay (increase in OD450 corresponds to increase in COL7 binding to Laminin332).
Figure 4B depicts Lam332 binding of COL7 in LV-COL7-transduced fibroblasts (as transduced by the INXN-2002 vector) culture supernatants. Drug Substance vials were thawed and cultured for 3 days. Conditioned cell culture supernatants were collected and assayed for binding to Lam332 compared with BSA control. Results show virus dose-dependent binding to Lam332.

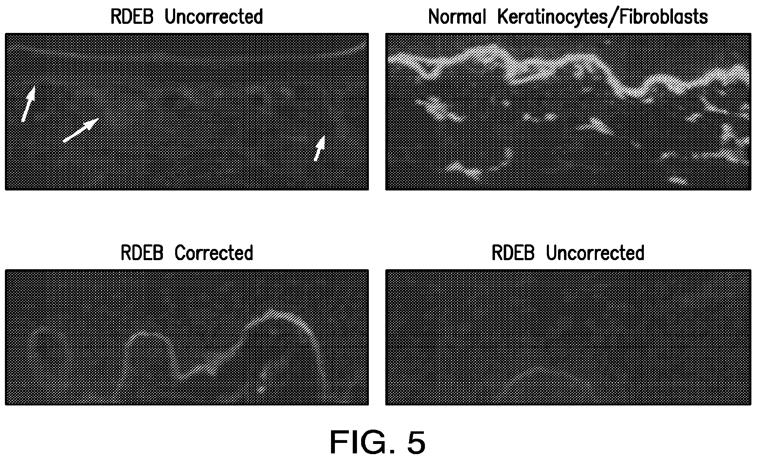

Figure 5 depicts composite RDEB skin grafts on the dorsum of SCID mice that were injected intradermally with 1 × 10⁶ GM-HDF (as transduced by the INXN-2002 vector) and analyzed by immunofluorescent staining with human COL7 specific antibodies. Representative images are shown. Localization of COL7 was observed in composite grafts (n=4) generated with RDEB keratinocytes at Day 10-post intradermal injection of 1 × 10⁶ GM-HDF. Positive control grafts generated from normal keratinocytes and fibroblasts showed intense COL7 staining and negative control grafts did not show COL7 staining at baseline measurements (arrows at DEJ for negative baseline comparison).
Figure 6 provides a schematic of the pSMPUW lentiviral expression plasmid vector.
Figure 7 compares the features of the pSMPUW lentiviral expression vector relative to 3rd generation lentivirus expression vectors.
Figure 8 provides a schematic of the pFUGW lentiviral expression plasmid vector.
Figure 9 depicts an electron micrograph of HIV-1 virus particles.
Figure 10 provides the immunoprecipitation results for TR8 GM-HDFs detecting the formation of C7 trimers.
Figure 11A provides the immunoprecipitation results for TR10 and ER1 GM-HDFs detecting the formation of C7 trimers.
Figure 11B provides the immunoprecipitation results for TR9 detecting the formation of C7 trimers.
Figure 12 shows the results of Lam332 binding results by C7 as expressed by GM-HDFs.
Figure 13 provides a schematic of the lentiviral vector plasmid construct, which encodes a *COL7A1* gene.
Figure 14 presents a schematic of the proviral RNA genome structure of the INXN-2002 lentiviral vector.
Figure 15 shows a schematic depicting the details of the mature INXN-2002 virus particle.
Figure 16 provides a schematic of the *in vitro* immortalization assay to assess the potential for insertional genotoxicity of INXN-2002.
Figure 17 represents typical cell morphology and structures of FXC-007 in culture.
Figure 18 provides a graph of the C7 expression levels as determined by ELISA for particular dosages of the TR8 GM-HDFs.
Figure 19 provides the western blot showing the immunoprecipitation of C7 trimers against NC1-specific antibodies as produced by FCX-007 GM-HDFs.
Figure 20 shows the assay readout (optical density at 450 nm (OD450)) for a binding assay to detect the interaction of C7 with laminin 332 or bovine serum albumin (BSA)-coated wells. Bound C7 was detecting using a C7 NC1-specific antibody and an HRP-conjugated secondary antibody.
Figure 21A graphically represents the amount of cell migration of normal and RDEB patient fibroblasts transduced with the LV-COL7 over time.
Figure 21B provides images of the cell migration of normal and RDEB patient fibroblasts transduced with the LV-COL7 over time.
Figure 22 provides the schematic for the cloning of human *COL7A1* gene.
Figure 23 provides the schematic of the cloning of the *COL7A1* gene into the pSMPUW expression vector to produce the INXN-2002 lentiviral vector transfer plasmid, IGE230.
Figure 24 provides the schematic representation of the construction of the INXN-2004 lentiviral vector transfer plasmid (IGE308).
Figure 25A schematically depict the gene elements of the IGE308 plasmid.
Figure 25B schematically depict the gene elements of the IGE308 plasmid.
Figure 26 provides the immunofluorescence analysis of GM-HDF injected composite grafts.

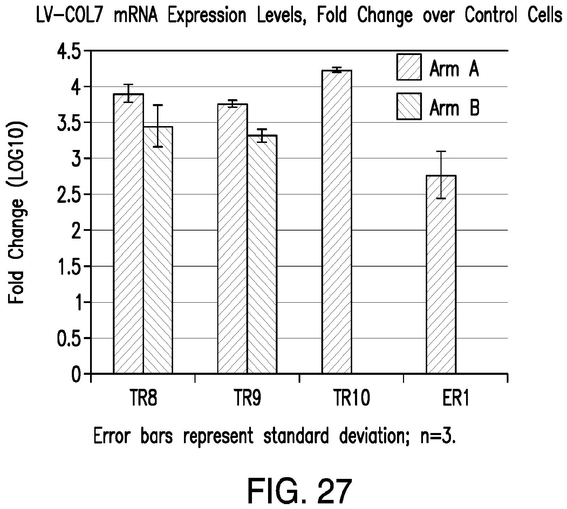

Figure 27 provides a graph representing the LV-COL7 transcript levels as measured by its fold change value over control cells.
Figure 28 provides representative images of C7 staining for INXN-2002 and LV-HA-COL7-transduced RDEB fibroblasts.
Figure 29 provides a graphical representation of the C7 protein levels as measured in the cell supernatants of GM-HDFs transduced by TR8, TR9, T10 and ER1.
Figure 30 graphically represents the correction of hypermotility of GM-HDFs over time for TR8, TR9, TR10 and ER1.
Figure 31 depicts representative indirect immunofluorescence (IF) images to examine C7 protein expression by GM-HDF cells using an antibody specific for C7 at two magnifications, 20x and 5x. (Only Arm A was tested for TR12.1 and TR13. Only Arm A of TR8 is shown for comparison of previous processes.)
Figure 32 provides the percentage of C7-positive (C7+) cells of GM-HDFs for each training run arm of TR11, TR12.1 and TR13.
Figure 33 provides a graph representation of the amount of C7 protein levels expressed for TR8, TR11, TR12.1 and 13. Error bars represent standard deviation; n=3. Only Arm A results are shown for TR8, TR12.1, and TR13.
Figure 34 provides the SDS-PAGE/immunoblots for the detection of C7 trimers (a) TR11, (b) TR12.1 and TR13 GM-HDFs.
Figure 35 depicts a graph of C7 expressed by GM-HDF that bind to Laminin 332 (Lam332) for TR11, TR12.1 and TR13.
Figure 36 provides the results of cell migration assays depicting the correction of hypermotility (% migration) over time for GM-HDFs for TR11, TR12.1 and TR13. The hypermotility of normal human dermal fibroblasts (NHDF) and a control fibroblasts from recessive dystrophic epidermolysis bullosa cells are provided.

### DETAILED DESCRIPTION OF THE INVENTION

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

In order that the present invention may be more readily understood, certain terms are herein defined. Additional definitions are set forth throughout the detailed description.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects. Typically, the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. It is contemplated that any embodiment discussed in this specification can be implemented with respect to any method or composition of the invention, and vice versa. Furthermore, compositions of the invention can be used to achieve methods of the invention.

Nucleic acids and/or nucleic acid sequences are "homologous" when they are derived, naturally or artificially, from a common ancestral nucleic acid or nucleic acid sequence. Proteins and/or protein sequences are homologous when their encoding DNAs are derived, naturally or artificially, from a common ancestral nucleic acid or nucleic acid sequence. The homologous molecules can be termed homologs. For example, any naturally occurring proteins, as described herein, can be modified by any available mutagenesis method. When expressed, this mutagenized nucleic acid encodes a polypeptide that is homologous to the protein encoded by the original nucleic acid. Homology is generally inferred from sequence identity between two or more nucleic acids or proteins (or sequences thereof). The precise percentage of identity between sequences that is useful in establishing homology varies with the nucleic acid and protein at issue, but as little as 25% sequence identity is routinely used to establish homology. Higher levels of sequence identity, e.g., 30%, 40%, 50%, 60%, 70%, 80%, 90%, 95% or 99% or more can also be used to establish homology. Methods for determining sequence identity percentages (e.g., BLASTP and BLASTN using default parameters) are described herein and are generally available.

The terms "identical" or "sequence identity" in the context of two nucleic acid sequences or amino acid sequences of polypeptides refers to the residues in the two sequences which are the same when aligned for maximum correspondence over a specified comparison window. A "comparison window", as used herein, refers to a segment of at least about 20 contiguous positions, usually about 50 to about 200, more usually about 100 to about 150 in which a sequence may be compared to a reference sequence of the same number of contiguous positions after the two sequences are aligned optimally. Methods of alignment of sequences for comparison are well-known in the art. Optimal alignment of sequences for comparison may be conducted by the local homology algorithm of Smith and Waterman (1981) Adv. Appl. Math. 2:482; by the alignment algorithm of Needleman and Wunsch (1970) J. Mol. Biol. 48:443; by the search for similarity method of Pearson and Lipman (1988) Proc. Nat. Acad. Sci U.S.A. 85:2444; by computerized implementations of these algorithms (including, but not limited to CLUSTAL in the PC/Gene program by Intelligentics, Mountain View Calif., GAP, BESTFIT, BLAST, FASTA, and TFASTA in the Wisconsin Genetics Software Package, Genetics Computer Group (GCG), 575 Science Dr., Madison, Wis., U.S.A.); the CLUSTAL program is well described by Higgins and Sharp (1988) Gene 73:237-244 and Higgins and Sharp (1989) CABIOS 5:151-153; Corpet et al. (1988) Nucleic Acids Res. 16:10881-10890; Huang et al (1992) Computer Applications in the Biosciences 8:155-165; and Pearson et al. (1994) Methods in Molecular Biology 24:307-331. Alignment is also often performed by inspection and manual alignment.

In one class of embodiments, the polypeptides herein are at least 70%, generally at least 75%, optionally at least 80%, 85%, 90%, 98% or 99% or more identical to a reference polypeptide, or a fragment thereof, e.g., as measured by BLASTP (or CLUSTAL, or any other available alignment software) using default parameters. Similarly, nucleic acids can also be described with reference to a starting nucleic acid, e.g., they can be 50%, 60%, 70%, 75%, 80%, 85%, 90%, 98%, 99% or more identical to a reference nucleic acid or a fragment thereof, e.g., as measured by BLASTN (or CLUSTAL, or any other available alignment software) using default parameters. When one molecule is said to have certain percentage of sequence identity with a larger molecule, it means that when the two molecules are optimally aligned, said percentage of residues in the smaller molecule finds a match residue in the larger molecule in accordance with the order by which the two molecules are optimally aligned.

The term "substantially identical" as applied to nucleic acid or amino acid sequences means that a nucleic acid or amino acid sequence comprises a sequence that has at least 90% sequence identity or more, preferably at least 95%, more preferably at least 98% and most preferably at least 99%, compared to a reference sequence using the programs described above (preferably BLAST) using standard parameters. For example, the BLASTN program (for nucleotide sequences) uses as defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix (see Henikoff & Henikoff, Proc. Natl. Acad. Sci. USA 89:10915 (1992)). Percentage of sequence identity is determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Preferably, the substantial identity exists over a region of the sequences that is at least about 50 residues in length, more preferably over a region of at least about 100 residues, and most preferably the sequences are substantially identical over at least about 150 residues. In a most preferred embodiment, the sequences are substantially identical over the entire length of the coding regions.

A "functional variant" of a protein disclosed herein can, for example, comprise the amino acid sequence of the reference protein with at least or about 1, 2, 3, 4, 5, 6, 7, 8, 9, 10, 11, 12, 13, 14, 15, 16, 17, 18, 19, or 20 conservative amino acid substitutions. The phrase "conservative amino acid substitution" or "conservative mutation" refers to the replacement of one amino acid by another amino acid with a common property. A functional way to define common properties between individual amino acids is to analyze the normalized frequencies of amino acid changes between corresponding proteins of homologous organisms (Schulz, G. E. and Schirmer, R. H., Principles of Protein Structure, Springer-Verlag, New York (1979)). According to such analyses, groups of amino acids may be defined where amino acids within a group exchange preferentially with each other, and therefore resemble each other most in their impact on the overall protein structure (Schulz, G. E. and Schirmer, R. H., supra). Examples of conservative mutations include amino acid substitutions of amino acids within the sub-groups above, for example, lysine for arginine and vice versa such that a positive charge may be maintained; glutamic acid for aspartic acid and vice versa such that a negative charge may be maintained; serine for threonine such that a free --OH can be maintained; and glutamine for asparagine such that a free --NH₂ can be maintained. In one embodiment of the present invention, a *COL7A1* gene encodes for the C7 protein or a functional variant thereof, provided that the variant is capable of anchoring the fibrils between the epidermis and dermis.

Alternatively or additionally, the functional variants can comprise the amino acid sequence of the reference protein with at least one non-conservative amino acid substitution. "Non-conservative mutations" involve amino acid substitutions between different groups, for example, lysine for tryptophan, or phenylalanine for serine, etc. In this case, it is preferable for the non-conservative amino acid substitution to not interfere with, or inhibit the biological activity of, the functional variant. The non-conservative amino acid substitution may enhance the biological activity of the functional variant, such that the biological activity of the functional variant is increased as compared to the reference sequence.

Proteins disclosed herein (including functional portions and functional variants thereof) may comprise synthetic amino acids in place of one or more naturally-occurring amino acids. Such synthetic amino acids are known in the art, and include, for example, aminocyclohexane carboxylic acid, norleucine, α-amino n-decanoic acid, homoserine, S-acetylaminomethyl-cysteine, trans-3- and trans-4-hydroxyproline, 4-aminophenylalanine, 4-nitrophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, α-(2-amino-2-norbornane)-carboxylic acid, α,γ-diaminobutyric acid, α,β-diaminopropionic acid, homophenylalanine, and α-tert-butylglycine.

The invention includes the vectors INXN-2002; and IGE308 also referred to herein as INXN-2004, as well as vectors substantially identical and/or homologous thereto as well as functional variants thereof.

The term "patient" or "subject" refers to mammals, including humans and animals.

The term "treating" refers to reducing or alleviating the symptoms, and/or preventing relapses and/or the progression of DEB, including RDEB and/or DDEB.

The term "autologous cells" refers to cells obtained and then returned to the same individual. In particular, cells may be removed from the body of a DEB patient, which may subsequently be genetically modified, cultivated to proliferate, and then returned to the body of the patient from which they were originally removed.

The term "transduction" refers to the delivery of a gene(s) using a viral or retroviral vector by means of viral infection rather than by transfection.

The term "transducing lentiviral vector" or "transducing lentiviral vector particle" refers to the infectious lentiviral vector particles formed from the co-transfection of a packaging cell line with the lentiviral expression/transfer plasmid vector comprising the *COL7A1* gene or functional variant thereof, a packaging vector(s), and an envelope vector. The transducing lentiviral vector is harvested from the supernatant of the producer cell culture after transfection. Suitable packaging cell lines are known in the art and include, for example, the 293T cell line.

The term "lentiviral vector" refers to a vector containing structural and functional genetic elements outside the LTRs that are primarily derived from a lentivirus.

The term "self-inactivating vector" (SIN) refers to vectors in which the 3' LTR enhancerpromoter region, known as the U3 region, has been modified (e.g., by deletion or substitution) to prevent viral transcription beyond the first round of viral replication. Consequently, the vectors are capable of infecting and then integrating into the host genome only once, and cannot be passed further. SIN vectors greatly reduce risk of creating unwanted replication-competent virus since the 3' LTR U3 region has been modified to prevent viral transcription beyond the first round of replication, eliminating the ability of the virus to be passed.

The term "pharmaceutically acceptable carrier" is employed herein to refer to liquid solutions which are, within the scope of sound medical judgment, suitable for use in contact with the autologous, genetically-modified cells without affecting their activity, and without being toxic to the tissues of human beings and animals or causing irritation, allergic response, or other complications, commensurate with a reasonable benefit/risk ratio. A useful pharmaceutically acceptable carrier may be an injectable solution which is biocompatible with the autologous, genetically-modified cells and does not reduce their activity or cause their death.

The present disclosure relates to an autologous, genetically-modified cell therapy for C7-deficient patients. "C7-deficient patients" either lack or have substantially reduced production of type VII collagen (C7), which is important for anchoring fibril formation at the dermal-epidermal junction (DEJ), resulting in dystrophic epidermolysis bullosa (DEB). The present disclosure relates to genetically modifying DEB cells obtained from a patient, such as fibroblasts or keratinocytes, to insert a functional *COL7A1* gene or functional variant thereof, expanding the genetically modified cells, and administering a population of the genetically-modified cells back into the DEB patient.

Cells may be obtained from a patient suffering from DEB. Fibroblasts or keratinocytes are preferred. The cells may be obtained through known methods, including from patient skin samples, scrapings, and biopsies, for example. In one embodiment, the cells are obtained from the non-blistering skin of a DEB patient. In another embodiment, the cells are obtained from the blistering skin of a DEB patient. The cells obtained from a DEB patient have either a mutated, non-functional or missing *COL7A1* gene. These cells are cultured using standard cell culture techniques. The cells obtained from the patient are subsequently treated *ex vivo* with genetic material encoding a functional type VII collagen gene (C7) or functional variant thereof.

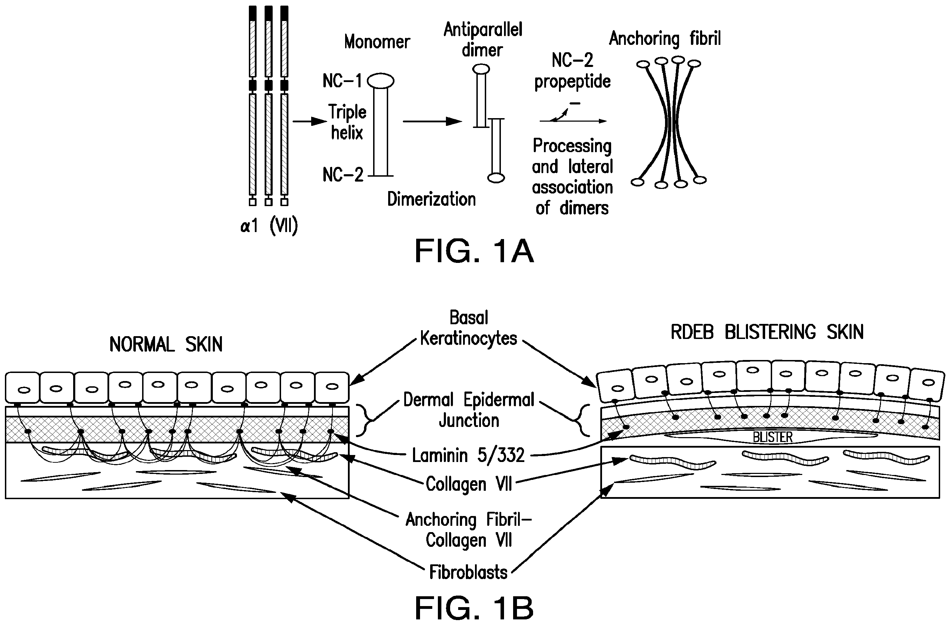

The *COL7A1* gene has the nucleic acid, SEQ ID NO:1, which encodes for a 290 kDa alpha chain, wherein three chains form a triple helix (trimer), as depicted in Figure 1A. C7 has the amino acid sequence of SEQ ID NO: 2 and is the protein important for anchoring fibril formation at the dermal-epidermal junction (DEJ). Mutations in the C7 gene cause an absence or reduction of C7, which make up anchoring fibrils that maintain binding of the epidermis to the dermis. C7 anchoring fibrils bind to other collagens, extracellular matrix proteins, and Lam332 that mediate the attachment of the dermis to the epidermis, as shown in Figure 1B. The absence of anchoring fibrils leads to blistering of the skin. Vectors may comprise nucleic acids and polypeptides substantially identical to *COL7A1* and C7 respectively. In another aspect, vectors may comprise nucleic acids that encode a functional variant of the COL7A1 or C7 protein, provided that the variant is capable of anchoring the fibrils between the epidermis and dermis. In another aspect, only the open reading frame of the *COL7A1* gene, which encodes the C7 protein or a functional variant thereof may be used. In some embodiments, the *COL7A1* nucleic acid is codon-optimized for increased expression of C7 in the transduced cell, as is known in the art.

In accordance with the present invention, the *COL7A1* can be transduced into the DEB cells obtained from a patient using a transducing lentiviral vector, which is replication defective and self-inactivating. Self-inactivating lentiviral vectors are derived from the human immunodeficiency virus (HIV-1), which are pseudotyped with a heterologous VSV-G envelope protein instead of the HIV-1 envelope protein. As previously reported, a 400 bp deletion is introduced to the U3 region of the LTR resulting in a self-inactivating (SIN) vector (Zuffery 1998). The vectors of the present invention are distinguished from 2nd and 3rd generation SIN vectors. In particular, the vectors of the present invention have been modified to enhance the safety features and increase the cloning capacity as further described herein.

Vectors used to construct the transducing lentiviral vectors of the present invention are introduced via transfection or infection into a packaging cell line. The packaging cell line produces transducing lentiviral vector particles that contain the vector genome. After cotransfection of the packaging vectors, transfer vector, and an envelope vector to the packaging cell line, the recombinant virus is recovered from the culture media and titered by standard methods used by those of skill in the art. Thus, the packaging constructs can be introduced into human cell lines by calcium phosphate transfection, lipofection or electroporation, generally together with a dominant selectable marker, such as kanamycin, neomycin, DHFR, or Glutamine synthetase, followed by selection in the presence of the appropriate drug and isolation of clones. The selectable marker gene can be linked physically to the packaging genes in the construct.

Stable cell lines wherein the packaging functions are configured to be expressed by a suitable packaging cell are known. For example, see U.S. Pat. No. 5,686,279; and Ory et al., (1996), which describe packaging cells. The packaging cells with a lentiviral vector incorporated in them form producer cells. Producer cells are thus cells or cell-lines that can produce or release packaged infectious viral particles carrying the therapeutic gene of interest. An example of a suitable lentiviral vector packaging cell lines includes 293 cells.

According to the invention, the transducing lentiviral vector is constructed from a lentiviral expression plasmid vector comprising (a) a modified 5' long terminal repeat (LTR), wherein the promoter of the modified 5' LTR is a cytomegalovirus promoter, (b) the *COL7A1* gene, (c) at least one lentiviral central polypurine tract element, and (d) a modified 3' LTR, wherein the modified 3' LTR comprises a deletion relative to the wild-type 3' LTR, wherein a hepatitis B virus post- transcriptional regulatory element (PRE) has been deleted.

In another aspect of the invention, the deleted hepatitis B virus post- transcriptional regulatory element (PRE) is a woodchuck post-transcriptional regulatory element PRE (WPRE).

In another aspect of the invention, the lentiviral expression plasmid vector comprises two or more lentiviral central polypurine tract (cPPT) elements. Incorporation of cPPT element has been found to increase gene expression levels.

In another aspect of the invention, the 3' LTR may be modified to delete a 400 bp fragment (as disclosed by Zuffery 1998), instead of the commonly used 133 bp deletion of standard 3rd generation SIN LV vectors. The 400 bp deletion is believed to increase safety by preventing read-through transcription as well increase viral titer because the vector transcript is more stable in packaging cells.

In yet another aspect of the disclosure, the lentiviral expression plasmid may incorporate a hepatitis B virus post-transcriptional regulatory element (PRE), which is preferably woodchuck post-transcriptional regulatory element PRE (WPRE). WPRE has been found to increase the viral titer of the vector.

The addition of the cPPT, and modified 3'LTR deletion has been shown to improve the function of the lentiviral vector comprising the *COL7A1* gene of the present invention to increase gene expression levels, viral titer, and safety.

The present invention further includes the use of lentiviral transfer vectors in which certain elements common for 3rd generation lentiviral expression vectors are deleted. In particular, safety concerns for use of lentiviral vectors in humans hinge on the fear of generating a replication-competent lentivirus, which may arise from recombination between split genomes of 3rd generation lentiviral vectors. (Tareen *et al.,* 2013.). The gag sequence and/or the revresponsive elements (RRE) may be deleted from the viral vector, in order to reduce sequence homology with other helper plasmids and thereby increase safety to humans. Accordingly, in one embodiment of the invention, the gag sequence is absent in the vector. In another embodiment of the invention, both the gag sequence and RRE is absent in the vector.

The starting materials to generate the lentiviral vectors of the present invention is preferably a lentiviral expression plasmid vector that comprises a cPPT and PRE that can accommodate the insertion of the large *COL7A1* gene (8.89 kbp) or a functional variant thereof. Preferably, the starting material to construct the transducing lentiviral vector of the present invention is selected from the lentiviral expression plasmid vectors, pSMPUW (Cell Biolabs, Inc., San Diego, CA) and pFUGW (Addgene, Cambridge, MA).

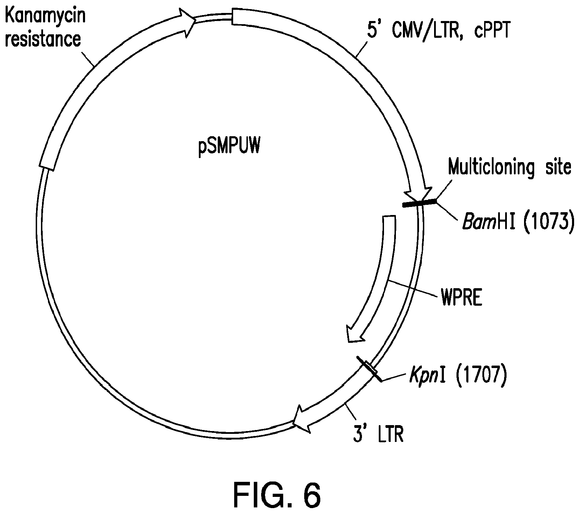

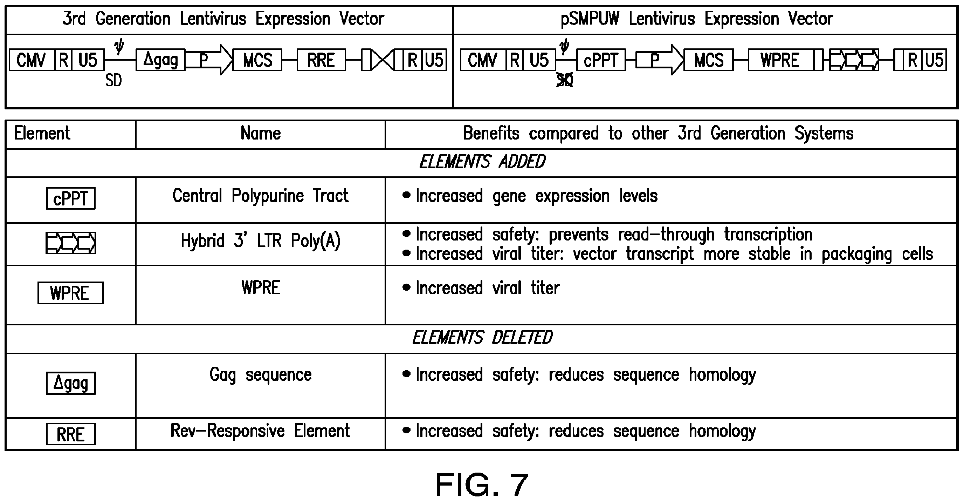

In one aspect of the invention, the pSMPUW lentiviral expression plasmid vector is selected for construction of the transducing lentiviral vector. Figure 6 shows a schematic of the genetic elements in the pSMPUW lentiviral expression plasmid vector. Features of the pSMPUW lentiviral expression vector have been modified from 3rd generation lentivirus expression vectors in order to enhance gene expression levels and safety features, as depicted in Figure 7. In particular, the pSMPUW lentiviral expression vector encodes for a multicloning site (MCS) followed by the Woodchuck Hepatitis Virus Post-transcriptional regulatory element (WPRE). The residual gag (Δgag) and the RRE element were removed from the pSMPUW vector construct. Additionally, the pSMPUW vector construct utilized a larger 400 bp deletion in the 3'LTR U3 region instead of the commonly used 133 bp deletion in a standard 3rd generation SIN LV vector. Wild type *COL7A1* gene was incorporated into the pSMPUW vector.

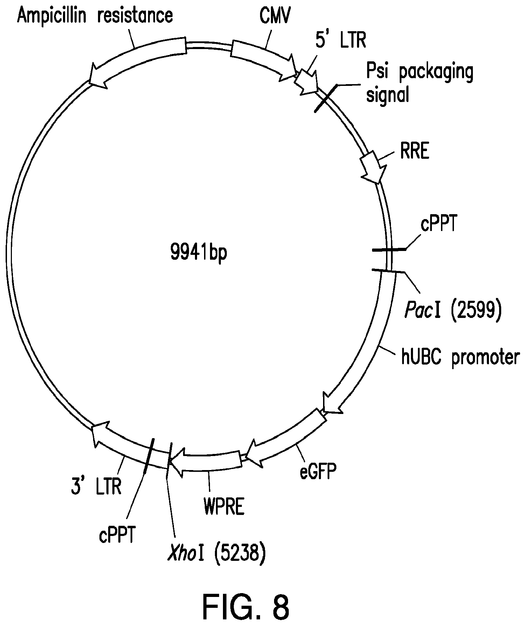

In another aspect of the invention, the pFUGW lentiviral expression plasmid is selected for construction of the transducing lentiviral vector. Figure 8 shows a schematic of the genetic elements in this plasmid vector. Features of the pFUGW vector include a RRE, two cPPT elements, and a WPRE element, which may improve lentiviral production and transgene expression.

In another aspect of the invention, the WPRE element has been deleted from the lentiviral vector.

In an embodiment of the present invention, transducing lentiviral vector particles of the present invention are designated INXN-2002 (vector transfer plasmid - IGE230) or INXN-2004 (vector transfer plasmid - IGE308), or a substantially identical vector comprising functional variants thereof.

The cells obtained from the DEB patient are transformed with a functional *COL7A1* gene. In one aspect, the cells are transduced with a lentiviral vector having the *COL7A1* gene. In another aspect, the lentiviral vector is a self-inactivating vector. The copy number of the integrated transgene can be assessed using any known methods. For example, copy number may be determined through quantitative PCR, multiplex ligationdependent probe amplification, fluorescent in situ hybridization (FISH), microarray-based copy number screening, and conventional karyotyping. The number of copies of the transgene integrated into each cell may be modulated by the virus dose given to the cells during production. The integrated transgene copy number per cell in the obtained RDEB cells transduced with a *COL7A1*-containing vector is dose dependent.

Cells obtained from the DEB patient and transformed with the functional *COL7A1* gene will have a functional *COL7A1* gene and exhibit normal cell morphologies. These cells may be caused to proliferate or expanded in culture using standard cell culture techniques.

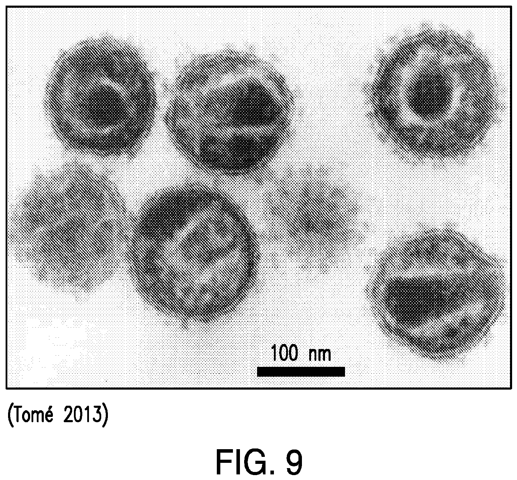

Normal fibroblast morphological characteristics are observed among obtained fibroblasts transduced with *COL7A1*-transducing vectors within the scope of the present invention. For example, normal fibroblast morphologies include cells displaying elongated, fusiform or spindle appearance with slender extensions. Normal morphologies further include cells appearing larger, flattened stellate cells which may have cytoplasmic leading edges. Figure 9 displays an example of normal cell morphology for fibroblasts transduced with a *COL7A1-*transducing vector.

The production of C7 produced by DEB cells obtained from a patient and transduced with a *COL7A1*-transducing vector has also been observed. In particular, the formation of C7 trimers is important in the assembly of anchoring fibrils. It has been found that DEB patient cells transduced with a *COL7A1*-transducing vector are capable of forming the C7 trimers with the correct structure, size, and function.

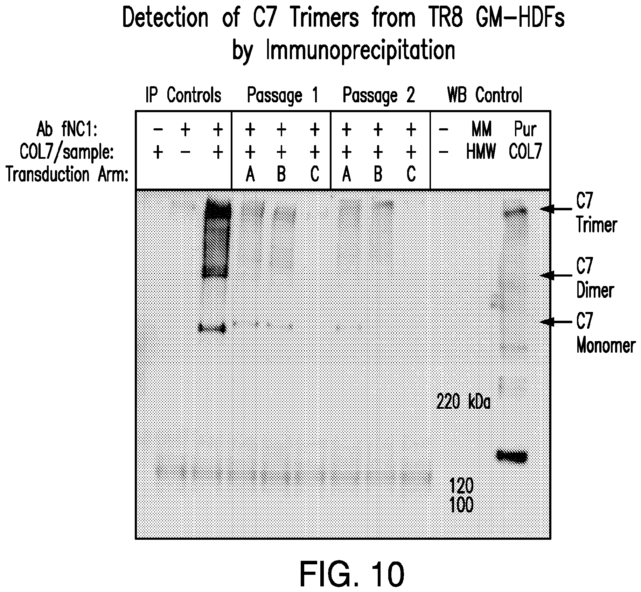

In one aspect of the invention, it is possible to verify that the C7 expressed by fibroblasts transduced with a *COL7A1*-transducing vector, as defined by the claims, will be capable of forming anchoring fibrils using immunoprecipitation with an anti-C7 specific antibody. For example, the anti-C7 specific antibody, fNC1, may be used for selective capture and the concentration of C7 from culture supernatants for detection by SDS-PAGE/immunoblot. For example, Figures 9 and 10 demonstrate that C7 produced by fibroblasts transduced with a COL7AI-transducing vector were predominantly trimeric.

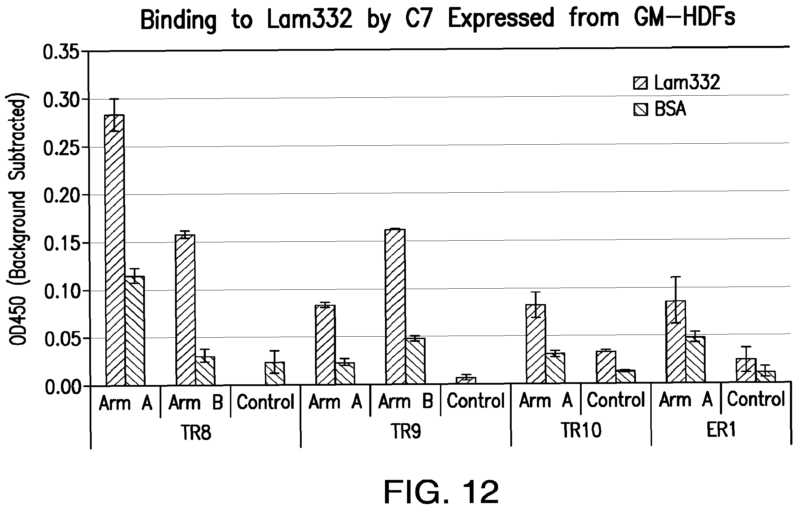

In another aspect of the invention, the function of C7 may be assessed using known methods, such as using a laminin binding assay or cell migration assay. C7 has been shown to bind immobilized extracellular matrix (ECM) components, including fibronectin, Laminin 332 (Lam332), COL1, and COL4 (Chen, et al., 2002a). The interaction between C7 and Lam332 occurs through the NH2-terminal NC1 domain of C7 and is dependent upon the native conformation of both Lam332 and C7 NC1 (Rousselle, et al., 1997). The association between C7 and Lam332 is important for establishing correct Lam332 architecture at the dermal-epidermal junction. Such organization is important for interactions with extracellular ligands and cell surface receptors, and for cell signaling (Waterman, et al., 2007). In one aspect of the disclosure, an ELISA using an antibody against the C7 NC1 domain to detect binding of C7 to purified Lam332 has been developed. Although a C7/Lam332 binding ELISA has already been described in the literature (Chen, et al., 2002a), to our knowledge it has never been used to test C7 present in the supernatants of transduced cells. Results in Figure 12 show dose-dependent binding to Lam332 by C7 expressed by GM-HDFs from the Training and Engineering runs.

In addition, a cell migration assay can be used to assess functional C7 activity. Previous studies have shown that RDEB fibroblasts and keratinocytes show an increase in motility relative to their normal counterparts, and that normal motility can be restored by expression of C7 (Chen, et al., 2000; Chen, et al., 2002b; Cogan, et al. 2014; Baldeschi et al., 2003). Suitable assay includes the colloidal gold salt migration assay to measure the migration of fibroblasts and keratinocytes, or a wound healing assay to measure the migration of keratinocytes. Cells having functional C7 activity will exhibit a reduced motility relative to RDEB cells in such assays.

The present invention is directed, in one aspect, to pharmaceutical formulations comprising the autologous, genetically-modified cells from DEB patients. These cells may be present in any amount suitable for the delivery to the patient in which the cells were originally harvested. For example, that cells may be present in a cell concentration of 1.0 - 5.0 × 10⁷ cells/mL, 1.0 - 4.0 × 10⁷ cells/mL, 1.0 - 3.0 × 10⁷ cells/ mL, or 1.0 - 2.0 × 10⁷ cells/mL. The cells are present in present in a suspension suitable to sustain the viability of the cells, such as in Dulbecco's Modified Eagle's Medium (DMEM). In particular, the viability of the cells in the formulation are present in an amount of ≥ 60%, 70%, 75% or 80%. Suitable excipients may also be present in the formulation, such as phosphate buffered saline which may be used to wash the cells from thawed vials containing the autologous, genetically-modified. Preferably, no phenol red is present in the final formulation.

The autologous genetically modified cells of the present disclosure have a transducing vector copy number of at least or about 0.05, 0.09, 0.41, or 0.74 or in the range of about 0.1 to about 6.0, about 0.1 to about 5.5, about 0.1 to about 5.0, about 0.1 to about 4.5, about 0.1 to about 4.0, about 0.1 to about 3.5, about 0.1 to about 3.0, about 0.1 to about 1, 0.4 to about 1, or 0.4 to about 0.75. Preferably, the transducing vector copy number is in the range of about 0.1 about 5.0. It has been found that the the transduced human dermal fibroblast or keratinocytes have at least or about 1, 2, 5, 10, 20, 25, 27, 28, 29, 30, 35, 40, 45, or 50-fold greater integrated transgene copy number per cell relative to a transduction composition not subjected to spinoculation or super-transduction. Moreover, the C7 protein expression values of the FCX-007 cells of the present invention are ≥ 300, 350, 400, 450, 500, 550, or 600 ng/day/E6 cell. Preferably, the C7 protein expression values of the autologous genetically-modified cells of the present invention are ≥ 500 ng/day/E6 cell. The present invention has also found that expression of C7 has increased by 10, 25, 50, 100, 150, or 200-fold relative to transduced human dermal fibroblasts or keratinocytes not subjected to spinoculation or a second transduction.

The multiplicity of infection (MOI) refers to the number of vector particles per cell used in transduction. In accordance with the present invention, the desired transducing vector copy number has been achieved even as the MOIs decreases, as shown in the examples below. For example, the invention relates to MOIs of ≤ 15, < 14, ≤ 13, ≤ 12, ≤ 11, or ≤ 10. Preferably, the MOI is between about 1 to 10.

The formulations of the present invention may be for use in treating various maladies of patients suffering from Type VII deficiency. In particular, the formulations of the present invention are suitable to treat DEB, including DDEB or RDEB. In an embodiment of the invention, the medical use is for treating subtypes of RDEB, including but not limited to Hallopeau-Seiemens, non-Hallopeau-Siemens RDEB, RDEB inversa, pretibial RDEB, acral RDEB, and RDEB centripetalis.

The invention further relates to the medical use for the treatment, reduction, prevention, and/or inhibition of various symptoms attributed to C7 deficiencies. For example, DEB is known to cause scarring after blisters, which may cause contracture deformities, swallowing difficulty if the mouth and esophagus are affected, fusion of fingers and toes, and limited mobility. Therefore, in one aspect of the invention envisions the medical use for the treatment, reduction, inhibition, and/or prevention of pseudosyndactyly, also known as mitten hand syndrome. In another aspect, the invention relates to the medical use for the treatment, reduction, inhibition, and/or prevention of deep fibrosis and/or scarring associated with RDEB and/or DDEB, which may result in milia, joint contractures, generalized soft tissue fibrosis, organ fibrosis, corneal lesions, scarring plaques, scarring alopecia, nail dystrophy, ankyloglosia, and increased frequency of dental caries, for example. In another aspect, the invention relates to the medical use for the treatment, reduction, prevention, and/or inhibition of oral mucosa lesions and gastrointestinal lesions associated with RDEB. Another embodiment relates to the medical use for treatment, prevention, reduction and/or inhibition of blisters associated with DEB patients.

Administration of the autologous genetically modified cells may be done at any appropriate time as one skilled in the art would be capable of determining based on the needs of the patient. For example, administration may be done once, once a day, once a month, once a quarter, or 1-2 times per year.

The formulation of the present invention containing the autologous genetically modified cells may be administered to the patient by any known method, including but not limited to injection, topically, orally, or embedded in a biocompatible matrix. The injection may be, e.g., parenteral, intradermal, subcutaneous, intramuscular, intravenous, intraosseous, intraarterial, ocular and intraperitoneal or direct injection into a specific organ/tissue, e.g. prostate or liver. Topically, the formulation may be administered directly to an affected site, such as at the site of a lesion. Debridement of the affected tissue can precede the direct application of the formulation to the site. Alternatively, the formulation may be encapsulated in a suitable delivery system, such as in a polymer capsule, or embedded in a biocompatible matrix or graft, e.g., collagen matrix, in a hydrogel, skin graft, or a mesh.

The autologous genetically modified cells of the present invention may be administered solely or in combination with other treatments for a patient suffering from DEB. Examples of therapeutic agents used for treating DEB include topical care, such as Zorblisa, skin grafts, antiinflammatories, antibodies, other potential gene or cell-based therapies. In another embodiment, the modified cells of the present invention may be administered for the skin or muoscal areas where bone marrow transplant therapies (or other system cellular therapies such as mesenchymal stem cells) did not provide sufficient therapy. The C7-expressing ability of the autologous genetically modified cells is preferably not impaired by a combination with other therapeutic agents.

The present invention further relates to a method of enhancing or increasing the integrated transgene copy number per cell in the genetically modified cells obtained from C7-deficient patients and transduced according to the present invention. In particular, it has been found that certain steps during transduction substantially enhanced the copy number of the transgene in these cells. According to the invention, the cells obtained from the C7-deficient patient are contacted with the transducing lentiviral vector of the present invention and that composition is subjected to spinoculation, also known as spin transduction. In this manner, the lentiviral vectors are spun onto the targeted cells. The addition of spinoculation has unexpectedly increased the copy number per cells in the genetically modified human dermal fibroblasts by a level of at least 5, 10, 15, 20, 25, 27, 28, 29, 30, 35, 40, 45, or 50- fold relative to a transduction without spinoculation or transduction. However, in order to identify additional methods to increase copy number, it has unexpectedly been found that a second transduction step (or super-transduction) in which the targeted cells are passaged through the first transduction with spinoculation, and then subsequently passaged through a second transduction optionally with spinoculation. In this manner, it has been found that this super-transduction with spinoculation increases the copy number by an additional 2-fold increase relative to the original traditional transduction method that did not include spinoculation or super-transduction.

In another embodiment, it has been found that changing the lentiviral vector from INXN-2002 to INXN-2004 increased the transgene copy number in the genetically modified human dermal fibroblasts by 4-fold relative to traditional transduction without spinoculation or super-transduction.

Through extensively evaluating integrated transgene copy numbers, it has been found the cumulative change of adding spinoculation, adding super-transduction with spinoculation, and changing from INXN-2002 to INXN-2004, relative to the original transduction process that did not include spinoculation or super-transduction, unexpectedly increased the copy number > 27-fold (*see* Example 9: comparing TR12.1 and TR3 to TR8 in original studies (which did not include spinoculation or super-transduction). Accordingly, the present invention relates to increasing the copy number of the transduced cells from the C7-deficient patient.

To further illustrate the invention, the following non-limiting examples are provided.

### EXAMPLES

FCX-007 is a suspension of live, autologous human dermal fibroblast cells genetically modified using either a lentiviral vector (INXN-2002) (as shown in Example 1) or a lentiviral vector (INXN-2004) (as shown in Example 2) to express the human collagen type 7 protein.

### EXAMPLE 1

### A. Elucidation of Structure and Characteristics of FCX-007 transduced with Lentiviral Vector INXN-2002

The FCX-007 cells derived from the lentiviral vector INXN-2002 cells are suspended in a cryopreservation medium consisting of Iscove's Modified Dulbecco's Medium (IMDM) without fetal bovine serum (50.0%), Profreeze-CDMTM (42.5%) and dimethyl sulfoxide (DMSO) (7.5%). The structural features for FCX-007 Drug Substance (DS) include the primary structure of the autologous human dermal fibroblast (HDF) cells and the structure of the lentiviral vector used to transduce and gene modify the HDF cells.

### B. Lentiviral Vector (INXN-2002)

INXN-2002 Lentiviral Vector (LV), which is used to transduce and to introduce the human collagen 7A1 gene into the HDF cells, is a recombinant lentiviral vector encoding the human collagen 7A1 gene. INXN-2002 LV is a self-inactivating (SIN) lentiviral vector that is constructed based on the human immunodeficiency virus type 1 (HIV-1) pseudotyped with a heterologous VSV-G envelope protein. The virus particle for INXN-2002 lentiviral vector is approximately 120 nm in diameter and is comprised of numerous proteins with two copies of a single-stranded RNA genome. A specific molecular formula, molecular weight, or stereochemistry is not available.

Structural features for INXN-2002 LV include the primary structure of the RNA viral genome and the structure of the viral particle. The primary structure of the RNA genome of INXN-2002 is determined by the full nucleotide sequencing of the viral genome. The viral particles structure is deduced from the particle structure of HIV-1 with added VSV-G protein pseudotyping. An overview of the nucleic acid structure and the structure of the viral particle are provided below.

### C. INXN-2002 RNA Viral Genome Structure

INXN-2002 LV is a self-inactivating (SIN) lentiviral vector that is constructed based on the human immunodeficiency virus type 1 (HIV-1) pseudotyped with a heterologous VSV-G envelope protein instead of the HIV-1 envelope protein. A 400 bp deletion is introduced to the U3 region of the LTR resulting in a self-inactivating (SIN) vector (Zuffery 1998). The pSMPUW lentiviral expression plasmid vector (Cell Biolabs, Inc., San Diego, CA) was selected for construction of the INXN-2002 LV. Figure 6 shows a schematic of the genetic elements in the pSMPUW lentiviral expression plasmid vector.

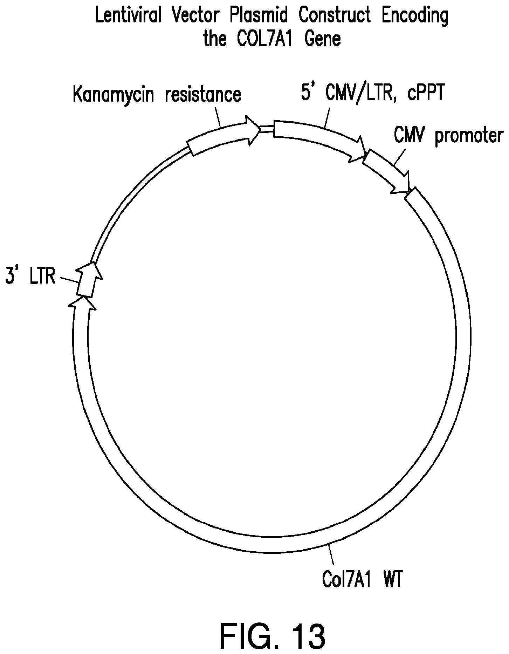

The coding elements between the 5' and 3' LTRs of the HIV-1 virus are fully gutted. The vector encodes for a multicloning site followed by the Woodchuck Hepatitis Virus Posttransscriptional Regulatory Element (WPRE). In order to maximize the cloning capacity of the vector, these elements were removed by digesting the vector with BamHI from the multicloning site and KpnI at the 5' end of the 3' left terminal repeat. The COL7A1 gene expression cassette with a CMV promoter is cloned into the digested vector by single strand annealing to generate the lentiviral vector plasmid construct encoding the COL7A1 gene as shown in Figure 13.

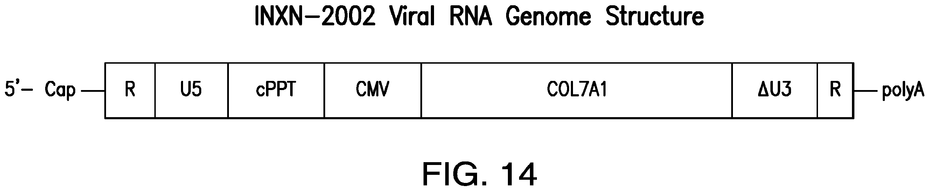

The lentiviral vector plasmid construct is co-transfected into HEK293T cells with three helper plasmids (pCMV-G, pCMV-Rev2 and pCgp) to produce the INXN-2002 lentiviral vector particles. pCMV-G plasmid provides the VSV-G pseudotyping surface protein, pCMV-Rev2 provides the HIV-1 Rev protein for efficient RNA transport and packaging, and pCgp provides the structural and viral enzyme proteins for production of the INXN-2002 lentiviral vector particles. Figure 14 shows a schematic of the proviral RNA genome structure of the INXN-2002 lentiviral vector.

### D. INXN-2002 Lentiviral Vector Particle Structure

INXN-2002 lentiviral vector is constructed based on a HIV-1 derived vector backbone and has a similar viral particle structure to the HIV-1 virus. Figure 9 shows an electron micrograph of HIV-1 particles.

The HIV-1 particle has a spherical shape of approximately 120 nm in diameter, with an estimated molecular weight of 277MDa (Carlson 2008). The particle has a lipid bilayer membrane stubbed with envelope protein. The envelope protein interacts with the receptors on the target cells for infection and delivery of the RNA viral genome to the target cells. A cone shaped nucleo-core, where two strands of viral RNA genome is housed, can be observed inside the virus particle. The cone shaped nucleo-core is formed by the viral capsid protein.

For INXN-2002, VSV-G (glycoprotein of the vesicular stomatitis virus (VSV-G)) is used as a substitute for the HIV-1 envelope proteins resulting in improved vector stability, target cell tropism, and transduction efficiency (Cronin 2005).

During production, INXN-2002 viral particles assemble and bud out from the surface of transfected HEK293T cells. The VSV-G protein is provided by the pCMV-G helper plasmid, the vector core and enzyme proteins are provided by the pCgp helper plasmid, and the Rev protein, which is needed for efficient RNA genome transport and packaging into the viral particle, is provided by the pCMV-Rev2 plasmid. It is noted that all the other HIV-1 accessory proteins including Vpu, Vif, Vpr, Nef, and Tat, are deleted from the INXN-2002 vector.

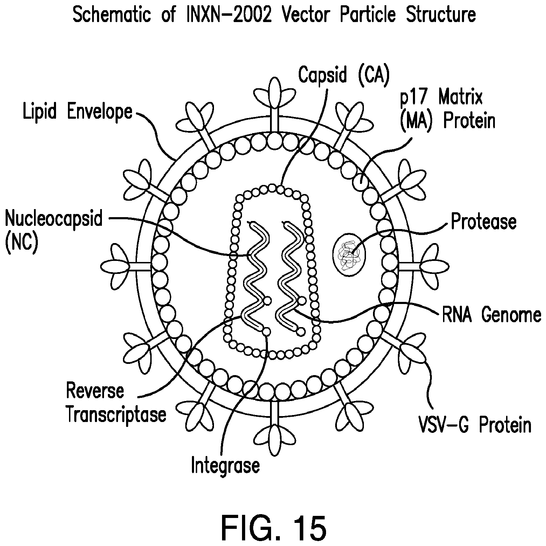

After budding from the producer cell surface, the protease enzyme packaged inside the virus particle cleaves the Gag precursor protein into its constituent proteins (MA, CA, NC), to convert the immature virion into a mature infectious INXN-2002 vector particle. Figure 15 shows a schematic depicting the details of the mature INXN-2002 virus particle.

Two strands of the INXN-2002 RNA genome are packaged inside the cone shaped core formed by the Capsid protein (CA). The nucleocapsid (NC) protein forms a stable complex with the RNA genome inside the capsid core. The matrix protein forms a coat on the inner surface of the membrane. The virus buds through the cell plasma membrane spiked with the VSV-G envelope protein and forms the lipid envelope. Based on the recent three dimensional analysis of HIV-1 virus particle structure (Carlson 2008), Table 1 shows the component proteins that make up the INXN-2002 lentiviral vector and a brief description of the function of each of the components.

**Table 1: INXN-2002 Lentiviral Vector Major Component Proteins**

| **Component Proteins** | **Protein MW (kD)** | **Protein functions** |
|---|---|---|
| Capsid (CA) | 24 | Forms the nucleo-core |
| Matrix (MA) | 17 | Forms the protein coat on the inner surface of the lipid membrane |
| Nucleocapsid (NC) | 7 | Forms a stable complex with the RNA genome |
| VSV-G | 69 | Pseudotyped envelope protein interacts and binds to receptors on target cells for transduction to deliver the vector RNA genome to the cells |
| Protease | 11 | Plays an important role in the maturation of the INXN-2002 vector by cleaving the Gag proteins to its functional constituents, CA, MA, and NC |
| Reverse transcriptase | 66/51 | Builds a DNA copy of the viral RNA genome |
| Integrase | 31 | Inserts the DNA copy of the viral RNA genome into the infected cell genome |

### E. INXN-2002 Characterization

Table 2 provides a list of the characterization assays and specifications for INXN-2002 manufactured by City of Hope. Characterization assay results are provided in the attached CoA for INXN-2002 lot number 0786-240-0002-1, the lot intended for use in manufacture of the FCX-007 clinical product.

**Table 2: Characterization of INXN-2002**

| **Category** | **Assay** | **Test Method** | **Lab** | **SOP** | **Target Specification** |
|---|---|---|---|---|---|
| Identity | Vector identity | RT-PCR | CoH | QC-SOP-0843 | Band of correct size detected |
| | Vector insert stability | Southern blot analysis | Indiana University | VP-10-17.16 | Report result |
| Potency | Viral antigen Physical Titer (p24) | ELISA | Indiana University | VP-10-05.06 | Report result |
| | TU titer | H1299 transduction with qPCR readout | BioReliance | 016135.BSV | Report result |
| | C7 expression (Potency) | ELISA | BioReliance | 016136.BSV | Report result |
| Appearance and pH | Appearance | Visual inspection | CoH | QC-SOP-0734 | Opaque solution |
| | pH | pH meter | CoH | QC-SOP-0675 | 6.9 - 7.8 |

### F. Characterization of INXN-2002 by an in vitro Immortalization Assay

The potential for insertional genotoxicity of INXN-2002 was evaluated using an in vitro immortalization (IVIM) assay. The test was conducted at Cincinnati Children's Hospital Medical Center, Division of Experimental Hematology & Cancer Biology (CCHMC).

The principle of the IVIM test is based on the understanding that normal Lineage negative (Lin- ) bone marrow (BM) cells will stop proliferating after 3-4 weeks in vitro, but in the presence of certain vector integrations which cause upregulation of proto-oncogenes, some clones will continue to proliferate after 5 or more weeks. The number of such immortalized clones is representative of the oncogenic potential of the vector. The immortalized clones are expanded and further analyzed for stem cell markers by FACS, vector copy number by qPCR or the site of integration by LAM-PCR. Insertion in common integration sites (cis) such as Evil or Prdm 16 is frequently observed in immortalized clones generated in IVIM assays (Calmels et al., 2005; Modlich et al., 2008).

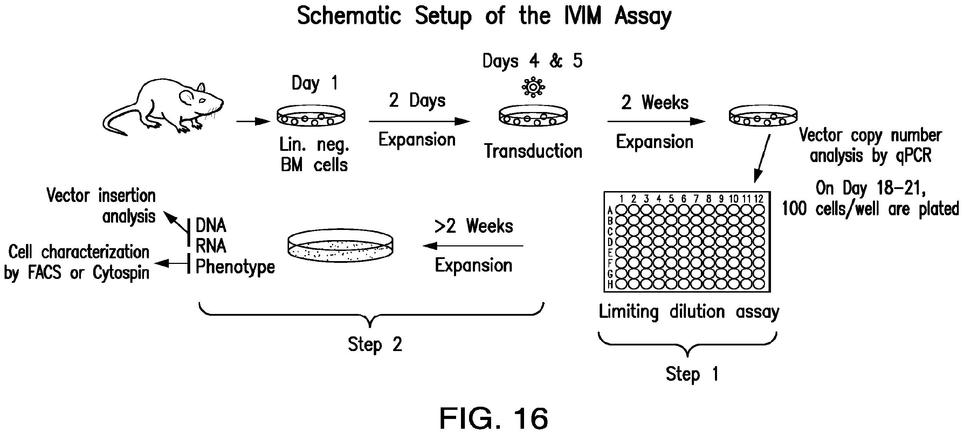

Lineage-negative (Lin-) bone marrow (BM) cells from C57BL/6 mice were isolated from complete BM by magnetic sorting using lineage-specific antibodies. The Lin- BM cells were then cultured and stimulated in complete growth medium for 2 days. On Day 4, cells were transduced with INXN-2002 vector in a 48-well plate coated with RetroNectin (10µg/cm², Takara). Figure 16 shows a schematic setup of the IVIM assay.

To enhance the INXN-2002 transduction efficiency on Lin- BM cells, INXN-2002 vector was concentrated approximately 14-fold using a spin concentrator. For transduction, 80 µL of the concentrated INXN-2002 was added to each well containing 1.5 × 10⁵ cells with 150 µL of transduction medium. The plate was centrifuged for 1000g at 32°C for 70 minutes (spinocculation) to further enhance the transduction efficiency. Cells were incubated at 37°C, 5% CO₂ incubator O/N.

In order to achieve a high INXN-2002 vector copy number in the transduced Lin- BM cells, INXN-2002 transduction was repeated on Day 5, Day 6, and Day 7 for a total of 4 consecutive transductions.

The transduced Lin- BM cells were expanded for 10 days and cell concentration was adjusted to 2 - 4 × 10⁵ cells/ml every two days, and fresh medium was provided as needed. On Day 10 post transduction, cells were harvested and counted. A portion of the cells was then submitted for DNA isolation and qPCR to determine the vector copy number (VCN). The remaining cells were placed back into culture for potential plating of the IVIM assay between days 18 - 21.

Table 3 shows the pilot test run result of INXN-2002 transduced Lin- BM cells harvested on Day 10.

**Table 3: Day 10 Post-Transduction Lin- BM Cells Analysis**

| **Test Article** | **Total Cells** | **Cell Viability** | **Vector Copy per Cell** |
|---|---|---|---|
| INXN-2002 | 1.5 x 10⁶ | 89% | 0.09 |
| Control vector-1 | 2.7 x 10⁶ | 95% | 0.81 |
| Control vector-2 | 2.1 x 10⁶ | 91% | 3.58 |
| Mock | 3.0 x 10⁶ | 88% | 0.0 |

Despite the use of concentrated INXN-2002 vector and 4x consecutive spinocculation transduction, the vector copy number reached in the Lin- BM cells was significantly lower than the targeted copy number of 1-3 expected for the IVIM test. The pilot test was terminated.

Because of the low vector copy numbers experienced in the IVIM assays as well as the low vector copy numbers achieved in all the RDEB fibroblast cell transduction runs, it is believed that this lot of INXN-2002 poses minimal risk of insertional genotoxicity when used to transduce RDEB fibroblast cells. No further analysis of this lot of INXN-2002 by the IVIM assay was pursued.

### G. FCS-007 Transduced with INXN-2002

The human dermal fibroblast cells used for the manufacture of FCX-007 are derived from a live skin biopsy. The biopsy is digested using Liberase (Roche) to release the dermal fibroblast cells and then the cells are expanded in culture using standard cell culture techniques. At the completion of culture expansion after INXN-2002 transduction, the cells are harvested and washed, then formulated to contain 1.0 - 3.0 × 10⁷cells/mL. The DS is tested for purity and confirmed to contain ≥ 98% fibroblasts by CD90 staining with cell viability of ≥ 85%.

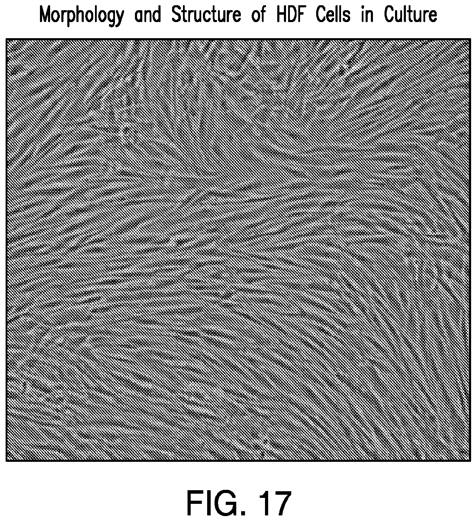

FCX-007 cells transduced with the INXN-2002 LV in the DS formulation display typical fibroblast morphologies when cultured on tissue culture surfaces. Specifically, cells may display an elongated, fusiform or spindle appearance with slender extensions, or cells may appear as larger, flattened stellate cells which may have cytoplasmic leading edges. A mixture of these morphologies may also be observed. Figure 17 shows a typical cell morphology and structure of FCX-007 DS in culture.

The cells express proteins characteristic of normal fibroblasts including the fibroblast specific marker, CD90 (Thy-1), a 35 kDa cell-surface glycoprotein, and the extracellular matrix proteins, such as various types of collagen.

### H. FCX-007 Derived from Lentiviral Vector INXN-2002: Drug Substance Release Characterization

FCX-007 Drug Substance is characterized for release at three stages during manufacturing: in- process, bulk harvest, and after cryopreservation (DS). Table 4 provides a list of the characterization assays and specifications for release of FCX-007 DS manufactured by PCT. Characterization assay results for the finished Drug Substance are provided in the attached CoTs for Training Run 8 Arm A, Arm B and Arm C (Non-Transduced Control).

**Table 4: FCX-007 Drug Substance Release Characterization**

| **Stage** | **Assay** | **Test Method** | **SOP** | **Specification** |
|---|---|---|---|---|
| In-Process | Morphology | Microscopic evaluation | PCT SOP-0334 | Pass |
| | Cell confluence | Microscopic evaluation | PCT SOP-0334 | Pass |
| Bulk Harvest | Cell count | Hemacytometer | PCT SOP-0329 | ≥1E9 |
| | Cell viability | Hemacytometer | PCT SOP-0329 | ≥ 85% |
| Drug Substance-Cryovial | Cell Count | Hemacytometer | PCT SOP-0329 | 1.0-2.7E7 cells/mL |
| | Cell Viability | Hemacytometer | PCT SOP-0329 | >/= 85% |
| | Purity | FACS | PCT SOP WI-1067 | >/= 98% CD-90+ |
| | COL7A1 gene copy # | qPCR | BioReliance109011.BSV | Report result |
| | C7 expression | ELISA | PCT SOP-0338 | Report result |

### I. Additional FCX-007 DS Derived from INXN-2002 Characterization

Additional characterization of FCX-007 has been performed on the training production runs to confirm expression of functional C7. The representative assessments shown below are from Training Run 8 (TR8) where cells were transduced with either a high dose (3.4 IU/cell) or low dose (1.7 IU/cell) of LV-COL7, or were mock-transduced. These transduction arms are also referred to as Arm A, B, and C, respectively.

### J. FCX-007 DS Transduced with INXN-2002: C7 Expression Level

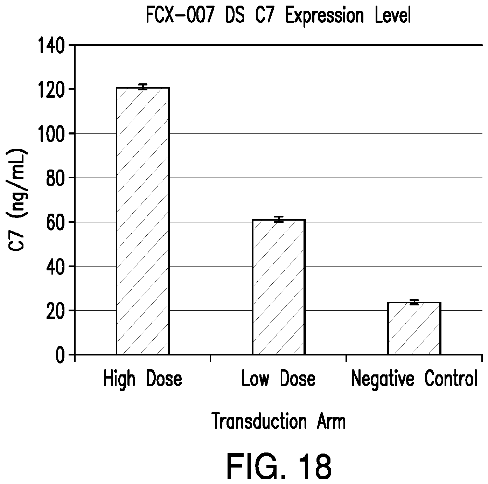

An enzyme-linked immunofluorescence assay (ELISA) was developed for the purpose of quantifying C7 expression by FCX-007. For this assay, TR8 Drug Substance vials (high dose, low dose, and mock-transduced) were thawed and cultured for 3 days and conditioned cell culture supernatants were collected and assayed for C7. Results in Figure 18 show virus dose-dependent protein expression that ranges from 60 to 120 ng/mL C7 in LV-COL7-transduced cells.

### K. FCX-007 DS Transduced with INXN-2002: C7 Trimer Formation

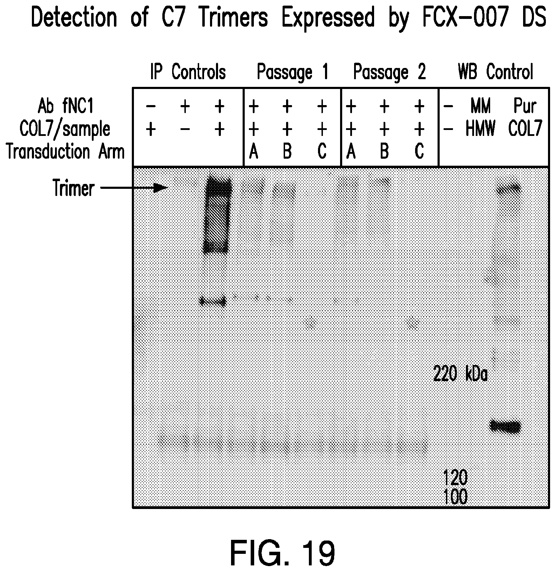

Anchoring fibrils are formed from the assembly of C7 trimers. The proper expression and formation of C7 trimers by FCX-007 can be detected by immunoprecipitation of C7 followed by non-denaturing SDS-PAGE/immunoblot analyses. For Figure 19, C7 was immunoprecipitated from FCX-007 cell culture supernatants at passage 1 and passage 2 post- thaw using a NC1-specific antibody and was separated on non-denaturing SDS-PAGE then visualized by western blot. The C7 produced by RDEB fibroblasts was predominantly trimeric (red arrow; ∼870 kDa) with LV-COL7-transduced cells (Transduction Arms A and B) expressing more C7 than mock-transduced cells (Arm C, starred). Monomeric (290 kDa) and dimeric (580 kDa) forms were also observed. Assay controls included immunopreciptation of purified C7 (Pur COL7) and immunoprecipitation without antibody or test sample (IP Controls).

### L. FCX-007 Transduced with INXN-2002: C7 Binding to Lam332

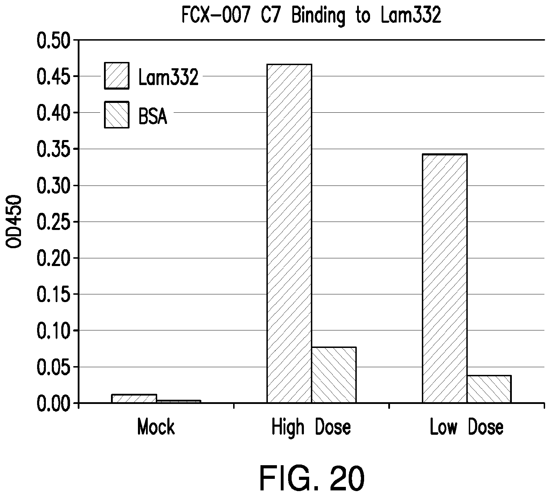

C7 interacts with Laminin332 at the dermal/epidermal junction (DEJ). The interaction between C7 and Lammin332 is important for anchoring fibril functionality (Chen 2002, Rousselle 1997, Waterman 2007). A binding assay for detection of this interaction was developed at Intrexon. Drug Substance vials (high dose, low dose, and mock-transduced) were thawed and cultured for 2 days. Conditioned cell culture supernatants were collected and were incubated with Lam332 or bovine serum albumin (BSA)-coated wells and bound C7 was detected using a C7 NC1- specific antibody and an HRP-conjugated secondary antibody. The assay readout is optical density at 450 nm (OD450). Results in Figure 20 show virus dose-dependent binding to Lam332 compared with a BSA control.

### M. FCX-007 Derived from INXN-2002 Migration Assessment

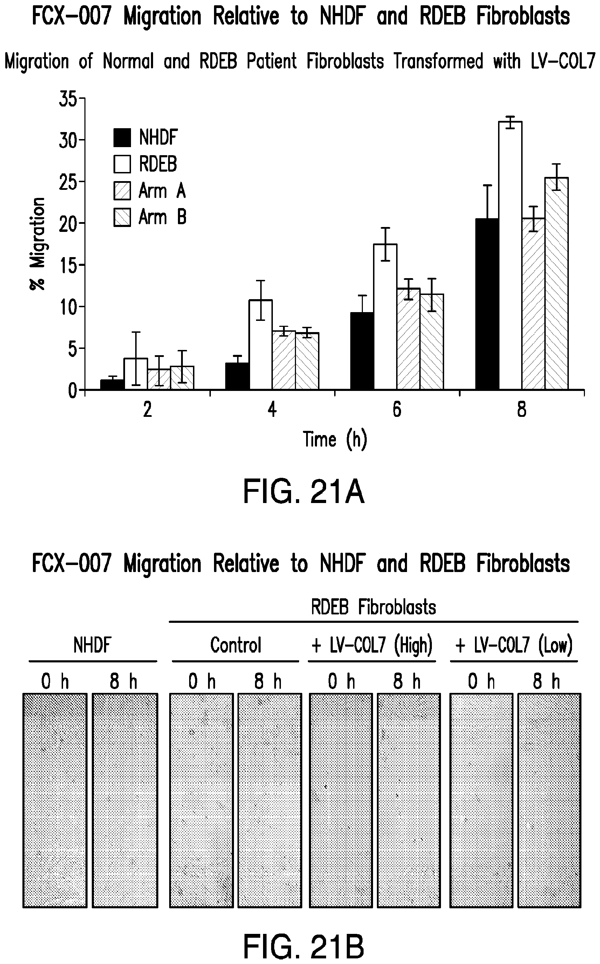

A migration assay was developed to assess function of C7. Chen 2000 has demonstrated that RDEB patient skin cells migrate faster than normal skin cells into an artificial wound margin created on tissue culture vessels, and that application of C7 can restore the migration rate. Normal human dermal fibroblasts (NHDFs; Lonza) and cells from FCX-007 Drug Substance vials were thawed and cultured. Cells were seeded into culture dishes with an insert to prevent cell adherence in a small strip of the culture dish. The strip was then removed and the rate at which the cells migrated into the open area was monitored by microscopy and quantified using the irregular shape delineation plugin for ImageJ software. Figures 21A-21B show both the percent migration (A) and the images of migration (B) for this assay. The results show that the mock-transduced RDEB patient fibroblasts migrate into the open area faster than NHDFs, and transduction with LV-COL7 reverts the patient cells to a rate of migration similar to NHDFs. These results are consistent with those described by Chen 2000.

### Example 2: FCX-007 Transduced with INXN-2004 Lentiviral Vector

FCX-007 is an autologous fibroblast cell product genetically modified by INXN-2004 lentiviral vector (LV-COL7) to express the human collagen 7 protein (C7). The materials used to manufacture FCX-007 DS/INXN-2004 are described below are similar to that set forth above in Example 1, except that the IGE-230 LV-COL7 vector plasmid was used for the production of INXN-2002. IGE-308 LV-COL7 vector plasmid was used to make the INXN-2004 vector. The same helper plasmids, pCMV-G, pCMV-Rev2, and pCgp were used to co-transfect a 293 WCB cell line.

### INXN-2004 Lentiviral Vector

### IGE308 LV-COL7 Vector Transfer Plasmid

The IGE308 plasmid was constructed using standard molecular cloning methods. The construction process involved cloning of the human *COL7A1* gene and introduction of the cloned *COL7A1* gene into a lentiviral vector (SIN) backbone, pFUGW, to produce the IGE308 LV-COL7 vector transfer plasmid.

### Cloning of the Human COL7A1 Gene

The *COL7A1* gene cloned into the IGE308 LV-COL7 vector transfer plasmid is the same *COL7A1* gene cloned into INXN-2002 (IGE230 vector transfer plasmid).

Primers were designed and produced with the Takara PrimeScript reverse transcriptase to amplify the *COL7A1* gene from human genomic cDNA. Four primer pairs, as shown in Table 5 were designed, each to amplify approximately 2 kb of the *COL7A1* gene.

**Table 5: Primer Sets used to Amplify the Human COL7A1 Gene**

| **Forward Primer** | **Primer Sequence** | **Reverse Primer** | **Primer Sequence** | **Expected Amplicon Size (bp)** |
|---|---|---|---|---|
| CollF1 (SEQ ID NO: 3) | CGACTTGTGTTGGGACTGG CTAGCGCCACCATGACGCT GCGGCTTCTGGTGGC | CollR3 (SEQ ID NO: 7) | CCCGCACAGTGTAGCTAA GCCC | 1753 |
| CollF3 (SEQ ID NO: 4) | GGGCTTAGCTACACTGTGC GGG | CollR2 (SEQ ID NO: 8) | CCTTTGGACAATACACTG GGCAGG | 2059 |
| CollF2 (SEQ ID NO: 5) | CCTGCCCAGTGTATTGTCC AAAGG | CollR4 (SEQ ID NO: 9) | CAGATGCCTTGATGCCAG CAG | 2127 |
| CollF4 (SEQ ID NO: 6) | CTGCTGGCATCAAGGCATC TG | CollR1 (SEQ ID NO: 10) | CGTGATTTCATTTGCTAC ACGTAATCGATTCAGTCC TGGGCAGTACCTGTCCC | 2993 |

Products were amplified with two primer pairs (CollF2/CollR4 and CollF4/CollR1) designed to amplify the 5' end 3790 base pairs (bp) of *COL7A1* from human genomic cDNA along with a 5' overhang for cloning into the expression vector. The two 5' PCR products (2127 bp and 2993 bp) were joined by overlap extension PCR to generate a 3790 bp final product.

In order to clone the remainder of the gene, a fragment of DNA (341bp, Col1A1-3, Table 6 was synthesized encompassing the final 322 bp of the *COL7A1* gene with overlaps to the PCR amplified 5' 3790bp gene fragment and the cloning vector.

**Table 6: Col1A1-3 Fragment Sequence**

| **Fragment** | **Sequence** |
|---|---|
| Col1A1-3 (SEQ ID NO: 11) | CGCTCCCAGAACATCACCTACCACTGCAAGAACAGCGTGGCCTACATG GACCAGCAGACTGGCAACCTCAAGAAGGCCCTGCTCCTCCAGGGCTCC AACGAGATCGAGATCCGCGCCGAGGGCAACAGCCGCTTCACCTACAGC GTCACTGTCGATGGCTGCACGAGTCACACCGGAGCCTGGGGCAAGACA GTGATTGAATACAAAACCACCAAGACCTCCCGCCTGCGCATCATCGAT GTGGCCCCCTTGGACGTTGGTGCCCCAGACCAGGAATTCGGCTTCGAC GTTGGCCATGTCTGCTTCCTGTAAATCGATTACGTGTAGCAAATGAAAT CACG |

The two *COL7A1* gene fragments (3790 bp and Col1A1-3) were assembled into an inducible expression vector (VVN-257673) that had previously been digested with NheI and ClaI using the In-Fusion HD Cloning Kit. Bacterial clones were screened by PCR with primers specific to the backbone vector and *COL7A1* sequence to identify candidates for sequencing. Positive clones were confirmed by digestion and *COL7A1* fragment DNA sequencing. The resulting plasmid was named VVN-4311835.

VVN-4311835 and a *COL7A1* expression plasmid (SC300011) purchased from Origene were digested with BstZ17I and SapI. A 6276 bp fragment of the *COL7A1* gene was cloned from SC300011 into VVN-4311835. The resulting plasmid, VVN-4311835 (used the same plasmid number), was completely sequenced and the *COL7A1* sequence was confirmed to be complete and without mutations.

VVN-4311835 plasmid was digested with NheI and ClaI, two restriction enzymes with sites just outside of the *COL7A1* coding sequence. The excised *COL7A1* gene was cloned into a constitutive expression vector, VVN-257231 which was also digested with NheI and ClaI, to produce VVN-4319958. VVN-4319958 expresses the full length human *COL7A1* under the control of the constitutive CMV promoter.

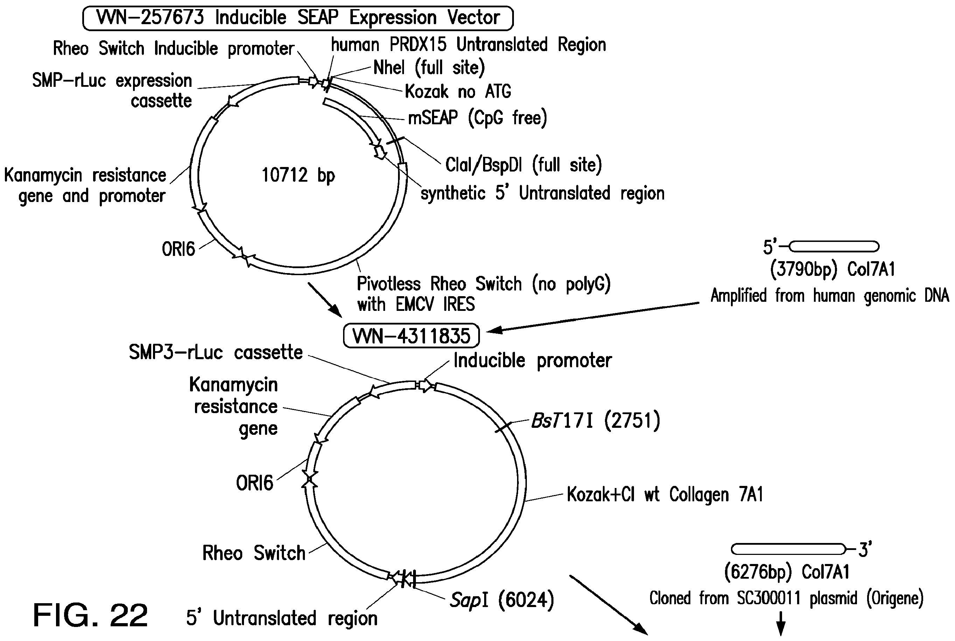

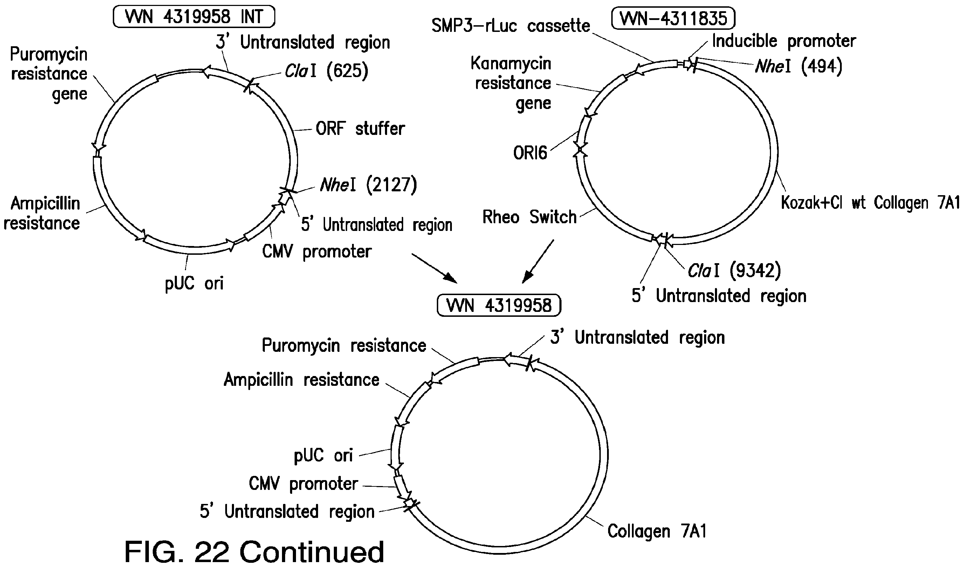

Figure 22 provides a schematic for the cloning of the human *COL7A1* gene.

### Introduction of the Cloned COL7A1 Gene into a Lentiviral Vector (SIN)

The pSMPUW lentiviral expression vector (VPK-211, Cell Biolabs, Inc., San Diego, CA) was initially selected for construction of INXN-2002 lentiviral vector encoding the *COL7A1* gene based on its enhanced safety features and large cloning capacity.

Figure 7 compares the pSMPUW vector to a standard 3rd generation SIN LV vector. The pSMPUW vector encodes for a multicloning site (MCS) followed by the Woodchuck Hepatitis Virus Post-transcriptional regulatory element (WPRE). The residual gag (Δgag) and the RRE element were removed from the pSMPUW vector construct. Additionally, the pSMPUW vector construct utilized a larger 400 bp deletion in the 3'LTR U3 region instead of the commonly used 133 bp deletion in a standard 3rd generation SIN LV vector.

Details of the cloning of the *COL7A1* gene into the pSMPUW lentiviral expression vector for construction of INXN-2002 lentiviral vector were described above in Example 1, and are further described below.

An insert was generated containing a CMV promoter followed by a Kozak sequence and a truncated version of the *COL7A1* gene with Bell and SapI restriction sites to use for introduction of the entire coding sequence. This insert was synthesized in two fragments, CColG and ColG2 (IDT) (Table 8). These two fragments were assembled with the digested pSMPUW vector using the In-Fusion HD Cloning Kit. Positive clones were identified using colony PCR with a primer specific to the plasmid backbone (63968-3R), and a primer specific to the *COL7A1* gene (ColS14). Plasmids from positive clones were purified and sequence confirmed to generate IGE228. IGE228 was digested with FspI and BamHI.

Initial attempts to clone the *COL7A1* gene using the BclI and SapI sites were unsuccessful. An alternate strategy was devised using a PCR product as a linker to bypass the BclI restriction site. Primers EPF5 and CollR3 were used to generate a PCR product from the plasmid template VVN-4319958. This product was digested with BamHI, which cuts 48 bp upstream of the 5' end of *COL7A1,* and FspI, which cuts 1630 bp into the 5' end of *COL7A1* to generate the 5' linker.

The wild type *COL7A1* gene was cut with FspI and SapI from VVN-4319958. This fragment was ligated to the digested IGE228 and the 5' linker to generate a lentivirus construct for the expression of *COL7A1*, VVN-4580853 (also named IGE230). Positive clones were identified using the primers ColS13, specific to *COL7A1* and 63968-3R, specific to the lentiviral backbone. The plasmid was sequenced from the CMV promoter to the 3' end of *COL7A1.*

Table 7 below shows the synthetic gene sequences and primers used for introduction of the *COL7A1* gene into the pSMPUW lentiviral expression vector.

**Table 7: Synthetic Gene Elements and Primers**

| **Gene Element / Primer Name** | **Sequence** |
|---|---|
| CColG (SEQ ID NO: 12) | TTCAAATTTTCGGGGGATCGCATTAGTTATTAATAGTAATCAATTACGGGG TCATTAGTTCATAGCCCATATATGGAGTTCCGCGTTACATAACTTACGGTAA ATGGCCCGCCTGGTTGACCGCCCAACGACCCCCGCCCATTGACGTCAATAA TGACGTATGTTCCCATAGTAACGCCAATAGGGACTTTCCATTGACGTCAAT GGGTGGAGTATTTACGGTAAACTGCCCACTTGGTAGTACATCAAGTGTATC ATACGCCAAGTACGCCCCCTATTGACGTCAATGACGGTAAATGGCCCGCCT GGTATTATGCCCAGTACATGACCTTATGGGACTTTCCTACTTGGCAGTACAT CTGCGTATTAGTCATCGCTATTACCATGGTGATGCGGTTTTGGCAGTACATC AATGGGCGTGGATAGCGGTTTGACTCACGGGGATTTCCAAGTCTCCACCCC ATTGACGTCAATGGGAGTTTGTTTTGGCACCAAAATCAACGGGACTTTCCA AAATGTCGTAACAACTCCGCCCCATTGACGCAAATGGGCGGTAGGCGTGTA CGGTGGGAGGTCTATATAAGCAGAGCTGGTTTAGTGAACCGTCAGATCCGC TAGCATTGTGTGCTTTTCGGGCCACCATGACGCTGCGGCTTCTGGTGGCCGC GCTCTGCGCCGGGATCCTGGCAGAGGCGCCCCGAGTGCGAGCCCAGCACA GGGAGAGAGTGAC |
| ColG2 (SEQ ID NO: 13) | AGTGCGAGCCCAGCACAGGGAGAGAGTGACCTGCACGCGCCTTTACGCCG CTGACATTGTGTTCTTACTGGATGGCTCCTCATCCATTGGCCGCAGCAATTT CCGCGAGGTCCGCAGCTTTCTCGAAGGGCTGGTGCTGCCTTTCTCTGGAGC AGCCAGTGCACAGGGTGTGCGCTTTGCCACAGTGCAGTACAGCGATGACCC ACGGACAGAGTTCGGCCTGGATGCACTTGGCTCTGGGGGTGATGTGATCCG CGCCATCCGTGAGCTTAGCTACAAGGGGGGCAACACTCGCACAGGGGCTG CAATTCTCCATGTGGCTGACCATGTCTTCCTGCCCCAGCTGGCCCGACCTGG TGTCCCCAAGGTCTGCATCCTGATCACGTCTCTCATGCAGAGGAGGAAGAG CGGGTACCCCCTGAGGATGATGAGTACTCTGAATACTCCGAGTATTCTGTG GAGGAGTACCAGGACCCTGAAGCTCCTTGGGATAGTGATGACCCCTGTTCC CTGCCACTGGATGAGGGCTCCTGCACTGCCTACACCCTGCGCTGGTACCAT CGGGCTGTGACAGGCAGCACAGAGGCCTGTCACCCTTTTGTCTATGGTGGC TGTGGAGGGAATGCCAACCGTTTTGGGACCCGTGAGGCCTGCGAGCGCCGC TGCCCACCCCGGGTGGTCCAGAGCCAGGGGACAGGTACTGCCCAGGACTG AGTACCTTTAAGACCAATGACTTACAA |
| 63968-3R (SEQ ID NO: 14) | ATGGAAAAACGCCAGCAACG |
| ColS14 (SEQ ID NO: 15) | CTGTCACCCTTTTGTCTATG |
| EPF5 (SEQ ID NO: 16) | GTTGCCGGACACTTCTTGTCCTCT |
| CollR3 (SEQ ID NO: 17) | CCCGCACAGTGTAGCTAAGCCC |
| ColS13 (SEQ ID NO: 18) | AGAGGCCCCGAAGGACTTCA |

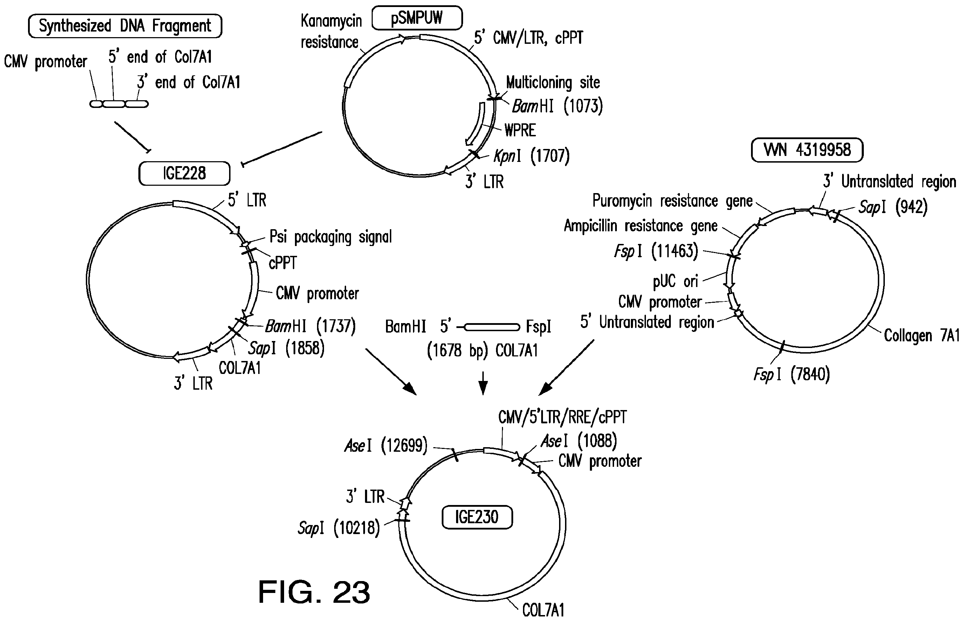

Figure 23 provides a schematic of the cloning of the *COL7A1* gene into the pSMPUW expression vector to produce the INXN-2002 lentiviral vector transfer plasmid, IGE230.

Results from continuing development indicated that deletion of the RRE element and the use of a large 400 bp deletion in the 3'LTR U3 region in the pSMPUW vector construct, combined with the requirement to package a large *COL7A1* gene (8.8 kbp), resulted in a negative impact on LV-COL7 vector production (infectious titer) and subsequent transduction and C7 protein expression in RDEB fibroblast cells.

A 3^{rd} GEN SIN vector, pFUGW (FUGW, plasmid #14883, www.addgene.org) was selected to construct a second generation LV-COL7 vector, INXN-2004. INXN-2004 was constructed to improve the vector copy number. Figure 8 provides a schematic of the genetic elements in the pFUGW lentiviral expression plasmid vector.

The pFUGW vector contains a RRE, two cPPT elements, as well as a WPRE (woodchuck hepatitis virus posttranscriptional regulatory element) element for achieving improved lentiviral vector production and transgene expression. The IGE308 lentiviral vector transfer plasmid was constructed to maximize the transgene cloning capacity to accommodate the insertion of the large *COL7A1* gene (8.8 kbp).

The WPRE element, the hUBC promoter and the GFP reporter gene were removed through digestion of the vector with PacI and XhoI. An insert was generated containing the 5' end of the CMV promoter followed by a small fragment of the 3' end of *COL7A1* gene. This insert (ColCN) was synthesized at IDT as a G-Block fragment (sequence in Table 8). The fragment was assembled with the digested pFUGW vector using a Gibson cloning derivative method. Positive clones were identified using colony PCR with primers specific to the plasmid backbone (FugwQCF and FugwQCR). Plasmids from positive clones were purified and sequence confirmed to generate IGE301.

IGE301 was then digested with BaeI. IGE230 was digested with AseI and SapI to create a fragment containing the 3' end of the CMV promoter, the 5' UTR, and the majority of the *COL7A1* gene. The fragment was transferred to the BaeI-digested IGE301 vector using a Gibson cloning derivative method. Positive clones were identified using the primers ColS13, specific to *COL7A1* and FugwQCR, specific to the lentiviral backbone. Plasmids from positive clones were purified and sequence-confirmed to generate IGE308.

Table 8 below provides the synthetic gene sequences and primers used for introduction of the *COL7A1* gene into the pFUGW lentiviral expression vector.

Plasmids were Maxi-prepped with the Qiagen MaxiPrep Kit per the manufacturer's protocol.

**Table 8: Synthetic Gene Elements and Primers used in the Final Steps of IGE308 Creation**

| **Gene Element / Primer Name** | **Sequence** |
|---|---|
| ColCN (g-block) (SEQ ID NO: 19) | Acagcagagatccagtttggttaattaagcattagttattaatagtaatcaattacggggtcattaagactcggcctta gaaccccagtatcagcagaaccgcgtctctcatgcagaggaggaagagcgggtaccccctgaggatgatgagt actctgaatactccgagtattctgtggaggagtaccaggaccctgaagctccttgggatagtgatgacccctgttcc ctgccactggatgagggctcctgcactgcctacaccctgcgctggtaccatcgggctgtgacaggcagcacaga ggcctgtcacccttttgtctatggtggctgtggagggaatgccaaccgttttgggacccgtgaggcctgcgagcgc cgctgcccaccccgggtggtccagagccaggggacaggtactgcccaggactgatcgataccgtcgacctcga gacctagaaaaacat |
| FugwQCF (SEQ ID NO: 20) | GTAGACATAATAGCAACAGAC |
| FugwQCR (SEQ ID NO: 21) | TATTGCTACTTGTGATTGCTC |
| ColS13 (SEQ ID NO: 22) | AGAGGCCCCGAAGGACTTCA |

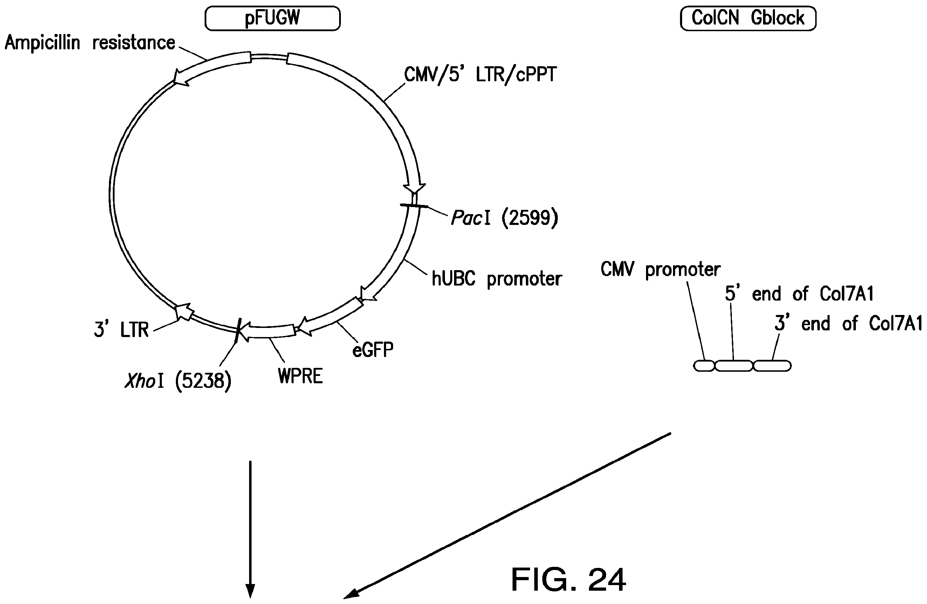

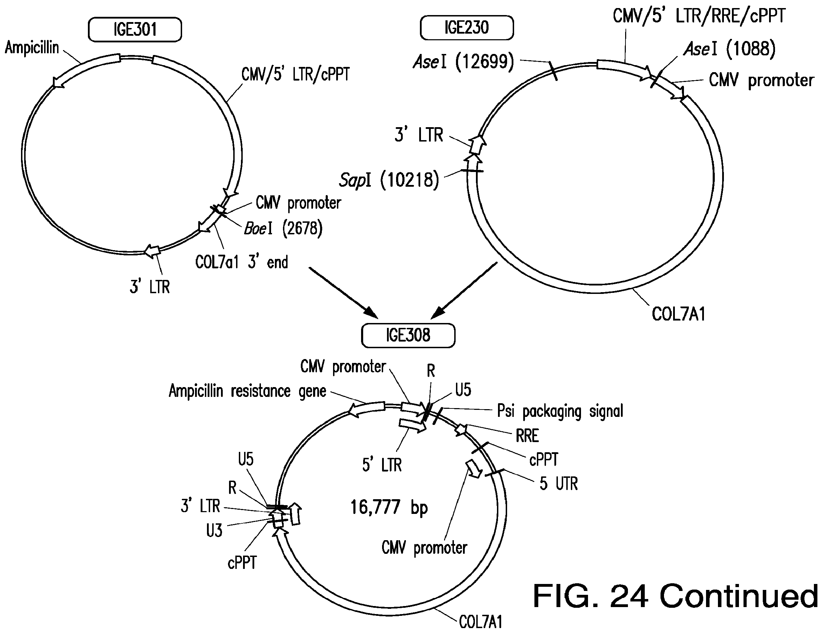

Figure 24 provides a schematic representation of the construction of the INXN-2004 lentiviral vector transfer plasmid (IGE308).

### Production of IGE-308 Plasmid

The IGE308 plasmid was produced using a process consisting of five steps: transformation, glycerol stock production, scale up, capture, diafiltration and formulation.

Transformation: a seed stock of the IGE308 plasmid was provided to Aldevron for transformation into the competent *E. coli* (DH10B). Transformation plates were stored at 34°C which is the standard for lentiviral constructs.

Glycerol stock production - Seed cultures were created by picking isolated colonies from the transformation plate. Each culture was mini prepped and the best seed culture, as determined via agarose gel, was used to make the working glycerol stocks. Scale Up - One 500 mL culture was grown in each of the following media types: Rapid Growth media and Maximum Yield media (for a total of two 500 mL cultures) in order to compare media. The cultures were inoculated using the glycerol stocks created in step two and a full panel of QC assays was run on each prep. This also allowed Aldevron to test the stability of the plasmid in each media type.

Based on the initial QC results Rapid Growth media was chosen for growth of the transformed *E. coli* in shaker flasks at 34°C. A total of 8L of culture was prepared.

Capture - The 8 L of *E. coli* culture was lysed and initially purified using DMAE anion exchange chromatography which produced a total of 833.8 mg plasmid. An additional purification step was run using HIC (Hydrophobic Interaction Chromatography) chromatography over OS resin. This step yielded 663 mg of purified DNA.

Concentration and Formulation - The purified plasmid was then adjusted to the final buffer and concentration via serial diafiltration. The final buffer used was TE.

A total of 200 mg of IGE308 plasmid was shipped at a concentration of 1.4 mg/mL. The IGE308 plasmid was analyzed prior to further manufacture of the INXN-2004.

### Analysis of IGE308 Plasmid Sequence

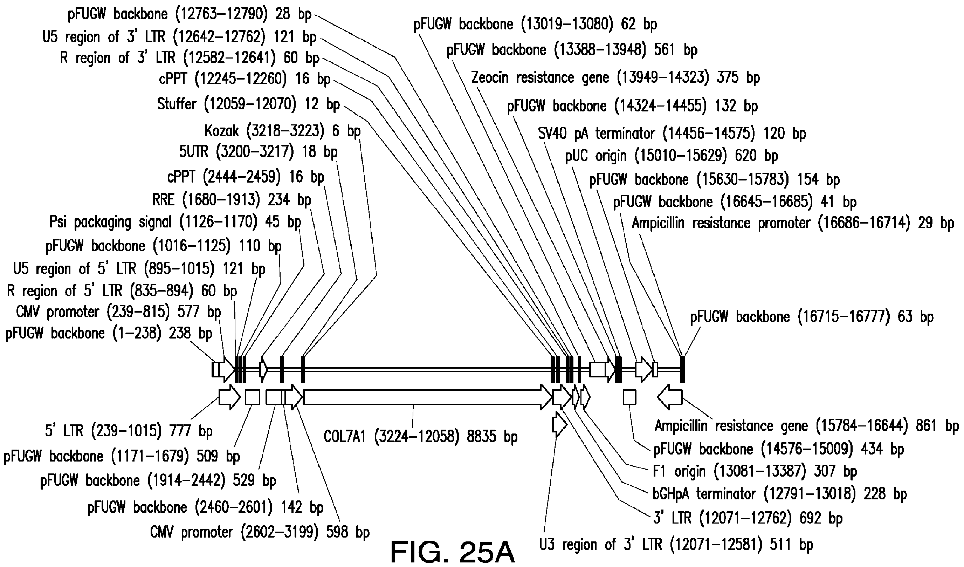

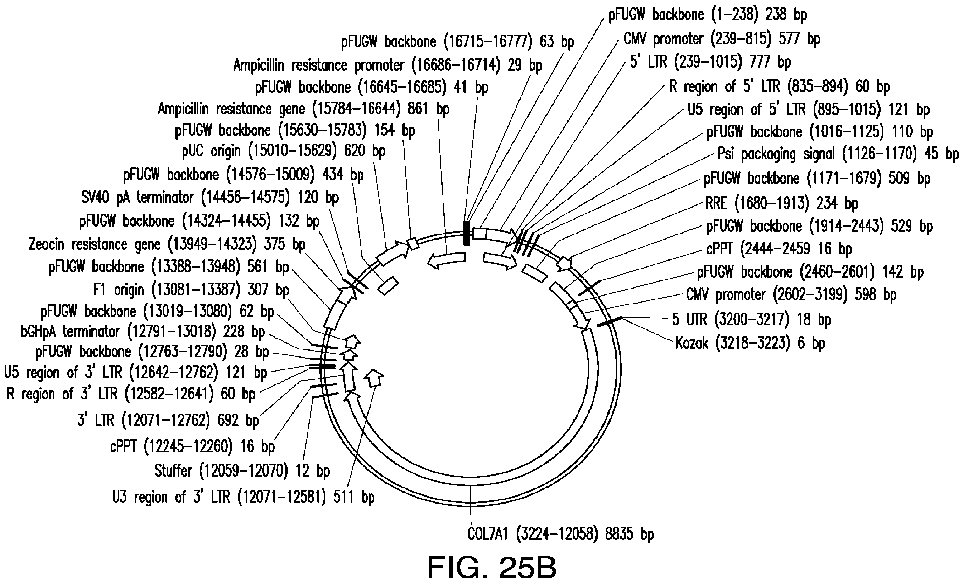

The IGE308 plasmid used for INXN-2004 manufacture was fully sequenced at SeqWright under GLP conditions using primer walking and oligonucleotide synthesis to yield 4-fold, bi-directional sequence coverage. The IGE308 plasmid has a size of 16,777 bp. The sequence showed 100% match to the IGE308 construction reference sequence provided to SeqWright. Gene elements in the IGE308 plasmid are illustrated in Figures 25A-25B. Table 9 provides a list of gene elements with their corresponding functions.

**Table 9: Size and Functions of Gene Elements in IGE308 Plasmid**

| **Location** | **Size (bp)** | **Element** | **Function** |
|---|---|---|---|
| 1-238 | 238 | pFUGW backbone | Backbone element |
| 239-815 | 577 | CMV promoter | Promoter to drive expression of the proviral genome |
| 239-1015 | 777 | 5' LTR | Proviral genome packaging and integration |
| 835-894 | 60 | R region of 5' LTR | Repeat region in 5' and 3' LTRs, transcription initiation site |
| 895-1015 | 121 | U5 region of 5' LTR | Unique 5' sequence contains the Tat binding site and packaging sequences of HIV |
| 1016-1125 | 110 | pFUGW backbone | Backbone element |
| 1126-1170 | 45 | Psi packaging signal | RNA target site for packaging the viral RNA genome into viral capsid during replication |
| 1171-1679 | 509 | pFUGW backbone | Backbone element |
| 1680-1913 | 234 | RRE | Facilitates mRNA transcript nucleus export when bound to Rev protein |
| 1914-2443 | 529 | pFUGW backbone | Backbone element |
| 2444-2459 | 16 | cPPT | Recognition site for proviral DNA synthesis, increases transduction efficiency and transgene expression |
| 2460-2601 | 142 | pFUGW backbone | Backbone element |
| 2602-3199 | 598 | CMV promoter | Promoter to drive the expression of *COL7A1* gene inside the LV-COL7 vector |
| 3200-3217 | 18 | 5UTR | Synthetic 5'UTR fragment to improve the translation of the *COL7A1 (derived from* Rahnella aquatilis, ATCC 33071) |
| 3218-3223 | 6 | Kozak | Translation enhancement |
| 3224-12058 | 8835 | Collagen 7A1 ORF with TGA stop codon | Collagen 7A1 ORF encoding the human wild type collagen 7 protein |
| 12059-12070 | 12 | Stuffer | Remnant sequence resulted from DNA cloning |
| 12071-12762 | 692 | 3'LTR | Polyadenylation signal and proviral genome packaging and integration |
| 12071-12581 | 511 | U3 region of 3' LTR | SIN deletion (133 bp) located in this region |
| 12245-12260 | 16 | cPPT | Recognition site for proviral DNA synthesis, increases transduction efficiency and transgene expression |
| 12582-12641 | 60 | R region of 3' LTR | Repeat region in 5' and 3' LTRs, transcription initiation site |
| 12642-12762 | 121 | U5 region of 3' LTR | Unique 5' sequence contains the Tat binding site and packaging sequences of HIV |
| 12763-12790 | 28 | pFUGW backbone | Backbone element |
| 12791-13018 | 228 | bGHpA terminator | Sequence to stop transcription of DNA |
| 13019-13080 | 62 | pFUGW backbone | Backbone element |
| 13081-13387 | 307 | F1 origin | F 1 phage origin of replication |
| 13388-13948 | 561 | pFUGW backbone | Backbone element |
| 13949-14323 | 375 | Zeocin resistance gene | Mammalian selection marker |
| 14324-14455 | 132 | pFUGW backbone | Backbone element |
| 14456-14575 | 120 | SV40 pA terminator | Sequence to stop transcription of DNA |
| 14576-15009 | 434 | pFUGW backbone | Backbone element |
| 15010-15629 | 620 | pUC origin | Bacterial DNA replication start |
| 15630-15783 | 154 | pFUGW backbone | Backbone element |
| 15784-16644 | 861 | Ampicillin resistance gene | Plasmid selection marker |
| 16645-16685 | 41 | pFUGW backbone | Backbone element |
| 16686-16714 | 29 | Ampicillin resistance promoter | Promoter to drive expression of the ampicillin resistance gene |
| 16715-16777 | 63 | pFUGW backbone | Backbone element |

The *COL7A1* gene sequence in the IGE308 plasmid (without the stop codon TGA) was compared to the GenBank Homo sapiens collagen, type VII, alpha 1 (*COL7A7*) sequence (NM_000094.3). Sequence alignment is provided in Appendix 4. Three silent point mutations were identified as listed in Table 10 below. No sequence gap was identified. The three silent point mutations have no impact on the encoded C7 protein.

**Table 10: Sequence Comparison of COL7A1 in IGE308 and GenBank Consensus**

| **Nucleotide Position** | ***COL7A1* in IGE308** | **GenBank Consensus (NM_000094.3)** | **Impact** |
|---|---|---|---|
| 6040 | G | A | Silent point mutation |
| 8674 | A | G | Silent point mutation |
| 8731 | G | C | Silent point mutation |

### Helper Plasmids, pCMV-G, pCMV-Rev2 and pCgp

The three helper plasmids used for INXN-2004 LV vector production, pCMV-G, pCMV-Rev2 and pCgp, were provided by CoH.

### 293T Working Cell Bank (WCB)

The 293T WCB used for INXN-2004 LV vector production was provided by CoH.

### Other Starting Materials for INXN-2004 Manufacture

Other starting materials used for INXN-2004 LV vector production were also provided by CoH.

### Biopsy Tissue

Three 3-4 mm punch skin biopsies (dermis and epidermis layers) are collected from an un-blistered area of the RDEB subject's body using standard aseptic practices. The biopsies are collected by the treating physician, placed into a vial containing cold, sterile phosphate buffered saline (PBS), and shipped via next day delivery in a refrigerated Infectious Shipper container, which is appropriate for shipping potentially biohazardous tissue and is designed to maintain a temperature of 2-8°C.

### a. Reagents, Solvents, and Auxiliary Materials

### i. Reagents and Solvents

Two animal-sourced reagents are used in the FCX-007 Drug Substance manufacturing process: Fetal Bovine Serum (FBS) and Trypsin-EDTA solution. Complete Growth Medium

Complete Growth Medium (CGM) is used in FCX-007 manufacturing during all the cell culture expansion steps. CGM is prepared by mixing 18 L of IMDM in a 20 L bag with 2 L of FBS through aseptic transfer. The formulated CGM bag is stored in a 5±3°C refrigerator.

### a. Initiation Growth Medium

Initiation Growth Medium (IGM) is used in FCX-007 manufacturing for the initial seeding of fibroblasts after biopsy digestion into a T-75 cell culture flask in the presence of antibiotics. Initiation Growth Medium is prepared fresh by adding 0.6 mL of the100-fold concentrated antibiotic solution (GA) aseptically inside an ISO5 BSC to 58.2 mL of CGM in a 500 mL square media bottle. Final concentrations are 0.4 mg/mL gentamicin, 0.295 µg/mL amphotericin B. GSH solution (1.2 mL of 50X solution) is also added to the 500 mL square media bottle. The bottles are closed and inverted 3-5 times to mix. The IGM is warmed inside a 37°C incubator for at least 60 minutes before immediate use.

The Initiation Growth Medium is prepared fresh immediately prior to use and therefore no expiration date is applied and no Quality Control release tests are performed.

### b. Transduction Medium

Transduction Medium (TM) is used during the INXN-2004 transduction of fibroblast cells. Transduction Medium is prepared fresh inside an ISO5 BSC by adding 40 mL INMM to a 250 mL centrifuge tube containing 10 mL CGM to dilute the FBS concentration to 2% in the medium. The 250 mL tube is mixed and placed inside a 37.0°C Incubator to warm until needed for INXN-2004 transduction.

The Transduction Medium is prepared fresh immediately prior to use and therefore no expiration date is applied and no Quality Control release tests are performed.

### c. GSH Solution

GSH is supplemented to the Initiation Growth Medium (IGM) and CGM used in the early stage T-75 and T-175 fibroblast cell cultures to enhance cell growth. A 50X GSH stock solution is prepared inside an ISO5 BSC by dissolving 51.1 g of GSH into 1 L of IMDM. The solution is 0.22 µm filtered. The filtered solution is aliquoted into 50 mL centrifuge tubes at 20 mL per tube and stored frozen at -80°C for further use.

### d. RetroNectin^{™} Solution

RetroNectin^{™} is used in the FCX-007 production process to enhance the INXN-2004 transduction efficiency on fibroblast cells. Inside an ISO5 BSC, using a 5 mL syringe and a 18g needle, 2.5 mL WFI is aseptically transferred into a vial of clinical grade RetroNectin^{®} to reconstitute RetroNectin^{®} at 1.0 mg/mL. RetroNectin^{®} is dissolved thoroughly by swirling gently. The entire contents of the RetroNectin^{®} vial are withdrawn using the attached 5 mL syringe. The 18g needle is aseptically replaced with a 0.22 µm syringe filter onto the syringe, and the reconstituted RetroNectin^{®} is sterile filtered into a 250 mL centrifuge tube. Using an appropriately sized pipette, 122.5 mL PBS is transferred to the reconstituted RetroNectin^{®} in the 250 mL centrifuge tube to dilute the RetroNectin^{®} to 20µg/mL, which is then mixed well using a pipette. Using an appropriately sized pipette, 6.3 mL of the diluted RetroNectin^{®} solution is added to each of T-25 flasks to coat the surface with RetroNectin^{®} at 5 µg/cm². For the first round of INXN-2004 transduction, 5x to 10x T-25 flasks are prepared. For INXN-2004 super transduction, 18x T-25 flasks are prepared.

The RetroNectin^{™} solution is prepared fresh immediately prior to use and therefore no expiration date is applied and no Quality Control release tests are performed.

### Cryopreservation Medium

Cryopreservation Medium is a two-fold concentrated solution that is added to harvested and washed fibroblasts during the FCX-007 manufacturing process to formulate the cell seed stock and FCX-007 Drug Substance for storage in liquid nitrogen. Cryopreservation Medium is prepared by mixing 0.85 volume ProFreeze^{™}-CDM (2X) with 0.15 volume DMSO to obtain one volume Cryopreservation Medium.

The Cryopreservation Medium is prepared fresh immediately prior to use and therefore no expiration date is applied and no Quality Control release tests are performed.

### Example 3: Training Run 8, 9 and 10 and Enhanced Biopsy Enzymatic Digestion

### Training Run 8 and Enhanced Biopsy Enzymatic Digestion

In the above TRs, the biopsy digestion method was suspected to not be able to achieve effective digestion of the 3-4mm sized biopsy tissue. In TR8, the application of additional shear force during the digestion process was incorporated into the process to improve the overall digestion efficiency. The digestion process was modified to the following: pulse vortex the centrifuge tube at maximum setting for 5 seconds, every 15 ± 2 minutes during the digestion, returning the centrifuge tube to the orbital shaker after each vortex; at the end of the 60 minutes of incubation, pulse vortex the centrifuge tube at maximum setting for 10 seconds.

A biopsy from an RDEB donor was processed and digested using the enhanced digestion method. Cells from the digestion were seeded into a T-75 flask using culture medium supplemented with GSH. Cells showed good growth and reached 90% confluence on day 14 for passaging. To evaluate the INXN-2002 transduction conditions, cells harvested from the T-75 flask were seeded into 3x T-175 flasks, as Control, Arm A, and Arm B. Cells in all three arms were expanded, transduced with the 10 L pilot LV-COL7 vector in 1-CS (the control arm was mock transduced), and then further expanded into 2x 10-CS before being harvested for cryopreservation.

**Table 11: Cell Growth and Cell Yields in Training Run 8**

| **Activity** | | **Days in Culture** | **Viable Cells** | **Cell Viability** | **Note** |
|---|---|---|---|---|---|
| Biopsy digestion, seed into one T-75 flask, with 35 mL culture medium, 37°C and 5% CO₂ | | 0 | 3.4 x 10⁵ | 99% | Good cell growth was observed in the T-75 flask; approximately 90% confluence on Day 14. |
| Passage 1: to three T-175 flasks with 50 mL culture medium, 37°C and 10% CO₂ | | 14 | 6.5 x 10⁶ | 97% | Flask 1: mock transduction control |
| | | | | | Flask 2: LV transduction (Arm A) |
| | | | | | Flask 3: LV transduction (Arm B) |
| Passage 2: to 1-CS and LV transduction | | 20 | 1.4 x 10⁷ | 98% | Cells were harvested from each of the T-175 flask and expanded into corresponding 1-CS for LV-COL7 transduction (CoH Pilot LV-COL7). |
| | Control | 20 | 1.0 x 10⁷ | 97% | |
| | Arm A | 20 | 1.0 x 10⁷ | 98% | |
| | Arm B | | | | |
| Passage 3: to 10-CS | | | | | Cells from each arm were harvested and expanded to one corresponding 10-CS. |
| | Control | 26 | 6.1 x 10⁷ | 99% | |
| | Arm A | 27 | 6.6 x 10⁷ | 99% | |
| | Arm B | 26 | 6.4 x 10⁷ | 99% | |
| Passage 4: to 2x 10-CS | | | | | Cells from each arm were harvested and expanded to two corresponding 10-CS. |
| | Control | 35 | 4.0 x 10⁸ | 99% | |
| | Arm A | 39 | 3.3 x 10⁸ | 98% | |
| | Arm B | 38 | 3.8 x 10⁸ | 98% | |
| Harvest: 2x10-CS | | | | | Cells were harvested from each of the 2x10-CS for cryopreservation and testing. |
| | Control | 45 | 6.9 x 10⁸ | 95% | |
| | Arm A | 47 | 4.0 x 10⁸ | 98% | |
| | Arm B | 46 | 5.3 x 10⁸ | 97% | |

The total number of cells harvested from the 10-CS was considered adequate for further production of FCX-007 drug product for injection. The harvested cells were tested for COL7A1 gene copy number (transduction efficiency), C7 protein expression, cell viability, and cell purity. Table 12 provides the analysis results for the harvested cells from all three arms.

**Table 12: Analysis of Cells Harvested from Training Run 8**

| **Condition** | **INXN-2002 Transduction MOI (IU/cell)¹** | **COL7 Algene copy/cell²** | **C7 protein expression² (ng/mL)** | **Purity² (% CD90⁺)** | **Cell Viability (%)** | **Endotoxin** |
|---|---|---|---|---|---|---|
| Arm A | 3.4 | 0.021 | 59.4 | 100% | 95% | Pass |
| Arm B | 1.7 | 0.017 | 46.6 | 100% | 93% | Pass |
| Control | Mock | BLQ³ | BLQ³ | 100% | 96% | Pass |

| | | | | | | |
|---|---|---|---|---|---|---|
| ¹ INXN-2002 IU titer: 9.2 × 10⁶ IU/mL which was determined at Intrexon using an early development stage H1299 infectious titer assay. ² Details of the assays are provided in Section 3.2. 5.4. ³ BLQ: below limit of quantitation | | | | | | |

A Multiplicity of Infection (MOI) dose dependent increase in COL7A1 gene copy number and C7 protein expression was observed. Details of the INXN-2002 transduction development and optimization are described below. Although low INXN-2002 IU titer limited the transduction MOI, and the subsequent COL7A1 gene copy number, C7 protein expression was observed to be biologically relevant in the preclinical setting as evidenced by the in vitro and in vivo results. Overall, the results show that RDEB fibroblast cells can be transduced with INXN-2002 and express functional C7 protein.

The Arm A cell product from TR8 was used for Proof of Concept studies and toxicology and biodistribution studies.

### Training Run 9

A biopsy from an RDEB donor was processed and digested using the enhanced digestion method. Cells from the digestion were seeded into a T-75 flask using culture medium supplemented with GSH. Similar to TR8, cells showed good growth and reached 90% confluence on Day 19 for passaging. To evaluate the LV-COL7 transduction conditions, cells harvested from the T-75 flask were seeded into 3x T-175 flasks, as Control, Arm A, and Arm B. Cells in all three arms were expanded, transduced with LV-HA-COL7 vector (containing an HA tag in the construct) in 1-CS (the control arm was mock transduced), then further expanded into 2x 10-CS before being harvested. The harvested cells were tested for COL7A1 gene copy number (transduction efficiency), C7 protein expression, and cell purity. Table 13 provides the cell growth in TR9.

**Table 13: Cell Growth and Cell Yields in Training Run 9**

| **Activity** | | **Days in Culture** | **Viable Cells** | **Cell Viability** | **Note** |
|---|---|---|---|---|---|
| Biopsy digestion, seed into one T-75 flask, with 35 mL culture medium, 37°C and 5% CO₂ | | 0 | NA | NA | Good cell growth was observed in the T-75 flask; approximately 90% confluence on Day 19. |
| Passage 1: to three T-175 flasks with 50 mL culture medium, 37°C and 10% CO₂ | | 19 | 4.5 x 10⁶ | 99% | Flask 1: mock transduction control |
| | | | | | Flask 2: LV transduction (Arm A) |
| | | | | | Flask 3: LV transduction (Arm B) |
| Passage 2: to 1-CS and LV transduction | | | | | Cells were harvested from each of the T-175 flask and expanded into corresponding 1-CS for LV-COL7 transduction (CoH Pilot LV-HA-COL7, a research vector) 1. |
| | Control | 26 | 1.0 x 10⁷ | 99% | |
| | Arm A | 25 | 7.1 x 10⁶ | 97% | |
| | Arm B | 25 | 7.8 x 10⁶ | 98% | |
| Passage 3: to 10-CS | | | | | Cells from each arm were harvested and expanded to one corresponding 10-CS. |
| | Control | 33 | 3.8 x 10⁷ | 99% | |
| | Arm A | 32 | 5.3 x 10⁷ | 98% | |
| | Arm B | 31 | 2.5 x 10⁷ | 97% | |
| Passage 4: to 2x 10-CS | | | | | Cells from each arm were harvested and expanded to two corresponding 10-CS.² |
| | Control | 46 | 2.6 x 10⁸ | 97% | |
| | Arm A | 42 | 3.6 x 10⁸ | 98% | |
| | Arm B | 45 | 2.8 x 10⁸ | 97% | |
| Harvest: 2x10-CS | | | | | Cells were harvested from each of the 2x10-CS for cryopreservation and testing. |
| | Control | 61 | 7.6 x 10⁸ | 100% | |
| | Arm A | 53 | 5.3 x 10⁸ | 97% | |
| | Arm B | 60 | 5.5 x 10⁸ | 98% | |

| | | | | | |
|---|---|---|---|---|---|
| ¹ LV-HA-COL7 vector is a research vector which incorporated an HA tag to the COL7A1 sequence in the INXN-2002 LV vector. ² The total number of cells harvested from the 10-CS is considered adequate for further production of FCX-007 drug product for injection. | | | | | |

Table 14 provides the analysis results for the harvested cells from all three arms.

**Table 14: Analysis of Cells Harvested from Training Run 9**

| **Condition** | **LV-HA-Col7 Transductio n MOI (IU/cell)¹** | **Col7 Gene Copy/Cell²** | **Col7 Protein Expression (ng/mL)** | **Purity² (% CD90⁺)** | **Cell Viability (%)** | **Endotoxin** |
|---|---|---|---|---|---|---|
| Arm A | 1.0 | 0.009 | 71.4 | 100% | 92% | Pass |
| Arm B | 1.9 | 0.021 | 91.6 | 99% | 95% | Pass |
| Control | Mock | BLQ³ | BLQ³ | 100% | 92% | Pass |

| | | | | | | |
|---|---|---|---|---|---|---|
| ¹ LV-HA-Col7 vector titer: 2.0 × 10⁶ IU/mL which was determined at Intrexon using an early development stage H1299 infectious titer assay. ² Details of the assays are provided in Section 3.2. 5.4. ³ BLQ: below limit of quantitation | | | | | | |

An MOI dose dependent increase in COL7A1 gene copy number and C7 protein expression was again observed. Again, the results show that RDEB fibroblast cells can be successfully grown, transduced with LV-HA-COL7 vector, and express C7 protein.

### Training Run 10 and Scale-Up to Six 10-CS

According to the proposed clinical protocol (See Module 5), 4 × 10⁸ FCX-007 drug product cells are needed for a single treatment dose, with a possibility of repeat dosing. To meet the projected FCX-007 product needs, it is necessary to scale-up the final cell expansion step to six 10-CSs based on the cell yields attained in TR8 and TR9.

A biopsy from an RDEB donor was processed and digested using the enhanced digestion method. Cells from the digestion were seeded into a T-75 flask using culture medium supplemented with GSH. Cells showed good growth and reached 90% confluence on Day 19 for passaging. Cells harvested from the T-75 flask were seeded into 2x T-175 flasks, one for INXN-2002 transduction, and one for cell substrate control. Cells from one flask were expanded, transduced with INXN-2002 in 1-CS, and then further expanded into 6x 10-CS before being harvested. Table 15 provides the cell growth in TR10.

**Table 15: Cell Growth and Cell Yields in Training Run 10**

| **Activity** | **Days in Culture** | **Viable Cells** | **Cell Viability** | **Note** |
|---|---|---|---|---|
| Biopsy digestion, seed into one T-75 flask, with 35mL culture medium, 37°C and 5% CO₂ | 0 | NA | NA | Good cell growth was observed in the T-75 flask; approximately 90% confluence on Day 19. |
| Passage 1: to two T-175 flasks with 50mL culture medium, 37°C and 10% CO₂ | 19 | 3.5 x 10⁶ | 98% | Flask 1: LV transduction Flask 2: control cells for analysis |
| Passage 2: to 1-CS and LV transduction | 30 | 4.6 x 10⁶ | 96% | Cells from Flask 1 were harvested and expanded into 1-CS for LV-COL7 transduction (GMP grade INXN-2002). |
| Passage 3: to 10-CS | 38 | 2.1 x 10⁷ | 97% | Cells were harvested and expanded to one 10-CS. |
| Passage 4: to 6x 10-CS | 60 | 1.9 x 10⁸ | 95% | Cells were harvested and expanded to six 10-CS. |
| Harvest: 6x 10-CS | 75 | 5.8 x 10⁸ | 99% | Cells were harvested from the 6x10-CS for cryopreservation and testing. |

Relative to cells in TR8 and TR9, cells in TR10 grew slower and yielded fewer cells at each passage step, indicating potential variability among different RDEB donors.

The total number of cells harvested from the 6x 10-CS is considered adequate for production of one dosage of FCX-007 drug product for injection.

Table 16 provides the analysis results for the harvested cells.

**Table 16: Analysis of Cells Harvested from TR10**

| **INXN-2002 Transduction MOI (IU/cell)¹** | **COL7 Gene Copy/Cell²** | **C7 Protein Expression² (ng/mL)** | **Purity² (% CD90⁺)** | **Cell Viability (%)** | **Endotoxin** |
|---|---|---|---|---|---|
| 2.0 | 0.013 | 60.09 | 100% | 92% | Pass |

| | | | | | |
|---|---|---|---|---|---|
| ¹ INXN-2002 IU titer: 9.2 × 10 IU/mL which was determined at Intrexon using an early development stage H1299 infectious titer assay. ² Details of the assays are provided in Section 3.2. 5.4. | | | | | |

### Example 4

### INXN-2002 Transduction Development and Optimization

Lentiviral transduction of dermal fibroblast cells was initially developed and optimized using normal human dermal fibroblast cells (NHDF; Lonza, CC-2511) cultured in 96-well plates with a model lentiviral GFP (GeneCopoeia, LP-EGFP-LV105-0205).

Using GeneCopoeia's LV transduction protocol as a starting point, initial optimization evaluated conditions including cell density at time of transduction, transduction culture volume, use of RetroNectin^{™}, super-infection (re-transducing cells with virus on two consecutive days), time of cell plating (at time of transduction versus one day prior to transduction), and serum content in transduction media. The optimal transduction procedure was then implemented in a GMP production setting. Details of the studies are described below.

### A. Effect of Cell Seeding Conditions and RetroNectin^{™} Coating for LV Transduction

NHDFs in exponential growth phase were harvested and seeded into 96-well plates on two different dates at different seeding densities. One set of non tissue culture treated 96-well plates were coated with RetroNectin^{™} per the manufacturer's recommendation (the RetroNectin^{™} coating density was 20 µg/cm²). For cells seeded one day before LV transduction (Day -1), the spent medium is removed and fresh medium (100 µL) with the LV vector is added to the wells on Day 0 for transduction. For the day of transduction condition (Day 0), cells and LV vector in 100 µL of fresh medium is added simultaneously to the wells. The MOI used was 2000 vp/cell for all conditions. After an overnight incubation, the medium was removed and the cells were fed with 100 µL fresh medium for further culture. At ninety-six hours post-transduction, the cells were harvested for transduction efficiency analysis by FACS analysis of GFP signal on a BD LSRII and analyzed using FlowJo software (v.10). Table 17 provides the LV transduction efficiencies under the different conditions.

**Table 17: Effect of Cell Seeding Condition and RetroNectin^{™} Coating on LV Transduction of Fibroblast Cells (% GFP Positive Cells)**

| **Cell Seeding Density (Cells/Well; Cells/Cm²)¹** | **Without RetroNectin^{™} Coating** | | **With RetroNectin^{™} Coating** | |
|---|---|---|---|---|
| | Day -1 Cell Seeding | Day 0 Cell Seeding | Day -1 Cell Seeding | Day 0 Cell Seeding |
| 3000; 9375 | 6.8% | 11.2% | NC | 22.6% |
| 1500; 4688 | 3.3% | 7.2% | NC | 18.2% |

| | | | | |
|---|---|---|---|---|
| Surface area for a 96-well plate well: 0.32 cm² NC = Not conducted | | | | |

The results demonstrate that it is not necessary to pre-seed the cells on the day before LV transduction. Cells and LV vector can be added simultaneously at the time of transduction, which is convenient for GMP manufacturing operations. Coating the culture surface with RetroNectin^{™} prior to LV transduction significantly increased the LV transduction efficiency. The higher cell seeding density of approximately 1 × 10⁴ cells/cm² is desired during LV transduction, as the lower cell seeding density resulted in lower LV transduction efficiency.

### B. Effect of MOI and Super-Transduction on LV Transduction Efficiency

In this study, the effects of MOI and super-transduction on LV transduction of fibroblast cells were examined.

A set of non tissue culture treated 96-well plates was pre-coated with RetroNectin^{™} (20 µg/cm²) before being used for transduction. At the time of transduction, 3000 cells with LV vector in a volume of 100 µL were added to a well. After an overnight incubation, the medium was removed and the cells were fed with 100 µL fresh medium for further culture. For the super-transduction, the culture medium was removed from the wells one day after the initial transduction (approximately 24 hours), and the same amount of LV vector in 100 µL fresh medium was added to the wells. After 3 hours of transduction, the medium was removed and the cells were fed with 100 µL fresh medium for further culture. Ninety-six hours post-transduction, the cells were harvested for transduction efficiency analysis by FACS analysis of GFP signal on a BD LSRII and analyzed using FlowJo software (v.10). Table 18 provides the LV transduction efficiencies under the different conditions.

**Table 18: Effect of MOI and Super-Transduction on LV Transduction of Fibroblast Cells (% GFP Positive Cells)**

| **MOI (vp/cell)** | **Without Super-Transduction** | **With Super-Transduction** |
|---|---|---|
| 0 | 1.6% | 2.0% |
| 200 | 4.3% | 5.8% |
| 500 | 7.7% | 10.0% |
| 1000 | 15.3% | 16.3% |
| 2000 | 22.6% | 27.5% |

An increasingly higher MOI resulted in higher transduction efficiencies with and without super-transduction. Super-transduction resulted in an incremental increase in LV transduction efficiency. However, the increase was not considered significant, and was not implemented in the GMP production setting.

### C. LV Transduction Culture Volume and FBS Concentration

Lentiviral vector particles first need to make contact with fibroblast cells in culture in order to achieve transduction. Like other viral particles, LV particles in solution follow Brownian motion, and a productive transduction is generally a random event. Reduction of culture medium depth to a minimum volume or the use of spino-transduction has been shown to improve viral vector transduction on target cells (Nyberg-Hoffman 1997). The effect of transduction volume/culture depth and medium FBS concentration on LV transduction of fibroblast cells were evaluated.

A set of non tissue culture treated 96-well plates was pre-coated with RetroNectin^{™} (20µg/cm²) before being used for transduction. At the time of transduction, 3000 cells with LV vector in a volume of 100 µL or 50 µL of medium with 10% FBS were added to the wells. The same transduction conditions were repeated in medium with 2% FBS. After an overnight incubation, the medium was removed and the cells were all fed with 100 µL fresh medium with 10% FBS for further culture. The MOI used was 2000 vp/cell for all conditions. Ninety-six hours post-transduction, the cells were harvested for transduction efficiency analysis by FACS analysis of GFP signal on a BD LSRII and analyzed using FlowJo software. Table 19 provides the LV transduction efficiencies under the different conditions.

**Table 19: Effect of Culture Volume (Depth) and FBS on LV Transduction (% GFP Positive Cells)**

| **Transduction Medium Volume in 96-well plate (µL)** | **Transduction Medium Depth (mm)** | **Transduction Medium FBS %** | |
|---|---|---|---|
| | | **2%** | **10%** |
| 50 | 1.6 | 46.7% | 46.2% |
| 100 | 3.1 | 31.8% | 27% |

As expected, reducing the transduction medium volume (depth) resulted in a noticeable increase in LV transduction efficiency. Additionally, results indicate that using a 2% FBS transduction medium is beneficial for LV transduction. Based on these results, a 2% FBS transduction medium was incorporated into the GMP manufacturing process, while keeping the transduction medium volume to a minimum, approximately 60 mL in 1-CS. In the GMP manufacturing process for FCX-007, a 1-layer CellSTACK^{®} with a surface area of 636 cm² will be used for LV transduction of fibroblast cells. It was determined that it is feasible to use a transduction volume of 60 mL (depth 0.9mm) without causing detrimental effect (e.g. drying) on the cells during the 3 hours transduction period.

### D. Evaluations of RetroNectin^{™} Coating and the Type of Culture Surface

RetroNectin^{™} coating of the culture surface significantly increased the LV transduction of fibroblast cells. The RetroNectin^{™} manufacturer recommends a RetroNectin^{™} coating amount in the range of 4 µg/cm² to 20 µg/cm². To minimize the amount of RetroNectin^{™} used without negatively impacting the LV transduction of fibroblast cells, the amount of RetroNectin^{™} used for coating was evaluated.

Furthermore, the RetroNectin^{™} manufacturer recommends use of a non tissue culture treated plastic surface for RetroNectin^{™} coating. However, culture flasks and CellSTACKs^{®} used for fibroblast cell culture are all tissue culture-treated. Both tissue culture treated and non tissue culture treated 96-well plates were coated with different amounts of RetroNectin^{™} and evaluated for LV transduction of fibroblast cells.

Both non tissue culture treated and tissue culture treated 96-well plates were pre-coated with different amounts of RetroNectin^{™} (20 µg/cm² 10 µg/cm² and 5 µg/cm² ) before being used for transduction. At the time of transduction, 3000 cells with LV vector in a volume of 50 µL of transduction medium with 2% FBS were added to each well. After 3 hours transduction, the medium was removed and the cells were all fed with 100 µL fresh medium with 10% FBS for further culture. The MOI used was 2000 vp/cell for all conditions. Ninety-six hours post-transduction, the cells were harvested for transduction efficiency analysis by FACS analysis of GFP signal on a BD LSRII and analyzed using FlowJo software. Table 20 provides the LV transduction efficiencies under the different conditions.

**Table 20: Effect of RetroNectin^{™} Amount and Culture Surface Type on LV Transduction of Fibroblast Cells (% GFP Positive Cells)**

| **Type of Culture Surface** | **Amount of RetroNectin^{™} Used for Coating (µg/cm²)** | | |
|---|---|---|---|
| | **5** | **10** | **20** |
| Tissue culture treated | 27.8% | 25.8% | 28.1% |
| Non tissue culture treated | 38.2% | 38.8% | 38.5% |

Comparable efficiencies of LV transduction of fibroblast were observed for all three doses of RetroNectin^{™} used for coating. As a result, a RetroNectin^{™} coating density of 5 µg/cm² was selected for use in the FCX-007 production process. Although lower LV transduction efficiency was observed in the tissue culture treated surface, the difference is not considered significant enough to prevent the use of tissue culture treated flasks in the INXN-2002 transduction step of the FCX-007 manufacturing process.

### Summary of the Development and Optimization of Fibroblast LV Transduction

Based on the study results described above using the LV-GFP model vector for fibroblast cell transduction, the following LV transduction protocol was selected for INXN-2002 transduction of fibroblast cells derived from RDEB donor:

Pre-coat the culture surface with RetroNectin^{™} at 5 µg/cm². Tissue culture treated flasks can be used for RetroNectin^{™} coating.

Add cells and LV vector simultaneously at the time of transduction, with a cell seeding density of approximately 1×10⁴ cells/cm².

Use a high MOI which does not cause toxic effect on cells to achieve high transduction efficiency (10 mL of INXN-2002 LV-Col7 vector per transduction).

Transduce for 3 hours at 37°C in a transduction medium with 2% FBS, and then change or feed cells with a medium which contains 10% FBS.

Use a low transduction medium volume, 50 µL/well for a 96-well plate or 60 mL for a 1-layer CellSTACK^{®}, for high transduction efficiency.

### Super-transduction is not required.

### F. INXN-2002 LV Transduction of Fibroblast Cells

Based on the LV transduction protocol developed above, INXN-2002 was used to transduce normal human fibroblast cells (NHDF, Lonza, CC-2511) in a 96-well plate. Considering the relative low titer of the INXN-2002, three doses of INXN-2002 were used for transduction, 12.5 µL (1:4 dilution), 3.1 µL (1:16 dilution), and 0.8 µL (1:64 dilution). The transduced cells were passaged three times to ensure stable integration of the LV-COL7 vector into the transduced cell genome. At passage 3, genomic DNA was extracted from the cells and analyzed by qPCR using a primer specific to the LV-COL7 vector sequence. The transduction efficiency was quantified as gene copy numbers per cell. Table 21 provides the transduction efficiency as measured by gene copy number per cell.

**Table 21: INXN-2002 Transduction of Fibroblast Cells**

| **INXN-2002 Vector Dosing¹** | | | **Gene Copy Number per Cell²** |
|---|---|---|---|
| **Dilution factor** | **Vector volume per well of 96-well plate (µL)** | **Calculated MOI (IU/cell)** | |
| 1/4 | 12.5 | 40 | 0.08 |
| 1/16 | 3.1 | 10 | 0.86 |
| 1/64 | 0.8 | 2.5 | 0.11 |

| | | | |
|---|---|---|---|
| ¹ INXN-2002 IU titer: 9.2 × 10⁶ IU/mL which was determined at Intrexon using an early development stage H1299 infectious titer assay. ² Gene copy number per cell was determined at Intrexon using an early development stage qPCR assay, which resulted in an approximately 10-fold higher gene copy number compared to the fully developed assay that was transferred to BioReliance for FCX-007 product analysis. The difference was due to the use of a different qPCR primer set and circular plasmid standards in the early development stage assay. | | | |

At the highest dose of INXN-2002 vector, ¼ dilution (MOI= 40), a significant toxic effect on cells were observed: most of the cells did not recover from the transduction step, resulting in a minimal gene copy number per cell at the end of the cell expansion. The optimal INXN-2002 MOI was approximately 10 IU/cell, resulting in a gene copy number of 0.86.

### INXN-2002 LV Transduction in Training Runs

Following the LV transduction protocol (Section [0260] ) and the INXN-2002 transduction vector dosing/MOI results from the 96-well studies, INXN-2002 transduction of RDEB fibroblast cells was conducted at scale in TR8, TR9, and TR10. The 1-layer CellSTACK^{®} used for INXN-2002 transduction was pre-coated with RetroNectin^{™} at 5 µg/cm². At the time of transduction, cells and INXN-2002 were added simultaneously in 60 mL of transduction medium with 2% FBS to the 1-layer CellSTACK^{®}. After 3 hours in the incubator at 37°C, the cells were fed with 130 mL of complete growth medium with 10% FBS. The transduced cells were passaged twice, first to one 10-layer CellSTACK^{®}, and then to two or six 10-layer CellSTACKs^{®} for cell harvest. Gene copy numbers in the harvested cells were analyzed by qPCR. Table 22 provides the INXN-2002 transduction results from the executed Training Runs.

**Table 22: Summary of LV-COL7 Transduction in Training Runs**

| **Training Run** | **LV-COL7 Vector** | **LV-COL7 Vector Titer (IU/mL)** | **Arm** | **Amount of Vector Used for Transduction (mL) (dilution factor)** | **Number of Cells for Transduction** | **MOI¹ (IU/cell)** | **Gene Copy per Cell²** |
|---|---|---|---|---|---|---|---|
| 8 | INXN-2002 LV-COL7 (Pilot) | 9.2x10⁶ | A | 3.75 (1/16) | 1.0 x 10⁷ | 3.4 | 0.021 |
| | | | B | 1.88 (1/32) | 1.0 x 10⁷ | 1.7 | 0.017 |
| | | | Control | 0 | 1.4 x 10⁷ | 0 | BLQ₁ |
| 9 | LV-HA-COL7 (Pilot) | 2.0x10⁶ | A | 3.75 (1/16) | 7.1 x 10⁶ | 1.0 | 0.009 |
| | | | B | 7.5 (1/8) | 7.4 x 10⁶ | 1.9 | 0.021 |
| | | | Control | 0 | 1.0 x 10⁷ | 0 | BLQ³ |
| 10 | INXN-2002 LV-COL7 (GMP) | 2.3x10⁶ | TR10 | 4 (1/15) | 4.6 x 10⁶ | 2.0 | 0.013 |

| | | | | | | | |
|---|---|---|---|---|---|---|---|
| ¹ MOI was determined based on the INXN-2002 LV-Col7 vector IU titer determined at Intrexon using an early development stage H1299 infectious titer assay, which resulted in approximately 10-fold higher titer value compared to the fully developed assay that was transferred to BioReliance for FCX-007 product analysis. The difference was due to the use of a different qPCR primer set and circular plasmid standards in the early development stage assay. ² Gene copy per cell was quantified using the fully developed qPCR assay, which was transferred to BioReliance for FCX-007 product analysis ³ BLQ: Below the limit of quantitation | | | | | | | |

Following the LV transduction protocol developed using the small scale 96-well plates, successful INXN-2002 transduction of RDEB fibroblast cells was achieved at scale for FCX-007 manufacture. Gene copy numbers in the transduced cells increased with an increased MOI of transduction. Because of the low titer of INXN-2002, gene copy numbers in the transduced, harvested cell product were relatively low. In order to produce FCX-007 cell product with a feasibly high gene copy number, 10 mL of INXN-2002 was selected for GMP production transduction, as it caused minimal toxic effects on cells during transduction.

### Engineering Run

In preparation for GMP manufacturing of FCX-007, and finalization of the production Master Batch Records (MBR), an engineering run was executed using a biopsy from an RDEB donor with approved production batch records. The GMP grade INXN-2002 LV vector was used for transduction. Table 23 provides cell growth figures from the Engineering Run.

**Table 23: Cell Growth and Cell Yields in Engineering Run**

| **Activity** | **Days in Culture** | **Viable Cells** | **Cell Viability** | **Note** |
|---|---|---|---|---|
| Biopsy digestion, seed into one T-75 flask, with 35mL culture medium, 37°C and 5% CO₂ | 0 | 9.6 x 10⁵ | 92% | Good cell growth in the T-75 flask, reached approximately 90% confluence on Day 19 |
| Passage 1: to one T-175 flask and one T-25 flask, 37°C and 10% CO₂ | 20 | 3.5 x 10⁶ | 99% | T-175 Flask: LV transduction |
| | | | | T-25 Flask: control cells for analysis |
| Passage 2: to 1-CS and LV transduction | 29 | 9.9 x 10⁶ | 98% | Cells from T-175 Flask were harvested and expanded into 1-CS for LV-COL7 transduction (GMP grade INXN-2002) |
| Passage 3: to 10-CS | 38 | 3.3 x 10⁷ | 98% | Cells were harvested and expanded to one 10-CS. |
| Passage 4: to 6x 10-CS | 49 | 3.1 x 10⁸ | 96% | Cells were harvested and expanded to six 10-CS. |
| Harvest: 6x 10-CS | 60 | 9.6 x 10⁸ | 97% | Cells were harvested from the 6x10-CS for cryopreservation and testing |

Cell growth and yields from the ER are comparable to those in TR8 and TR9, and are noticeably faster and higher relative to TR10, indicating cell growth variability among different RDEB donors. The harvested cells were filled and cryopreserved as shown in Table 24.

**Table 24: FCX-007 Drug Substance Vials Filled in Engineering Run**

| **Vials** | **Fill Volume (mL)** | **Number of Vials Filled** | **Purpose** |
|---|---|---|---|
| 2 mL cryovial | 1.2 | 10 | Bulk Drug Substance |
| 5 mL cryovial | 4.5 | 6 | Bulk Drug Substance |
| 2 mL cryovial | 1.2 | 2 | Sterility test |
| 2 mL cryovial | 0.6 | 4 | QC test |
| 2 mL cryovial | 0.6 | 6 | Bulk Drug Substance Stability Test |

The total number of cells harvested from the 6x 10-CS is considered adequate for production of two doses of FCX-007 drug product for injection.

The harvested cells were tested according to the proposed FCX-007 Drug Substance Specifications. Table 25 provides the analysis results for the harvested cells.

**Table 25: FCX-007 Drug Substance Engineering Run Analysis**

| **Test¹** | **Test Method** | **Lab** | **SOP** | **Specifications** | **Test Result** |
|---|---|---|---|---|---|
| Mycoplasma | USP<63> | BioReliance | 102063GMP.BSV | Negative | Negative |
| Replication Competent Lentivirus | C8166 cell | BioReliance | 009130GMP.BUK | No RCL detected | ND⁴ |
| Sterility | USP<71> | BioReliance | 510120GMP.BSV | No Growth | No growth |
| Endotoxin | Endosafe | PCT | SOP-0249 | ≤ 5.0 EU/mL | < 2.00 EU/mL |
| Cell Count | Hemacytometer | PCT | SOP-0329 | 1.0 - 3.0x10⁷ cells/mL | 1.6x10⁷ cells/mL |
| Cell Viability | Hemacytometer | PCT | SOP-0329 | ≥ 85% viability | 94% |
| Purity | FACS Calibur | PCT | SOP-1067 | ≥ 98% CD 90⁺ | 100% |
| Residual VSV-G Protein | VSV-G ELISA | Intrexon² | VSV-G ELISA | Report Result | BLQ |
| COL7A Gene Copy Number | qPCR | Intrexon² | COL7A Gene Copy Number | Report Result | 0.025 copies/cell |
| COL7A Gene Expression | Col7 ELISA | Intrexon³ | COL7A Gene Expression | Report Results | 66.4 ng/mL |

| | | | | | |
|---|---|---|---|---|---|
| ¹ Details of the assays are provided in Section 3.2. S.4.2. ² Intrexon in house assay. Assays are being transferred to BioReliance for GMP release test ³ Intrexon in house assay. Assays are being transferred to PCT for GMP release test ⁴ RCL testing was not performed on the cell product material as the material was not used for further studies | | | | | |

### Example 5 - Description of Manufacturing Process Development to Improve LV-Col7 Transduction Efficiency

FCX-007 cell products manufactured using the process described above had low levels of gene modification efficiency as indicated by the low gene copy number and C7 expression levels when INXN-2002 vector was used for cell transduction. Two approaches were pursued to increase the LV-Col7 transduction efficiency on RDEB fibroblast cells: 1) development of a new LV-Col7 lentiviral vector (INXN-2004) which offers better vector titer and improved stable transgene (collagen VII) expression in the transduced fibroblast cells, and 2) further optimization of the LV-Col7 transduction process on fibroblast cells. Details of the development of the new INXN-2004 vector are described in Section 3.2.S.2.3. Studies in further optimization of the LV-Col7 transduction process, as well as cell expansion procedures are described below.

### Spin Transduction (Spinoculation)

Spin transduction involves centrifugation of viral vectors onto the target cells under a g force environment. Spin transduction (spinoculation) has been reported in the literature to increase retroviral vector transduction efficiency on target cells (J Virol Methods. 1995 Aug;54(2-3):131-43. Centrifugal enhancement of retroviral mediated gene transfer. Bahnson AB1, Dunigan JT, Baysal BE, Mohney T, Atchison RW, Nimgaonkar MT, Ball ED, Barranger JA. and Hum Gene Ther. 1994 Jan;5(1):19-28. Improved methods of retroviral vector transduction and production for gene therapy. Kotani H1, Newton PB 3rd, Zhang S, Chiang YL, Otto E, Weaver L, Blaese RM, Anderson WF, McGarrity GJ.).

RDEB fibroblast cells from the previous TR8 were cultured. Cells in exponential growth phase were harvested and used for this study. The previous INXN-2002 vector lots were used for transduction. For spin transduction, 1×10³ cells with different MOIs of INXN-2002 in 50µL of transduction media (IMDM + 2%FBS) were added into each well of a 96-well tissue culture coated plates which were pre-coated with RetroNectin (5 µg/cm²). The plates containing cells plus INXN-2002 vector were centrifuged at 1300g, 4°C for 1.5 hours. After centrifugation, the plates were placed into a 37°C incubator to allow the cells to recover and grow for 3 hours. After which, 100 µl of complete culture media (IMDM +10FBS+GSH) was added to each well. The plates were returned to the incubator for further culture. The standard non-spin transduction described above was used as a control. After transduction, cells were passaged three times, 3-5 days culture between each cell passage. At the last passage culture supernatants were harvested to evaluate C7 protein expression levels, while the cells were harvested for gene copy number per cell analysis. Table 26 shows the Col7 expression levels for the different transduction conditions.

**Table 26: C7 Expression Level**

| **INXN-2002** | **MOI (IU/cell)** | **Vector Dilution used for Transduction** | **Transduction Method** | |
|---|---|---|---|---|
| | | | **Spin Transduction** | **Non-spin transduction** |
| GMP lot | 6.31 | | 257.4±62.17 | 311.75±70.89 |
| | 1.60 | | 539.50±70.49 | 251.27±22.41 |
| | 0.4 | | 243.94±3.40 | 207.51±25.26 |
| Pilot lot | 30 | | 327.76±21.69 | 238.56±27.93 |
| | 15 | | 391.59±42.82 | 208.39±16.80 |
| | 7.5 | | 269.07±25.51 | 214.23±16.83 |

Table 27 provides the vector copy number per cells of the harvested cells.

**Table 27: Vector Copy Number per Cell**

| **INXN-2002** | **MOI (IU/cell)** | **Vector dilution used for transduction** | **Transduction Method** | |
|---|---|---|---|---|
| | | | **Spin Transduction** | **Non-spin transduction** |
| GMP lot | 6.31 | | 0.11±0.02 | 0.10±0.02 |
| | 1.60 | | 0.11±0.01 | 0.06±0.00 |
| | 0.4 | | 0.10±0.03 | 0.04±0.01 |
| Pilot lot | 30 | | 0.09±0.01 | 0.06±0.02 |
| | 15 | | 0.09±0.02 | 0.05±0.01 |
| | 7.5 | | 0.05±0.03 | 0.07±0.02 |

| | | | | |
|---|---|---|---|---|
| INXN-2002, IU titer: 2.36 × 10 lU/mL (determined at Intrexon) INXN-2002, IU titer: 2.36 × 10⁶ lU/mL (determined at Intrexon) | | | | |

The results show a modest increase in both Col7 production levels and vector copy number per cell using the spin transduction method, especially at the lower MOIs and at the INXN-2002 vector dilutions tested.

### Optimization of Spin Transduction Parameters

The spin transduction parameters, including centrifugation time, speed and temperature, were evaluated with an aim to further increase the INXN-2002 transduction efficiency. The same cell culture condition as described above was used in this study. In the initial experiment, centrifugation temperature was examined. 96-well plates containing cells plus INXN-2002 vector were centrifuged at either 4°C or 25°C (RT) for 1.5 hours at 1300g. This was followed by another experiment where 96-well plates containing cells plus INXN-2002 vector were centrifuged at 4°C or 25°C (RT) for 1 or 2 hours, at either a high speed of 1300g, or a low speed of 300g, to examine the effect of centrifugation speed on INXN-2002 transduction efficiency. In both experiments, cells were returned to a 37°C incubator for incubation post centrifugation. Three hours later, 100 µl of complete culture media was added to each well. Cells were passaged three times before being harvested for Col7 expression analysis in the culture supernatant and vector copy number per cell analysis in the harvested cells.

Table 28 provides the effect of spin transduction temperature on Col7 expression levels and vector copy number per cell. The standard non-spin transduction was included as a control.

Again, increased INXN-2002 transduction, as indicated by higher C7 protein expression and vector copy number per cell, was demonstrated using spin transduction compared to the standard no spin transduction. Spin transduction temperature did not demonstrate impact on the overall transduction efficiency.

Table 29 below provides the effect of centrifugation speed as well as temperature and time on INXN-2002 transduction efficiency.

**Table 28: The Effect of Spin Transduction Temperature on INXN-2002 Transduction Efficiency**

| **INXN-2002 MOI** | **C7 Expression (ng C7/day/E6 cells)** | | | **Vector Copy Number per Cell** | | |
|---|---|---|---|---|---|---|
| | **No spin** | **Spin Transduction** | | **No Spin** | **Spin Transduction** | |
| | | **4°C** | **RT** | | **4°C** | **RT** |
| 18 | 105.84±11.69 | 177.36±24.98 | 313.59±39.13 | 0.07±0.04 | 0.07±0.02 | 0.08±0.03 |
| 6 | 117.82±14.79 | 196.26±23.75 | 143.61±19.01 | 0.02±0.01 | 0.08±0.03 | 0.05±0.00 |
| 2 | 50.05±13.60 | 136.55±6.82 | 105.57±17.90 | 0.01±0.00 | 0.02±0.00 | 0.03±0.02 |

| | | | | | | |
|---|---|---|---|---|---|---|
| INXN-2002 IU titer: 2.36 × 10⁶ ICT/mL (determined at Intrexon) | | | | | | |

**Table 29: Effect of Spin Transduction Temperature, Time, and Speed on INXN-2002 Transduction Efficiency**

| **INXN-2002 MOI** | **C7 Expression (ng C7/day/E6 cells)** | | | | | | | | **Vector Copy Number per Cell** | | | | | | | |
|---|---|---|---|---|---|---|---|---|---|---|---|---|---|---|---|---|
| Temp | 4°C | | | | RT | | | | 4°C | | | | RT | | | |
| Speed (xg) | 1300 | | 300 | | 1300 | | 300 | | 1300 | | 300 | | 1300 | | 300 | |
| Time ( hr) | 1 | 2 | 1 | 2 | 1 | 2 | 1 | 2 | 1 | 2 | 1 | 2 | 1 | 2 | 1 | 2 |
| 3.8 | 208.88 ±13.61 | 247.0 3±10. 09 | 221. 49±4 08 | 310. 93±0 .41 | 359.4 7±3.8 5 | 201.0 6±5.3 7 | 168. 98±3 34 | 201. 82±9 .41 | 0.08 ±0. 01 | 0.07 ±0. 01 | 0.06 ±0. 01 | 0.09 ±0. 03 | 0.11 ±0. 01 | 0.06 ±0. 01 | 0.07 ±0. 01 | 0.07 ±0. 02 |
| 1.9 | 180.34 ±12.33 | 265.4 4±1.3 3 | 213. 83±0 58 | 301. 82±1 1.54 | 240.6 1±2.6 8 | 180.3 6±3.1 0 | 212. 76±3 19 | 177. 16±5 92 | 0.06 ±0. 02 | 0.10 ±0. 02 | 0.05 ±0. 00 | 0.06 ±0. 01 | 0.10 ±0. 02 | 0.07 ±0. 02 | 0.06 ±0. 01 | 0.05 ±0. 01 |

| | | | | | | | | | | | | | | | | |
|---|---|---|---|---|---|---|---|---|---|---|---|---|---|---|---|---|
| INXN-2002 IU titer: 2.36 × 10⁶ ICT/mL (determined at Intrexon) | | | | | | | | | | | | | | | | |

The results indicate a modest improvement in INXN-2002 transduction efficiency, as indicated by C7 protein expression and vector copy number, when spin transduction was conduced at RT, 1300g, for 1 hour. The significance of the improvement is not known and the initially developed spin transduction parameters (centrifugation at 1300g for 1.5 hours at 4°C) were selected for future study use.

### Super Transduction to Further Increase INXN-2002 Transduction Efficiency

Super transduction (a second transduction) was evaluated in the early development of INXN-2002 transduction, which is described above. Super transduction was evaluated again in combination with spin transduction to further increase INXN-2002 transduction efficiency on fibroblast cells. Unlike the previous method, in the current study's super transduction was performed on cells one passage after the initial spin transduction. The inclusion of the one cell passage is expected to allow the cells to recover from the initial spin transduction and become more receptive to the 2^{nd} super transduction.

Similarly, RDEB fibroblast cells from the previous TR8 in exponential growth phase were harvested and used for this study. The optimized spin transduction method described above, centrifugation at 1300g for 1.5 hours at 4°C, was used for INXN-2002 transduction. The transduced cells were passaged once at 72-96 hours post the initial spin transduction. After 72-96 hours of culture in the second passage, cells were harvested and re-transduced using the same spin transduction conditions. After the super spin transduction, cells were passaged an additional three times before being harvested for Col7 expression analysis in the culture supernatant, and vector copy number per cell analysis in the harvested cells. The INXN-2002 vector was used for the study. Table 30 provides the effect of super spin transduction on INXN-2002 transduction efficiency.

**Table 30: Effect of Super Spin Transduction**

| **INXN-2002 MOI** | **C7 Protein Expression** | | **Vector Copy Number per Cell** | |
|---|---|---|---|---|
| | **One Round of Spin Transduction** | **Super Spin Transduction** | **One Round of Spin Transduction** | **Super Spin Transduction** |
| 1.9 | 257.8±3.37 | 431.9±95.5 | 0.07±0.03 | 0.22±0.07 |

| | | | | |
|---|---|---|---|---|
| INXN-2002 IU titer: 2.36 × 10⁶ IU/mL (determined at Intrexon) | | | | |

Super spin transduction resulted in a significant increase in INXN-2002 transduction efficiency on RDEB fibroblast cells. C7 protein expression increased approximately 1.7-fold and the vector copy number per cell increased approximately 3-fold.

The data demonstrate a 40% increase in Col7 expression and a 60% increase in copy number per cell with the additional transduction. Due to this increase, super-infection was added to the production protocol.

### Transduction with the INXN-2004 Vector

Concurrent with the development of the improved transduction method, a new LV-Col7 vector (referenced as "INXN-2004") based on a different lentiviral vector backbone (that is, pFUGW) was also developed.

### INXN-2004 Single Spin Transduction

RDEB fibroblast cells from the previous TR8 in exponential growth phase were harvested and used for this study. In the first study, single round of spin transduction as described above was carried out using a pilot lot of INXN-2004 vector. The standard no-spin transduction was included in the study as a control. Cells were passaged three times before being harvested for Col7 expression analysis in the culture supernatant, and vector copy number per cell analysis in the harvested cells. Table 31 provides the Col7 expression levels and vector copy number per cell using the INXN-2004 vector transduction.

**Table 31: C7 Protein Expression and Vector Copy Number per Cell using INXN-2004 Transduction**

| **INXN-2004 MOI** | **C7 Protein Expression** | | **Vector Copy Number per Cell** | |
|---|---|---|---|---|
| | **No Spin Transduction** | **Spin Transduction** | **No Spin Transduction** | **Spin Transduction** |
| 17 | 2312.46±0.03 | 3960.58±0.02 | 0.16±0.03 | 0.05±0.02 |
| 4.25 | 1668.46±0.06 | 3602.10±0.06 | 0.21±0.06 | 0.27±0.06 |
| 1.06 | 724.12±0.03 | 2566.88±0.10 | 0.06±0.03 | 0.21±0.10 |
| 0.27 | 168.61±0.00 | 1936.72±0.00 | 0.02±0.00 | 0.13±0.00 |

| | | | | |
|---|---|---|---|---|
| INXN-2004 IU titer: 2.36 × 10⁶ ICT/mL (determined at Intrexon) | | | | |

Relative to the INXN-2002 vector, a significant improvement in transduction efficiency was achieved with use of INXN-2004 in combination with the spin transduction method. Additionally, the INXN-2004 vector appears to be significantly more active in transducing RDEB fibroblast cells even under a no-spin transduction condition, as indicated by the approximately 10-fold higher C7 protein expression and vector copy number per cell. The results support the use of the pFUGW base for construction of the new INXN-2004 vector.

Consistent to what is reported above, spin transduction appears to exacerbate the INXN-2004 vector toxic effect on fibroblast cells at higher MOI conditions, as indicated by the lower C7 expression levels and vector copy numbers at MOI17. This does not impact FCX-007 manufacturing as the use of a lower MOI is demonstrated to be as effective.

### INXN-2004 Super Spin Transduction

In the second study, super spin transduction (as described above) was used for INXN-2004 transduction. The scale of transduction was increased from a 96-well plate to a T-25 flask. The transduction parameters remained unchanged, including the same RetroNectin^{™} coating density of 5µg/cm2, and the same cell density at transduction of 1×10⁴ cells/cm². Transduction volume in the T-25 flask was 4.2 mL. For transduction, the T-25 flask containing the cells and virus was centrifuged at 4°C, for 1.5 hours at 1300g. Cells were returned to a 37°C incubator and incubated for three hours. After the initial incubation, 4.2 mL of complete culture media was added to the flask for further incubation. Cells were passaged three times before being harvested for Col7 expression analysis in the culture supernatant, and vector copy number per cell analysis in the harvested cells. Table 32 provides the effect of super spin transduction on INXN-2004 vector transduction efficiency. A pilot lot of INXN-2004 vector was used for the study.

**Table 32: Effect of Super Spin Transduction for the INXN-2004 Vector**

| **INXN-2004 MOI** | **C7 Protein Expression** | | **Vector Copy Number per Cell** | |
|---|---|---|---|---|
| | **Single Spin Transduction** | **Super Spin Transduction** | **Single Spin Transduction** | **Super Spin Transduction** |
| 0.11 | 3178±20.53 | 4029±59.67 | 0.12±0.007 | 0.57±0.134 |

| | | | | |
|---|---|---|---|---|
| INXN-2004 IU titer: 2.36 × 10⁶ ICT/mL (determined at Intrexon) | | | | |

As observed for the previous INXN-2002 vector, super spin transduction resulted in a significant increase, approximately 5-fold, in vector copy number per cell in the transduction cells, despite a relatively modest increase in Col7 protein expression level, under a low MOI transduction condition.

### Summary of Transduction Development and Optimization

Significant improvements in LV-Col7 transduction efficiency on RDEB fibroblast cells was achieved in continued process development, both with the previous INXN-2002 and the new INXN-2004 vector. The improvements are to enable the manufacturing of a biologically potent FCX-007 cell product. Based on the manufacturing process development study results, the following transduction procedure was chosen for manufacturing of FCX-007 using INXN-2004 vector for transduction.

Pre-coat culture surface with RetroNectin at 5µg/cm². Tissue culture-treated flasks are used for RetroNectin^{™} coating.

Add cells and INXN-2004 vector simultaneously at the time of transduction. Cell seeding density is approximately 1 × 10⁴ cells/cm².

Centrifuge cells and INXN-2004 vector transduction mixture in the transduction vessel at 4°C, for 1.5 hours at 1300g.

Incubate cells for 1.5- 2.0 hours at 37°C post transduction in transduction medium with 2%FBS, and then change or feed cells with complete culture medium with 10%FBS.

Use a low transduction medium volume (50µL/well for a 96-well plate, or 0.35 mL/cm²).

Perform a super-transduction on cells after one passage post the initial transduction.

### Example 6 - Analysis of RDEB Genetically Modified Human Dermal Fibroblasts Transduced by INXN-2002

RDEB Genetically Modified Human Dermal Fibroblasts (GM-HDF) derived from INXN-2002 lentiviral vector was analyzed in order to:
Evaluate localization of COL7 expression in established composite human skin grafts (prepared on porcine dermis) comprised of RDEB keratinocytes by ID injection.
Assess potential carcinogenicity/tumorgenicity in composite RDEB human skin grafted (prepared on porcine dermis) on SCID mice treated with RDEB GM-HDF

An initial attempt to evaluate GM-HDF (derived from INXN-2002) function by seeding fibroblasts on the reticular side of composite graft cultures was unsuccessful, possibly due to inadequate migration of GM-HDF into the devitalized porcine dermis or insufficient time for adequate diffusion and deposition of rC7 at Day 19. An alternative approach was used to evaluate function by direct ID injection of existing grafts in six back-up animals on study. RDEB composite grafts prepared on porcine dermis appear to be more robust than anticipated, making direct injection into established RDEB skin grafts possible. This approach more closely mimics the disease and clinical route of administration.

This study report presents data from an initial evaluation of ID injection of GM-HDF into established RDEB grafts. Additional studies to achieve long term objectives for in vivo pharmacology are planned.

### A. STUDY MATERIALS

### A.1 Test Article(s)

Test article was prepared and tested for Drug Substance release specifications prior to shipment. The test article for skin graft injection was prepared one day prior to each scheduled day of treatment. GM-HDF was thawed, washed, resuspended in DMEM, and evaluated against release specifications. The GM-HDF was shipped for overnight delivery and administered within 48 hours. Non-corrected RDEB HDF (mock transduced) was isolated and expanded from the identical patient as the GM-HDF by the same methods.

The test article and cells to be used for preparation of composite skin grafts was shipped frozen. Cells used for skin graft preparation will be thawed and cultured for testing.

Lot number TR8 (Non-Transduced Control) was used as the RDEB-HDF negative control. Lot number TR8 Arm A (GM-HDF-LV-COL7) was used as the GM-HDF test article.

Test article analysis was performed at the manufacturing facility prior to shipment. Remaining test article will be stored at -70°C for 1 year. The test article will be stored in Dulbecco's Modified Eagle Medium (DMEM).

### A.2. Other Chemicals and Materials

A list of other materials is provided below in Table 33.

**Table 33: Other Study Materials**

| **Description** | **Source** |
|---|---|
| Normal Porcine Skin | Porcine cadaver |
| RDEB keratinocytes | Human skin biopsy |
| RDEB fibroblasts | Human skin biopsy |
| Normal keratinocytes | Human skin biopsy |
| Normal fibroblasts | Human skin biopsy |

Group 1 (Negative Control) Cells: Untransduced RDEB cells from the donor from training run 8 were thawed, and passaged once prior to transfection. Two days after passaging the cells, the cells were collected and 1.2 × 10⁶ cells were transfected in a 50ml conical tube in low serum growth (ATCC PCS-201-030 and PCS-201-041) media using the lipid based transfection agent Transfex (ATCC) with COL7 plasmid VVN 4317513. After a 10 minutes incubation with complexed Transfex, DNA and Optimem (LifeTech), 1 × 10⁶ transfected cells were transferred to a T150 flask, and 2 × 10⁵ cells were placed in a T25 and placed in a 37°C 5% CO2 incubator for 16 hours, at which time the media was removed and replace with full growth media (IMDM (Sigma), 920mg/L of L-Glutathione Reduced (Sigma) 10% heat inactivated FBS (Atlantic Biologicals) and 1XGlutamax (LifeTech)). Cells were then returned to the 37°C 5%CO2 incubator until time of shipment. At time of shipment all cell flask was filled to capacity, such that all parts of the flask were in contact with media regardless of the flask orientation.

### B. STUDY DESIGN

### B.1. Randomization

Due to high failure rates of RDEB human skin grafts, mice in these groups of animals are not randomized.

### B.2. Justification for Species and Number on Study

The NOD. CB.17-PRKDC scid/scid mouse is the standard species for use in proof of concept preclinical efficacy/toxicology studies and is a rodent species of choice for such evaluations using the human xenograft models. This study was designed to minimize the number of animals on study that would provide sufficient data to evaluate the test articles in.

### B.3. Route of Administration

Fibroblasts will be administered by intradermal injection for normal skin grafts. Composite grafts will be administered cells by seeding prior to grafting.

### B.4. Dose Administration

Intradermal cell injections of lentivirus transduced (COL7A1-corrected) RDEB GM-HDF (using INXN-2002) and negative controls (vehicle or RDEB-HDF) will be done with a 30-gauge needle. The injection will be performed by first piercing the skin, then directing the needle as superficially as possible back upward toward the surface.

Composite grafts will be administered cells by seeding prior to grafting according to protocol outlined in the in vitro study with the distinction that the composite culture system will not be lifted to air-fluid interface prior to grafting.

### B.4.1. Preparation of Fibroblasts for Injection (Composite Grafts)

Fibroblasts were removed from the liquid nitrogen storage at one week prior to injection, thawed in 37°C water bath until a small ice crystal remains, then disinfected and transferred to BSC. Vials were resuspended in 10ml of PBS wash volume and spun for 10 minutes at 1000 RPM. The supernatant was discarded and the cells were washed in DMEM+10% FBS 1x Antibiotic media and re-spun for 10minutes at 1000RPM. The pellet was resuspended in appropriate volume and plated on 15cm TC dishes. Media was changed every other day and passaged at 80% confluence until grafts are ready to inject. On the day of injection, fibroblasts were trypsinized, neutralized in DMEM+10% FBS media and spun 10min at 10000 RPM, the pellet of cells was resuspended and an aliquot of cells was taken, cells were counted with trypan blue stain to determine viability. The cells were resuspended such that 1.0 × 10⁶ cells in 50uL volume is drawn up into syringe. A 30g needle was attached and placed at 4°C until mouse was ready tobe injected.

**Table 34: Composite Skin Grafts (Fibroblast Seeding)**

| **Grp** | **N** | **Sex** | **Composite Skin Graft** | | **Number of Cells** | | **Collection of Specimens** |
|---|---|---|---|---|---|---|---|
| | | | **Keratinocytes** | **Fibroblasts** | **Keratinocytes** | **Fibroblasts** | |
| 1 | 3 | M | WT | WT | 1 × 10⁶ | 1 × 10⁶ | Day 15 |
| 2 | 3 | M | RDEB Keratinocytes | RDEB HDF | 1 × 10⁶ | 1 × 10⁶ | Day 15 |
| 3 | 3 | M | RDEB Keratinocytes | RDEB-GM-HDF | 1 × 10⁶ | 1 × 10⁶ | Day 15 |
| 4 | 3 | M | RDEB Keratinocytes | RDEB - GM-HDF | 1 × 10⁶ | 5 × 10⁵ | Day 15 |
| 5 | 3 | M | RDEB Keratinocytes | RDEB - GM-HDF | 1 × 10⁶ | 2.5 × 10⁵ | Day 15 |
| 6 | 3 | M | RDEB Keratinocytes | RDEB - GM-HDF | 1 × 10⁶ | 1.25 × 10⁵ | Day 15 |

**Table 35: Composite Skin Grafts (Fibroblast Intradermal Injections)**

| **Grp** | **N** | **Sex** | **Test Article** | **Dose** | **Injection** | **Graft Description** |
|---|---|---|---|---|---|---|
| 1 | 2 | M | RDEB-HDF | 1 x 10⁶ | Composite Graft | 1 million RDEB GM-HDF, 1 million RDEB KC |
| . | . | .. | | | | |
| 2 | 4 | M | RDEB-GM-HDF | 1 x 10⁶ | Composite Graft | 0.125 million RDEB GM-HDF, 1 million RDEB KC |
| . | . | .. | | | | |

**Table 36: Study Groups for the Evaluation of ID-Injected Xenografts**

| **Condition** | **Graft Pre-Seeding Prep Conditions** | | **Injected Fibroblasts** | **Date of Harvest** |
|---|---|---|---|---|
| **Control Groups** | **Keratinocytes** | **Fibroblasts** | | |
| Normal | Normal KC | Normal FB | N/A | Day 19 post-grafting |
| FDEB | RDEB | RDEB | N/A | Day 19 post-grafting |

| **Experimental Groups** | | | | |
|---|---|---|---|---|
| 1A | RDEB | TR8/A 1 million | TR8 nontransduced | 10 days post-injection |
| 1B | RDEB | TR8/A 1 million | TR8 nontransduced | 10 days post-injection |
| 2A | RDEB | TR8/A 0.125 mil | TR8/ArmA | 10 days post-injection |
| 2B | RDEB | TR8/A 0.125 mil | TR8/ArmA | 10 days post-injection |
| 2C | RDEB | TR8/A 0.125 mil | TR8/AmiA | 10 days post-injection |
| 2D | RDEB | TR8/A 0.125 mil | TR8/ArmA | 10 days post-injection |

### C. Skin Grafts

### C.1 RDEB Human Skin Grafts

### C.1.1 Fibroblasts

Genetically engineered human fibroblasts (RDEB GM-HDF) produced by lentiviral gene transfer, including the INXN-2002 lentiviral vector, along with normal fibroblasts and non-corrected RDEB fibroblasts as controls, are the cell types used for initially infusing dermis skin. Cells are cultured onto devitalized dermis to form skin equivalents approximately 2 cm square. Regenerated skin composites consist of human keratinocytes cultured atop fibroblast-infused human devitalized dermis.

### Isolation and culture of primary keratinocytes

Wild-type keratinocyte cells are isolated from neonatal foreskin, whereas primary RDEB keratinocytes are isolated from patient skin samples. The latter is obtained as a 6mm punch biopsy submerged in 15 mL of keratinocyte growth medium 50/50V in a 15 mL centrifuge tube shipped on wet ice. No bubbles can be present in shipped samples. Medium 50/50V contains 50% medium 154 (Invitrogen) supplemented with HKGS (0.2% bovine pituitary extract [BPE], bovine insulin [5 mg/mL], hydrocortisone [0.18 mg/mL], bovine transferrin [5 mg/mL], human epidermal growth factor [0.2 ng/mL]) and 50% keratinocyte SFM (KSFM; Invitrogen) supplemented with recombinant human EGF1-53 BPE. Antibiotics AV100 (amikacin/vancomycin) are added to reduce bacterial contamination.

Separate dermis and epidermis by treating with 25 U/mL dispase (Becton Dickinson) overnight at 4°C. Epidermis is then peeled from dermis and placed in a 15 mL centrifuge tube with 5 mL TrypLE 10x. Epidermal "peel" is incubated in a 37°C water bath for 15-30 min with gentle agitation every 5 min. TrypLE is neutralized with equal volume DTI (defined trypsin inhibitor) or DMEM+10%FBS. Cells are pelleted at 1200 rpm and plated in 12 mL 50/50V onto T75 Corning^{®} Purecoat^{™}collagen 1 mimetic flasks. In the event of PureCoat^{™} flask unavailability from manufacturer, 1ml of collagen 1 coating solution per 10cm plastic dish for 15 minutes at 37°C can be used instead. Collagen 1 coating is a 0.2 uM filtered solution of Vitrogen 100 Collagen (1ml), HEPES (2ml), BSA (100uL of 100mg/ml) and 1x HBSS (100ml). The excess collagen coating is aspirated prior to cell seeding. Flasks are swirled to ensure an even distribution of cells and placed in a 37°C 5% CO₂ humidified incubator for at least one night before any manipulation or observation. Cells are allowed to adapt to tissue culture conditions for 3 days. After adaptation, medium is changed once every 2 days or as needed. Keratinocytes are passaged onto normal tissue culture plastic when they are approximately 70% confluent.

In the 50/50V media keratinocytes grow well for five passages. Keratinocytes are utilized within the first four passages.

### Preparation of porcine devitalized dermis

Obtain sheets of split thickness porcine skin. This may be prepared in similar fashion to devitalized human dermis (as above) from porcine skin harvested by dermatome. Wash the porcine dermis 3x in sterile PBS containing 1x penicillin/streptomycin/Amphotericin/gentamicin and incubate in PBS with 1M NaCl and 2x antibiotics at 37°C for 72 hours. Separate epidermis and discard, wash 3x with 1XPBS containing antibiotics to remove excess salt and gentamicin in solution.

Confirm sterility by culturing a piece of porcine dermis in 50/50V or KGM for 4 days. Dermis can be stored at 4°C in antibiotic solution, changing the PBS weekly. Dermis should not be stored for more than a few months before use.

### Organotypic Culture Seeding Fibroblasts

Cut devitalized dermis into 2 cm square pieces. Place dermis basement membrane side down into 6 well culture plates and allow dermis to dry slightly.

Seed fibroblasts into each well, centrifuge 1200 rpm for 5 minutes and return to 37°C with 5% humidified CO₂ for 3-4 days, changing media every day. During this time, fibroblasts attach to the dermis and begin to migrate into the tissue.

### Seeding Keratinocytes

After the fibroblasts have migrated through the dermis, carefully detach dermis and transfer to annular dermal support (ADS). Add a thin layer of Matrigel to seal and secure dermis in device. Add 5 mL KGM to stromal compartment of ADS. KGM is a 3:1 mixture of DMEM:Ham's F12 supplemented with FBS (10%), adenine (1.8 × 10-4 M), hydrocortisone (0.4 µg/mL), insulin (5 µg/mL), cholera toxin (1 × 10-10 M), EGF (10 ng/mL), transferrin (5 µg/mL), and triiodo-L-thyronine (1.36 ng/mL). Trypsinize, count, and seed keratinocytes in a total volume of 100 µL of KGM per ADS. Return ADS assembly to incubator. Do not disturb ADS for at least 24hrs. The keratinocytes at the top of the dermis must remain at the air liquid interface. Change the KGM in the lower chamber every day. Organotypic culture will continue for 1-2 weeks.

### Grafting Organotypic Culture

Organotypic cultures will be ready to graft after 7-10 days. Grafting will be conducted as described above for normal human skin grafts. Bandages and sutures will be removed after 7-10 days and animal will be rewrapped with fresh bandaging for another week. Thereafter graft will be exposed to air to mature.

### Harvesting Grafts and Sectioning

Humanely sacrifice mice and carefully harvest graft. Bisect graft and cut an additional 1 mm slice. Trim slice down to a 1 mm square and send for immunoelectron microscopy (IEM). Freeze remaining halves in OCT on dry ice while noting the central edge. Sectioning will start at this edge. One half will be sectioned for immediate analysis and remaining half will be stored for later analysis. Cut ten 8 µm sections starting from the central edge and sequentially label. Discard the next forty sections. Repeat until end of graft is reached. Air dry and fix with ice cold 50/50 acetone/methanol.

### Injection of Established RDEB Composite Grafts

Fibroblasts will be intradermally injected directly into the skin following of the established graft. Injections will be no greater than 50 µL in volume.

Injections were performed on mice previously grafted with composite grafts as described herein: Devitalized porcine dermis was seeded by centrifugation with variable dosage of RDEB GM- HDF and grown in tissue culture for 1 week. Subsequently, the dermis was flipped over and 1 million RDEB keratinocytes were seeded and allowed to grow in culture for one more week. After this two week period in tissue culture as described above, the grafts were placed on SCID mice and sutured in place, bandaged for 13 days. The dressings were removed at that time, and at 38-41 days post grafting, grafts were injected with 50uL volume of fibroblasts.

### D. EXPERIMENTAL PROCEDURES

### D. 1. Localization of COL7

Immunofluorescent microscopy was used to evaluate expression of type VII collagen. For immunofluorescence (IF) analyses of skin cultures with anti-human C7 specific antibody NP185.

### D.1.2. Methods for NP 185 Antibody Immunofluorescence Microscopy

Composite grafts were harvested from SCID mice immediately after CO₂ euthanasia was performed. The grafts were bisected and placed in OCT blocks. Frozen blocks of tissue were sectioned on a Leica CM1850 cryostat at 8µM thickness at -21°C and fixed in ice cold 50% methanol/50% acetone. Slides were rehydrated and washed in 1XPBS three times, incubated in primary antibody (NP185 10ug/ml; mouse anti-Human Collagen VII) for 1 hour at room temperature. Slides were washed and incubated in Alexa 488 tagged goat anti-mouse IgG antibody (1:400, 1hour, Room Temperature, life technologies) and a nuclear Hoescht 33342 counterstain (Life technologies). Samples were washed three times in 1xPBS, mounted using fluoromount prior to imaging. Images were taken on a Zeiss Observer.Z1 fluorescence microscope at 20X magnification.

Grafts harvested at Day 19 were included as positive and negative control arms. Experimental arms were injected Day 38, 39, or 41 and harvested Day 48, 49, or 51 (Table 37).

**Table 37: Graft, Injection, and Harvest Days**

| **Study Arm** | **N** | **Day of Graft** | **Day of Injection** | **Day of Harvest** |
|---|---|---|---|---|
| Control Arms | | | | |
| Positive (Normal keratinocytes, normal fibroblasts) | 1 | 0 | N/A | 19 |
| Negative (RDEB keratinocytes, RDEB fibroblasts) | 1 | 0 | N/A | 19 |
| Experiment Arms | | | | |
| Non-transduced RDEB fibroblasts | 2 | 0 | 41 | 51 |
| GM-HDF | 4 | 0 | 38 (n=3) or 39 (n=1) | 48 (n=3) or 49 (n=1) |

### E. DATA ANALYSIS

IF images were prepared and provided to the PI in a blinded fashion. Data was analyzed and interpreted by the PI prior to unblinding.

### F. RESULTS

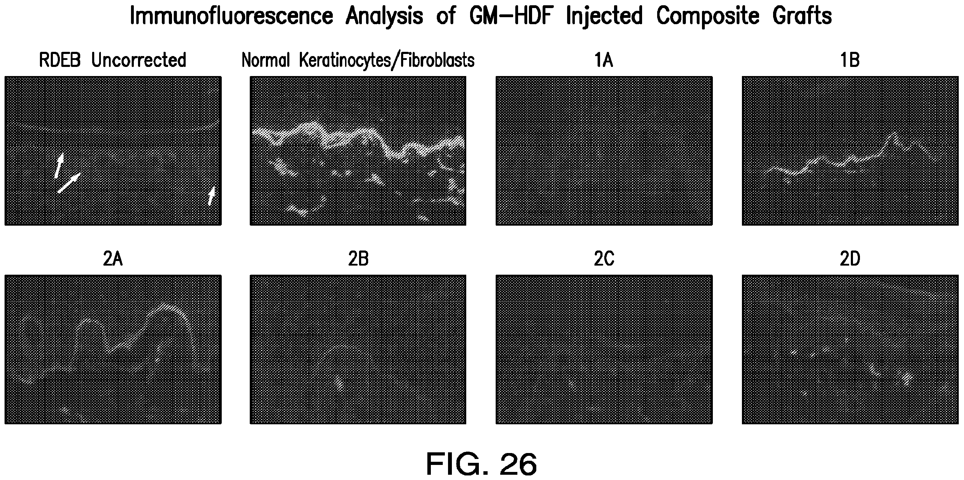

Representative results from the IF analysis of the harvested graft tissue are presented in Figure 26.

C7 staining at DEJ was visualized in all NP185 staining conditions excluding the negative control (image "RDEB uncorrected" - see arrows at DEJ for negative baseline comparison). With regard to the positive controls, there was intense DEJ staining seen in the DEJ of normal keratinocytes/fibroblasts seeded grafts. There was a small focus of positive staining seen in the group 1 injected with uncorrected fibroblasts (Image 1b) in a small section that could represent previously seeded corrected fibroblasts, however the remainder of the uncorrected fibroblast injected grafts in both mice appear to be less intense (as represented in image 1a). There was C7 staining seen at the DEJ in the representative images of four mouse grafts injected with corrected fibroblasts (4a-d) were observed even after only 10-days post-injection.

No tumors were observed in any grafts during the course of the study.

### G. CONCLUSIONS

RDEB GM-HDF TR8 intradermally injected at the approximate proposed clinical dose produced C7 that localized to the DEJ in vivo in composite grafts of RDEB keratinocytes on devitalized pig dermis. Additionally results from seeding of GM-HDF in composite grafts prior to grafting indicate that GM-HDF could persist in expression C7 that localizes to the DEJ. This model can be used to characterize the localization, durability, persistence and phenotype correction approximating the effect of GM-HDF predictive of the clinical dose.

Results of these studies show intradermal injections of autologous GM-HDF as an effective treatment of RDEB in patients.

### Example 7 - Non-GLP In Vitro GM-HDF Cell Characterization and Proof-of-Concept Assessments

The objectives of this study are the following: (a) characterize the GM-HDFs produced using the anticipated at-scale GMP production method to be used for the treatment of RDEB patients; (b) assess copy number of integrated LV and expression levels of C7; and (c) confirm the functionality of C7 expressed by the GM-HDFs

### A. STUDY MATERIALS

### A.1. Test Article(s)

GM-HDFs from Training runs 8, 9, and 10, and the Engineering run (as described above) were characterized.

**Table 38: General production information for TR8, TR9, TRIO, and ER1**

| **Production Run** | **Arm** | **MOI (IU/cell)** | **LV-COL7 and TU titer** | **Production Scale** |
|---|---|---|---|---|
| TR8 | A | 3.4 | INXN-2002 (Pilot) 9.2x10⁶ IU/mL | 2 x 10-layer CellSTACKs^{®} |
| | B | 1.7 | | |
| | Control ¹ | 0 | | |
| TR9 | A | 1.0 | LV-HA-COL7 (Pilot) 2.0×10⁶ IU/mL | 2 x 10-layer CellSTACKs^{®} |
| | B | 1.9 | | |
| | Control¹ | 0 | | |
| TR10² | A | 2.0 | INXN-2002 (GMP) 2.3x10⁶ ICT/mL | 6 x 10-layer CellSTACKs^{®} |
| | Control¹ | 0 | | |
| ER1² | A | 2.6 | INXN-2002 (GMP) 2.3x10⁶ ICT/mL | 6 x 10-layer CellSTACKs^{®} |

| | | | | |
|---|---|---|---|---|
| ¹ Control arm cells were mock-transduced ²Only one LV-COL7 transduction arm was generated for TRIO and for ER1 | | | | |

### B. Primer and Probe sequences used in qPCR analyses

**Table 39: Primer and Probe Sequences used for qPCR Assays**

| **Name** | **Sequence¹** | **Assay** |
|---|---|---|
| PR13843 forward² | ACCTGAAAGCGAAAGGGAAAC (SEQ ID NO:25) | LV-COL7 copy number |
| PR13843 reverse² | CACCCATCTCTCTCCTTCTAGCC (SEQ ID NO: 26) | LV-COL7 copy number |
| PR13843 probe² | 6-FAM- AGCTCTCTCGACGCAGGACTCGGC- 3'IB FQ (SEQ ID NO: 27) | LV-COL7 copy number |
| PR13653 forward | CACTCCCAACGAAGACAAGAT (SEQ ID NO: 28) | COL7A1 mRNA expression |
| PR13653 reverse | GTCTAACCAGAGAGACCCAGTA (SEQ ID NO: 29) | COL7A1 mRNA expression |
| PR13653 probe | 6-FAM TTTGTAAACCGGTGCAGCTGCTTT 3'IB FQ (SEQ ID NO: 30) | COL7A1 mRNA expression |

| | | |
|---|---|---|
| ¹ Primers/probes purchased from IDT. ²LV-specific primer/probe sequences derived from Greenberg et al. (2006). | | |

### C. EXPERIMENTAL PROCEDURES

### C.1 LV-COL7 Copy Number

Nucleic acid isolation was performed using Qiagen's AllPrep kit according to the manufacturer's instructions. gDNA isolated from 3×10⁵ GM-HDF cells was normalized to 12.5 ng/µl. 8 µl of the normalized gDNA was used in a 20 µL assay (10 µL Taqman^{®} Gene Express, 1.8 µL nuclease-free water, 0.06 µL of 100 µM forward primer, 0.06 µL of 100 µM reverse primer, 0.04 µL of 100 µM Taqman^{®} probe). A standard curve of serially diluted linearized C7 lentiviral shuttle vector (1e6 copies/reaction to 5 copies/reaction), plus 4 µL of human gDNA (1.5e4 cells/reaction) were also assayed in the 20 µL assay mentioned above. The Taqman^{®} assay used was PR13843. 8 µL of an additional standard curve of commercial human gDNA (Clontech) (1.5e4 cells/reaction to 2e2 cells/reaction) in a 20 µL assay (10 µL Taqman^{®} Gene Express, 1.0 L nuclease-free water, 1 µL of 20X ACTB primer/probe set) was also performed. All samples were run on an ABI7900 using the following cycling parameters: 2 minutes at 50°C, 10 minutes at 95°C, and 40 cycles of 15 sec at 95°C and 1 minute at 60°C.

### C.2 COL7A1 RT-qPCR

Vials of GM-HDF cells were thawed and gDNA and RNA were isolated using Qiagen's AllPrep^{™} kit. RNA (800 µg) was used to generate cDNA using Quanta's qScript^{™} per manufacturer's instructions. The cDNA and gDNA were then tested using Taqman ^{®} assay PR13653 as described in above. Data were normalized to housekeeping gene ACTB (dCT), and then compared to Control data (ddCT). The resulting ddCT is converted to fold change using the formula 2^-(ddCT).

### C.3 C7 Immunofluorescence

For immunofluorescence analyses, 1.2 × 10⁴ GM-HDFs were allowed to attach to PDL/Lamin- coated coverslips in 24-well plates overnight and then fixed and permeabilized with a 50%/50% mix of methanol/acetone. The coverslips were washed 3 times with 1X PBS and then blocked with 10% goat serum in PBS for 30 minutes at room temperature. After three additional washes with PBS, the coverslips were incubated with 1.25 µg/mL fNC1 antibody in 1% goat serum/PBS, followed by 3 additional washes with PBS and incubation with 5 µg/mL Alexa Fluor^{®} 555- conjugated goat anti-rabbit IgG in 1% goat serum/PBS for 1 hour at room temperature. Coverslips were stained with NucBlue^{®} Live Cell Stain Ready Probes Reagent before being mounted onto slides. Images were acquired on a Zeiss Axio Observer microscope at 20X magnification using an exposure time of 290 ms. NHDFs or GM-HDFs were fixed, permeabilized, and stained with NucBlueLive^{®} Cell Stain to visualize nuclei (blue) or with the fNC1 antibody and Alexa Fluor^{®} 555-congugated goat anti-rabbit IgG (5 µg/mL) to visualize C7 expression (red). Images were acquired at 20X magnification using an exposure time of 290 ms.

### C.4 C7 Protein ELISA

Briefly, a standard curve of purified His-NC1 fragment (9.8 - 625 ng/mL) or collected supernatants containing C7 protein (from GM-HDF in culture for three to five days) were immobilized to a Nunc MaxiSorp^{®} 96-well plate overnight at 4°C. Standards and samples were tested in the same sample matrix (20% RDEB fibroblast conditioned media). Coated wells were washed with PBST and blocked with 3% BSA/PBS for 1 hour at 37°C. Detection was accomplished using a polyclonal anti-NC1 Ab (fNC1, 0.5 µg/mL) followed by incubation with secondary antibody donkey anti-rabbit IgG HRP (Jackson ImmunoResearch, 0.08 µg/mL). Bound antibodies were detected via colorimetric development with TMB substrate solution. Following quenching of the reaction, absorbance was measured at 450 nm on the SpectraMax^{®} Plus 384 (Molecular Devices).

### C.5 Immunoprecipitation of C7 trimers

First, magnetic Protein G beads were washed with 1 X PBS-T. Beads were bound to the magnet for a minimum of 2 minutes prior to removal of supernatant and in all subsequent steps. Following the washes, the beads were coated with 5 µg of anti-C7 fNC1 and incubated for 10 minutes with rotation (Glas-Col, setting 30 ~ 14 rpm at room temperature). Beads were bound to the magnet to remove the supernatant and washed with Ab binding/Wash buffer. Supernatants were collected from GM-HDFs in culture for three to five days. C7 containing supernatant was added to the bead/C7 supernatant mix and was incubated overnight at 4° C with rotation (setting 30 ~ 14 rpm). The next day the beads were bound to the magnet and washed three times using Wash buffer. Target antigen was eluted in 20 µL of elution buffer (50 mM glycine, pH 2.8, and 10 µl of 4X Loading Dye). The samples were denatured at 70°C for 10 minutes. 12 µl of a total of 30 µl (remaining sample was stored at 4°C) were then loaded (per well) into a 12 well 3-8% Tris Acetate gel and run for 4 hours at 150 volts. The gel was removed from the cassette and soaked in Transfer Buffer containing 10% methanol for 20 minutes. Overnight wet transfer of the gel to a nitrocellulose membrane was performed at 15 volts at 4°C. The next day, the voltage was increased to 50 volts for 30 min on ice. Upon completion of transfer, the blot was blocked using 5% milk in TBS-T with agitation (Labnet ProBlot^{™} Rocker 25, setting 60 rpm) for 2 hours at room temperature and incubated with commercially available anti-C7 LH7.2 (0.25 µg/mL) with agitation for 2 hours at room temperature. The blot was washed 3 times with 1X TBS-T (5 min each) with agitation before being probed with HRP-conjugated goat anti-mouse IgG (0.1 µg/mL) with agitation for 1 hour at room temperature. The blot was developed using Lumiglo Ultra^{™}Chemiluminescent substrate and Fujifilm LAS 3000 Imaging System.

### C.6 Lam332 Binding

96-well MaxiSorp^{™} plates were coated with 1 µg Lam332 or BSA in 100 mM carbonate buffer, pH 9.3 overnight at 4°C. The plates were rinsed five times with PBS-T, and then blocked with 1% BSA in PBS-T at room temperature for 1 hour. Coated wells were rinsed five times with PBS-T and incubated with supernatants from transduced cells overnight at 4°C. Following three more washes with PBS-T, bound C7 was incubated with the fNC1 antibody (0.5 µg/mL, PBS-T) at room temperature for 3 hours, followed by incubation with HRP-conjugated donkey anti- rabbit IgG (0.8 µg/mL final concentration in PBS-T) at room temperature for 2 hrs. Detection of the C7-bound antibodies was via a colorimetric reaction using TMB and measured by reading the absorbance of the product at 450nm on a SpectraMax^{®} Plus 384 plate reader (Molecular Devices). All incubations were carried out on an orbital shaker (GeneMate) at a speed setting of 2.

### C.7. Cell Migration Assay

CytoSelect^{™} 24-well wound-healing assay kit from Cell Biolabs, Inc. was used for the cell migration assay. GM-HDFs (0.8 × 10⁵) were seeded in the presence of a wound insert in 24-well plates and grown for 48 hours. The wound insert was then removed and the cells were further cultured in reduced (1%) serum media containing 15 µg/mL type I collagen (COLI) for 8 hours. Brightfield images of the wounds were acquired 0, 2, 4, 6, and 8 hours after removal of the wound insert using the Celigo^{®} Imaging Cytometry System (Cyntellect). The surface area of the wound at each time point was measured using ImageJ (National Institutes of Health). The data is reported as the percentage of migration (% Migration) covering the wound area.

### C.7. DATA ANALYSIS

qPCR was performed using an ABI 7900 instrument utilizing SDS2.3 software. The experimental data were viewed and exported in SDS 2.4 software. ImagJ software was used to quantitate percent cell migration for the assay described above. ImageJ is an open source image processing program developed by the Nation Institute of Health and designed for scientific multidimensional images.

### D. RESULTS

### D.1. Characterization of C7 Expression

### D.1.1. LV-COL7 Copy Number and Transcript Levels

Taqman^{®} assays were designed against various regions of the viral shuttle vector. Of the Taqman^{®} assays tested, designs targeting the C7 coding sequence demonstrated unacceptable levels of background amplification. The Taqman^{®} assay selected, PR13843, was targeted to a sequence found at the 5' end of the LV backbone as described in Greenberg et al. (2006).

GM-HDF training run cells were obtained, washed with dPBS, and resuspended in RLT buffer. Qiagen's AllPrep kit was used to isolate the DNA (for copy number) and RNA (for mRNA transcript level) from the drug substance vials from each arm of each training run. The isolated DNA and RNA were then subjected to qPCR and RT-qPCR assays as described above in Section C.1 and C.2, respectively. Table 40 shows the copy number/cell from Training Runs 8, 9, and 10 (TR8, TR9 and TRIO) and the Engineering Run (ER1). The data demonstrate that the number of copies of transgene integrated into each cell can be modulated by the virus dose given to the cells during production, and is less than 1 copy/cell. Similarly, the LV-COL7 transcript levels, shown in Figure 40, also correlate with the virus dose and copy number, with Arms A and B from the training runs expressing higher levels (3 to 5 LOG) of the lentiviral C7 sequence compared to Control cells.

**Table 40: LV-COL7 Copy Number in GM-HDF Production Run Cells**

| **Production Run** | **LV-COL7 Used** | **Arm** | **MOI (IU/cell)** | **Gene Copy per Cell** |
|---|---|---|---|---|
| TR8 | INXN-2002 (Pilot) | A | 3.4 | 0.021±0.007 |
| | | B | 1.7 | 0.017±0.007 |
| | | Control¹ | 0 | BLQ² |
| TR9 | LV-HA-COL7 (Pilot) | A | 1.0 | 0.009±0.002 |
| | | B | 1.9 | 0.021±0.002 |
| | | Control¹ | 0 | BLQ² |
| TR10³ | INXN-2002 (GMP) | A (3) | 2.0 | 0.013±0.002 |
| | | Control¹ | 0 | BLQ² |
| ER1³ | INXN-2002 (GMP) | A (3) | 2.6 | 0.025±0.005 |
| | | Control¹ | 0 | BLQ² |

| | | | | |
|---|---|---|---|---|
| ¹ Control arms were mock-transduced and were not exposed to LV-COL7 ² BLQ: below limit of quantitation ³ Only a single transduction arm was generated for TRIO and for ER1 | | | | |

### D. 1.2. C7 Protein Expression

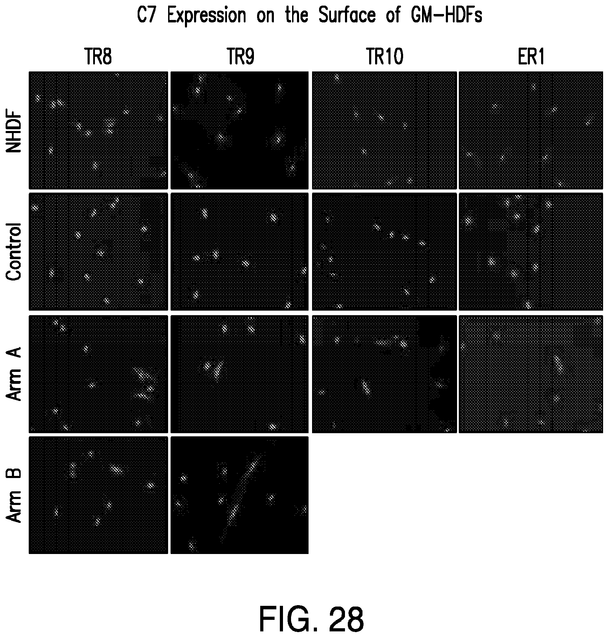

Indirect immunofluorescence (IF) was used to examine C7 protein expression by GM-HDF cells using an antibody specific for the C7. Normal human dermal fibroblasts (NHDFs) were used as a positive control for this assay and the control arms of the training runs were used as negative controls. In the representative images shown in Figure 28 below, a small percentage (<10%) of INXN-2002 and LV-HA-COL7-transduced RDEB fibroblasts show C7 staining, which is consistent with the copy number of integrated LV (Table 18). The signal from the GM-HDFs that are C7-positive is brighter than the signal from NHDFs, indicating greater amounts of C7 produced by GM-HDFs.

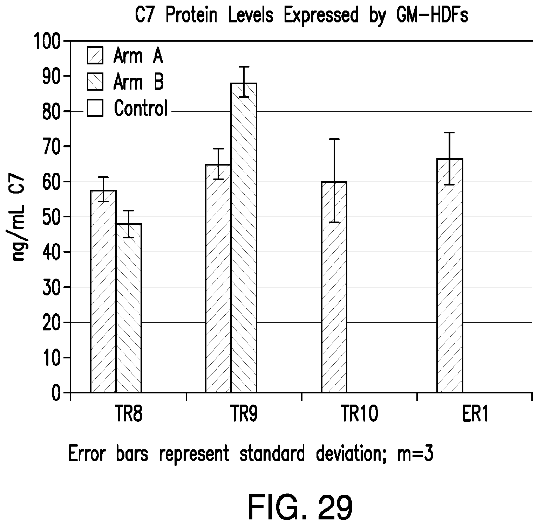

A direct ELISA was developed by Intrexon to measure C7 secreted by GM-HDFs into the cell culture media. This assay, as described above in Section C.4, utilized an NC1 region-specific polyclonal antibody. ELISA assay results of the GM-HDFs from the three training runs show that C7 expression is LV dose dependent with expression levels ranging from 45-90 ng/mL of cell supernatants (Figure 29).

### D.2. Functional Assessment of C7 Expressed by GM-HDFs 9.2.1. Formation of C7 Trimers

The formation of C7 trimers is important in the assembly of anchoring fibrils. To verify that the C7 expressed by GM-HDFs will be capable of forming anchoring fibrils, we assessed whether or not the expressed C7 formed trimers using immunoprecipitation (IP) with an anti-C7 specific antibody (fNC1) for selective capture, then concentration of C7 from culture supernatants for detection by SDS-PAGE/immunoblot.

The optimal conditions for immunoprecipitation were established by testing a commercial C7 antibody and the fNC1 C7 antibody. The coating of protein G beads with fNC1 resulted in higher levels of C7 binding compared to coating with the commercially available antibody. Controls used in this assay include a no antibody control to rule out non-specific binding of C7 to the beads, as well as antibody coated beads incubated with and without purified C7 spiked into conditioned media as controls for specificity of C7 isolated from GM-HDF culture supernatants.

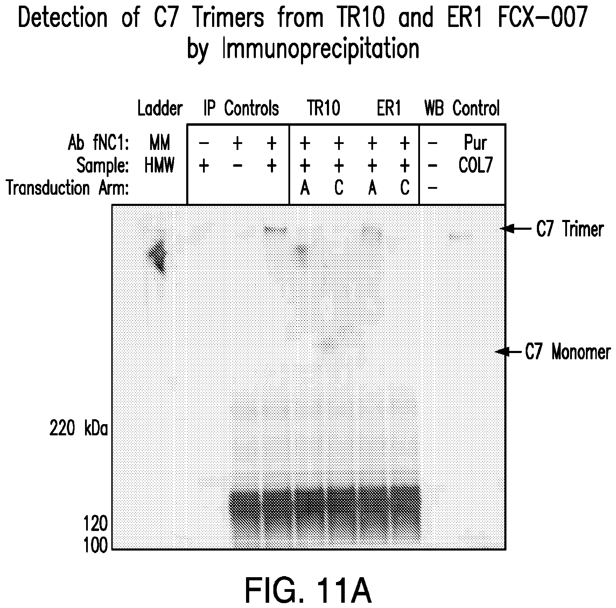

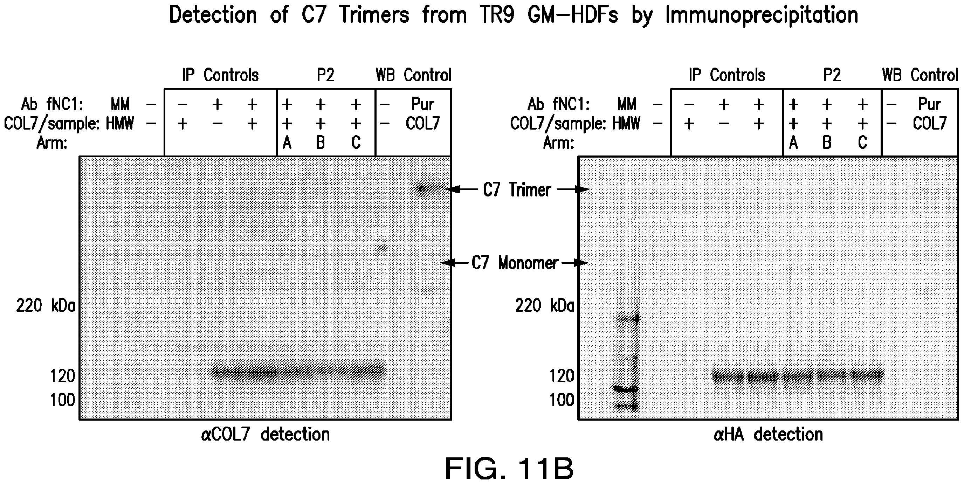

Immunoprecipitation results for TR8 and for TRIO and ER1 showed that the C7 produced by GM-HDF was predominantly trimeric (Figure 10, and Figure 11, respectively). Some lower molecular weight species showing immunoreactivity were also observed, and included the dimeric and 290 kDa monomeric forms. IP of C7 from GM-HDFs of TR9 was detected on the immunoblot at low levels by both an anti-C7 antibody (left panel, Figure 11B) as well as an antibody specific for the HA tag incorporated into the C7 protein (right panel, Figure 11B).

### D.2.2. Lam332 Binding by C7 Expressed from GM-HDFs

C7 has been shown to bind immobilized extracellular matrix (ECM) components, including fibronectin, Laminin 332 (Lam332), COL1, and COL4 (Chen, et al., 2002a). The interaction between C7 and Lam332 occurs through the NH2-terminal NC1 domain of C7 and is dependent upon the native conformation of both Lam332 and C7 NC1 (Rousselle, et al., 1997). The association between C7 and Lam332 is important for establishing correct Lam332 architecture at the dermal-epidermal junction. Such organization is important for interactions with extracellular ligands and cell surface receptors, and for cell signaling (Waterman, et al., 2007). Intrexon developed an ELISA using an antibody against the C7 NC1 domain to detect binding of C7 to purified Lam332. Although a C7/Lam332 binding ELISA has already been described in the literature (Chen, et al., 2002a), to our knowledge it has never been used to test C7 present in the supernatants of transduced cells. Results in Figure 12 show dose-dependent binding to Lam332 by C7 expressed by GM-HDFs from the Training and Engineering runs.

### D.2.3. Cell Migration Assay

Previous studies have shown that RDEB fibroblasts and keratinocytes show an increase in motility relative to their normal counterparts, and that normal motility can be restored by expression of C7 (Chen, et al., 2000; Chen, et al., 2002b; Cogan, et al. 2014; Baldeschi et al., 2003). Most of these studies employed either the colloidal gold salt migration assay to measure the migration of fibroblasts and keratinocytes, or a wound-healing assay to measure the migration of keratinocytes. Here we used the CytoSelect^{™} 24-well wound-healing assay kit (Cell Biolabs, Inc.) to measure the motility of NHDFs and GM-HDFs.

Immunofluorescent staining for C7 revealed that only a small percentage (<10%) of transduced cells expressed high levels of the protein, raising the question as to whether a small number of C7-producing cells could have a global effect on cell migration. To determine if the presence of C7 in the culture media is sufficient to reverse the RDEB hyper-motility phenotype, the wound healing assay was carried out on GM-HDFs from the three training runs, with NHDFs as a positive control and the mock-transduced Control (RDEB) arms as the negative control.

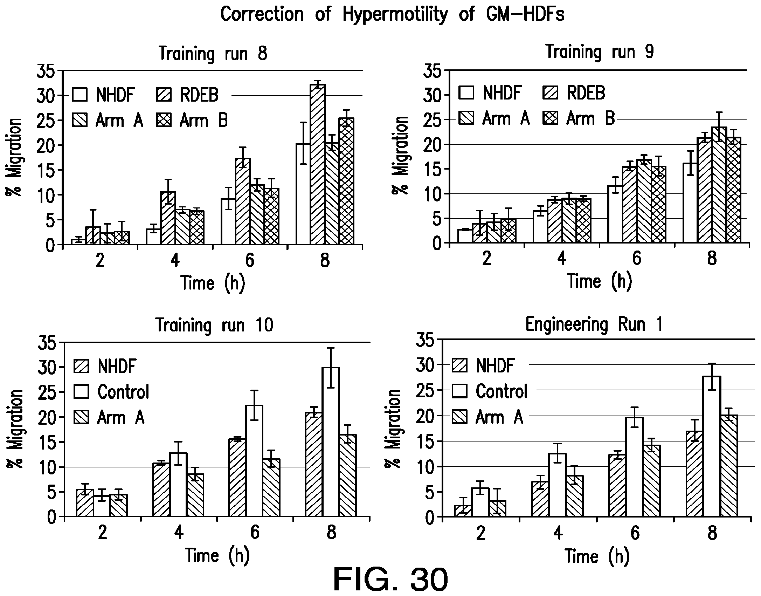

Control fibroblasts from TR8, TRIO, and ER1 exhibit a hyper-motility phenotype that is reversed by the expression of C7 (Figure 30). However, the Control fibroblasts from TR9 do not exhibit a significant hyper-motility phenotype, making it difficult to investigate the functionality of HA- tagged C7 in this assay.

### CONCLUSIONS

The integrated transgene copy number per cell in GM-HDFs was dependent on the virus dose ranging from 0.009 to 0.03 transgene copies per cell.

Dose-dependent C7 expression from the GM-HDF cells was confirmed by qRT-PCR, immunofluorescence staining, and ELISA.

The structure of the C7 expressed by the GM-HDF cells was confirmed to be predominantly trimeric by immunoprecipitation/SDS-PAGE/immunoblot analysis for TR8, TRIO, and ER1. TR9 showed minimal trimer formation.

The C7 produced from the TR8 GM-HDF cells was demonstrated to be functional by binding to Laminin332 in an in vitro binding assay and by correction of the hypermotility phenotype of Control RDEB cells in an in vitro migration assay. TR9 showed binding to Laminin332, however, migration assay results were equivocal. TR9 HA-GM-HDFs generated from transduction with LV-HA-COL7 showed low levels of trimer, less binding to Lam332, and lack of clear hypermotility correction.

Due to lack of confirmation of in vitro functionality of HA-C7 expressed by TR9 HA- GM-HDFs, TR8 cells were chosen for in vitro and in vivo hybrid pharmacology/toxicology evaluations. This eliminated the ability to assess expression, localization and persistence in normal human skin or RDEB composite grafts prepared with devitalized human skin. Ultimately, composite grafts prepared with devitalized porcine dermis were used for these studies. Immunostaining with human specific anti-C7 antibody was used to distinguish rC7 form native C7 in this approach.

### EXAMPLE 8 - DRUG PRODUCT

FCX-007 Drug Product consists of a sterile suspension of each patient's own living autologous fibroblast cells, which are gene modified using INXN-2004 LV-COL7 to encode the human wild type *COL7A1* gene, formulated in Dulbecco's Modified Eagle's Medium (DMEM) without phenol red.

### Formulation Development

Development of FCX-007 formulation and cell dosage was based on Fibrocell's manufacturing experience with azficel-T (LAVIV^{®}); a live, autologous fibroblast cell product for injection. Fibroblast cells have been successfully formulated in DMEM at a cell concentration range of 0.5 - 3.0 × 10⁷ cells/mL without viscosity concerns (azficel-T BLA #125348). Data suggest viscosity becomes a concern for product injection when cell concentration reaches 5.8 × 10⁷ cells/mL. As a result, DMEM at a cell concentration of 1.0-3.0 × 10⁷ cells/mL was selected as the formulation for FCX-007 DP.

### Excess

Up to ten FCX-007 DP vials are shipped to the clinic as one dose for treatment injection. At the time of administration, the DP vial is gently inverted three times, and then 1 mL from each vial is drawn aseptically into a sterile 1 mL syringe with a 21 gauge needle. The 21 gauge needle is then replaced with a 30 gauge needle for intradermal administration. The process is repeated for the remaining 9 product vials.

FCX-007 DP vials are filled with 1.2 mL of the fibroblast cell suspension into a 2.0 mL capacity vial for a recoverable volume of 1.0 mL. The 0.2 mL excess is intended to ensure that 1.0 mL can be obtained from the vial at the time of administration in the clinic.

### a. Physicochemical and Biological Properties

### i. Physicochemical Properties

FCX-007 DP is presented as a gene modified autologous fibroblast cell suspension in DMEM without phenol red with a 1.2 mL fill in a 2 mL cryovial. The cells are demonstrated by assays to be a living culture. Table 41 shows the physicochemical properties of FCX-007 DP.

**Table 41: Physicochemical Properties of FCX-007 DP**

| **Physicochemical Property** | **Value** |
|---|---|
| Cell Count | 1.0-3.0 x 10⁷ cells |
| Cell Viability | ≥ 70% |

### Biological Properties

FCX-007 is a sterile autologous fibroblast cell product that is genetically modified to express the human collagen 7 protein (C7). INXN-2004 vector copy number per cell and expression of the C7 protein are unique biological properties of FCX-007 DP. FCX-007 DP is a live cell suspension product that is produced from FCX-007 DS by washing the thawed FCX-007 DS cells in PBS and DMEM buffers. INXN-2004 vector copy number per cell and C7 protein expression are analyzed on the FCX-007 DS prior to release to manufacture FCX-007 DP Table 42 shows the biological properties of FCX-007 DP.

**Table 42: Biological Properties of FCX-007 DP**

| **Biological Property** | **Value** | **Stage Measured** |
|---|---|---|
| Cell Type Purity / Identity (fibroblast) | ≥ 98% CD 90+ | Drug Substance |
| INXN-2002 Vector Copy Number | 0.1 - 5.0 | Drug Substance |
| C7 Protein Expression | ≥ 500ng/day/E6 cells | Drug Substance |
| Sterility | No Growth (Sterile) | Drug Product |

### BATCH FORMULA

To prepare the FCX-007 Drug Product, FCX-007 Drug Substance vials are removed from the vapor phase of liquid nitrogen frozen storage, thawed, and pooled inside an ISO 5 BSC. The cells are washed with phosphate buffered saline (PBS), followed by a wash with Dulbecco's Modified Eagle's Medium (DMEM) without phenol red before being suspended in DMEM without phenol red to a target concentration of 1.0 - 3.0 × 10⁷ cells/mL. The formulated cells are then filled into 2 mL cryovials at 1.2 mL/vial. Drug Product material prepared for each injection treatment is defined as a batch. The proposed treatment batch size is up to ten (10) 2 mL vials containing 1.2 mL of cell suspension.

Table 43 provides a list of all components used in the Drug Product manufacturing process.

**Table 43: Composition and Quantity of FCX-007 Drug Product Batch**

| **Component** | **Function** | **Quality Standard** | **Quantity per DP Vial¹** | **Quantity per Batch (10 vials per batch)** |
|---|---|---|---|---|
| FCX-007 DS | Drug Substance | Drug Product Certificate of Analysis | 1.2-3.6 x 10⁷ cells | 1.2 - 3.6 x 10⁸ cells |
| PBS | Diluent used for washing Drug Substance cells | Manufacturer 's Certificate of Analysis | Minimal residual trace² | Minimal residual trace² |
| DMEM without phenol red | Buffer for cell suspension | Manufacturer 's Certificate of Analysis | 1.2 mL | 12 mL |

| | | | | |
|---|---|---|---|---|
| ¹ Vial contains 1.2 mL of FCX-007 cell suspension ² PBS wash residual is diluted with DMEM without phenol red such that trace amounts of PBS is possible but not quantifiable | | | | |

### DESCRIPTION OF MANUFACTURINIG PROCESS AND PROCESS CONTROLS

### b. Manufacturing Process Flow Diagram

Manufacturing of the FCX-007 Drug Product commences when FCX-007 Drug Substance vials are removed from liquid nitrogen storage and thawed using a ThermoMixer^{®}. In an ISOS BSC the thawed FCX-007 DS cells are pooled into a 250 mL centrifuge tube containing >5x bulk volume of phosphate buffered saline (PBS) wash buffer for re-suspension. The cells are centrifuged at 1000 rpm for 10 minutes at 5 ± 3°C and the PBS wash buffer is removed. This is followed by a wash with > 5x bulk volume of Dulbecco's Modified Eagle Medium (DMEM) by re-suspension and centrifugation at 1000 rpm for 10 minutes at 5 ± 3°C. The DMEM wash buffer is removed. The washed cells are re-suspended in DMEM without phenol red to a target concentration of 2.0 × 10⁷ cells/mL. The cell suspension is manually filled into sterile 2.0 mL cryovials in an ISOS BSC to a volume of 1.2 mL per vial to produce the FCX-007 DP vials. The Drug Product vials are stored at 2-8°C until released by QA for shipment in a Credo Cube^{™} shipper to the clinical site.

### Manufacturing Process Description

FCX-007 Drug Product is manufactured as indicated above. INXN-2002 is a synonymous name for FCX-007.

Step 1: Removal of FCX-007 Drug Substance Vials from Liquid Nitrogen Storage and Lot Verification

The FCX-007 Drug Substance, which is stored in a liquid nitrogen freezer, is requested from Materials Management. FCX-007 Drug Substance vials are then pulled for this batch of FCX-007 Drug Product production before being transferred to the cleanroom suite.

### Step 2: Thaw Cryovials

A Line Clearance of the cleanroom suite is performed by QA per SOP-0099 GMP Production Line Clearance/GTP Line Cleaning in order to release the room for processing use. Operators initiate room, personnel, and BSC environmental monitoring (EM) per SOP-0129 (In Process Environmental and Personnel Monitoring) prior to processing and non-viable EM sampling in the BSC during processing.

The FCX-007 DS cyrovials are disinfected and thawed in a ThermoMixer^{®} equilibrated to 37°C until cell suspension is almost completely thawed with a sliver of ice crystals remaining. The thawed vials are transferred to a BSC.

### Step 3: PBS Wash

Inside the BSC, using a pipette, transfer approximately 150 mL of PBS (> 5 volumes of the FCX-007 DS cell suspension) into a 250 mL centrifuge tube. The contents of the thawed cryovials are transferred into the 250 mL centrifuge tube. Use the diluted cell suspension to rinse the pipette. Use a pipette to transfer 3.0 mL of fresh PBS to each cryovial as a rinse. Transfer the rinse to a 250 mL centrifuge tube. Swirl the tube gently to mix the cell suspension. Transfer the centrifuge tube to a centrifuge and centrifuge the cells at 1000 RPM for 10 minutes at 5 ± 3°C. After centrifugation, remove the PBS wash supernatant from the tube.

### i. Step 4: DMEM Wash

Using a pipette, add approximately 200 mL of DMEM without phenol red (> 5 volumes of the FCX-007 DS cell suspension) to the tube to re-suspend the cell pellet. Swirl the tube gently to mix the cell suspension. Transfer the centrifuge tube to a centrifuge and centrifuge the cells at 1000 RPM for 10 minutes at 5 ± 3°C. After centrifugation, using a pipette, transfer 0.9 mL of the supernatant from the centrifuge bottle into each of 2 separate 2 mL cryovials (1.8 mL total) labeled "Lot, QC-Sterility". Set the cryovials labeled for sterility aside in the BSC. Aspirate the remaining DMEM wash supernatant from the centrifuge tube.

### Step 5: Cell Pellet Re-suspension in DMEM

The volume of DMEM used to re-suspend the cell pellet is calculated as:

Volume of thawed FCX-007 DS cell suspension divided by 1.5

The 1/1.5 volume factor compensates for possible cell loss during the wash steps, in order to result in a cell concentration in the specified range of 1.0-3.0 × 10⁷ cells/mL after re-suspension.

Use a pipette to transfer the calculated amount of DMEM to re-suspend the cell pellet in the centrifuge tube. Use a pipette to transfer 0.1 mL of the cell suspension into a 2 mL cryovial labeled "Lot, QC- Count and Viability" and submit the cryovial to QC for cell counting. Use a pipette to measure the remaining total cell suspension volume and place the cell suspension into a 2-8°C refrigerator for later final fill use.

### Step 5a: Re-dilution (if needed)

If the QC cell concentration is above 3.0 × 10⁷cells/mL, perform an additional dilution using fresh DMEM to target a cell concentration of 2.0 × 10⁷ cells/mL. Resubmit a cell suspension sample to QC for cell count to confirm final cell concentration.

### Step 6: Final Fill

Retrieve the cells from the refrigerator and transfer the cells to the BSC for final fill. Use a pipette to fill the well-mixed cell suspension manually into 2 mL cryovials in the following order:
Fill 0.1 mL of cell suspension into the first sterility sample vial (added to retained sample from Step 4)
Fill the product vials at 1.2 mL per vial
Fill 0.1 mL of cell suspension into the second sterility sample vial (added to retained sample from Step 4)
Fill the QC vial at ≤1.8 mL per vial
Submit the sterility and QC vials for testing.

Step 7: Store DP Vials for Release and Shipment

Hold the DP vials at 5 ± 3°C for release and shipment to the clinical site.

### Description of the In-Process Controls

### Environmental Controls

All cell culture manipulations are carried out in a certified ISO 5 Biological Safety Cabinets (BSCs) within an ISO7 environmental background using aseptic techniques. The final Drug Product 2 mL cryovial containers are purchased pre-sterilized from Corning. The operators performing the final fill are qualified for aseptic fill operations.

### In-Process Controls and Testing

The process controls and testing performed for release of FCX-007 Drug Product are described below.

### Step 1

The correct FCX-007 Drug Substance lot is verified by QA before use in FCX-007 DP manufacture.

### Step 2

The temperature of the ThermoMixer^{®} used to thaw FCX-007 Drug Substance vials is controlled at 37°C. The content of each vial is thawed until a sliver of ice still remains in the vial to prevent overheating.

### Step 3

The PBS wash volume is controlled at ≥ 5 volumes of the FCX-007 DS cell pool to ensure adequate washing of the cells. The centrifugation conditions are controlled at 1000 rpm, 10 minutes and 5±3°C to ensure sufficient cell pelleting.

### Step 4

Similarly to the PBS wash step, the DMEM wash volume is controlled at ≥ 5 volumes of the FCX-007 DS cell pool to ensure adequate washing of the cells. The centrifugation conditions are controlled at 1000 rpm, 10 minutes and 5±3°C to ensure sufficient cell pelleting. Two 900 µL aliquots of DMEM wash supernatant are taken for use in sterility testing of the final product.

### Step 5 and 5(a) (if needed)

The re-suspended cell concentration is controlled at 1.0 -3.0 × 10⁷ cells/mL, and viability ≥ 70%.

### Step 6

Fill volume of the FCX-007 DP vials is controlled at 1.2 mL fill per vial. A final cell count and viability measurement, as well as Gram stain is performed on the filled QC vial. The two sterility vials are submitted for sterility testing. The results of this sterility test are not received until after Drug Product release, so release of DP is done on the basis of a negative Gram Stain. The use of a rapid microbial test in this situation is indicated in Guidance for FDA Reviewers and Sponsors "Content and Review of Chemistry, Manufacturing, and Control (CMC) Information for Human Somatic Cell Therapy Investigation New Drug Applications (INDs)" of April 2008.

### ii. Control of Materials

The FCX-007 Drug Product manufacturing process utilizes single use, disposable products that will come in contact with raw materials (e.g. pipets, centrifuge tubes, and cryovials) to remove cleaning and carryover issues from consideration. Raw materials selected for use in the manufacturing process are acceptable for pharmaceutical processing as being USP VI compliant. All disposable items are purchased pre-sterilized by gamma irradiation. The PBS and DMEM wash buffers are purchased sterile from commercial suppliers. Steps in the process have the appropriate process parameters identified, such as time and temperature.

### EXAMPLE 9 - In Vitro Characterization of Optimized GM-HDF

This example describes characterization of GM-HDFs generated using an enhanced LV transduction procedure the LV-COL7 construct INXN-2004. Three training runs (TR11, TR12.1, and TR13) were executed to generate cells for the analyses at GMP-scale. TR11 was transduced with the LV-COL7 vector INXN-2002 and was used to evaluate a centrifugation-enhanced (spinoculation) LV transduction procedure with and without a second transduction step (super-transduction). TR12.1 and TR13 were transduced with the LV-COL7 vector INXN-2004 utilizing enhanced transduction procedures as determined through evaluation of TR11.

GM-HDFs from each training run were characterized using assays to assess results such as copy number of integrated LV-COL7, protein expression of C7, and functionality of the C7 expressed by GM-HDFs.

LV integration and C7 expression levels increased first with the addition of the super-transduction step and increased further with use of the LV-COL7 INXN-2004. C7 expressed from GM-HDFs from all three training runs assembled into the trimeric form and bound to Laminin332, which is important in the formation of anchoring fibrils. Expression of C7 by the GM-HDFs was able to reverse hypermotility observed in RDEB fibroblasts. GM-HDFs from TR12.1 and 13 were selected to be used in *in vivo* GLP toxicology studies.

Objectives in this study were to (a) demonstrate improvement in copy number of integrated LV and expression of C7 resulting from 1) an enhanced transduction procedure incorporated into the at-scale GMP production method and 2) adoption of the INXN-2004 LV-COL7 construct and (b) to confirm the functionality of C7 expressed by the GM-HDFs

### 1. STUDY MATERIALS

### 1.1. Test Article(s)

GM-HDFs from Training Runs (TR) 11, 12.1, and 13 were characterized.

**Table 44: General production information for TR11, TR12.1, and TR13**

| **Production Run** | **LV-COL7 and TU titer** | **Arm** | **MOI (IU/cell)** | **Super-transduction** | **Production Scale** |
|---|---|---|---|---|---|
| TR11 | INXN-2002 (GMP) 1.2x10⁵ IU/mL | A | 0.77 | Yes | 2 x 10-layer CellSTACKs^{®} |
| | | B | 0.77 | No | |
| | | Control¹ | 0 | n/a | |
| TR12.1 | INXN-2004 (GMP) 1.4x10⁵ IU/mL | A | 0.12 | Yes | 2 x 10-layer CellSTACKs^{®} |
| | | B | 0.12 | No | T-175² |
| TR13 | INXN-2004 (GMP) 1.4x10⁵ IU/mL | A | 0.12 | Yes | 2 x 10-layer CellSTACKs^{®} |
| | | B | 0.12 | No | T-175² |

| | | | | | |
|---|---|---|---|---|---|
| ¹ Control arm cells were mock-transduced ² Arm B of TR12.1 and TR13 were for research only. Passaging of these arms terminated early and, thus, did not complete the full-scale production process. | | | | | |

### 1.2. Primer and Probe Sequences

**Table 45: Primer and Probe Sequences used for qPCR Assays**

| **Name** | **Sequence¹** | **Assay** |
|---|---|---|
| PR13843 forward² | ACCTGAAAGCGAAAGGGAAAC (SEQ ID NO: 31) | LV-COL7 copy number |
| PR13843 reverse² | CACCCATCTCTCTCCTTCTAGCC (SEQ ID NO: 32) | LV-COL7 copy number |
| PR13843 probe² | 6-FAM- AGCTCTCTCGACGCAGGACTCGGC-3'IB FQ (SEQ ID NO: 33) | LV-COL7 copy number |

| | | |
|---|---|---|
| ¹ Primers/probes purchased from IDT. ² LV-specific primer/probe sequences derived from Greenberg *et al.* (2006). | | |

### 2. EXPERIMENTAL PROCEDURES

### 2.1. LV-COL7 Copy Number

Nucleic acid isolation was performed using Qiagen's AllPrep kit according to the manufacturer's instructions. gDNA was isolated from 6.7×10⁵ GM-HDF cells, and 8 µL of gDNA was used in a 20 µL assay (10 µL Taqman^{®} Gene Express, 1.8 µL nuclease-free water, 0.06 µL of 100 µM forward primer, 0.06 µL of 100 µM reverse primer, 0.04 µL of 100 µM Taqman^{®} probe). A standard curve of serially diluted linearized LV-COL7 lentiviral shuttle vector (1e6 copies/reaction to 5 copies/reaction), plus 4 µL of human gDNA (1.5e4 cells/reaction) were also assayed in the 20 µL assay mentioned above. The Taqman^{®} assay used was PR13843. 8 µL of an additional standard curve of commercial human gDNA (Clontech) (1.5e4 cells/reaction to 2e2 cells/reaction) in a 20 µL assay (10 µL Taqman^{®} Gene Express, 1.0 L nuclease-free water, 1 µL of 20X ACTB primer/probe set) was also performed. All samples were run in triplicate on an ABI7900HT using the following cycling parameters: 2 minutes at 50°C, 10 minutes at 95°C, and 40 cycles of 15 sec at 95°C and 1 minute at 60°C.

### 2.2. C7 Immunofluorescence

For immunofluorescence analyses, 1.2 × 10⁴ GM-HDFs were allowed to attach to PDL/Lamin-coated coverslips in 24-well plates overnight and then fixed and permeabilized with a 50%/50% mix of methanol/acetone. The coverslips were washed 3 times with 1X PBS and then blocked with 10% goat serum in PBS for 30 minutes at room temperature. After three additional washes with PBS, the coverslips were incubated with 1.25 µg/mL of a polyclonal fNC1 antibody (α-fNC1) in 1% goat serum/PBS, followed by 3 additional washes with PBS and incubation with 5 µg/mL Alexa Fluor^{®} 555-conjugated goat anti-rabbit IgG in 1% goat serum/PBS for 1 hour at room temperature. Coverslips were stained with NucBlue^{®} Live Cell Stain Ready Probes Reagent before being mounted onto slides. Images were acquired on a Zeiss Axio Observer microscope at 20X magnification using an exposure time of 290 ms. NHDFs or GM-HDFs were fixed, permeabilized, and stained with NucBlue^{®} Live Cell Stain to visualize nuclei (blue) or with the fNC1 antibody and Alexa Fluor^{®} 555-congugated goat anti-rabbit IgG (5 µg/mL) to visualize C7 expression (red). Images were acquired at 20X and at 5X magnification using an exposure time of 290 ms.

### 2.3. C7 Flow Cytometry

GM-HDFs (~500,000 cells) were collected into each well of a 96-well plate V-bottom plate, concentrated by centrifugation (1500 rpm, 5 min, room temp.), and the culture media supernatants removed. The cells were then resuspended in 200 µL of CytoFix, transferred to a 96-well V-bottom plate, and incubated for 30 min at 4°C. Following fixation, the fixative is then removed by centrifugation (1500 rpm, 5 min, 4°C) and the cells permeabilized with 200 µL ice-cold 100% methanol for 30 min on ice. Cells were then held at -20°C prior to staining procedure. For staining, permeabilized cells were washed twice with 200 µL incubation buffer (0.5 g BSA in 100 mL PBS), with centrifugation (300 rpm, 5 min, room temp.) in between washes to remove the buffer. Washed cells were resuspended in 100 µL of incubation buffer containing α-fNC1 diluted 1:400 and incubated for 1hour at room temp. Cells were then washed twice as described above and resuspended in 100 µL of incubation buffer containing AlexaFluor 555-Rabbit IgG secondary antibody diluted 1:2000 and incubated for 30 min at room temp. Cells were then washed twice as described above and resuspended in 100 µL of PBS. C7-expressing cells were enumerated by flow cytometry using BD LSRII and BD FACSDiva Software (V6.2) with the following parameters: FSC Voltage = 484, SSC Voltage = 209, PE = 500.

### 2.4. C7 Protein ELISA

Briefly, a standard curve of purified His-NC1 fragment (9.8 - 625 ng/mL) or collected supernatants containing C7 protein (from GM-HDF in culture for three to five days) were immobilized to a Nunc MaxiSorp^{®} 96-well plate overnight at 4°C. Standards and samples were tested in the same sample matrix (10% RDEB fibroblast conditioned media). Coated wells were washed with PBST and blocked with 3% BSA/PBS for 1 hour at 37°C. Detection was accomplished using α-fNC1 Ab (0.5 µg/mL) followed by incubation with secondary antibody donkey anti-rabbit IgG HRP (Jackson ImmunoResearch, 0.08 µg/mL). Bound antibodies were detected via colorimetric development with TMB substrate solution. Following quenching of the reaction, absorbance was measured at 450 nm on the SpectraMax^{®} Plus 384 (Molecular Devices).

### 2.5. Immunoprecipitation of C7 trimers

First, magnetic Protein G beads were washed with 1 X PBS-T. Beads were bound to the magnet for a minimum of 2 minutes prior to removal of supernatant and in all subsequent steps. Following the washes, the beads were coated with 5 µg of α-fNC1 and incubated for 10 minutes with rotation (Glas-Col, setting 30 ~ 14 rpm at room temperature). Beads were bound to the magnet to remove the supernatant and washed with Ab binding/Wash buffer. Supernatants were collected from GM-HDFs in culture for three to five days. C7 containing supernatant was added to the bead/C7 supernatant mix and was incubated overnight at 4° C with rotation (setting 30 ~ 14 rpm). The next day the beads were bound to the magnet and washed three times using Wash buffer. Target antigen was eluted in 20 µL of elution buffer (50 mM glycine, pH 2.8, and 10 µL of 4X Loading Dye). The samples were denatured at 70°C for 10 minutes. 12 µL of a total of 30 µL (remaining sample was stored at 4°C) were then loaded (per well) into a 12 well 3-8% Tris Acetate gel and run for 4 hours at 150 volts. The gel was removed from the cassette and soaked in Transfer Buffer containing 10% methanol for 20 minutes. Overnight wet transfer of the gel to a nitrocellulose membrane was performed at 15 volts at 4°C. The next day, the voltage was increased to 50 volts for 30 min on ice. Upon completion of transfer, the blot was blocked using 5% milk in TBS-T with agitation (Labnet ProBlot^{™} Rocker 25, setting 60 rpm) for 2 hours at room temperature and incubated with commercially available anti-C7 LH7.2 (0.25 µg/mL) with agitation for 2 hours at room temperature. The blot was washed 3 times with 1X TBS-T (5 min each) with agitation before being probed with HRP-conjugated goat anti-mouse IgG (0.1 µg/mL) with agitation for 1 hour at room temperature. The blot was developed using Lumiglo Ultra^{™} Chemiluminescent substrate and Fujifilm LAS 3000 Imaging System.

### 2.6. Lam332 Binding

96-well MaxiSorp^{™} plates were coated with 1 µg Lam332 or BSA in 100 mM carbonate buffer, pH 9.3 overnight at 4°C. The plates were rinsed five times with PBS-T, and then blocked with 1% BSA in PBS-T at room temperature for 1 hour. Coated wells were rinsed five times with PBS-T and incubated with supernatants from transduced cells overnight at 4°C. Following three more washes with PBS-T, bound C7 was incubated with the α-fNC1 (0.5 µg/mL, PBS-T) at room temperature for 3 hours, followed by incubation with HRP-conjugated donkey anti-rabbit IgG (0.8 µg/mL final concentration in PBS-T) at room temperature for 2 hrs. Detection of the C7-bound antibodies was via a colorimetric reaction using TMB and measured by reading the absorbance of the product at 450nm on a SpectraMax^{®} Plus 384 plate reader (Molecular Devices). All incubations were carried out on an orbital shaker (GeneMate) at a speed setting of 2.

### 2.7. Cell Migration Assay

CytoSelect^{™} 24-well wound-healing assay kit from Cell Biolabs, Inc. was used for the cell migration assay. GM-HDFs (0.8 × 10⁵) were seeded in the presence of a wound insert in 24-well plates and grown for 48 hours. The wound insert was then removed and the cells were further cultured in reduced (1%) serum media containing 15 µg/mL type I collagen (COLI) for 8 hours. Brightfield images of the wounds were acquired 0, 2, 4, 6, and 8 hours after removal of the wound insert using the Celigo^{®} Imaging Cytometry System (Cyntellect). The surface area of the wound at each time point was measured using ImageJ (National Institutes of Health). The data is reported as the percentage of migration (% Migration) covering the wound area.

### 3. DATA ANALYSIS

qPCR was performed using an ABI 7900 instrument utilizing SDS2.3 software. The experimental data were viewed and exported in SDS 2.4 software. FlowJo V10 software was used to analyze Flow Cytometry data. ImagJ software was used to quantitate percent cell migration for the assay described in Section 2.7. ImageJ is an open source image processing program developed by the Nation Institute of Health and designed for scientific multidimensional images.

### 4. RESULTS

### 4.1. Characterization of C7 Expression

The overall objectives of this evaluation were to increase copy number and protein expression through a comparison of transduction methods and LV vectors. Specific objectives were to:
1. Evaluate the effect of super-transduction on integrated copy number
2. Evaluate the effect of spinoculation on integrated copy number
3. Compare copy number values between INXN-2002 and INXN-2004 under super-transduced and spinoculated conditions.

Two GMP lots of LV-COL7 constructs were used for these studies: the INXN-2002 and the construct INXN-2004 as described in this Example.

### 4.1.1. LV-COL7 Copy Number

GM-HDF training run cells were obtained and copy number was determined as described above in Section 2.1. Table 46 below provides the copy number/cell from Training Runs utilizing the new transduction methods. Initial optimization implemented the addition of centrifugation during the transduction step ("spinoculation"). Standard transduction had been used previously with INXN-2002 and resulted in integrated copy numbers ranging from 0.013 to 0.025. Addition of only the spinoculation step for transduction with INXN-2002 resulted in a ≥2-fold increase in the integrated copy number to 0.05 per cell (TR11 Arm B, Table 46) compared to TR8 results from previous studies.

To further enhance the integrated copy number, a second transduction ("super-transduction") was added to the GM-HDF production process. Addition of super-transduction, also utilizing spinoculation, further increased integrated copy numbers of INXN-2002 by approximately 2-fold (TR11 Arm A, Table 46).

Small-scale research studies (not shown) demonstrated a second-generation INXN-2004 LV-COL7 construct, which utilizes an alternative 3^{rd}-generation self-inactivating LV backbone, had the potential to achieve higher integrated copy numbers, resulting in higher expression levels, while utilizing a lower multiplicity of infection (MOI) than used for INXN-2002. When used at GMP-scale with the optimized transduction procedure (spinoculations with super-transduction), INXN-2004 had further improved integrated LV copy numbers of approximately 4- to 8-fold over the INXN-2002 construct to achieve average copy numbers of 0.41 to 0.74 (TR12.1 and TR13, respectively, Table 46).

Of note, Arms B of TR12.1 and TR13 were not tested for copy numbers as they were not passaged through the entire production process (Table 44). Accurate copy number assessments require cells that have been passaged several times following transduction to ensure that episomal copies of LV, which can artificially inflate measured copy numbers, no longer remain.

**Table 46: LV-COL7 Copy Number in GM-HDF Production Run Cells**

| **Production Run** | **LV-COL7 Used** | **Arm** | **MOI (IU/cell)** | **Super-transduction¹** | **Gene Copy per Cell** |
|---|---|---|---|---|---|
| TR8² | INXN-2002 (pilot) | A | 3.4 | No | 0.021 ±0.005 |
| TR11 | INXN-2002 (GMP) | A | 0.77 | Yes | 0.09±0.03 |
| | | B | 0.77 | No | 0.05±0.01 |
| | | Control³ | n/a | No | BLQ⁴ |
| TR12.1⁵ | INXN-2004 (GMP) | A | 0.12 | Yes | 0.41±0.19 |
| TR13⁵ | INXN-2004 (GMP) | A | 0.12 | Yes | 0.74±0.10 |

| | | | | | |
|---|---|---|---|---|---|
| ¹ Only indicated arms were super-transduced; all TR11, TR12.1, and TR13 arms were spinoculated ² TR8 Arm A data is presented as a comparator for previous results presented in RDEB-ADSO-2 ³ Control arm was mock-transduced and was not exposed to LV-COL7 ⁴ BLQ: below lower limit of quantitation ⁵ Arm B for both TR12.1 and TR13 was not carried through the full production process and not tested for LV-COL7 copy numbers | | | | | |

### 4.1.2. C7 Protein Expression

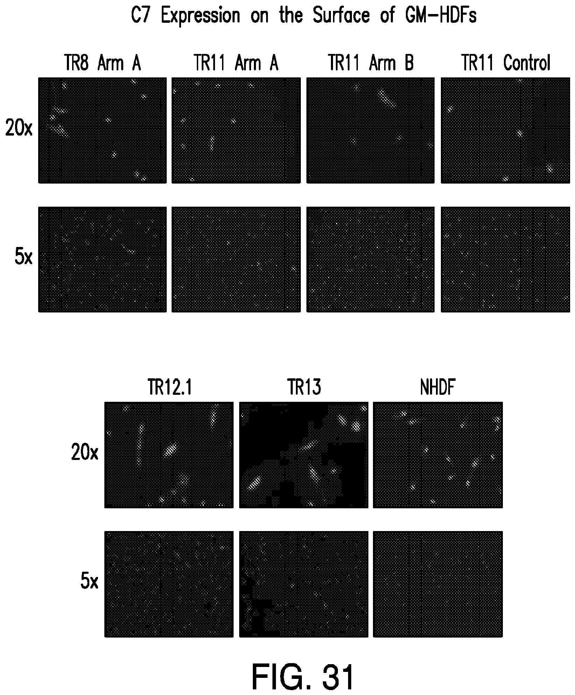

Indirect immunofluorescence (IF) was used to examine C7 protein expression by GM-HDF cells using an antibody specific for the C7. TR11 Arms A & B, and only Arm A of TR12.1 and TR13 were analyzed by IF. Normal human dermal fibroblasts (NHDFs) were used as a positive control and the control arm of TR11 was used as a negative control. Representative images at two magnifications, 20x and 5x are shown below in Figure 31. Also included as a comparator for the original process are IF images of cells from TR8 Arm A which had integrated LV-COL7 copy numbers of 0.015 per cell. Consistent with the copy number of integrated LV presented in Table 46 above, only a small number of TR11 GM-HDFs were visualized with C7 antibodies, though still greater than the number of C7-positive cells from TR8. Furthermore, the higher copy numbers measured from TR12.1 and TR13 GM-HDFs correlate to higher numbers of cells positive for C7 detection. Of note, the signal from the GM-HDFs that are C7-positive is brighter than the signal from NHDFs, suggesting greater amounts of C7 produced by GM-HDFs per cell.

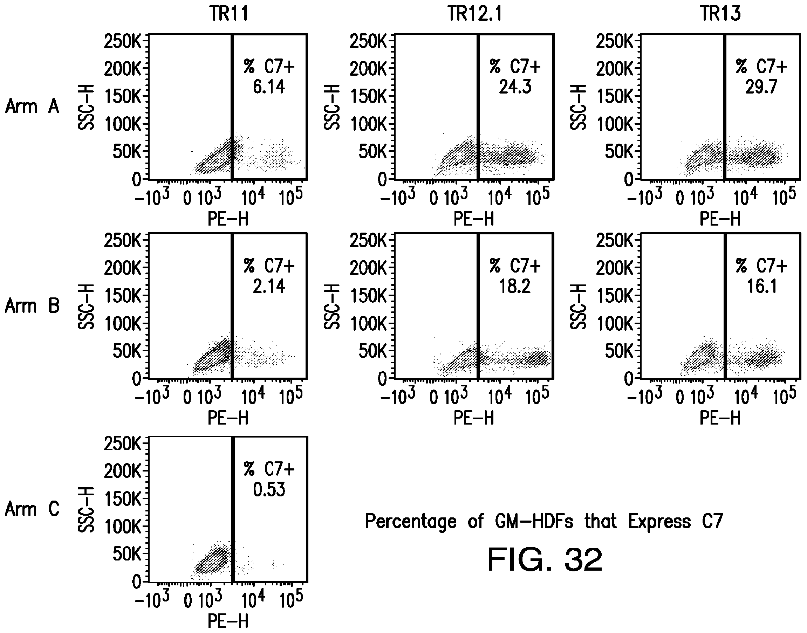

The specific number of cells expressing C7 was then quantified using a flow cytometry assay. As described in Section 2.3, this assay utilized an NC1 region-specific polyclonal antibody. For this experiment, Arm B cells from TR12.1 and TR13 were included to compare the single transduction procedure (Arm B) with the super-transduction procedure (Arm A) using second-generation LV-COL7 construct (INXN-2004). As shown in Figure 32 below, the percentage of C7-positive (C7+) cells for each training run arm is consistent with the qualitative number of cells expressing C7 as detected by IF (Figure 32), and correlates to the measured copy number (Table 46). Comparison of C7+ cells between Arm A and Arm B for TR12.1 and TR13 confirms the near doubling of expression levels that would be predicted by the addition of a second transduction.

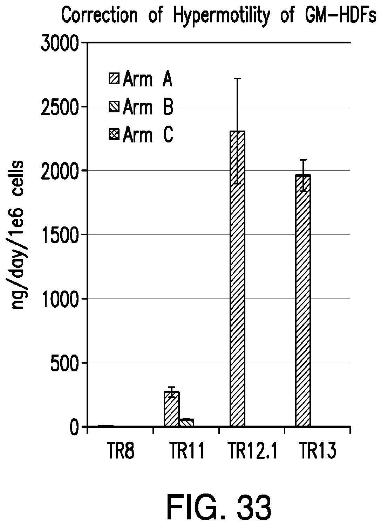

A direct ELISA was used to measure C7 secreted by GM-HDFs into the cell culture media. This assay, as described in Section 2.4, also utilized the NC1 region-specific polyclonal antibody. ELISA assay results of the GM-HDFs from the three training runs show that C7 expression is dependent on the level of integrated LV copy numbers with expression levels reaching approximately 2300 ng/day/1e6 cells (Figure 33). Compared to TR8 Arm A as a representative result from previous studies (RDEB-ADSO-2), this represents a >200-fold increase in C7 expression (Figure 33).

### 4.2. Functional Assessment of C7 Expressed by GM-HDFs

### 4.2.1. Formation of C7 Trimers

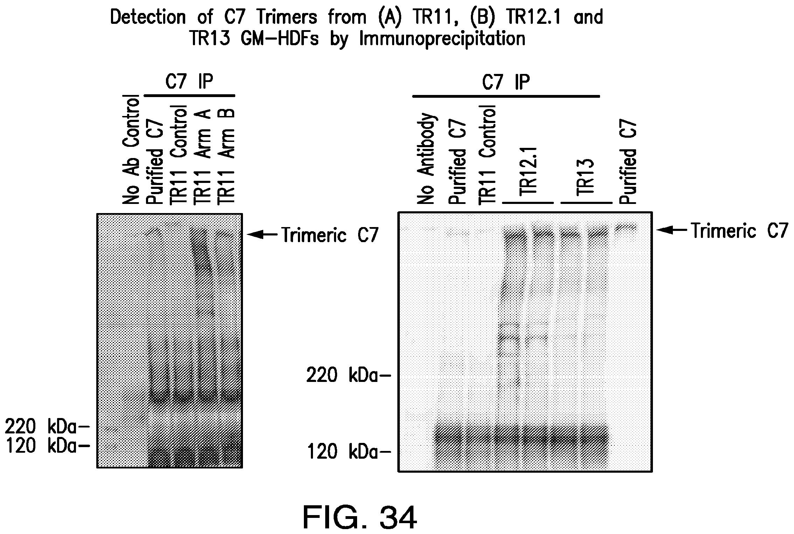

The formation of C7 trimers is essential in the assembly of anchoring fibrils (Bruckner-Tuderman, 1999). To verify that the C7 expressed by GM-HDFs will be capable of forming anchoring fibrils, GM-HDFs were assessed for the formation of C7 trimers using immunoprecipitation (IP) with an anti-C7 specific antibody (fNC1) for selective capture, then concentration of C7 from culture supernatants for detection by SDS-PAGE/immunoblot. Purified recombinant C7 was used as a positive control and the capture step without C7-specific antibody was used as a negative control. IP results for the three training runs showed that the C7 produced by the GM-HDFs was predominantly trimeric (Figure 34) with some lower molecular weight species showing immunoreactivity present, likely the dimeric and monomeric forms of C7.

### 4.2.2. Lam332 Binding by C7 Expressed from GM-HDFs

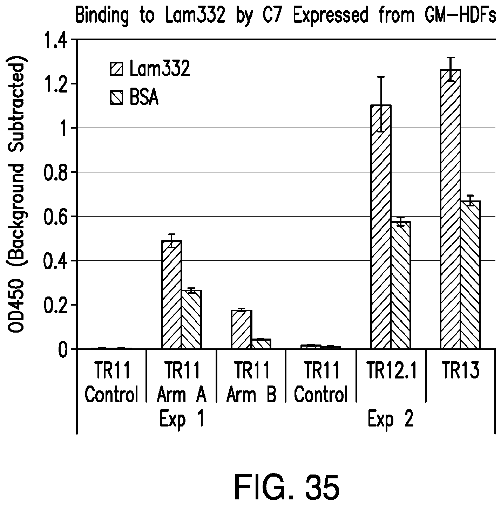

C7 has been shown to bind immobilized extracellular matrix (ECM) components, including fibronectin, Laminin 332 (Lam332), COL1, and COL4 (Chen, et al., 2002a). The interaction between C7 and Lam332 occurs through the NH2-terminal NC1 domain of C7 and is dependent upon the native conformation of both Lam332 and C7 NC1 (Rousselle, et al., 1997). The association between C7 and Lam332 is important for establishing correct Lam332 architecture at the dermal-epidermal junction. Such organization is critical for interactions with extracellular ligands and cell surface receptors, and for cell signaling (Waterman, et al., 2007). Intrexon developed an ELISA using an antibody against the C7 NC1 domain to detect binding of C7 to purified Lam332. Results from two experiments, presented in Figure 35, show copy number-dependent binding to Lam332 by C7 expressed by GM-HDFs from the three Training runs. TR11 Control cells were used as a negative control in both experiments.

### 4.2.3. Cell Migration Assay

Previous studies have shown that RDEB fibroblasts and keratinocytes show an increase in motility relative to their normal counterparts, and that normal motility can be restored by expression of C7 (Chen, et al., 2000; Chen, et al., 2002b; Cogan, et al. 2014; Baldeschi et al., 2003). Most of these studies employed either the colloidal gold salt migration assay to measure the migration of fibroblasts and keratinocytes, or a wound-healing assay to measure the migration of keratinocytes. In this Example a CytoSelect^{™} 24-well wound-healing assay kit (Cell Biolabs, Inc.) was used to measure the motility of NHDFs and GM-HDFs.

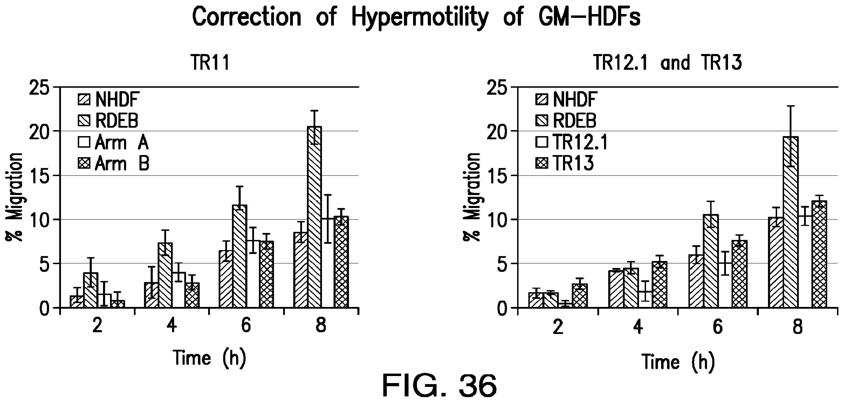

To determine whether the presence of C7 in the culture media is sufficient to reverse the RDEB hyper-motility phenotype, the wound healing assay was carried out on GM-HDFs from the three training runs, with NHDFs as a positive control and the mock-transduced Control (RDEB) arms as the negative control. Control fibroblasts from TR11 Arm C (RDEB) exhibited a hyper-motility phenotype that is reversed by the expression of C7 (Figure 36). Interestingly, the levels of phenotype reversal was not dependent upon the integrated copy number/C7 expression levels, suggesting that even low levels of C7 expression are enough to reverse the hyper-motility phenotype.

### 5. CONCLUSIONS

The integrated transgene copy number per cell in GM-HDFs was improved by both the addition of enhanced transduction methods (spinoculation and super-transduction) and a change to the LV-COL7 construct INXN-2004, with levels as high as 0.74 copies per cell achieved. The fold-improvements achieved with each process change are as follows:
- The addition of spinoculation: ≥2-fold
- The addition of super-transduction (with spinoculation): -2-fold
- The change from INXN-2002 to INXN-2004: >4-fold
- The total cumulative change from original process: average of >27-fold (TR12.1 and TR13 compared to TR8 used in original studies)

Integrated copy number-dependent C7 expression from the GM-HDF cells was confirmed by immunofluorescence staining, flow cytometry, and ELISA. A >200-fold improvement in C7 expression was achieved relative to TR8 results presented in IND submission 16582 Serial 0000.

The structure of the C7 expressed by the GM-HDF cells was confirmed to be predominantly trimeric by immunoprecipitation/SDS-PAGE/immunoblot analysis.

In addition, the C7 produced from the GM-HDF cells was confirmed to be functional with binding to Laminin332 *in vitro* and by correction of the hypermotility phenotype of control RDEB cells in an *in vitro* migration assay.

### APPENDIX 4

COL7A1 ATGACGCTGCGGCTTCTGGTGGCCGCGCTCTGCGCCGGGATCCTGGCAGAGGCGCCCCGA 60
Genbank ATGACGCTGCGGCTTCTGGTGGCCGCGCTCTGCGCCGGGATCCTGGCAGAGGCGCCCCG 60
************************************************************

COL7A1 GTGCGAGCCCAGCACAGGGAGAGAGTGACCTGCACGCGCCTTTACGCCGCTGACATTGTG 120
Genbank GTGCGAGCCCAGCACAGGGAGAGAGTGACCTGCACGCGCCTTTACGCCGCTGACATTGTG 120
************************************************************

COL7A1 TTCTTACTGGATGGCTCCTCATCCATTGGCCGCAGCAATTTCCGCGAGGTCCGCAGCTTT 180
Genbank TTCTTACTGGATGGCTCCTCATCCATTGGCCGCAGCAATTTCCGCGAGGTCCGCAGCTTT 180
************************************************************

COL7A1 CTCGAAGGGCTGGTGCTGCCTTTCTCTGGAGCAGCCAGTGCACAGGGTGTGCGCTTTGCC 240
Genbank CTCGAAGGGCTGGTGCTGCCTTTCTCTGGAGCAGCCAGTGCACAGGGTGTGCGCTTTGCC 240
************************************************************

COL7A1 CAGTGCAGTACAGCGATGACCCACGGACAGAGTTCGGCCTGGATGCACTTGGCTCTGGG 300
Genbank ACAGTGCAGTACAGCGATGACCCACGGACAGAGTTCGGCCTGGATGCACTTGGCTCTGGG 300
************************************************************

COL7A1 GGTGATGTGATCCGCGCCATCCGTGAGCTTAGCTACAAGGGGGGCAACACTCGCACAGGG 360
Genbank GGTGATGTGATCCGCGCCATCCGTGAGCTTAGCTACAAGGGGGGCAACACTCGCACAGGG 360
************************************************************

COL7A1 GCTGCAATTCTCCATGTGGCTGACCATGTCTTCCTGCCCCAGCTGGCCCGACCTGGTGTC 420
Genbank GCTGCAATTCTCCATGTGGCTGACCATGTCTTCCTGCCCCAGCTGGCCCGACCTGGTGTC 420
************************************************************

COL7A1 CCCAAGGTCTGCATCCTGATCACAGACGGGAAGTCCCAGGACCTGGTGGACACAGCTGCC 480
Genbank CCCAAGGTCTGCATCCTGATCACAGACGGGAAGTCCCAGGACCTGGTGGACACAGCTGCC 480
************************************************************

COL7A1 CAAAGGCTGAAGGGGCAGGGGGTCAAGCTATTTGCTGTGGGGATCAAGAATGCTGACCCT 540
Genbank CAAAGGCTGAAGGGGCAGGGGGTCAAGCTATTTGCTGTGGGGATCAAGAATGCTGACCCT 540
************************************************************

COL7A1 GAGGAGCTGAAGCGAGTTGCCTCACAGCCCACCAGTGACTTCTTCTTCTTCGTCAATGAC 600
Genbank GAGGAGCTGAAGCGAGTTGCCTCACAGCCCACCAGTGACTTCTTCTTCTTCGTCAATGAC 600
************************************************************

COL7A1 TTCAGCATCTTGAGGACACTACTGCCCCTCGTTTCCCGGAGAGTGTGCACGACTGCTGGT 660
Genbank TTCAGCATCTTGAGGACACTACTGCCCCTCGTTTCCCGGAGAGTGTGCACGACTGCTGGT 660
************************************************************

COL7A1 GGCGTGCCTGTGACCCGACCTCCGGATGACTCGACCTCTGCTCCACGAGACCTGGTGCTG 720
Genbank GGCGTGCCTGTGACCCGACCTCCGGATGACTCGACCTCTGCTCCACGAGACCTGGTGCTG 720
************************************************************

COL7A1 TCTGAGCCAAGCAGCCAATCCTTGAGAGTACAGTGGACAGCGGCCAGTGGCCCTGTGACT 780
Genbank TCTGAGCCAAGCAGCCAATCCTTGAGAGTACAGTGGACAGCGGCCAGTGGCCCTGTGACT 780
************************************************************

COL7A1 GGCTACAAGGTCCAGTACACTCCTCTGACGGGGCTGGGACAGCCACTGCCGAGTGAGCGG 840
Genbank GGCTACAAGGTCCAGTACACTCCTCTGACGGGGCTGGGACAGCCACTGCCGAGTGAGCGG 840
************************************************************

COL7A1 CAGGAGGTGAACGTCCCAGCTGGTGAGACCAGTGTGCGGCTGCGGGGTCTCCGGCCACTG 900
Genbank CAGGAGGTGAACGTCCCAGCTGGTGAGACCAGTGTGCGGCTGCGGGGTCTCCGGCCACTG 900
************************************************************

COL7A1         ACCGAGTACCAAGTGACTGTGATTGCCCTCTACGCCAACAGCATCGGGGAGGCTGTGAGC 960
Genbank         ACCGAGTACCAAGTGACTGTGATTGCCCTCTACGCCAACAGCATCGGGGAGGCTGTGAGC 960
                ************************************************************

COL7A1         GGGACAGCTCGGACCACTGCCCTAGAAGGGCCGGAACTGACCATCCAGAATACCACAGCC 1020
Genbank         GGGACAGCTCGGACCACTGCCCTAGAAGGGCCGGAACTGACCATCCAGAATACCACAGCC 1020
                ************************************************************

COL7A1         CACAGCCTCCTGGTGGCCTGGCGGAGTGTGCCAGGTGCCACTGGCTACCGTGTGACATGG 1080
Genbank         CACAGCCTCCTGGTGGCCTGGCGGAGTGTGCCAGGTGCCACTGGCTACCGTGTGACATGG 1080
                ************************************************************

COL7A1         CGGGTCCTCAGTGGTGGGCCCACACAGCAGCAGGAGCTGGGCCCTGGCAGGGTTCAGTG 1140
Genbank         CGGGTCCTCAGTGGTGGGCCCACACAGCAGCAGGAGCTGGGCCCTGGCAGGGTTCAGTG 1140
                ************************************************************

COL7A1         TTGCTGCGTGACTTGGAGCCTGGCACGGACTATGAGGTGACCGTGAGCACCCTATTTGGC 1200
Genbank         TTGCTGCGTGACTTGGAGCCTGGCACGGACTATGAGGTGACCGTGAGCACCCTATTTGGC 1200
                ************************************************************

COL7A1         CGCAGTGTGGGGCCCGCCACTTCCCTGATGGCTCGCACTGACGCTTCTGTTGAGCAGACC 1260
Genbank         CGCAGTGTGGGGCCCGCCACTTCCCTGATGGCTCGCACTGACGCTTCTGTTGAGCAGACC 1260
                ************************************************************

COL7A1         CTGCGCCCGGTCATCCTGGGCCCCACATCCATCCTCCTTTCCTGGAACTTGGTGCCTGAG 1320
Genbank         CTGCGCCCGGTCATCCTGGGCCCCACATCCATCCTCCTTTCCTGGAACTTGGTGCCTGAG 1320
                ************************************************************

COL7A1         GCCCGTGGCTACCGGTTGGAATGGCGGCGTGAGACTGGCTTGGAGCCACCGCAGAAGGTG 1380
Genbank         GCCCGTGGCTACCGGTTGGAATGGCGGCGTGAGACTGGCTTGGAGCCACCGCAGAAGGTG 1380
                ************************************************************

COL7A1         GTACTGCCCTCTGATGTGACCCGCTACCAGTTGGATGGGCTGCAGCCGGGCACTGAGTAC 1440
Genbank         GTACTGCCCTCTGATGTGACCCGCTACCAGTTGGATGGGCTGCAGCCGGGCACTGAGTAC 1440
                ************************************************************

COL7A1         CGCCTCACACTCTACACTCTGCTGGAGGGCCACGAGGTGGCCACCCCTGCAACCGTGGTT 1500
Genbank         CGCCTCACACTCTACACTCTGCTGGAGGGCCACGAGGTGGCCACCCCTGCAACCGTGGTT 1500
                ************************************************************

COL7A1         CCCACTGGACCAGAGCTGCCTGTGAGCCCTGTAACAGACCTGCAAGCCACCGAGCTGCCC 1560
Genbank         CCCACTGGACCAGAGCTGCCTGTGAGCCCTGTAACAGACCTGCAAGCCACCGAGCTGCCC 1560
                ************************************************************

COL7A1         GGGCAGCGGGTGCGAGTGTCCTGGAGCCCAGTCCCTGGTGCCACCCAGTACCGCATCATT 1620
Genbank         GGGCAGCGGGTGCGAGTGTCCTGGAGCCCAGTCCCTGGTGCCACCCAGTACCGCATCATT 1620
                ************************************************************

COL7A1         GTGCGCAGCACCCAGGGGGTTGAGCGGACCCTGGTGCTTCCTGGGAGTCAGACAGCATTC 1680
Genbank         GTGCGCAGCACCCAGGGGGTTGAGCGGACCCTGGTGCTTCCTGGGAGTCAGACAGCATTC 1680
                ************************************************************

COL7A1         GACTTGGATGACGTTCAGGCTGGGCTTAGCTACACTGTGCGGGTGTCTGCTCGAGTGGGT 1740
Genbank         GACTTGGATGACGTTCAGGCTGGGCTTAGCTACACTGTGCGGGTGTCTGCTCGAGTGGGT 1740
                ************************************************************

COL7A1         CCCCGTGAGGGCAGTGCCAGTGTCCTCACTGTCCGCCGGGAGCCGGAAACTCCACTTGCT 1800
Genbank         CCCCGTGAGGGCAGTGCCAGTGTCCTCACTGTCCGCCGGGAGCCGGAAACTCCACTTGCT 1800
                ************************************************************

COL7A1         GTTCCAGGGCTGCGGGTTGTGGTGTCAGATGCAACGCGAGTGAGGGTGGCCTGGGGACCC 1860
Genbank         GTTCCAGGGCTGCGGGTTGTGGTGTCAGATGCAACGCGAGTGAGGGTGGCCTGGGGACCC 1860
                ************************************************************

COL7A1      GTCCCTGGAGCCAGTGGATTTCGGATTAGCTGGAGCACAGGCAGTGGTCCGGAGTCCAGC 1920
Genbank     GTCCCTGGAGCCAGTGGATTTCGGATTAGCTGGAGCACAGGCAGTGGTCCGGAGTCCAGC 1920
            ************************************************************

COL7A1      CAGACACTGCCCCCAGACTCTACTGCCACAGACATCACAGGGCTGCAGCCTGGAACCACC 1980
Genbank     CAGACACTGCCCCCAGACTCTACTGCCACAGACATCACAGGGCTGCAGCCTGGAACCACC 1980
            ************************************************************

COL7A1      TACCAGGTGGCTGTGTCGGTACTGCGAGGCAGAGAGGAGGGCCCTGCTGCAGTCATCGTG 2040
Genbank     TACCAGGTGGCTGTGTCGGTACTGCGAGGCAGAGAGGAGGGCCCTGCTGCAGTCATCGTG 2040
            ************************************************************

COL7A1      GCTCGAACGGACCCACTGGGCCCAGTGAGGACGGTCCATGTGACTCAGGCCAGCAGCTCA 2100
Genbank     GCTCGAACGGACCCACTGGGCCCAGTGAGGACGGTCCATGTGACTCAGGCCAGCAGCTCA 2100
            ************************************************************

COL7A1      TCTGTCACCATTACCTGGACCAGGGTTCCTGGCGCCACAGGATACAGGGTTTCCTGGCAC 2160
Genbank     TCTGTCACCATTACCTGGACCAGGGTTCCTGGCGCCACAGGATACAGGGTTTCCTGGCAC 2160
            ************************************************************

COL7A1      TCAGCCCACGGCCCAGAGAAATCCCAGTTGGTTTCTGGGGAGGCCACGGTGGCTGAGCTG 2220
Genbank     TCAGCCCACGGCCCAGAGAAATCCCAGTTGGTTTCTGGGGAGGCCACGGTGGCTGAGCTG 2220
            ************************************************************

COL7A1      GATGGACTGGAGCCAGATACTGAGTATACGGTGCATGTGAGGGCCCATGTGGCTGGCGTG 2280
Genbank     GATGGACTGGAGCCAGATACTGAGTATACGGTGCATGTGAGGGCCCATGTGGCTGGCGTG 2280
            ************************************************************

COL7A1      GATGGGCCCCCTGCCTCTGTGGTTGTGAGGACTGCCCCTGAGCCTGTGGGTCGTGTGTCG 2340
Genbank     GATGGGCCCCCTGCCTCTGTGGTTGTGAGGACTGCCCCTGAGCCTGTGGGTCGTGTGTCG 2340
            ************************************************************

COL7A1      AGGCTGCAGATCCTCAATGCTTCCAGCGACGTTCTACGGATCACCTGGGTAGGGGTCACT 2400
Genbank     AGGCTGCAGATCCTCAATGCTTCCAGCGACGTTCTACGGATCACCTGGGTAGGGGTCACT 2400
            ************************************************************

COL7A1      GGAGCCACAGCTTACAGACTGGCCTGGGGCCGGAGTGAAGGCGGCCCCATGAGGCACCAG 2460
Genbank     GGAGCCACAGCTTACAGACTGGCCTGGGGCCGGAGTGAAGGCGGCCCCATGAGGCACCAG 2460
            ************************************************************

COL7A1      ATACTCCCAGGAAACACAGACTCTGCAGAGATCCGGGGTCTCGAAGGTGGAGTCAGCTAC 2520
Genbank     ATACTCCCAGGAAACACAGACTCTGCAGAGATCCGGGGTCTCGAAGGTGGAGTCAGCTAC 2520
            ************************************************************

COL7A1      TCAGTGCGAGTGACTGCACTTGTCGGGGACCGCGAGGGCACACCTGTCTCCATTGTTGTC 2580
Genbank     TCAGTGCGAGTGACTGCACTTGTCGGGGACCGCGAGGGCACACCTGTCTCCATTGTTGTC 2580
            ************************************************************

COL7A1      CTACGCCGCCTGAGGCTCCGCCAGCCCTGGGGACGCTTCACGTGGTGCAGCGCGGGGAG 2640
Genbank     ACTACGCCGCCTGAGGCTCCGCCAGCCCTGGGGACGCTTCACGTGGTGCAGCGCGGGGAG 2640
            ************************************************************

COL7A1      CACTCGCTGAGGCTGCGCTGGGAGCCGGTGCCCAGAGCGCAGGGCTTCCTTCTGCACTGG 2700
Genbank     CACTCGCTGAGGCTGCGCTGGGAGCCGGTGCCCAGAGCGCAGGGCTTCCTTCTGCACTGG 2700
            ************************************************************

COL7A1      CAACCTGAGGGTGGCCAGGAACAGTCCCGGGTCCTGGGGCCCGAGCTCAGCAGCTATCAC 2760
Genbank     CAACCTGAGGGTGGCCAGGAACAGTCCCGGGTCCTGGGGCCCGAGCTCAGCAGCTATCAC 2760
            ************************************************************

COL7A1      CTGGACGGGCTGGAGCCAGCGACACAGTACCGCGTGAGGCTGAGTGTCCTAGGGCC[?]GCT 2820
Genbank     CTGGACGGGCTGGAGCCAGCGACACAGTACCGCGTGAGGCTGAGTGTCCTAGGGCC[?]GCT 2820

*********************************************************.***

COL7A1 GGAGAAGGGCCCTCTGCAGAGGTGACTGCGCACTGAGTCACCTCGTGTTCCAAGCATT 2880
Genbank GGAGAAGGGCCCTCTGCAGAGGTGACTGCGCGCACTGAGTCACCTCGTGTTCCAAGCATT 2880
************************************************************

COL7A1 GAACTACGTGTGGTGGACACCTCGATCGACTCGGTGACTTTGGCCTGGACTCCAGTGTCC 2940
Genbank GAACTACGTGTGGTGGACACCTCGATCGACTCGGTGACTTTGGCCTGGACTCCAGTGTCC 2940
************************************************************

COL7A1 GGGCATCCAGCTACATCCTATCCTGGCGGCCACTCAGAGGCCCTGGCCAGGAAGTGCCT 3000
Genbank AGGGCATCCAGCTACATCCTATCCTGGCGGCCACTCAGAGGCCCTGGCCAGGAAGTGCCT 3000
************************************************************

COL7A1 GGGTCCCCGCAGACACTTCCAGGGATCTCAAGCTCCCAGCGGGTGACAGGGCTAGAGCCT 3060
Genbank GGGTCCCCGCAGACACTTCCAGGATCTCAAGCTCCCAGCGGGTGACAGGGCTAGAGCCT 3060
************************************************************

COL7A1 GGCGTCTCTTACATCTTCTCCCTGACGCCTGTCCTGGATGGTGTGCGGGGTCCTGAGGCA 3120
Genbank GGCGTCTCTTACATCTTCTCCCTGACGCCTGTCCTGGATGGTGTGCGGGGTCCTGAGGCA 3120
************************************************************

COL7A1 TCTGTCACACAGACGCCAGTGTGCCCCCGTGGCCTGGCGGATGTGGTGTTCCTACCACAT 3180
Genbank TCTGTCACACAGACGCCAGTGTGCCCCCGTGGCCTGGCGGATGTGGTGTTCCTACCACAT 3180
************************************************************

COL7A1 GCCACTCAAGACAATGCTCACCGTGCGGAGGCTACGAGGAGGGTCCTGGAGCGTCTGGTG 3240
Genbank GCCACTCAAGACAATGCTCACCGTGCGGAGGCTACGAGGAGGGTCCTGGAGCGTCTGGTG 3240
************************************************************

COL7A1 TTGGCACTTGGGCCTCTTGGGCCACAGGCAGTTCAGGTTGGCCTGCTGTCTTACAGTCAT 3300
Genbank TTGGCACTTGGGCCTCTTGGGCCACAGGCAGTTCAGGTTGGCCTGCTGTCTTACAGTCAT 3300
************************************************************

COL7A1 CGGCCCTCCCCACTGTTCCCACTGAATGGCTCCCATGACCTTGGCATTATCTTGCAAAGG 3360
Genbank CGGCCCTCCCCACTGTTCCCACTGAATGGCTCCCATGACCTTGGCATTATCTTGCAAAGG 3360
************************************************************

COL7A1 TCCGTGACATGCCCTACATGGACCCAAGTGGAACAACCTGGGCACAGCCGTGGTCACA 3420
Genbank ATCCGTGACATGCCCTACATGGACCCAAGTGGAACAACCTGGGCACAGCCGTGGTCAC 3420
************************************************************

COL7A1 GCTCACAGATACATGTTGGCACCAGATGCTCCTGGGCGCCGCCAGCACGTACCAGGGGTG 3480
Genbank GCTCACAGATACATGTTGGCACCAGATGCTCCTGGGCGCCGCCAGCACGTACCAGGGGTG 3480
************************************************************

COL7A1 ATGGTTCTGCTAGTGGATGAACCCTTGAGAGGTGACATATTCAGCCCCATCCGTGAGGCC 3540
Genbank ATGGTTCTGCTAGTGGATGAACCCTTGAGAGGTGACATATTCAGCCCCATCCGTGAGGCC 3540
************************************************************

COL7A1 CAGGCTTCTGGGCTTAATGTGGTGATGTTGGAATGGCTGGAGCGGACCCAGAGCAGCTG 3600
Genbank CAGGCTTCTGGGCTTAATGTGGTGATGTTGGAATGGCTGGAGCGGACCCAGAGCAGCTG 3600
************************************************************

COL7A1 CGTCGCTTGGCGCCGGGTATGGACTCTGTCCAGACCTTCTTCGCCGTGGATGATGGGCCA 3660
Genbank CGTCGCTTGGCGCCGGGTATGGACTCTGTCCAGACCTTCTTCGCCGTGGATGATGGGCCA 3660
************************************************************

COL7A1 GCCTGGACCAGGCAGTCAGTGGTCTGGCCACAGCCCTGTGTCAGGCATCCTTCACTACT 3720
Genbank AGCCTGGACCAGGCAGTCAGTGGTCTGGCCACAGCCCTGTGTCAGGCATCCTTCACTACT 3720
************************************************************

COL7A1 CAGCCCGGCCAGAGCCCTGCCCAGTGTATTGTCCAAAGGCCAGAAGGGGAACCTGGA 3780
Genbank CAGCCCGGCCAGAGCCCTGCCCAGTGTATTGTCCAAAGGGCCAGAAGGGGGAACCTGG 3780
************************************************************

COL7A1 GAGATGGGCCTGAGAGGACAAGTTGGGCCTCCTGGCGACCCTGGCCTCCCGGGCAGGACC 3840
Genbank GAGATGGGCCTGAGAGGACAAGTTGGGCCTCCTGGCGACCCTGGCCTCCCGGGCAGGACC 3840
************************************************************

COL7A1 GGTGCTCCCGGCCCCCAGGGGCCCCCTGGAAGTGCCACTGCCAAGGGCGAGAGGGGCTTC 3900
Genbank GGTGCTCCCGGCCCCCAGGGGCCCCCTGGAAGTGCCACTGCCAAGGGCGAGAGGGGCTTC 3900
************************************************************

COL7A1 CCTGGAGCAGATGGGCGTCCAGGCAGCCCTGGCCGCGCCGGGAATCCTGGGACCCCTGGA 3960
Genbank CCTGGAGCAGATGGGCGTCCAGGCAGCCCTGGCCGCGCCGGGAATCCTGGGACCCCTGGA 3960
************************************************************

COL7A1 GCCCCTGGCCTAAAGGGCTCTCCAGGGTTGCCTGGCCCTCGTGGGACCCGGGAGAGCGA 4020
Genbank GCCCCTGGCCTAAAGGGCTCTCCAGGGTTGCCTGGCCCTCGTGGGGACCCGGGAGAGCGA 4020
************************************************************

COL7A1 GGACCTCGAGGCCCAAAGGGGGAGCCGGGGGCTCCCGGACAAGTCATCGGAGGTGAAGGA 4080
Genbank GGACCTCGAGGCCCAAAGGGGGAGCCGGGGGCTCCCGGACAAGTCATCGGAGGTGAAGGA 4080
************************************************************

COL71 CCTGGGCTTCCTGGGCGGAAAGGGGACCCTGGACCATCGGGCCCCCCTGGACCTCGTGGA 4140
Genbank CCTGGGCTTCCTGGCGGAAAGGGGACCCTGGACCATCGGGCCCCCCTGGACCTCGTGGA 4140
************************************************************

COL7A1 CCACTGGGGGACCCAGGACCCCGTGGCCCCCCAGGGCTTCCTGGAACAGCCATGAAGGGT 4200
Genbank CCACTGGGGGACCCAGGACCCCGTGGCCCCCCAGGGCTTCCTGGAACAGCCATGAAGGGT 4200
************************************************************

COL7A1 GACAAAGGCGATCGTGGGGAGCGGGGTCCCCCTGGACCAGGTGAAGGTGGCATTGCTCCT 4260
Genbank GACAAAGGCGATCGTGGGGAGCGGGGTCCCCCTGGACCAGGTGAAGGTGGCATTGCTCCT 4260
************************************************************

COL7A1 GGGGAGCCTGGGCTGCCGGGTCTTCCCGGAAGCCCTGGACCCCAAGGCCCCGTTGGCCCC 4320
Genbank GGGGAGCCTGGGCTGCCGGGTCTTCCCGGAAGCCCTGGACCCCAAGGCCCCGTTGGCCCC 4320
************************************************************

COL7A1 CCTGGAAAGAAAGGAGAAAAAGGTGACTCTGAGGATGGAGCTCCAGGCCTCCCAGGACAA 4380
Genbank CCTGGAAAGAAAGGAGAAAAAGGTGACTCTGAGGATGGAGCTCCAGGCCTCCCAGGACAA 4380
************************************************************

COL7A1 CCTGGGTCTCCGGGTGAGCAGGGCCCACGGGGACCTCCTGGAGCTATTGGCCCCAAAGGT 4440
Genbank CCTGGGTCTCCGGGTGAGCAGGGCCCACGGGGACCTCCTGGAGCTATTGGCCCCAAAGGT 4440
************************************************************

COL7A1 GACCGGGGCTTTCCAGGGCCCCTGGGTGAGGCTGGAGAGAAGGGCGAACGTGGACCCCCA 4500
Genbank GACCGGGGCTTTCCAGGGCCCCTGGGTGAGGCTGGAGAGAAGGGCGAACGTGGACCCCCA 4500
************************************************************

COL7A1 GGCCCAGCGGGATCCCGGGGGCTGCCAGGGGTTGCTGGACGTCCTGGAGCCAAGGGTCCT 4560
Genbank GGCCCAGCGGGATCCCGGGGGCTGCCAGGGGTTGCTGGACGTCCTGGAGCCAAGGGTCCT 4560
************************************************************

COL7A1 GAAGGGCCACCAGGACCCACTGGCCGCCAAGGAGAGAAGGGGGAGCCTGGTCGCCCTGGG 4620
Genbank GAAGGGCCACCAGGACCCACTGGCCGCCAAGGAGAGAAGGGGGAGCCTGGTCGCCCTGGG 4620
************************************************************

COL7A1 GACCCTGCAGTGGTGGGACCTGCTGTTGCTGGACCCAAAGGAGAAAGGAGATGTGGGG 4680
Genbank GACCCTGCAGTGGTGGGACCTGCTGTTGCTGGACCCAAAGGAGAAAGGAGATGTGGGG 4680
************************************************************

COL7A1      CCCGCTGGGCCCAGAGGAGCTACCGGAGTCCAAGGGGAACGGGGCCCACCCGGCTTGGTT 4740
Genbank     CCCGCTGGGCCCAGAGGAGCTACCGGAGTCCAAGGGGAACGGGGCCCACCCGGCTTGGTT 4740
            ************************************************************

COL7A1      CTTCCTGGAGACCCTGGCCCCAAGGGAGACCCTGGAGACCGGGGTCCCATTGGCCTTACT 4800
Genbank     CTTCCTGGAGACCCTGGCCCCAAGGGAGACCCTGGAGACCGGGGTCCCATTGGCCTTACT 4800
            ************************************************************

COL7A1      GGCAGAGCAGGACCCCCAGGTGACTCAGGGCCTCCTGGAGAGAAGGGAGACCCTGGGCGG 4860
Genbank     GGCAGAGCAGGACCCCCAGGTGACTCAGGGCCTCCTGGAGAGAAGGGAGACCCTGGGCGG 4860
            ************************************************************

COL7A1      CCTGGCCCCCCAGGACCTGTTGGCCCCCGAGGACGAGATGGTGAAGTTGGAGAGAAAGGT 4920
Genbank     CCTGGCCCCCCAGGACCTGTTGGCCCCCGAGGACGAGATGGTGAAGTTGGAGAGAAAGGT 4920
            ************************************************************

COL7A1      GACGAGGGTCCTCCGGGTGACCCGGGTTTGCCTGGAAAAGCAGGCGAGCGTGGCCTTCGG 4980
Genbank     GACGAGGGTCCTCCGGGTGACCCGGGTTTGCCTGGAAAAGCAGGCGAGCGTGGCCTTCGG 4980
            ************************************************************

COL7A1      GGGGCACCTGGAGTTCGGGGGCCTGTGGGTGAAAAGGGAGACCAGGGAGATCCTGGAGAG 5040
Genbank     GGGGCACCTGGAGTTCGGGGGCCTGTGGGTGAAAAGGGAGACCAGGGAGATCCTGGAGAG 5040
            ************************************************************

COL7A1      GATGGACGAAATGGCAGCCCTGGATCATCTGGACCCAAGGGTGACCGTGGGGAGCCGGGT 5100
Genbank     GATGGACGAAATGGCAGCCCTGGATCATCTGGACCCAAGGGTGACCGTGGGGAGCCGGGT 5100
            ************************************************************

COL7A1      CCCCCAGGACCCCCGGGACGGCTGGTAGACACAGGACCTGGAGCCAGAGAGAAGGGAGAG 5160
Genbank     CCCCCAGGACCCCCGGGACGGCTGGTAGACACAGGACCTGGAGCCAGAGAGAAGGGAGAG 5160
            ************************************************************

COL7A1      CCTGGGGACCGCGGACAAGAGGGTCCTCGAGGGCCCAAGGGTGATCCTGGCCTCCCTGGA 5220
Genbank     CCTGGGGACCGCGGACAAGAGGGTCCTCGAGGGCCCAAGGGTGATCCTGGCCTCCCTGGA 5220
            ************************************************************

COL7A1      GCCCCTGGGGAAAGGGGCATTGAAGGGTTTCGGGGACCCCCAGGCCCACAGGGGGACCCA 5280
Genbank     GCCCCTGGGGAAAGGGGCATTGAAGGGTTTCGGGGACCCCCAGGCCCACAGGGGGACCCA 5280
            ************************************************************

COL71      GGTGTCCGAGGCCCAGCAGGAGAAAAGGGTGACCGGGGTCCCCCTGGGCTGGATGGCCGG 5340
Genbank     GGTGTCCGAGGCCCAGCAGGAGAAAAGGGTGACCGGGGTCCCCCTGGGCTGGATGGCCGG 5340
            ************************************************************

COL7A1      AGCGGACTGGATGGGAAACCAGGAGCCGCTGGGCCCTCTGGGCCGAATGGTGCTGCAGGC 5400
Genbank     AGCGGACTGGATGGGAAACCAGGAGCCGCTGGGCCCTCTGGGCCGAATGGTGCTGCAGGC 5400
            ************************************************************

COL7A1      AAGCTGGGGACCCAGGGAGAGACGGGCTTCCAGGCCTCCGTGGAGAACA[?]GGCCTCCCT 5460
Genbank     AAAGCTGGGGACCCAGGGAGAGACGGGCTTCCAGGCCTCCGTGGAGAACA[?]GGCCTCCCT 5460
            **************************************************.*********

COL7A1      GGCCCCTCTGGTCCCCCTGGATTACCGGGAAAGCCAGGCGAGGATGG[?]AAACCTGGCCTG 5520
Genbank     GGCCCCTCTGGTCCCCCTGGATTACCGGGAAAGCCAGGCGAGGATGG[?]AAACCTGGCCTG 5520
            *********************************************** ************

COL7A1      AATGGAAAAAACGGAGAACCTGGGGACCCTGGAGAAGACGGGAGGAAGGGAGAGAAAGGA 5580
Genbank     AATGGAAAAAACGGAGAACCTGGGGACCCTGGAGAAGACGGGAGGAAGGGAGAGAAAGGA 5580
            ************************************************************

COL7A1          GATTCAGGCGCCTCTGGGAGAGAAGGTCGTGATGGCCCCAAGGGTGAGCGTGGAGCTCCT 5640
Genbank         GATTCAGGCGCCTCTGGGAGAGAAGGTCGTGATGGCCCCAAGGGTGAGCGTGGAGCTCCT 5640
                ************************************************************

COL7A1          GGTATCCTTGGACCCCAGGGGCCTCCAGGCCTCCCAGGGCCAGTGGGCCCTCCTGGCCAG 5700
Genbank         GGTATCCTTGGACCCCAGGGGCCTCCAGGCCTCCCAGGGCCAGTGGGCCCTCCTGGCCAG 5700
                ************************************************************

COL7A1          GGTTTTCCTGGTGTCCCAGGAGGCACGGGCCCCAAGGGTGACCGTGGGGAGACTGGATCC 5760
Genbank         GGTTTTCCTGGTGTCCCAGGAGGCACGGGCCCCAAGGGTGACCGTGGGGAGACTGGATCC 5760
                ************************************************************

COL7A1          AAAGGGGAGCAGGGCCTCCCTGGAGAGCGTGGCCTGCGAGGAGAGCCTGGAAGTGTGCCG 5820
Genbank         AAAGGGGAGCAGGGCCTCCCTGGAGAGCGTGGCCTGCGAGGAGAGCCTGGAAGTGTGCCG 5820
                ************************************************************

COL7A1          AATGTGGATCGGTTGCTGGAAACTGCTGGCATCAAGGCATCTGCCCTGCGGGAGATCGTG 5880
Genbank         AATGTGGATCGGTTGCTGGAAACTGCTGGCATCAAGGCATCTGCCCTGCGGGAGATCGTG 5880
                ************************************************************

COL7A1          GAGACCTGGGATGAGAGCTCTGGTAGCTTCCTGCCTGTGCCCGAACGGCGTCGAGGCCCC 5940
Genbank         GAGACCTGGGATGAGAGCTCTGGTAGCTTCCTGCCTGTGCCCGAACGGCGTCGAGGCCCC 5940
                ************************************************************

COL7A1          AGGGGGACTCAGGCGAACAGGGCCCCCCAGGCAAGGAGGGCCCCATCGGCTTTCCTGGA 6000
Genbank         AAGGGGGACTCAGGCGAACAGGGCCCCCCAGGCAAGGAGGGCCCCATCGGCTTTCCTGGA 6000
                ************************************************************

COL7A1          GAACGCGGGCTGAAGGGCGACCGTGGAGACCCTGGCCCTCAGGGGCCACCTGGTCTGGCC 6060
Genbank         GAACGCGGGCTGAAGGGCGACCGTGGAGACCCTGGCCCTCAGGGGCCACCTGGTCTGGCC 6060
                ************************************************************

COL7A1          CTTGGGGAGAGGGGCCCCCCCGGGCCTTCCGGCCTTGCCGGGGAGCCTGGAAAGCCTGGT 6120
Genbank         CTTGGGGAGAGGGGCCCCCCCGGGCCTTCCGGCCTTGCCGGGGAGCCTGGAAAGCCTGGT 6120
                ************************************************************

COL71          ATTCCCGGGCTCCCAGGCAGGGCTGGGGGTGTGGGAGAGGCAGGAAGGCCAGGAGAGAGG 6180
Genbank         ATTCCCGGGCTCCCAGGCAGGGCTGGGGGTGTGGGAGAGGCAGGAAGGCCAGGAGAGAGG 6180
                ************************************************************

COL7A1          GGAGAACGGGGAGAGAAAGGAGAACGTGGAGAACAGGGCAGAGATGGCCCTCCTGGACTC 6240
Genbank         GGAGAACGGGGAGAGAAAGGAGAACGTGGAGAACAGGGCAGAGATGGCCCTCCTGGACTC 6240
                ************************************************************

COL7A1          CCTGGAACCCCTGGGCCCCCCGGACCCCCTGGCCCCAAGGTGTCTGTGGATGAGCCAGGT 6300
Genbank         CCTGGAACCCCTGGGCCCCCCGGACCCCCTGGCCCCAAGGTGTCTGTGGATGAGCCAGGT 6300
                ************************************************************

COL7A1          CCTGGACTCTCTGGAGAACAGGGACCCCCTGGACTCAAGGGTGCTAAGGGGGAGCCGGGC 6360
Genbank         CCTGGACTCTCTGGAGAACAGGGACCCCCTGGACTCAAGGGTGCTAAGGGGGAGCCGGGC 6360
                ************************************************************

COL7A1          GCAATGGTGACCAAGGTCCCAAAGGAGACAGGGGTGTGCCAGGCATCAAAGGAGACCGG 6420
Genbank         AGCAATGGTGACCAAGGTCCCAAAGGAGACAGGGGTGTGCCAGGCATCAAAGGAGACCGG 6420
                ************************************************************

COL7A1          GGAGAGCCTGGACCGAGGGGTCAGGACGGCAACCCGGGTCTACCAGGAGAGCGTGGTATG 6480
Genbank         GGAGAGCCTGGACCGAGGGGTCAGGACGGCAACCCGGGTCTACCAGGAGAGCGTGGTATG 6480
                ************************************************************

COL7A1          GCTGGGCCTGAAGGGAAGCCGGGTCTGCAGGGTCCAAGAGGCCCCCCTGGCCCAGTGGGT 6540
Genbank         GCTGGGCCTGAAGGGAAGCCGGGTCTGCAGGGTCCAAGAGGCCCCCCTGGCCCAGTGGGT 6540
                ************************************************************

COL7A1        GGTCATGGAGACCCTGGACCACCTGGTGCCCCGGGTCTTGCTGGCCCTGCAGGACCCCAA 6600
Genbank       GGTCATGGAGACCCTGGACCACCTGGTGCCCCGGGTCTTGCTGGCCCTGCAGGACCCCA 6600
              ************************************************************

COL7A1        GGACCTTCTGGCCTGAAGGGGGAGCCTGGAGAGACAGGACCTCCAGGACGGGGCCTGACT 6660
Genbank       GGACCTTCTGGCCTGAAGGGGGAGCCTGGAGAGACAGGACCTCCAGGACGGGGCCTGACT 6660
              ************************************************************

COL7A1        GGACCTACTGGAGCTGTGGGACTTCCTGGACCCCCCGGCCCTTCAGGCCTTGTGGGTCCA 6720
Genbank       GGACCTACTGGAGCTGTGGGACTTCCTGGACCCCCCGGCCCTTCAGGCCTTGTGGGTCCA 6720
              ************************************************************

COL7A1        CAGGGGTCTCCAGGTTTGCCTGGACAAGTGGGGGAGACAGGGAAGCCGGGAGCCCCAGGT 6780
Genbank       CAGGGGTCTCCAGGTTTGCCTGGACAAGTGGGGGAGACAGGGAAGCCGGGAGCCCCAGGT 6780
              ************************************************************

COL7A1        CGAGATGGTGCCAGTGGAAAAGATGGAGACAGAGGGAGCCCTGGTGTGCCAGGGTCACCA 6840
Genbank       CGAGATGGTGCCAGTGGAAAAGATGGAGACAGAGGGAGCCCTGGTGTGCCAGGGTCACCA 6840
              ************************************************************

COL7A1        GGTCTGCCTGGCCCTGTCGGACCTAAAGGAGAACCTGGCCCCACGGGGGCCCCTGGACAG 6900
Genbank       GGTCTGCCTGGCCCTGTCGGACCTAAAGGAGAACCTGGCCCCACGGGGGCCCCTGGACAG 6900
              ************************************************************

COL7A1        GCTGTGGTCGGGCTCCCTGGAGCAAAGGGAGAGAAGGGAGCCCCTGGAGGCCTTGCTGGA 6960
Genbank       GCTGTGGTCGGGCTCCCTGGAGCAAAGGGAGAGAAGGGAGCCCCTGGAGGCCTTGCTGGA 6960
              ************************************************************

COL7A1        GACCTGGTGGGTGAGCCGGGAGCCAAAGGTGACCGAGGACTGCCAGGGCCGCGAGGCGAG 7020
Genbank       GACCTGGTGGGTGAGCCGGGAGCCAAAGGTGACCGAGGACTGCCAGGGCCGCGAGGCGAG 7020
              ************************************************************

COL7A1        AGGGTGAAGCTGGCCGTGCAGGGGAGCCCGGAGACCCTGGGAAGATGGTCAGAAAGGG 7080
Genbank       AAGGGTGAAGCTGGCCGTGCAGGGGAGCCCGGAGACCCTGGGAAGATGGTCAGAAAGGG 7080
              ************************************************************

COL7A1        GCTCCAGGACCCAAAGGTTTCAAGGGTGACCCAGGAGTCGGGGTCCCGGGCTCCCCTGGG 7140
Genbank       GCTCCAGGACCCAAAGGTTTCAAGGGTGACCCAGGAGTCGGGGTCCCGGGCTCCCCTGGG 7140
              ************************************************************

COL7A1        CCTCCTGGCCCTCCAGGTGTGAAGGGAGATCTGGGCCTCCCTGGCCTGCCCGGTGCTCCT 7200
Genbank       CCTCCTGGCCCTCCAGGTGTGAAGGGAGATCTGGGCCTCCCTGGCCTGCCCGGTGCTCCT 7200
              ************************************************************

COL7A1        GGTGTTGTTGGGTTCCCGGGTCAGACAGGCCCTCGAGGAGAGATGGGTCAGCCAGGCCCT 7260
Genbank       GGTGTTGTTGGGTTCCCGGGTCAGACAGGCCCTCGAGGAGAGATGGGTCAGCCAGGCCCT 7260
              ************************************************************

COL7A1        GTGGAGAGCGGGGTCTGGCAGGCCCCCCAGGGAGAGAAGGAATCCCAGGACCCCTGGGG 7320
Genbank       AGTGGAGAGCGGGGTCTGGCAGGCCCCCAGGGAGAGAAGGAATCCCAGGACCCCTGGGG 7320
              ************************************************************

COL7A1        CCACCTGGACCACCGGGGTCAGTGGGACCACCTGGGGCCTCTGGACTCAAAGGAGACAAG 7380
Genbank       CCACCTGGACCACCGGGGTCAGTGGACCACCTGGGGCCTCTGGACTCAAAGGAGACAAG 7380
              ************************************************************

COL7A1        GGAGACCCTGGAGTAGGGCTGCCTGGGCCCCGAGGCGAGCGTGGGGAGCCAGGCATCCGG 7440
Genbank       GGAGACCCTGGAGTAGGGCTGCCTGGGCCCCGAGGCGAGCGTGGGGAGCCAGGCATCCGG 7440
              ************************************************************

COL7A1        GGTGAAGATGGCCGCCCCGGCCAGGAGGGACCCCGAGGACTCACGGGGCCCCCTGGCAGC 7500
Genbank       GGTGAAGATGGCCGCCCCGGCCAGGAGGGACCCCGAGGACTCACGGGGCCCCCTGGCAGC 7500

************************************************************

COL7A1 AGGGGAGAGCGTGGGGAGAAGGGTGATGTTGGAGTGCAGGACTAAAGGGTGACAAGGGA 7560
Genbank AGGGGAGAGCGTGGGGAGAAGGGTGATGTTGGGAGTGCAGGACTAAAGGGTGACAAGGG 7560
************************************************************

COL7A1 GACTCAGCTGTGATCCTGGGGCCTCCAGGCCCACGGGGTGCCAAGGGGGACATGGGTGAA 7620
Genbank GACTCAGCTGTGATCCTGGGGCCTCCAGGCCCACGGGGTGCCAAGGGGGACATGGGTGAA 7620
************************************************************

COL7A1 CGAGGGCCTCGGGGCTTGGATGGTGACAAAGGACCTCGGGGAGACAATGGGGACCCTGGT 7680
Genbank CGAGGGCCTCGGGGCTTGGATGGTGACAAAGGACCTCGGGGAGACAATGGGGACCCTGGT 7680
************************************************************

COL7A1 GACAAGGGCAGCAAGGGAGAGCCTGGTGACAAGGGCTCAGCCGGGTTGCCAGGACTGCGT 7740
Genbank GACAAGGGCAGCAAGGGAGAGCCTGGTGACAAGGGCTCAGCCGGGTTGCCAGGACTGCGT 7740
************************************************************

COL7A1 GGACTCCTGGGACCCCAGGGTCAACCTGGTGCAGCAGGGATCCCTGGTGACCCGGGATCC 7800
Genbank GGACTCCTGGGACCCCAGGGTCAACCTGGTGCAGCAGGGATCCCTGGTGACCCGGGATCC 7800
************************************************************

COL7A1 CCAGGAAAGGATGGAGTGCCTGGTATCCGAGGAGAAAAAGGAGATGTTGGCTTCATGGGT 7860
Genbank CCAGGAAAGGATGGAGTGCCTGGTATCCGAGGAGAAAAAGGAGATGTTGGCTTCATGGGT 7860
************************************************************

COL7A1 CCCCGGGGCCTCAAGGGTGAACGGGGAGTGAAGGGAGCCTGTGGCCTTGATGGAGAGAAG 7920
Genbank CCCCGGGGCCTCAAGGGTGAACGGGGAGTGAAGGGAGCCTGTGGCCTTGATGGAGAGAAG 7920
************************************************************

COL7A1 GGAGACAAGGGAGAAGCTGGTCCCCCAGGCCGCCCCGGGCTGGCAGGACACAAAGGAGAG 7980
Genbank GGAGACAAGGGAGAAGCTGGTCCCCCAGGCCGCCCCGGGCTGGCAGGACACAAAGGAGAG 7980
************************************************************

COL7A1 ATGGGGGAGCCTGGTGTGCCGGGCCAGTCGGGGGCCCCTGGCAAGGAGGGCCTGATCGGT 8040
Genbank ATGGGGGAGCCTGGTGTGCCGGGCCAGTCGGGGGCCCCTGGCAAGGAGGGCCTGATCGGT 8040
************************************************************

COL7A1 CCCAAGGGTGACCGAGGCTTTGACGGGCAGCCAGGCCCCAAGGGTGACCAGGGCGAGAAA 8100
Genbank CCCAAGGGTGACCGAGGCTTTGACGGGCAGCCAGGCCCCAAGGGTGACCAGGGCGAGAAA 8100
************************************************************

COL7A1 GGGGAGCGGGGAACCCCAGGAATTGGGGGCTTCCCAGGCCCCAGTGGAAATGATGGCTCT 8160
Genbank GGGGAGCGGGGAACCCCAGGAATTGGGGGCTTCCCAGGCCCCAGTGGAAATGATGGCTCT 8160
************************************************************

COL7A1 GCTGGTCCCCCAGGGCCACCTGGCAGTGTTGGTCCCAGAGGCCCCGAAGGACTTCAGGGC 8220
Genbank GCTGGTCCCCCAGGGCCACCTGGCAGTGTTGGTCCCAGAGGCCCCGAAGGACTTCAGGGC 8220
************************************************************

COL7A1 CAGAAGGGTGAGCGAGGTCCCCCCGGAGAGAGAGTGGTGGGGGCTCCTGGGGTCCCTGGA 8280
Genbank CAGAAGGGTGAGCGAGGTCCCCCCGGAGAGAGAGTGGTGGGGGCTCCTGGGGTCCCTGGA 8280
************************************************************

COL7A1 GCTCCTGGCGAGAGAGGGGAGCAGGGGCGGCCAGGGCCTGCCGGTCCTCGAGGCGAGAAG 8340
Genbank GCTCCTGGCGAGAGAGGGGAGCAGGGGCGGCCAGGGCCTGCCGGTCCTCGAGGCGAGAAG 8340
************************************************************

COL7A1 GGAGAAGCTGCACTGACGGAGGATGACATCCGGGGCTTTGTGCGCCAAGAGATGAGTCAG 8400
Genbank GGAGAAGCTGCACTGACGGAGGATGACATCCGGGGCTTTGTGCGCCAAGAGATGAGTCAG 8400
************************************************************

COL7A1 CACTGTGCCTGCCAGGGCCAGTTCATCGCATCTGGATCACGACCCCTCCCTAGTTATGCT 8460

Genbank CACTGTGCCTGCCAGGGCCAGTTCATCGCATCTGGATCACGACCCCTCCCTAGTTATGCT 8460
************************************************************

COL7A1 GCAGACACTGCCGGCTCCCAGCTCCATGCTGTGCCTGTGCTCCGCGTCTCTCATGCAGAG 8520
Genbank GCAGACACTGCCGGCTCCCAGCTCCATGCTGTGCCTGTGCTCCGCGTCTCTCATGCAGAG 8520
************************************************************

COL7A1 GAGGAAGAGCGGGTACCCCCTGAGGATGATGAGTACTCTGAATACTCCGAGTATTCTGTG 8580
Genbank GAGGAAGAGCGGGTACCCCCTGAGGATGATGAGTACTCTGAATACTCCGAGTATTCTGTG 8580
************************************************************

COL7A1 GAGGAGTACCAGGACCCTGAAGCTCCTTGGGATAGTGATGACCCCTGTTCCCTGCCACTG 8640
Genbank GAGGAGTACCAGGACCCTGAAGCTCCTTGGGATAGTGATGACCCCTGTTCCCTGCCACTG 8640
************************************************************

COL7A1 GATGAGGGCTCCTGCACTGCCTACACCCTGCGCTGGTACCATCGGGCTGTGACAGGCAGC 8700
Genbank GATGAGGGCTCCTGCACTGCCTACACCCTGCGCTGGTACCATCGGGCTGTGACAGGCAGC 8700
************************************************************

COL7A1 ACAGAGGCCTGTCACCCTTTTGTCTATGGTGGCTGTGGAGGGAATGCCAACCGTTTTGGG 8760
Genbank ACAGAGGCCTGTCACCCTTTTGTCTATGGTGGCTGTGGAGGGAATGCCAACCGTTTTGGG 8760
************************************************************

COL7A1 ACCCGTGAGGCCTGCGAGCGCCGCTGCCCACCCCGGGTGGTCCAGAGCCAGGGGACAGGT 8820
Genbank ACCCGTGAGGCCTGCGAGCGCCGCTGCCCACCCCGGGTGGTCCAGAGCCAGGGGACAGGT 8820
************************************************************

COL7A1 ACTGCCCAGGAC 8832 (SEQ ID NO: 23)
Genbank ACTGCCCAGGAC 8832 (SEQ ID NO: 24)
************

The following is a comparative analysis of 60010743-ITX-00001_CONTIG SEQUENCE (SEQ ID NO:34) relative to IGE308 REFSEQUENCE (SEQ ID NO: 34).

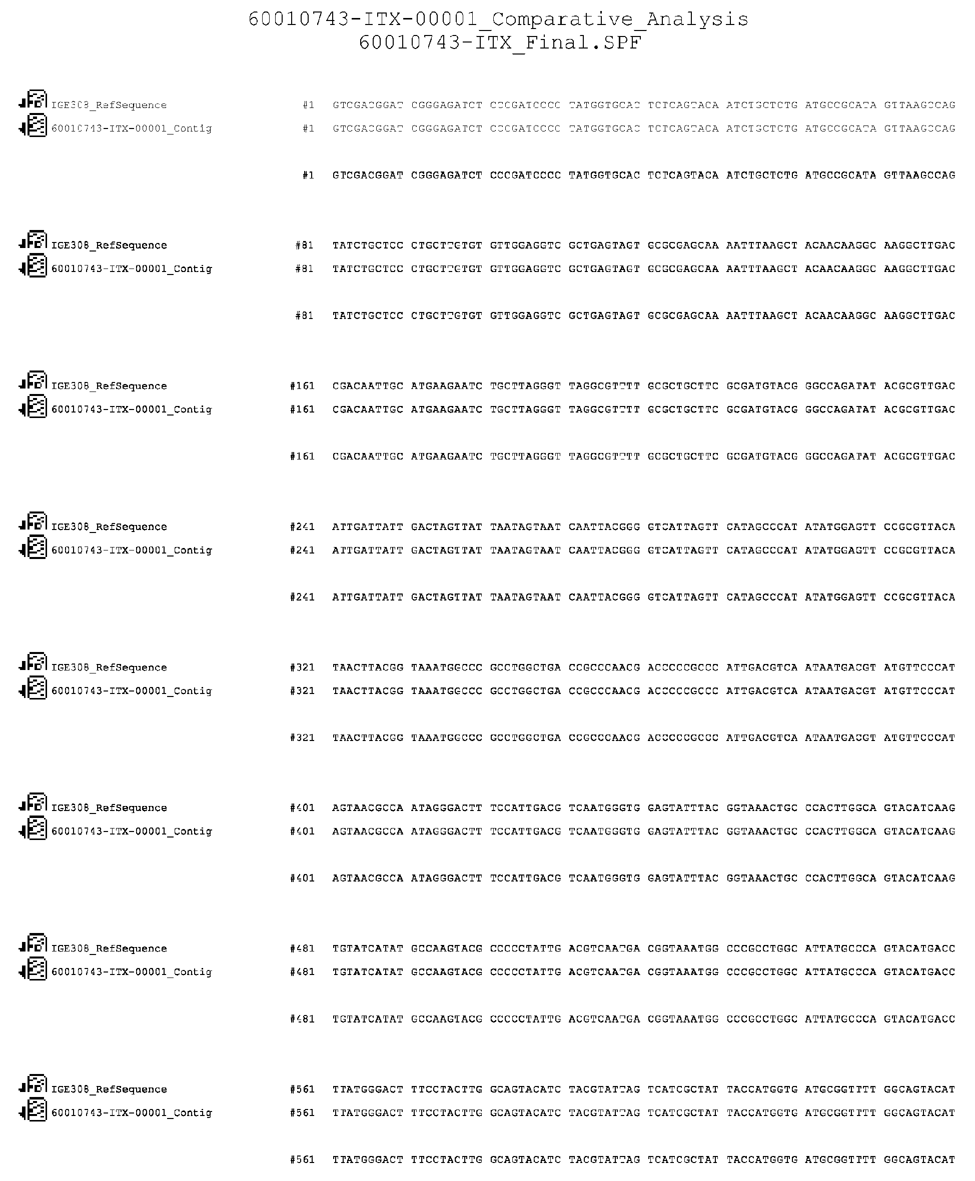

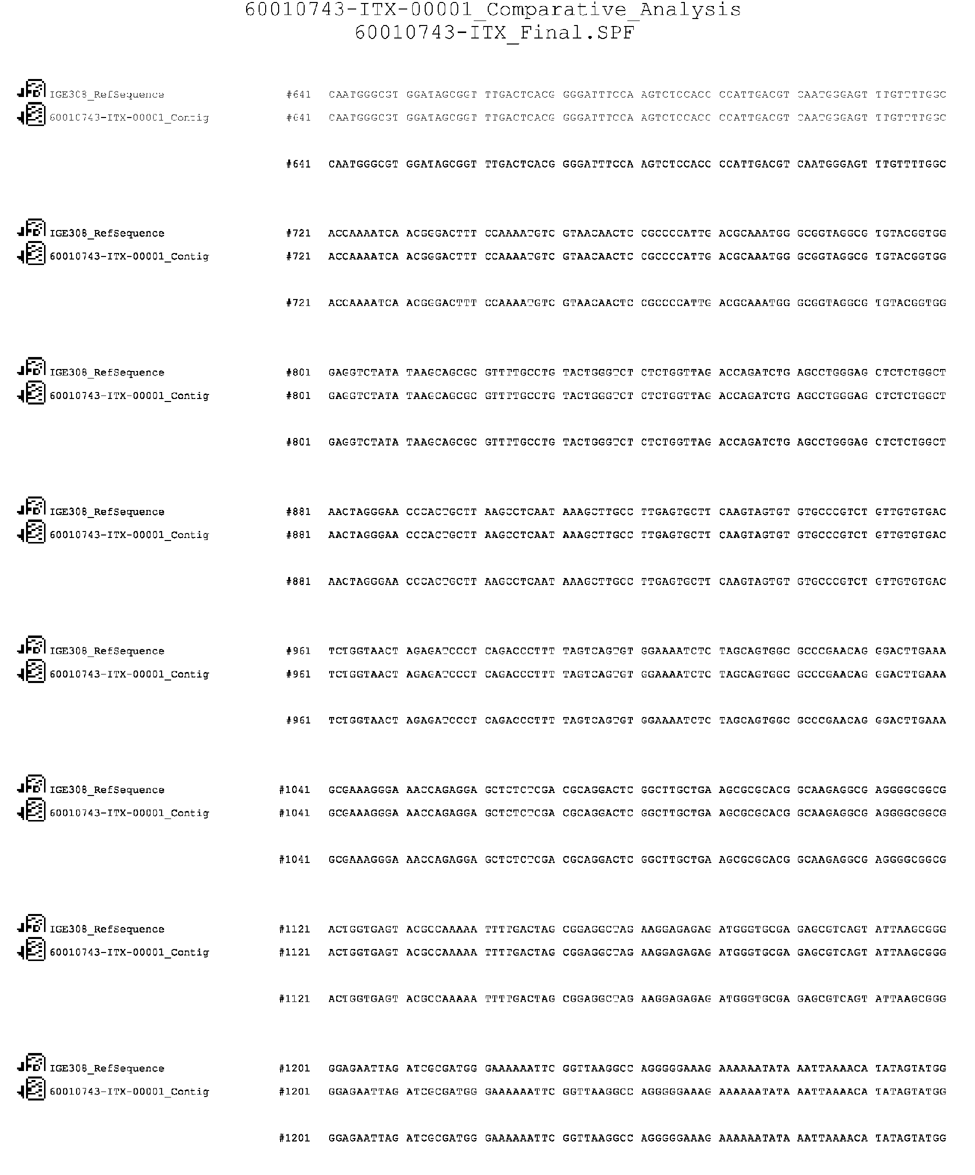

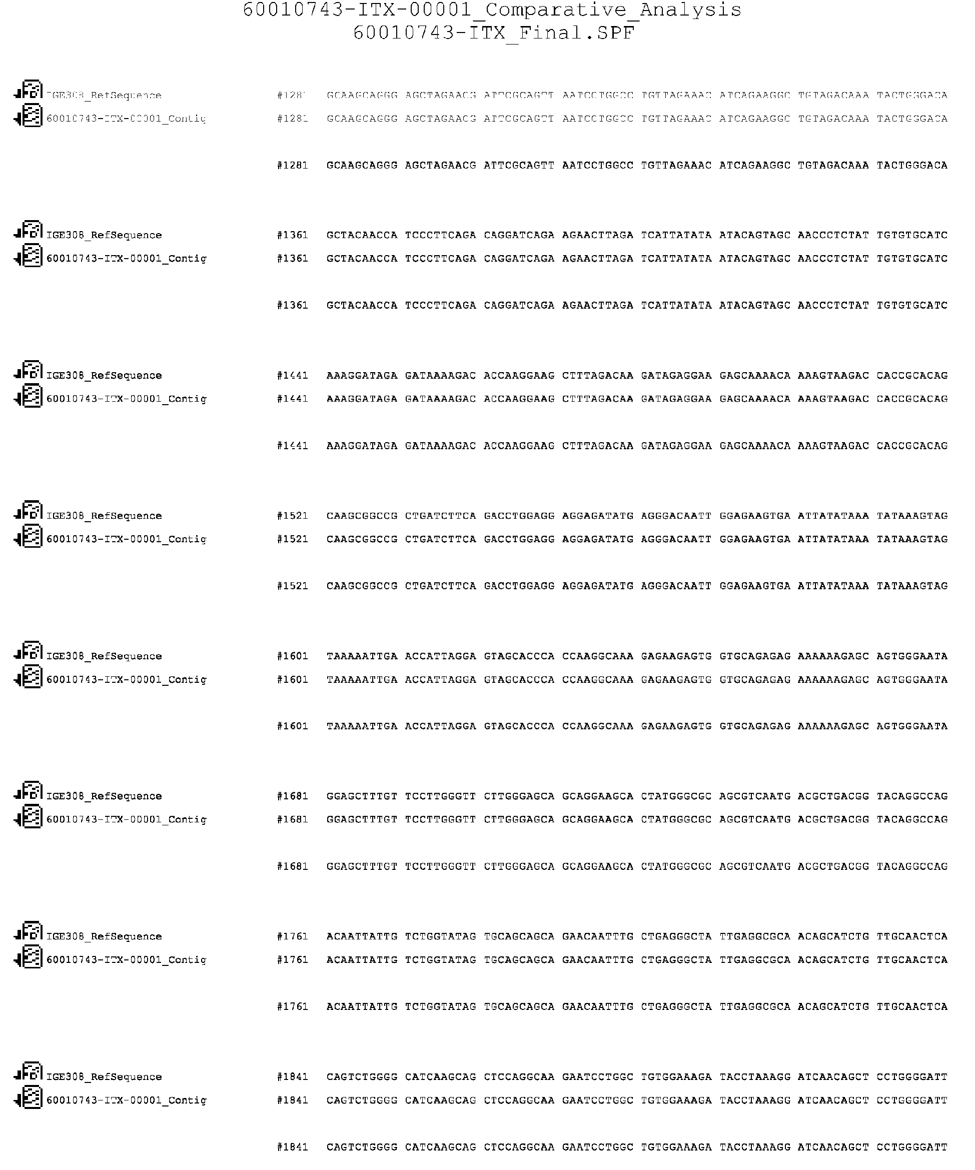

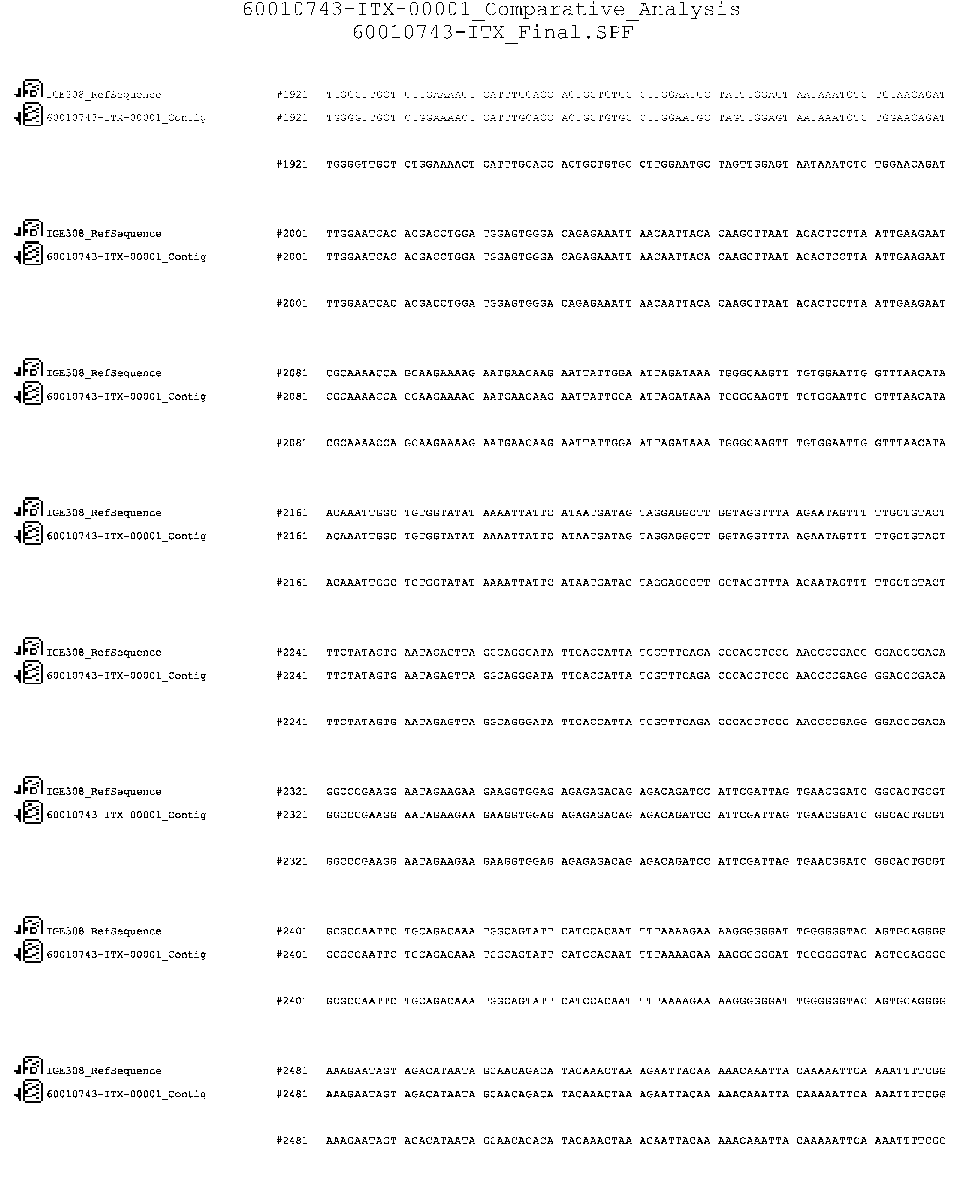

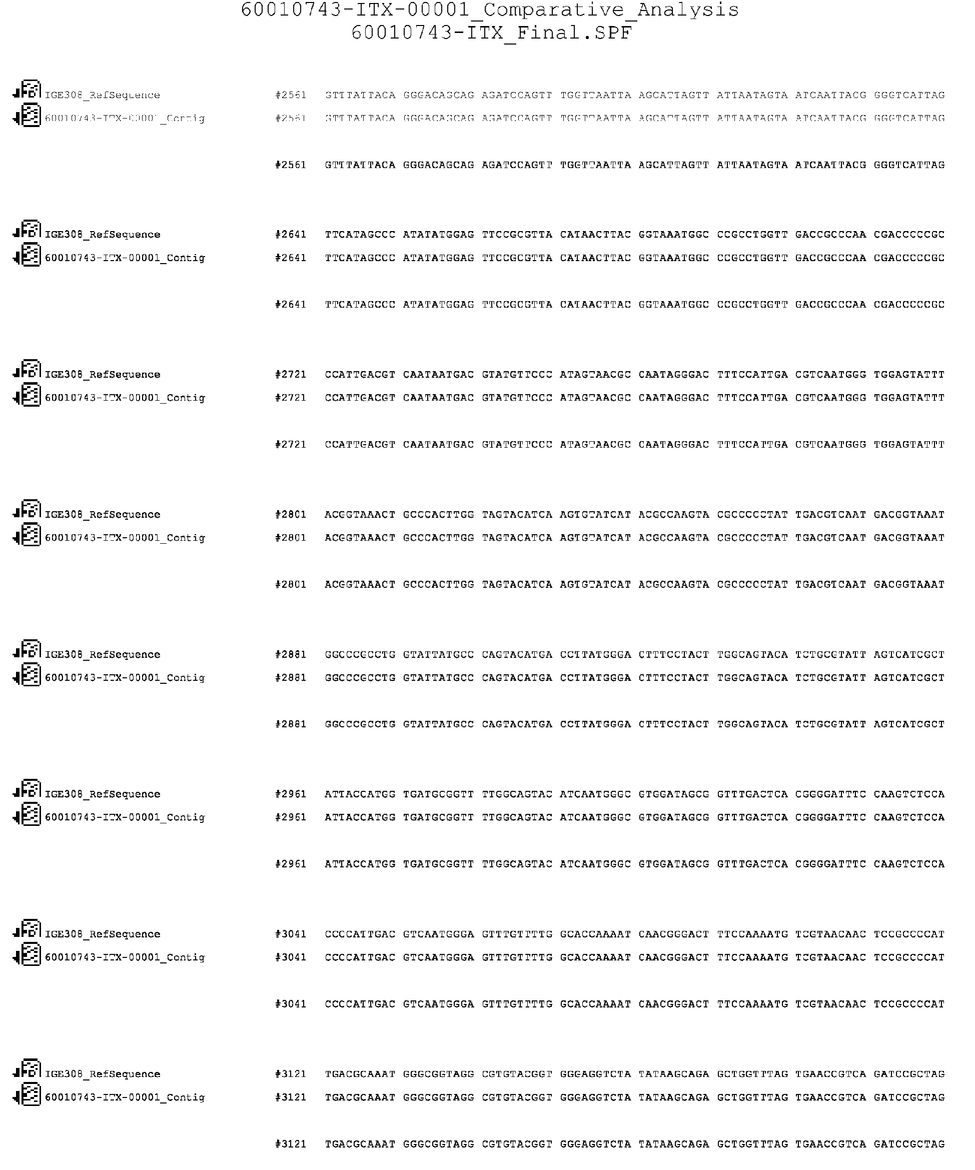

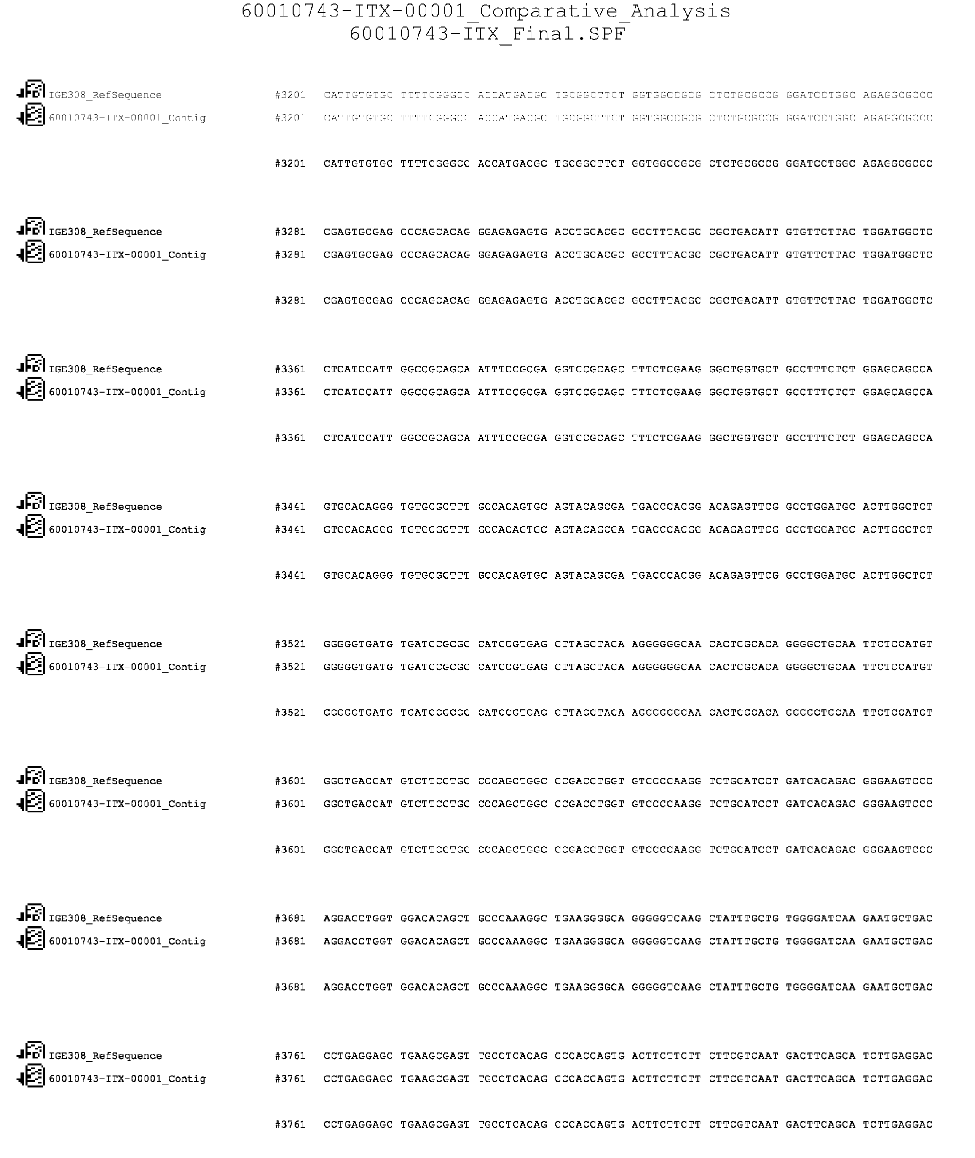

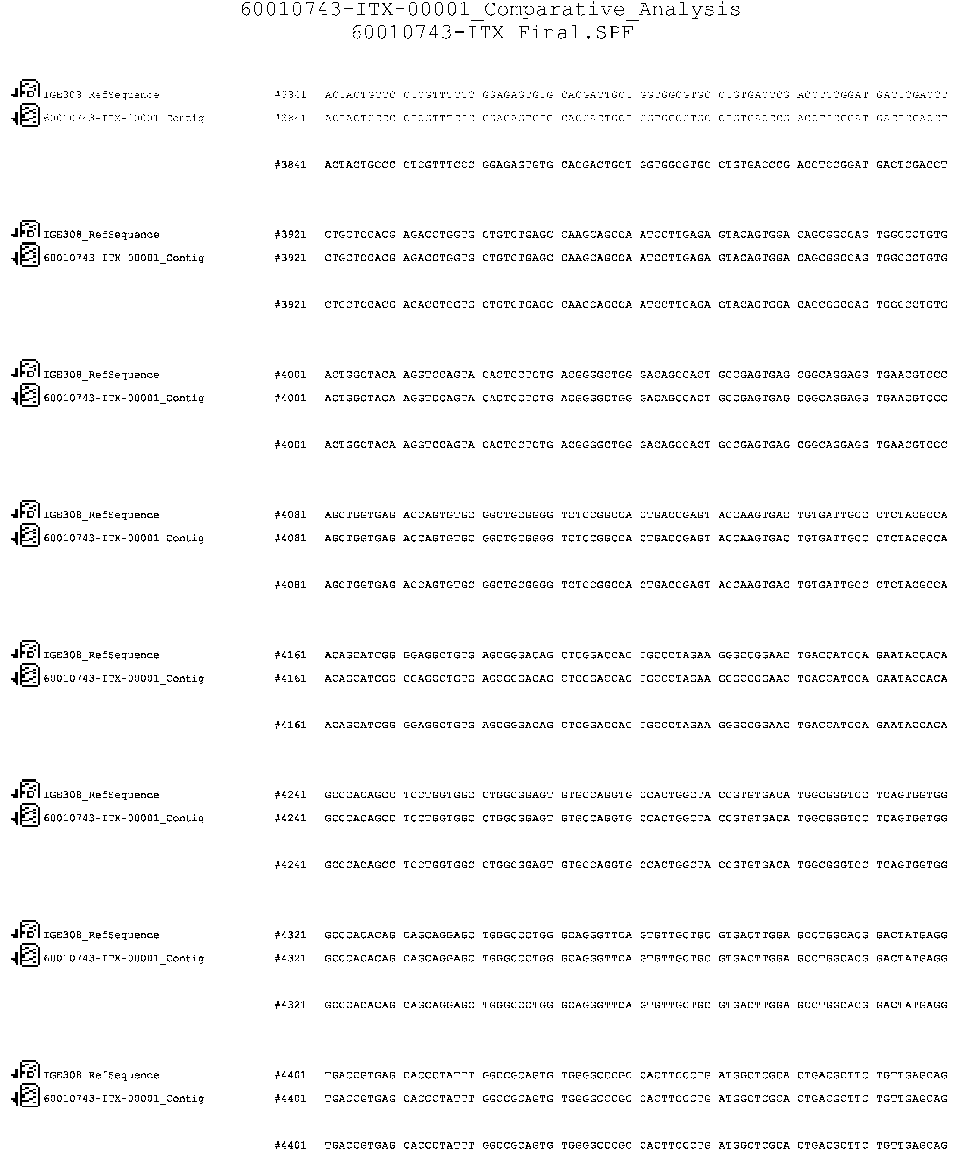

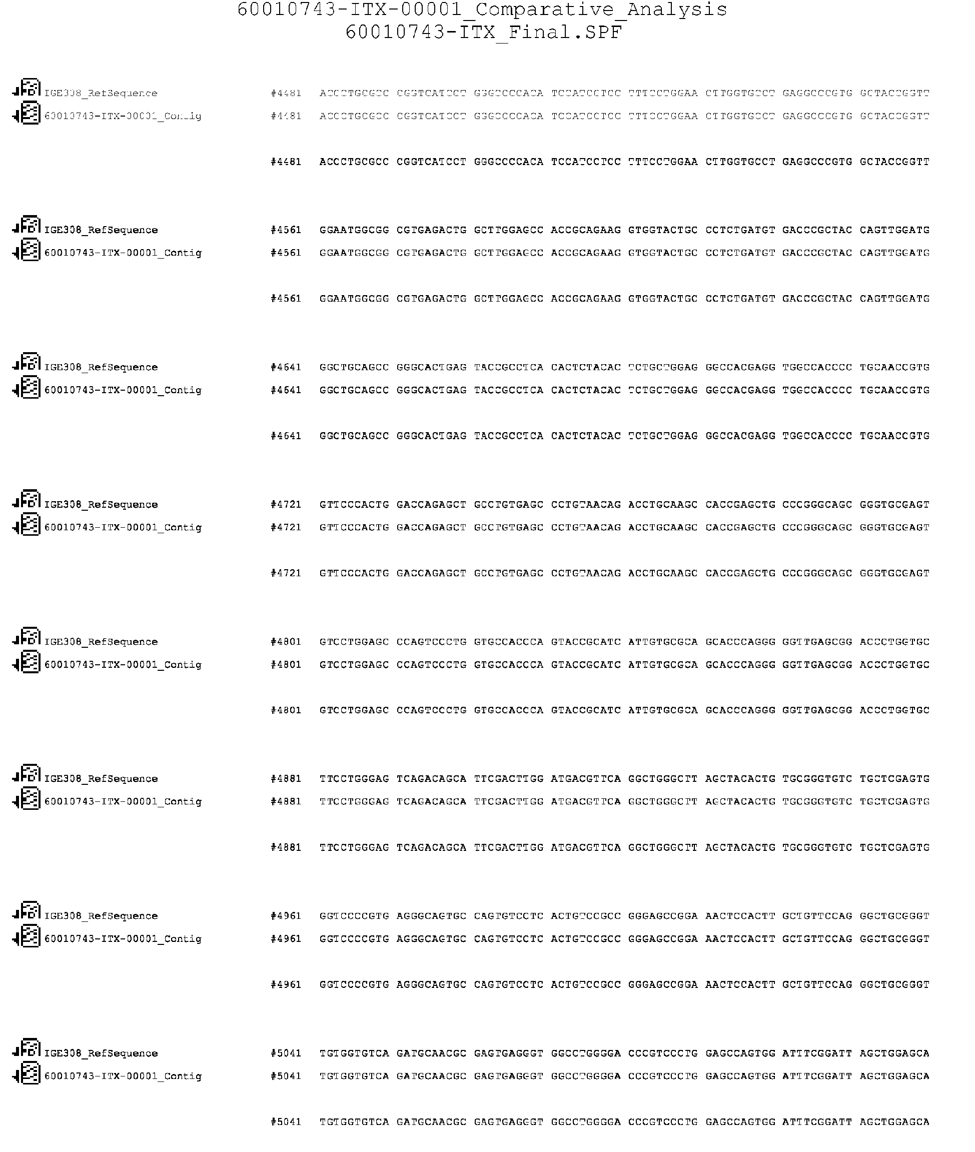

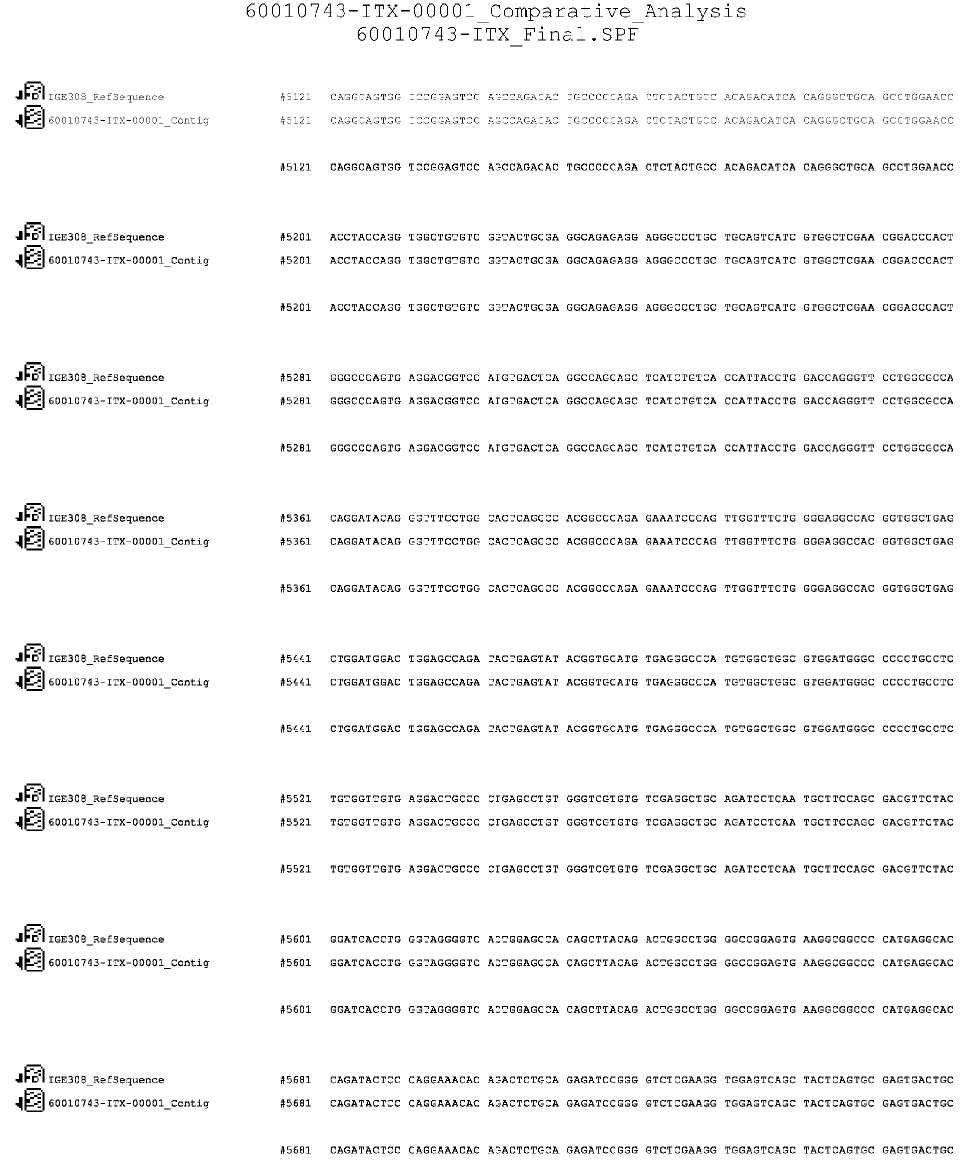

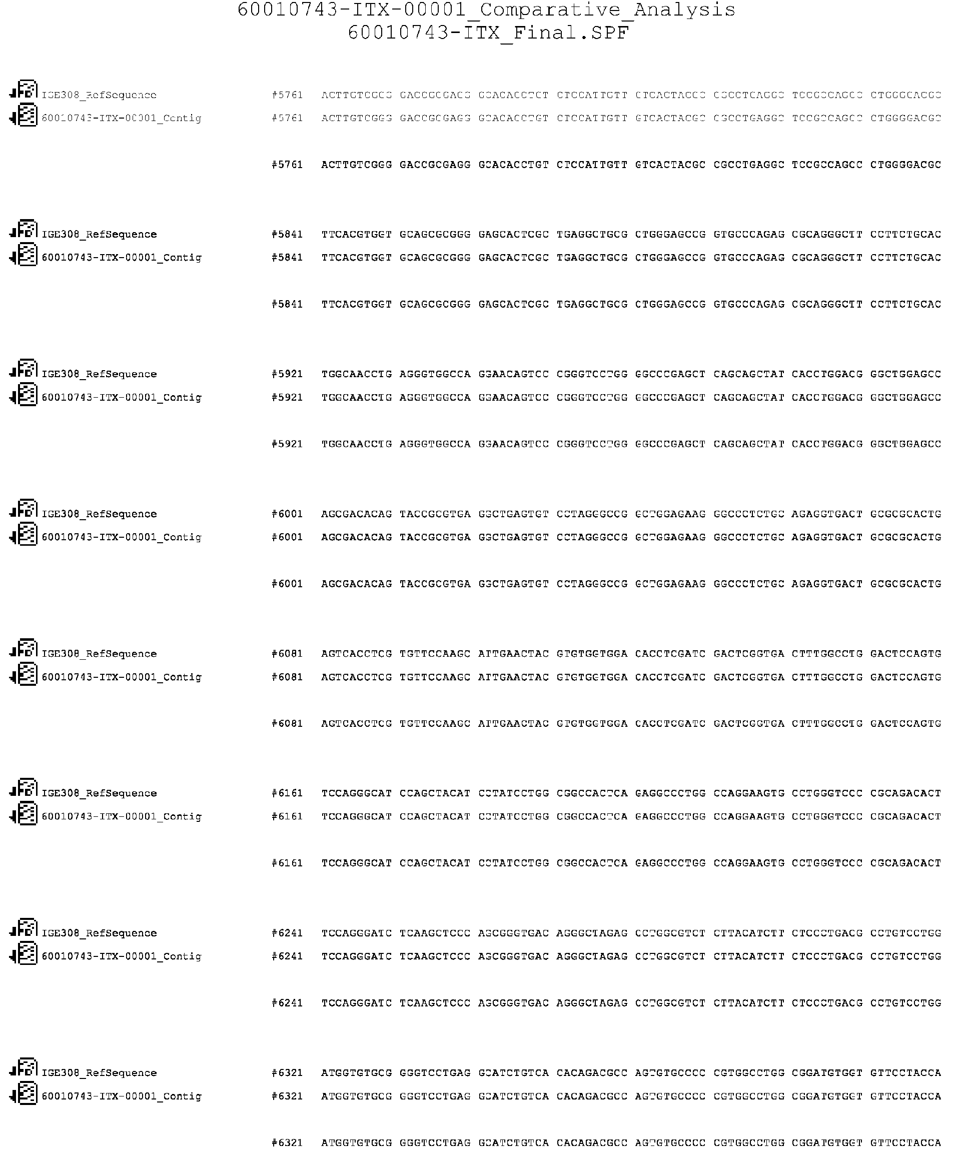

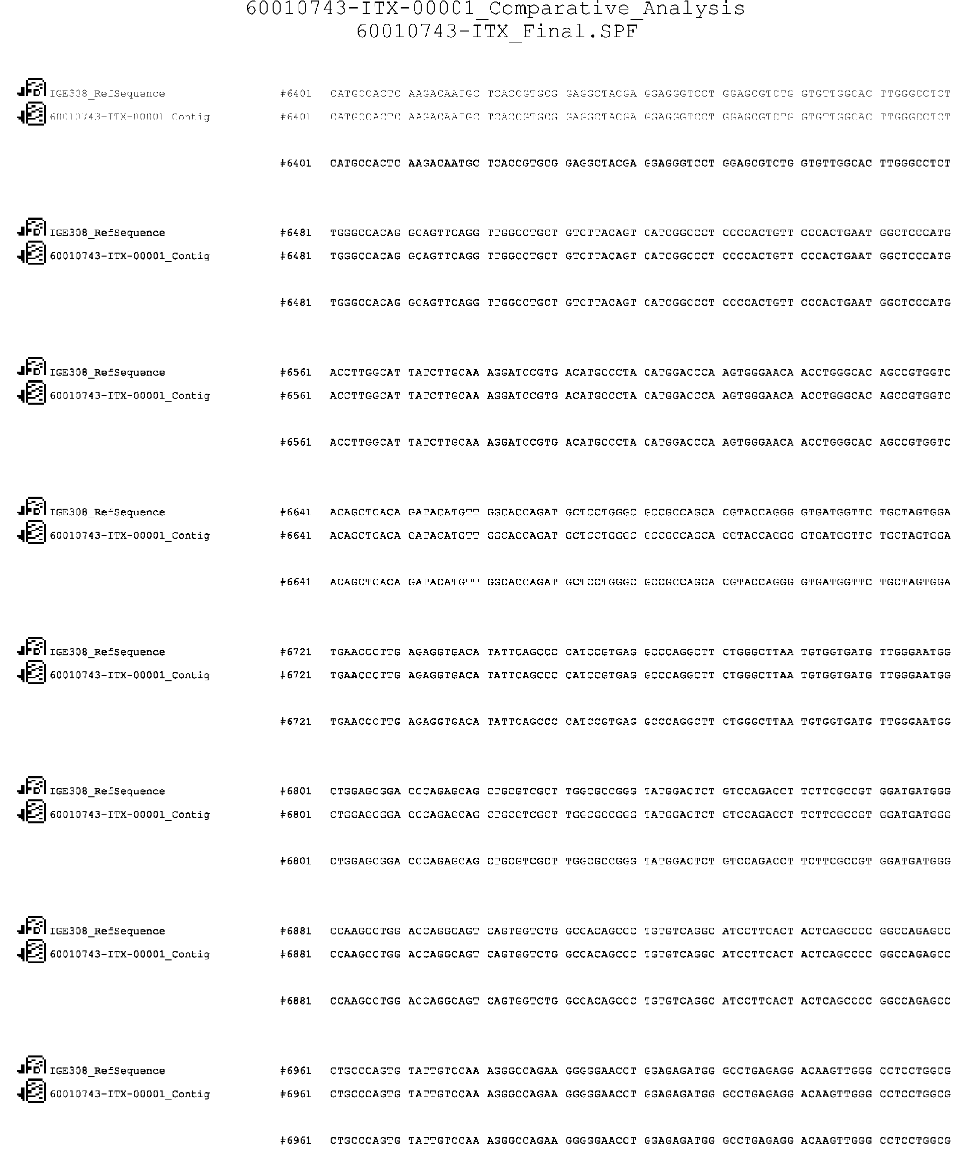

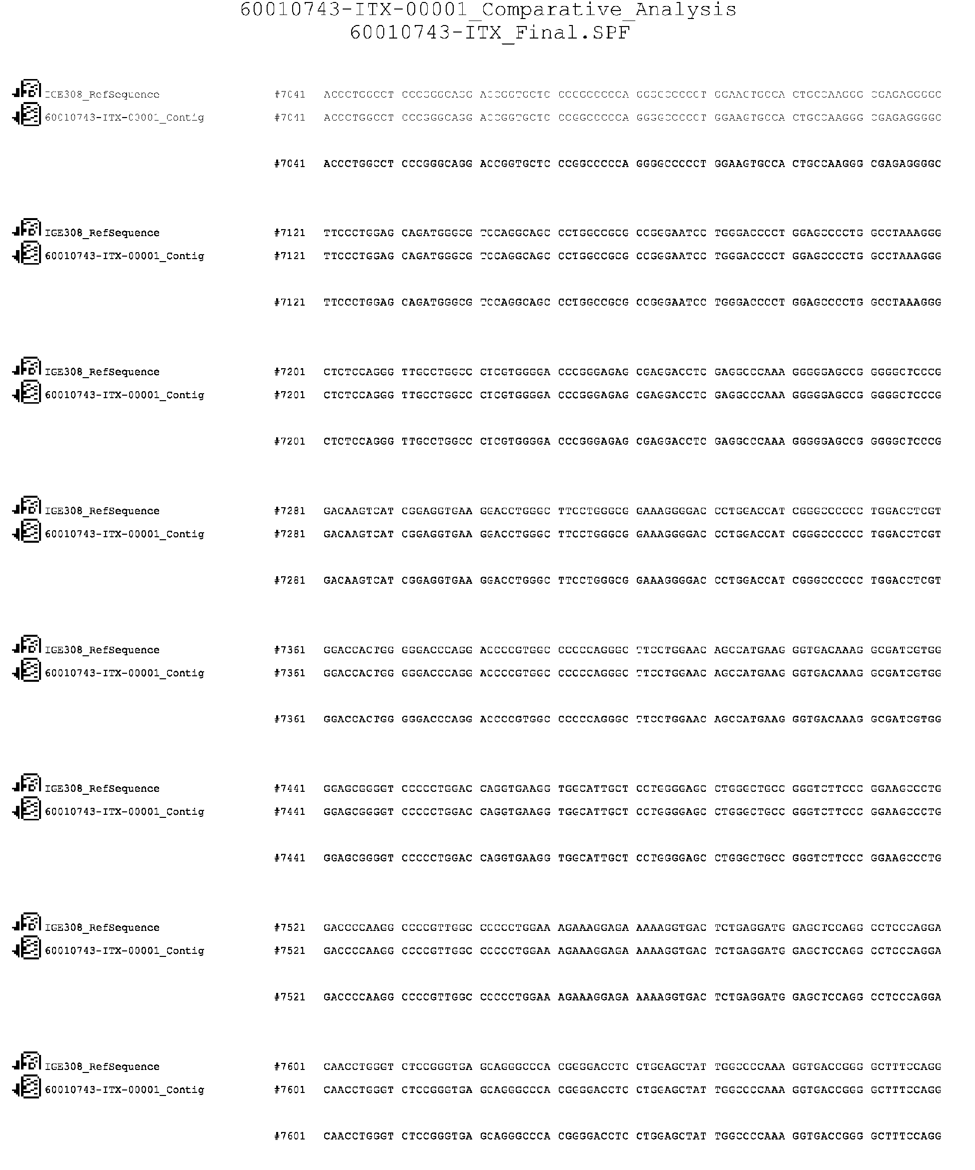

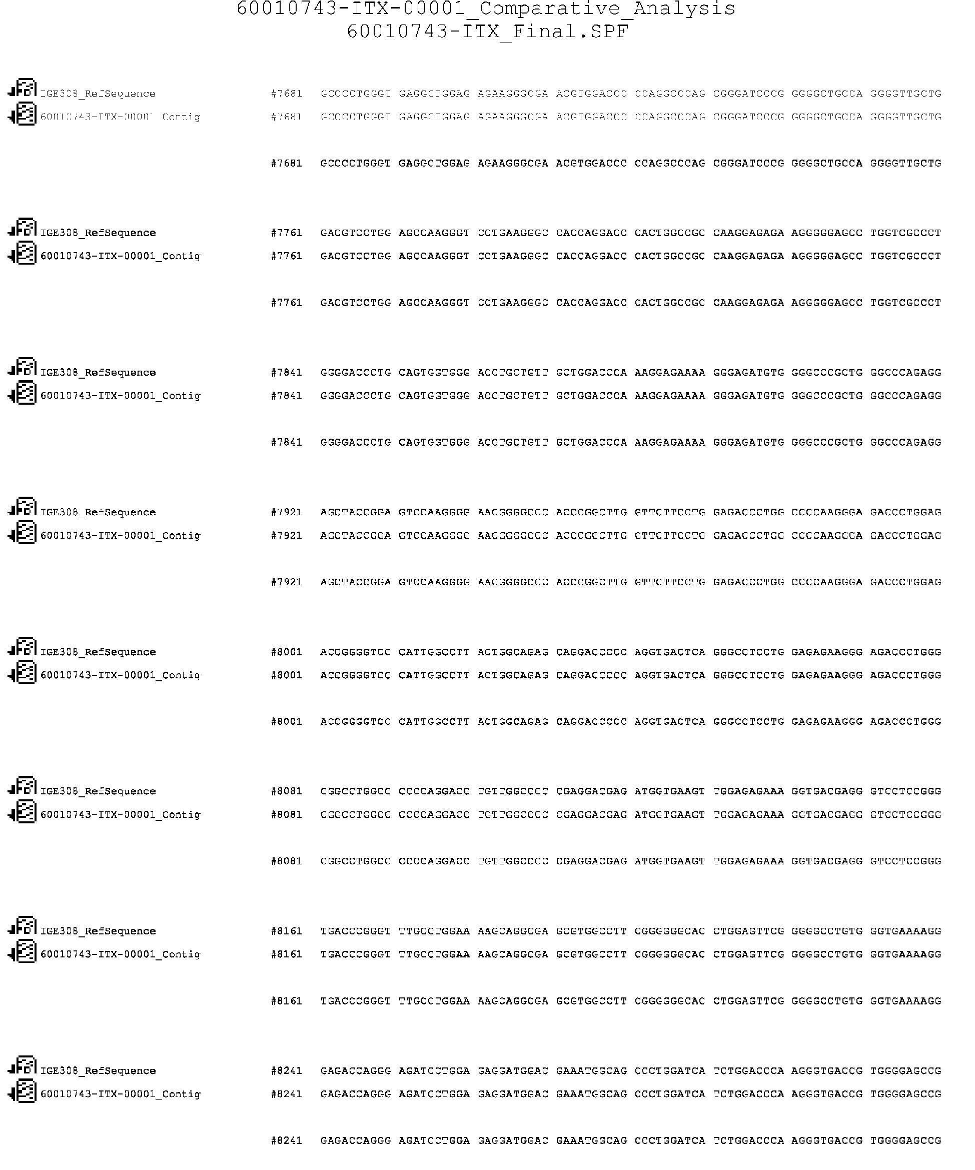

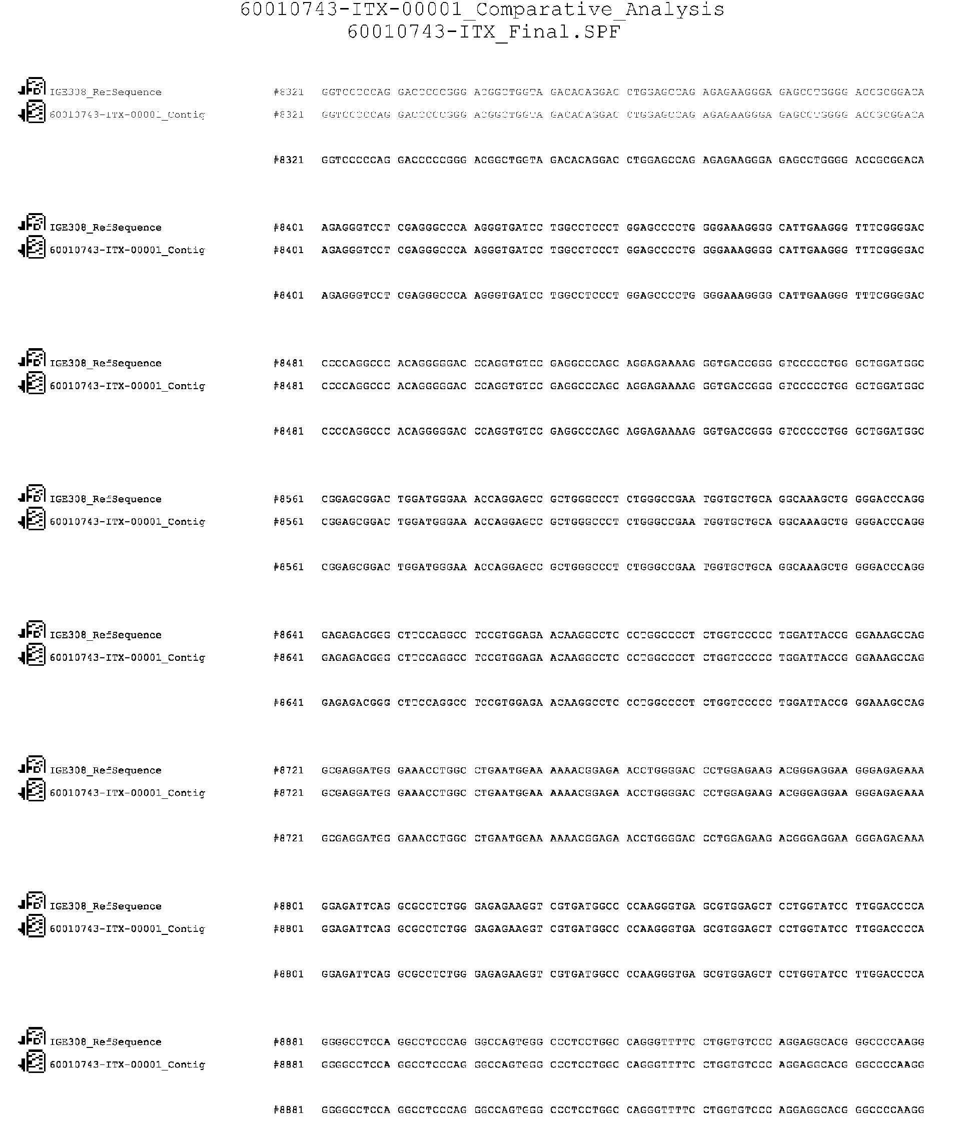

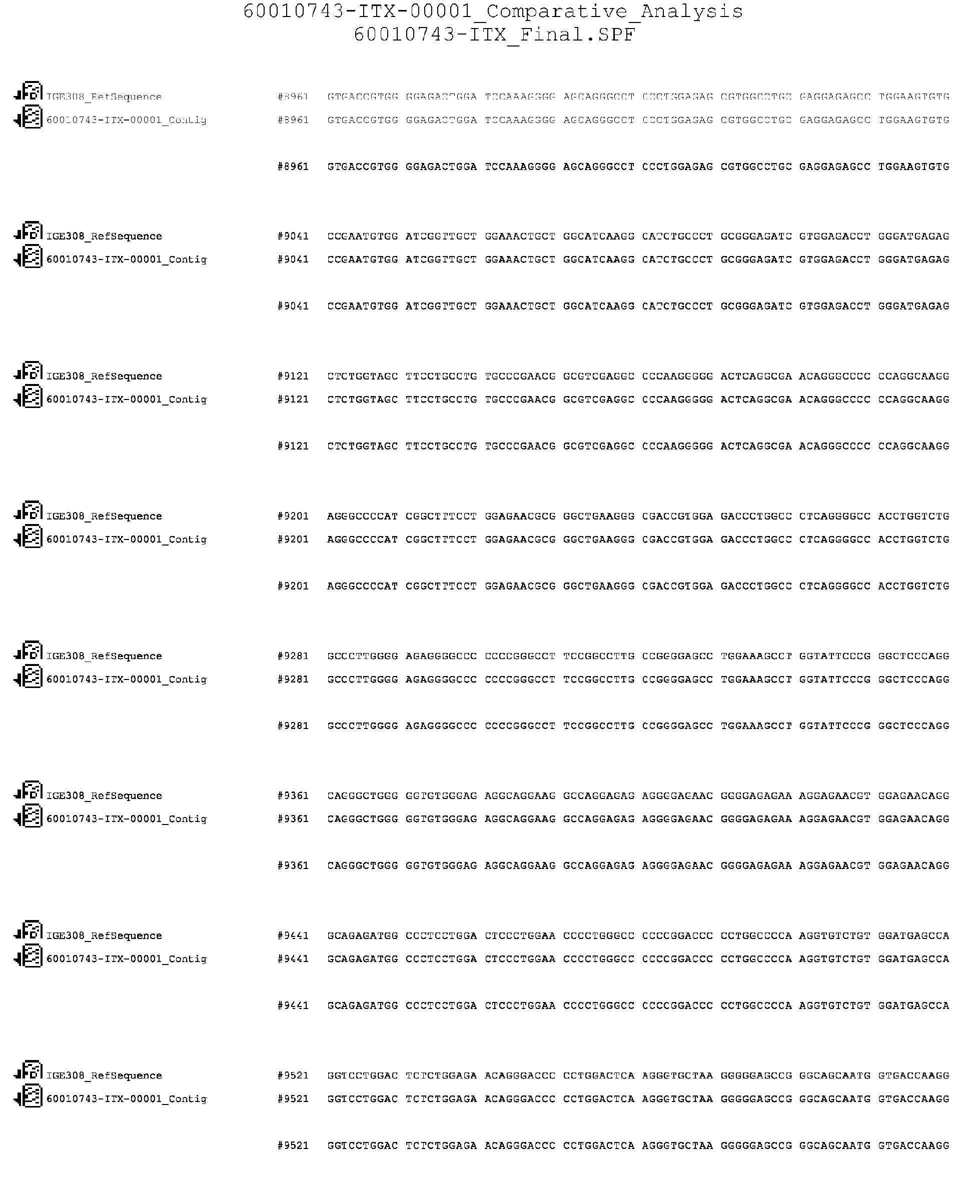

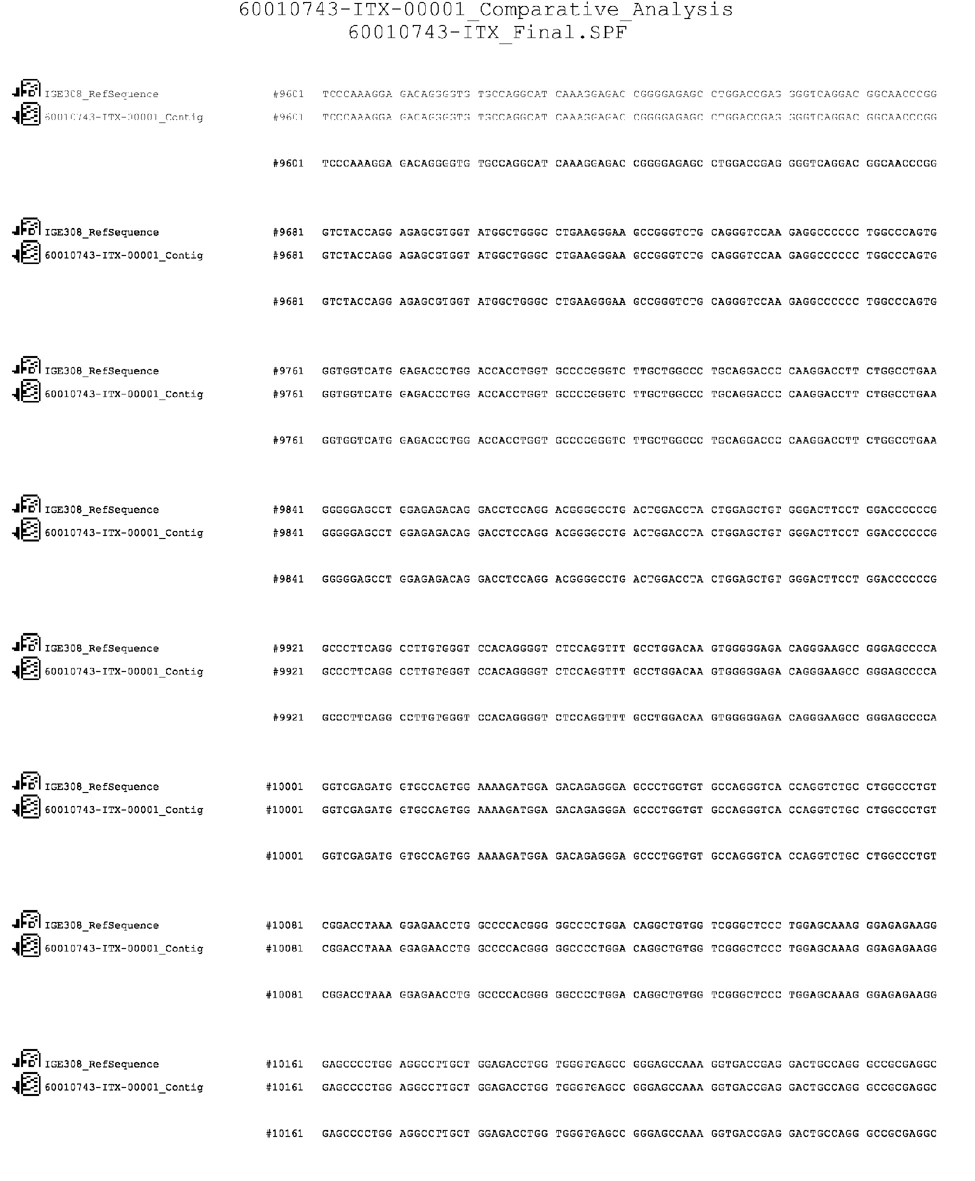

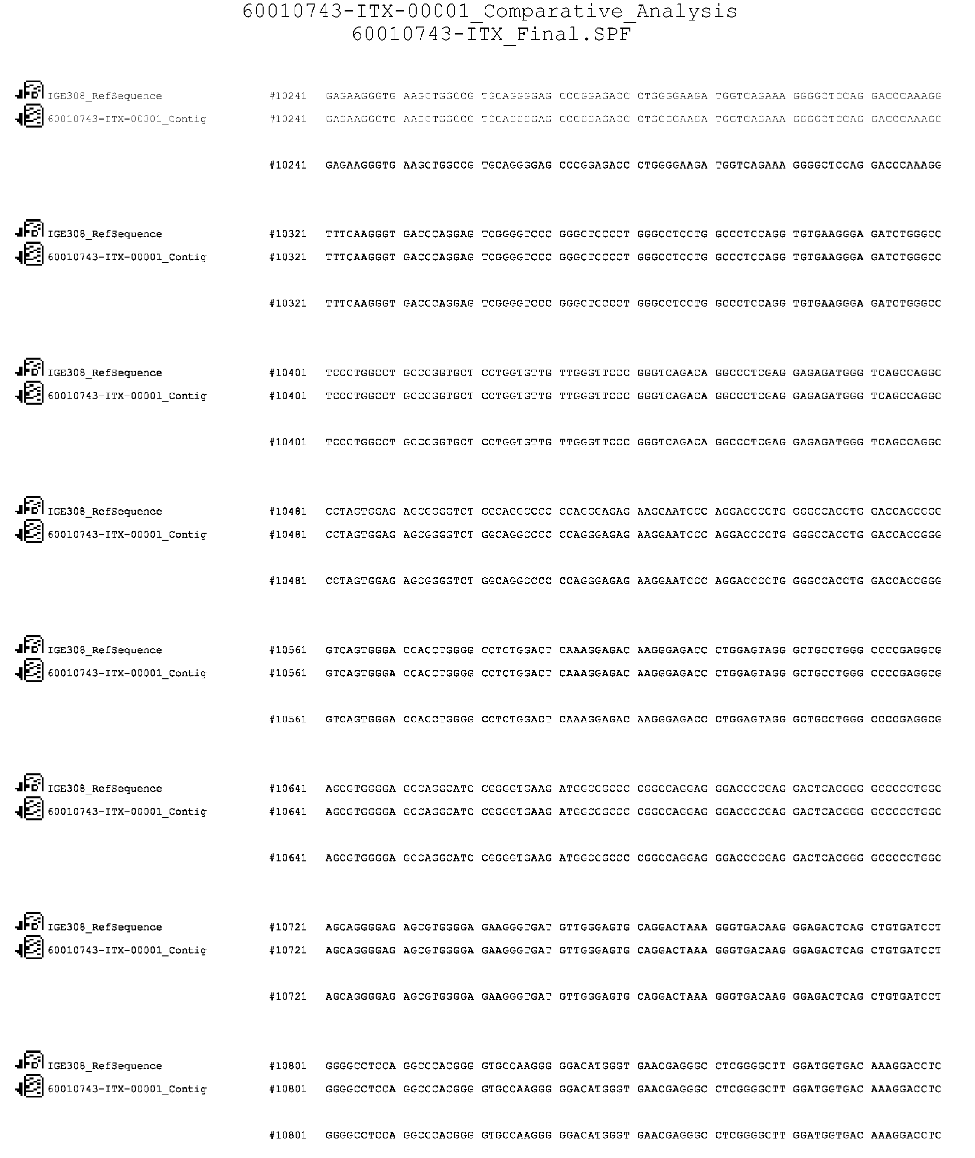

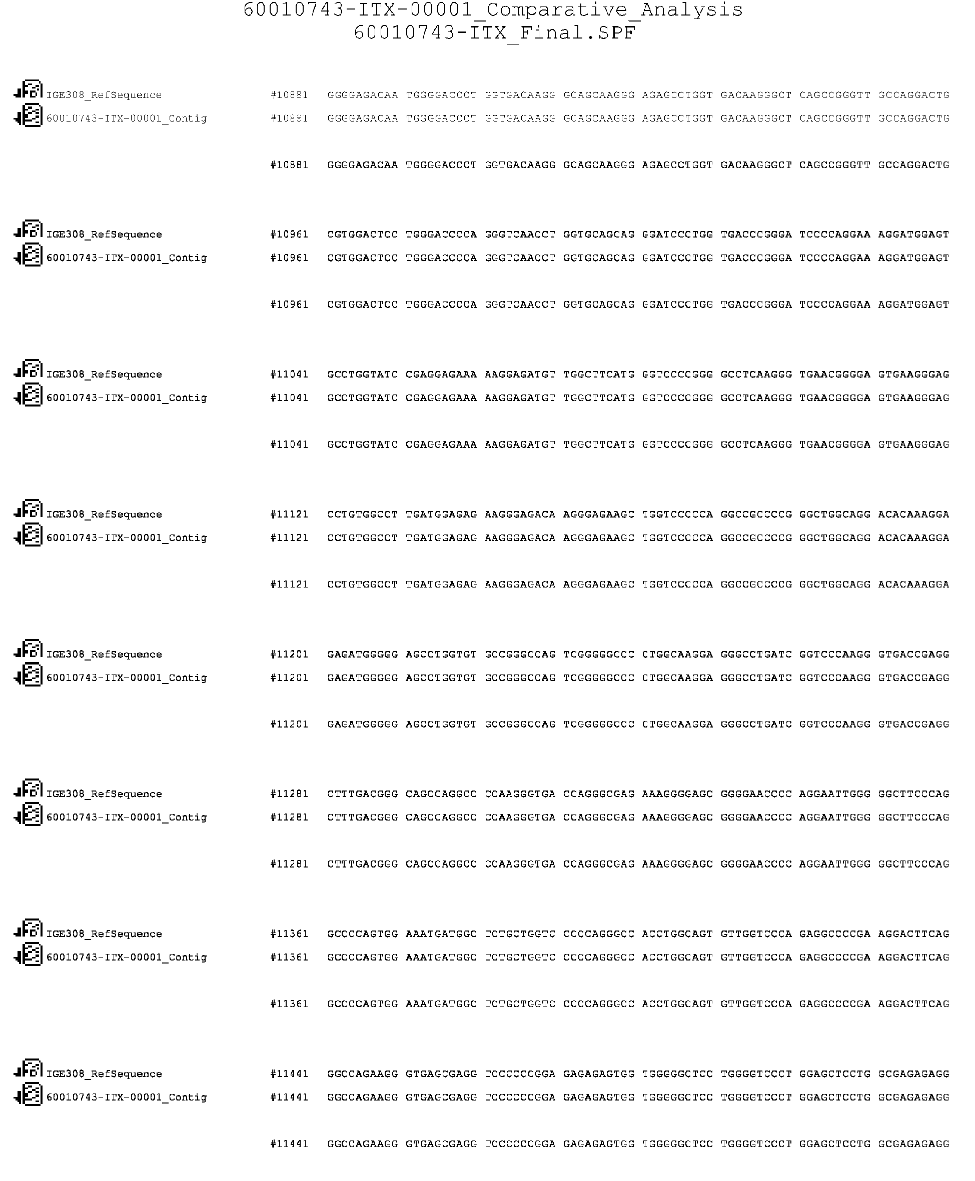

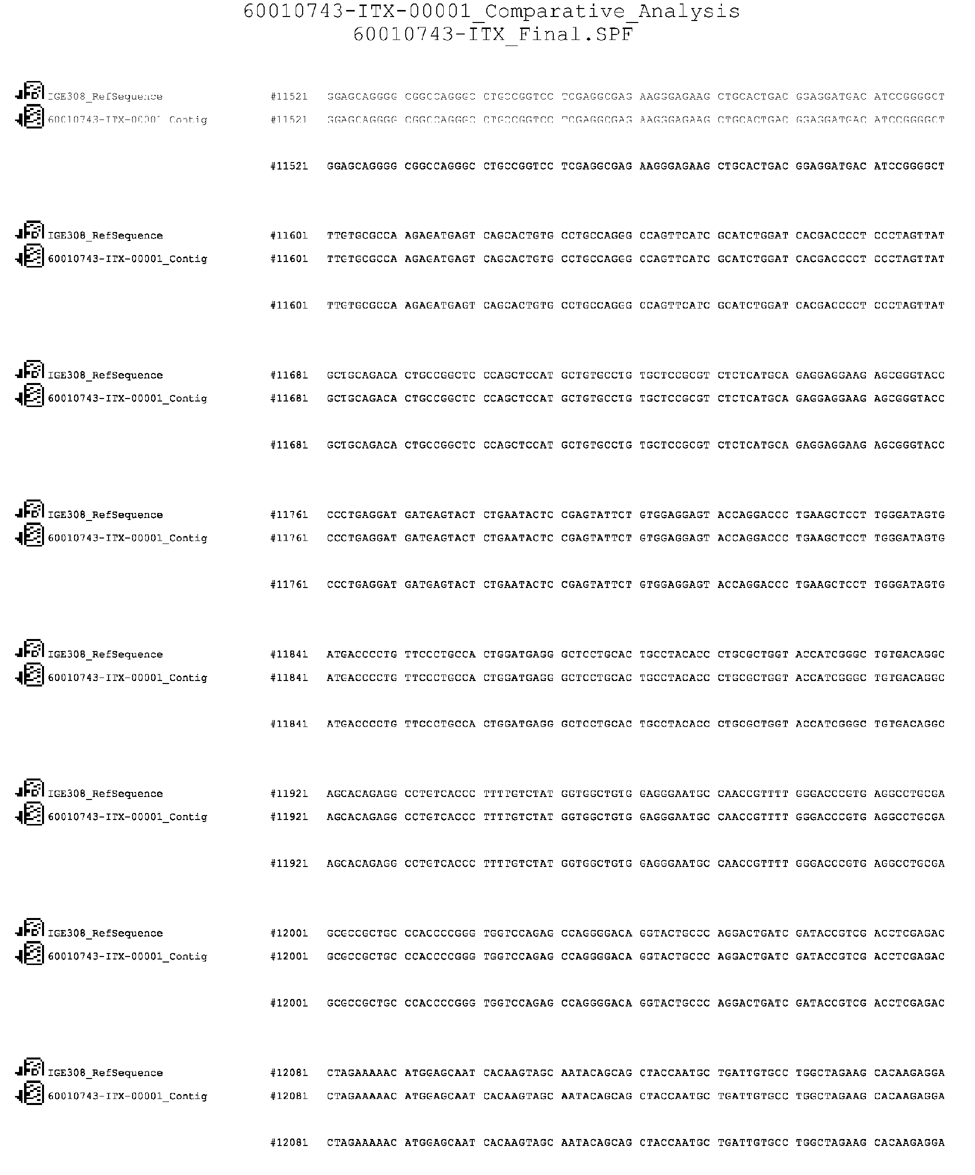

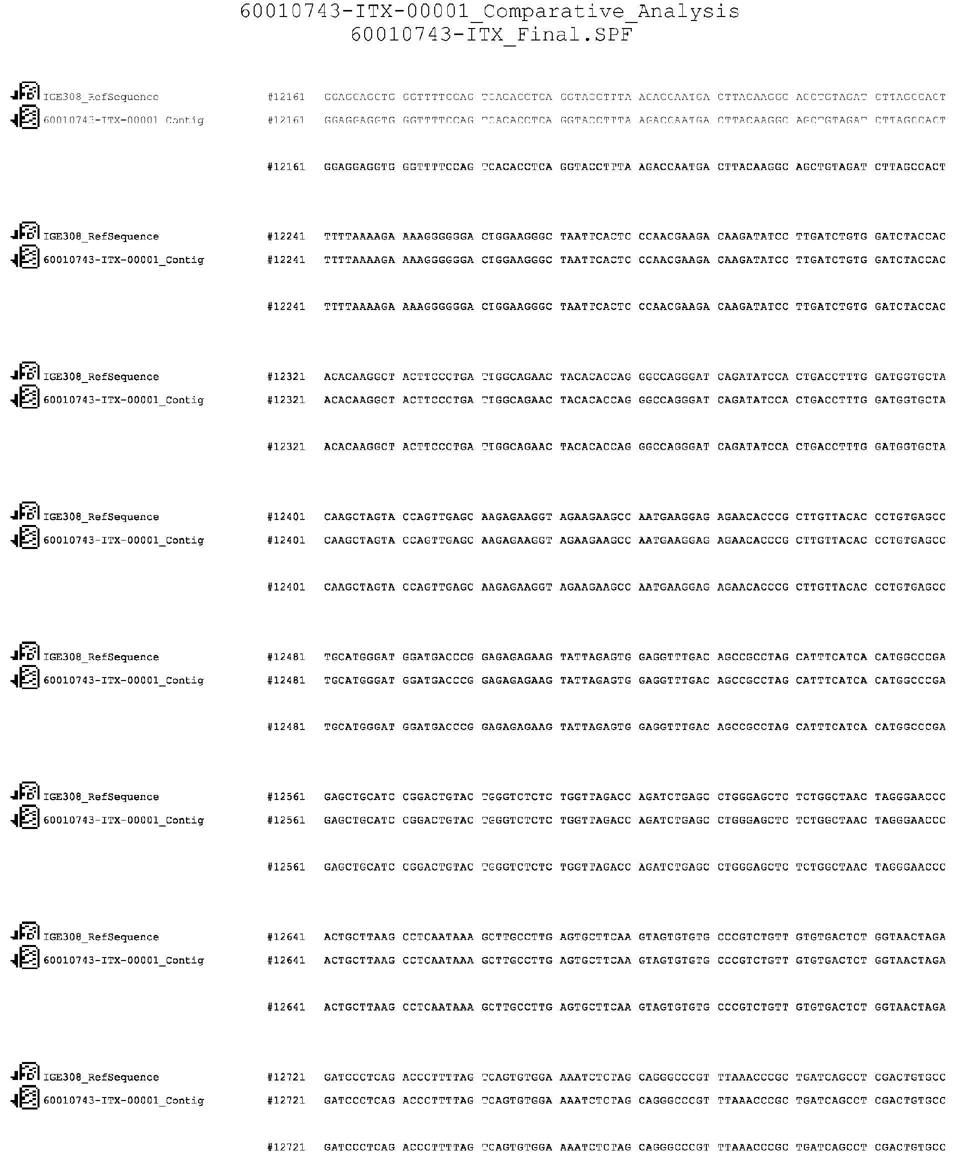

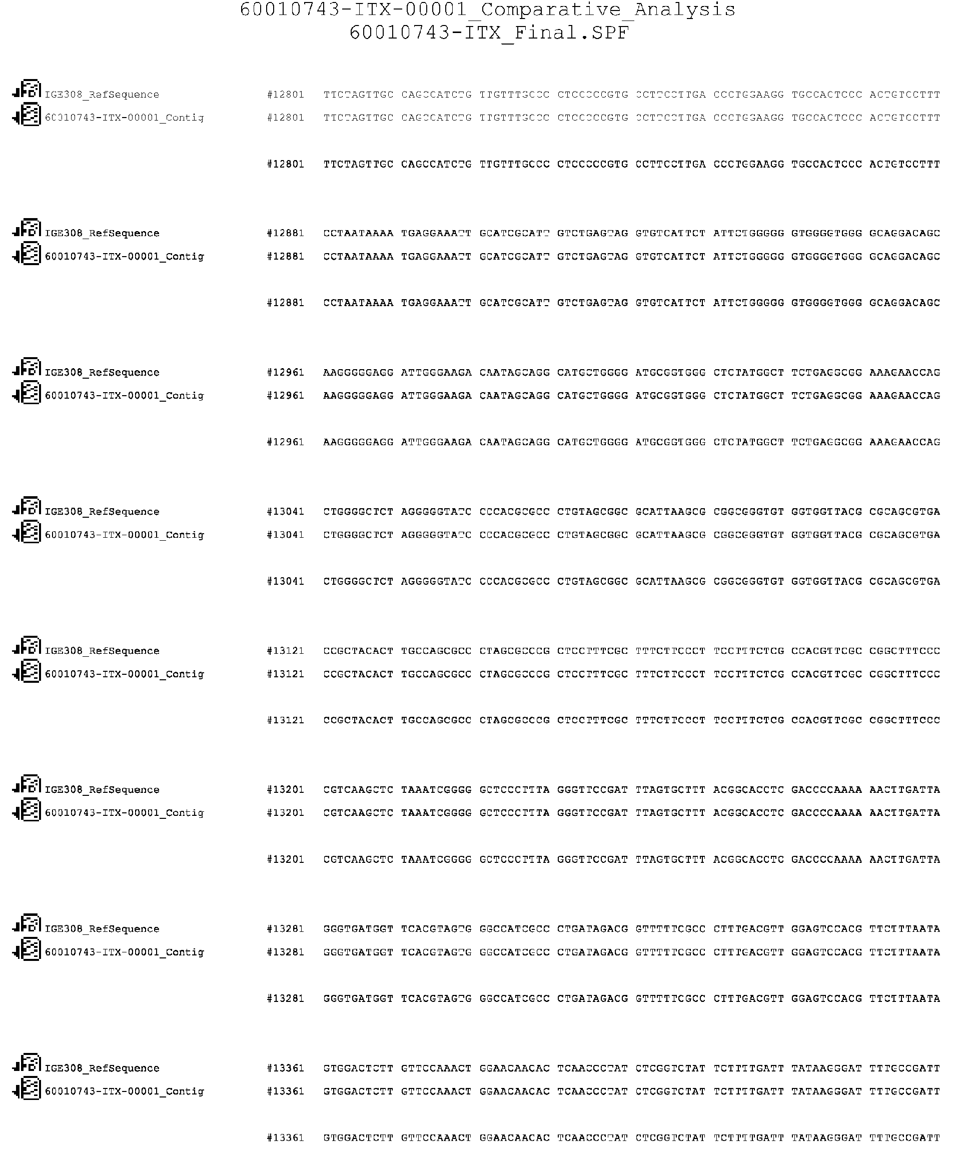

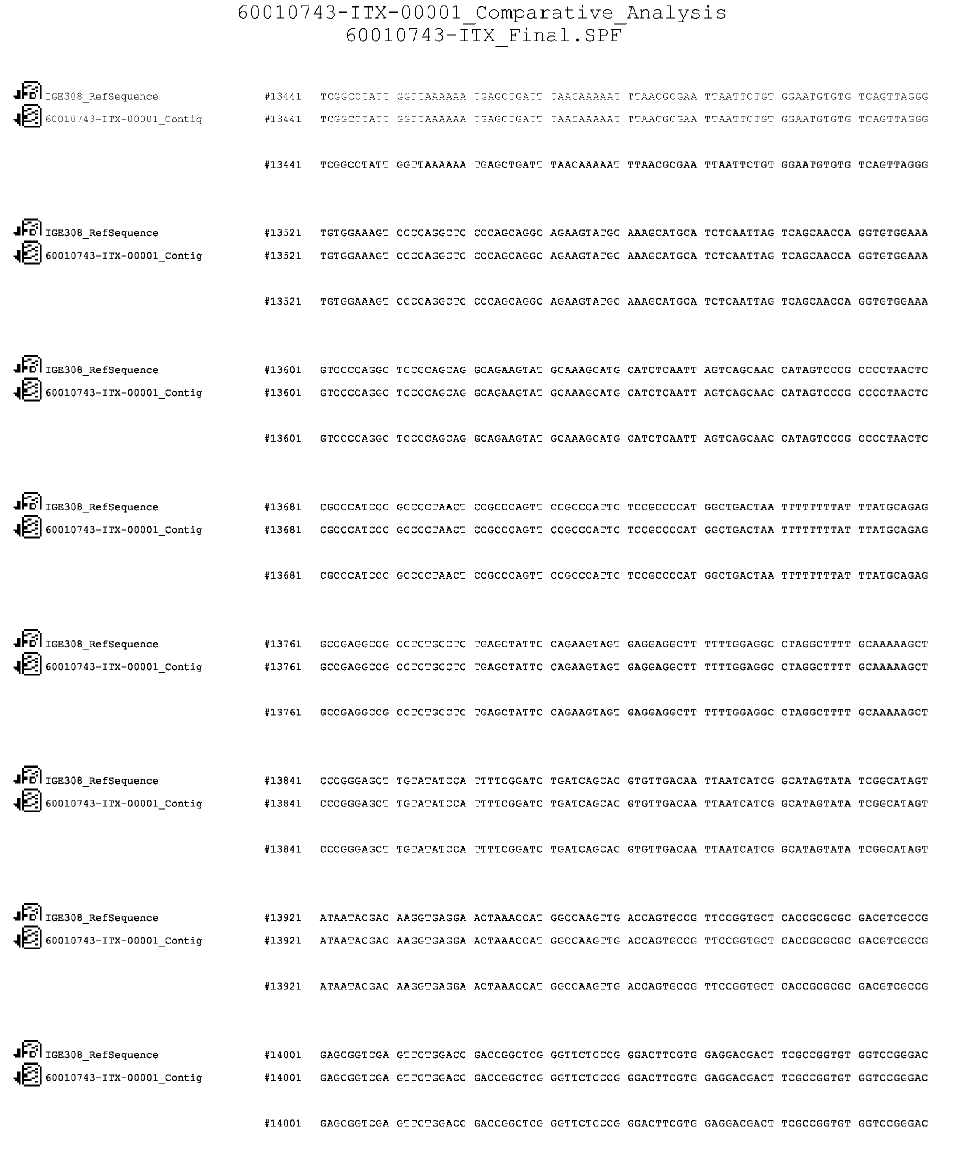

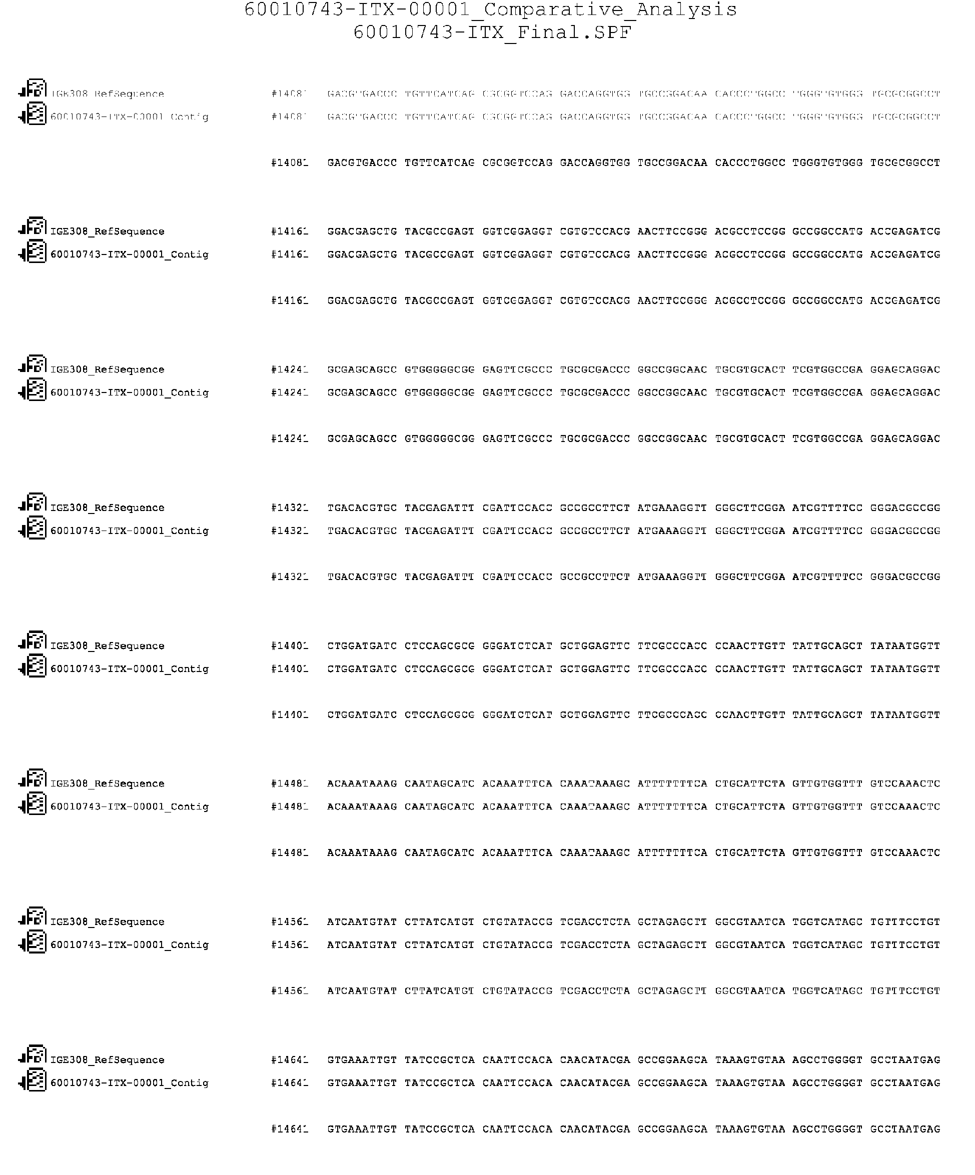

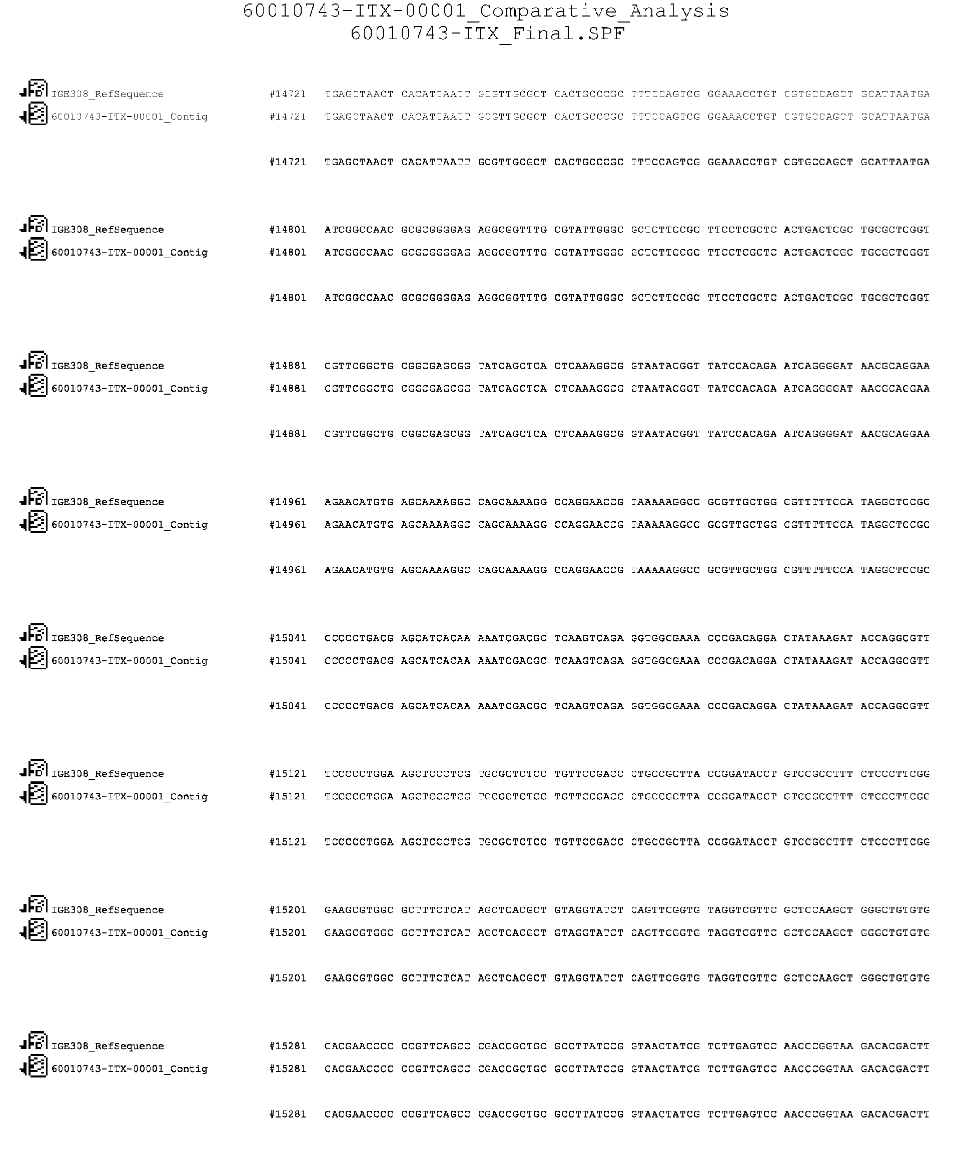

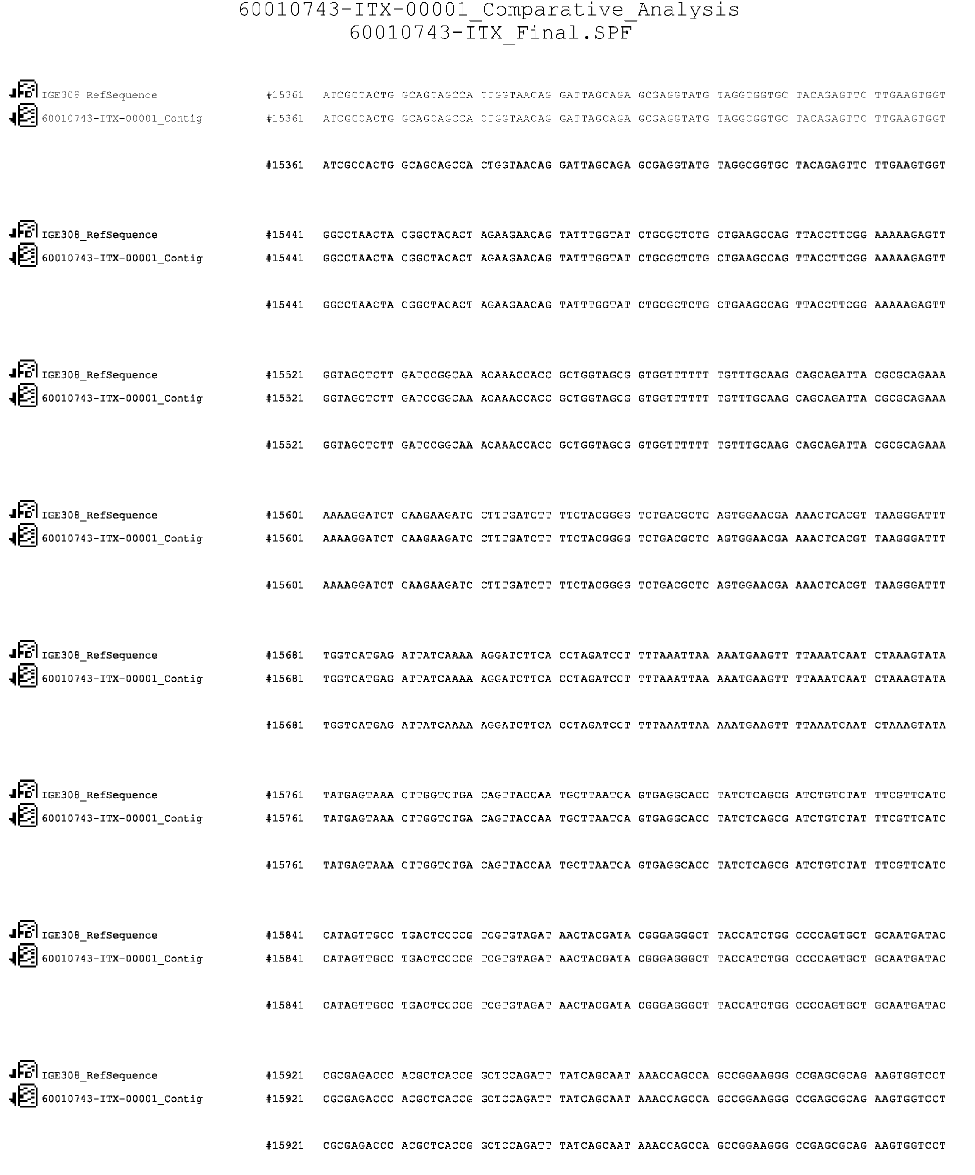

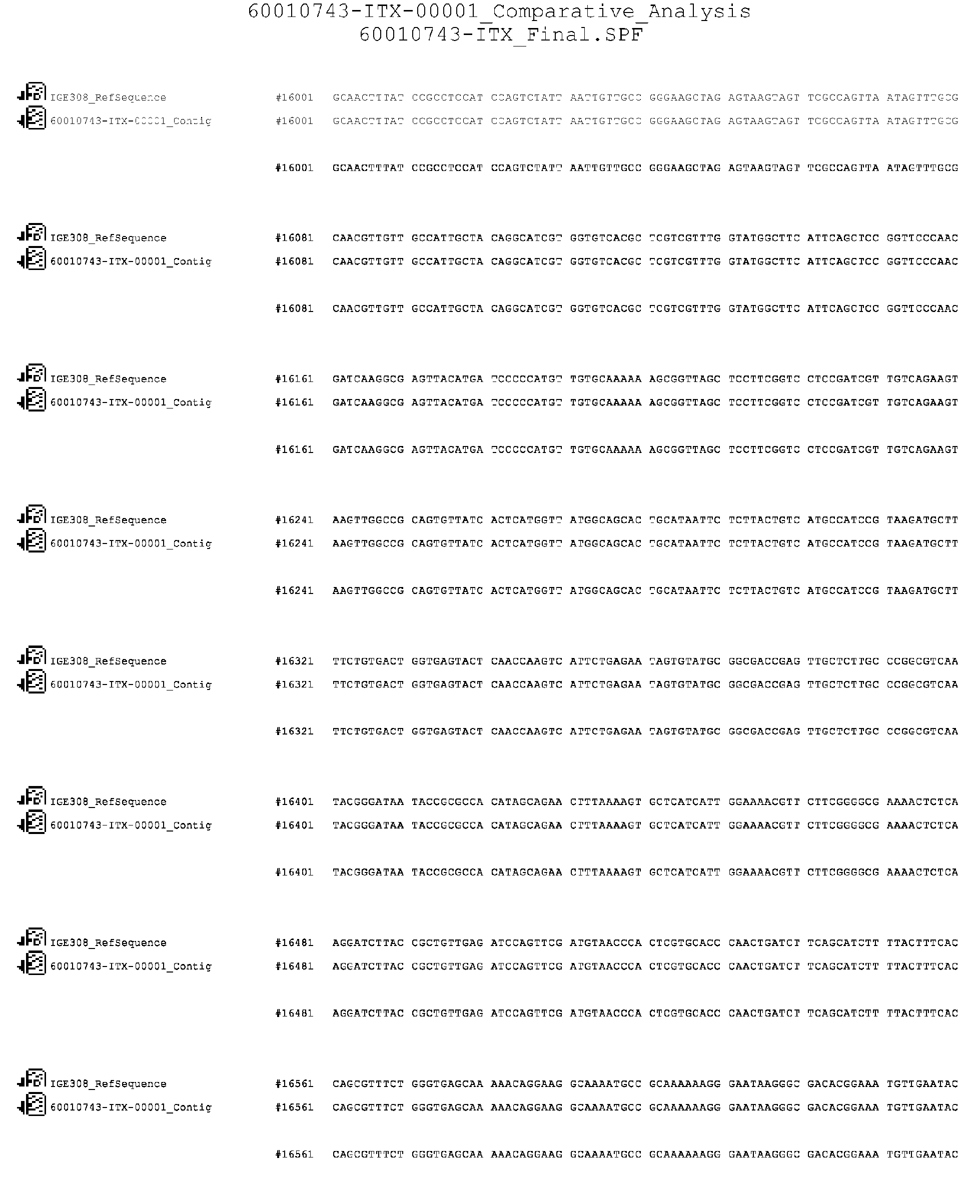

### REFERENCES

A. Aiuti et al. Lentiviral hematopoietic stem cell gene therapy benefits metachromatic leukodystrophy. Science, 2013. August; 341:6148.

Baldeschi C, Gache Y, Rattenholl A, Bouillé P, Danos O, Ortonne JP, Bruckner-Tuderman L, Meneguzzi G. 2003. Genetic correction of canine dystrophic epidermolysis bullosa mediated by retroviral vectors. Human Molecular Genetics 12: 1897-905.

A. Biffi et al. Lentiviral hematopoietic stem cell gene therapy benefits metachromatic leukodystrophy. Science, 2013. August; 341:6148.

Bruckner-Tuderman L, Hopfner B, Hammami-Hauasli N. 1999. Biology of anchoring fibrils: lessons from dystrophic epidermolysis bullosa. Matirx Biology 18: 43-54.

Burgeson R.E. Type VII collagen, anchoring fibrils, and epidermolysis bullosa. J. Invest. Dermatol. 1993;101:252-255.

Chen M, O'Toole EA, Muellenhoff M, Medina E, Kasahara N, Woodley DT. 2000. Development and characterization of a recombinant truncated type VII collagen "minigene". Implication for gene therapy of dystrophic epidermolysis bullosa. J Biol Chem 275: 24429-35.

Chen M, Costa FK, Lindvay CR, Han YP, Woodley DT. 2002a. The recombinant expression of full-length type VII collagen and characterization of molecular mechanisms underlying dystrophic epidermolysis bullosa. J Biol Chem 277: 2118-24.

Chen M, Kasahara N, Keene DR, Chan L, Hoeffler WK, Finlay D, Barcova M, Cannon PM, Mazurek C, Woodley DT. 2002b. Restoration of type VII collagen expression and function in dystrophic epidermolysis bullosa. Nature Genetics 32: 670-5.

Cogan J, Weinstein J, Wang X, Hou Y, Martin S, South AP, Woodley DT, Chen M. 2014. Aminoglycosides restore full-length type VII collagen by overcoming premature termination codons: therapeutic implications for dystrophic epidermolysis bullosa. Molecular Therapy 22: 1741-52.

Deshpande P, Ralston DR, and S McNeil. The use of allodermis prepared from Euro skin bank to prepare autologous tissue engineered skin for clinical use. Burns. 2013. 39(6): 1170-1177.

Goto, M., et al., Fibroblasts show more potential as target cells than keratinocytes in COL7A1 gene therapy of dystrophic epidermolysis bullosa. J Invest Dermatol, 2006. 126(4): p. 766-72.

Greenberg KP, Edwin SL, Schaffer DV, Flannery JG. 2006. Gene Delivery to the Retina Using Lentiviral Vectors. Advances in Experimental Medicine and Biology 572: 255-66

Kim YH, Woodley DT, Wynn KC, Giomi W, Bauer EA. Recessive Dystrophic Epidermylosis Bullosa Phenotype is Preserved in Xenografts Using SCID Mice: Development of an Experimental In Vivo Model. J Invest Dermatol. 1992 Feb;98(2):191-7.

Keene DR, Sakai LY, Lunstrum GP, Morris NP, Burgeson RE. Type VII Collagen Forms an Extended Network of Anchoring Fibrils. J Cell Biol. 1987 Mar;104(3):611-21.

Leigh I.M. Eady R.A. Heagerty A.H. Purkis P.E. Whitehead P.A. Burgeson R.E. Type VII collagen is a normal component of epidermal basement membrane, which shows altered expression in recessive dystrophic epidermolysis bullosa. J. Invest. Dermatol. 1988;90:639-642.

Mavilio F. Pellegrini G. Ferrari S. Di Nunzio F. Di Iorio E. Recchia A. Maruggi G. Ferrari G. Provasi E. Bonini C. Capurro S. Conti A. Magnoni C. Giannetti A. De Luca M. Correction of junctional epidermolysis bullosa by transplantation of genetically modified epidermal stem cells. Nat. Med. 2006;12:1397-1402.

Ortiz-Urda S. Lin Q. Green C.L. Keene D.R. Marinkovich M.P. Khavari P.A. Injection of genetically engineered fibroblasts corrects regenerated human epidermolysis bullosa skin tissue. J. Clin. Invest. 2003;111:251-255.

Remington J. Wang X. Hou Y. Zhou H. Burnett J. Muirhead T. Uitto J. Keene D.R. Woodley D.T. Chen M. Injection of recombinant human type VII collagen corrects the disease phenotype in a murine model of dystrophic epidermolysis bullosa. Mol. Ther. 2009;17:26-33.

Rousselle P, Keene DR, Ruggiero F, Champliaud MF, Rest M, Burgeson RE. 1997. Laminin 5 binds the NC-1 domain of type VII collagen. J Cell Bio, 138: 719-28.

Siprashvili Z, Ngon T. Nguyen, Maria Y. Bezchinsky, M. Peter Marinkovich, Alfred T. Lane, and Paul A. Khavari. Long-term type VII collagen restoration to human epidermylosis bullosa skin tissue. Hum Gene Ther. Oct 2010; 21(10): 1299-1310.

Tareen S, Nicolai C, Campbell D, Flynn P, Slough M, Vin C, Kelly-Clarke B, Odegard J, Robbins S. 2013. A Rev-Independent gag/pol Eliminates Detectable psi-gag Recombination in Lentiviral Vectors. Biores Open Access. 2013 Dec 1; 2(6): 421-430.

Waterman EA, Sakai N, Nguyen NT, Horst BA, Veitch DP, Dey CN, Ortiz-Urda S, Khavari PA, Marinkovich MP. 2007. A laminin-collagen complex drives human epidermal carcinogenesis through phosphoinositol-3-kinase activation. Cancer Research 67: 4264-70.

Woodley D.T. Keene D.R. Atha T. Huang Y. Ram R. Kasahara N. Chen M. Intradermal injection of lentiviral vectors corrects regenerated human dystrophic epidermolysis bullosa skin tissue in vivo. Mol. Ther. 2004;10:318-326.

Woodley D.T. Krueger G.G. Jorgensen C.M. Fairley J.A. Atha T. Huang Y. Clian L. Keene D.R. Chen M. Normal and gene-corrected dystrophic epidermolysis bullosa fibroblasts alone can produce type VII collagen at the basement membrane zone. J. Invest. Dermatol. 2003;121:1021-1028.

Woodley D.T. Keene D.R. Atha T. Huang Y. Lipman K. Li W. Chen M. Injection of recombinant human type VII collagen restores collagen function in dystrophic epidermolysis bullosa. Nat. Med. 2004;10:693-695.

## Claims

1. Autologous genetically modified cells for use in a method for treating a patient suffering from a type VII collagen (C7) deficiency, wherein said method comprises:
(i) contacting cells, obtained from the dermis or epidermis of the C7-deficient patient, with a transducing lentiviral vector comprising a nucleotide sequence encoding COL7A1 or a functional variant thereof to form the autologous genetically modified cells having a vector copy number wherein said lentiviral vector has a transducing vector copy number in the range of 0.1 to 5.0 copies per cell;
(ii) culturing said autologous genetically modified cells; and
(iii) administering an effective amount of the genetically modified cells to the C7-deficient patient,
wherein the transducing lentiviral vector further comprises (a) a modified 5' long terminal repeat (LTR), wherein the promoter of the modified 5' LTR is a cytomegalovirus promoter, (b) at least one lentiviral central polypurine tract element, and (c) a modified 3' LTR, wherein the modified 3' LTR comprises a deletion relative to the wild-type 3' LTR, wherein a hepatitis virus post-transcriptional regulatory element (PRE) has been deleted.

2. The autologous genetically modified cells for the use of claim 1, wherein the cells are selected from fibroblasts and keratinocytes.

3. The autologous genetically modified cells for the use of claim 1, wherein the C7-deficiency is dystrophic epidermolysis bullosa.

4. The autologous genetically modified cells for the use of claim 1, wherein the C7-deficiency is a dystrophic epidermolysis bullosa, recessive dystrophic epidermolysis bullosa (RDEB), dominant dystrophic epidermolysis bullosa (DDEB), Hallopeau-Siemens, non-Hallopeau-Siemens RDEB, RDEB inversa, pretibial RDEB, acral RDEB, or RDEB centripetalis.

5. The autologous genetically modified cells for the use of claim 1, wherein said transducing lentiviral vector is in the form of a lentiviral vector particle.

6. The autologous genetically modified cells for the use of claim 1 or claim 2, wherein the genetically modified cells are to be administered to the patient by injection, topically, orally, or embedded in a biocompatible matrix.

7. An autologous genetically modified fibroblast from a patient suffering from a Type VII collagen deficiency transduced with a lentiviral vector particle comprising a functional *COL7A1* gene or a functional variant thereof, and expressing type VII collagen, wherein said lentiviral vector particle has a transducing vector copy number in the range of 0.1 to 5.0 copies per cell, wherein said transducing lentiviral vector particle is constructed from a transfer lentiviral vector comprising (a) a modified 5' long terminal repeat in LTR, wherein the promoter of the modified 5' LTR is a cytomegalovirus promoter, (b) a functional *COL7A1* gene, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted, wherein the *COL7A1* gene or the functional variant thereof is incorporated into the cells to form genetically modified cells having a functional *COL7A1* gene.

8. A self-inactivating lentiviral vector formed from a transfer vector comprising (a) a modified 5' long terminal in LTR, wherein the promoter of the modified 5' LTR is a cytomegalovirus promoter, (b) the *COL7A1* gene or a functional variant thereof, (c) at least one lentiviral central polypurine tract element, and (d) a modified 3' LTR, wherein the modified 3' LTR comprises a deletion relative to the wild-type 3' LTR, and, wherein a hepatitis virus post-transcriptional regulatory element (PRE) has been deleted, wherein the deleted PRE is a woodchuck post-transcriptional regulatory element PRE (WPRE).

9. A transfer vector comprising the amino acid sequence set forth in SEQ ID NO: 34.

10. A lentiviral vector particle comprising the self-inactivating lentiviral vector of claim 8 or the transfer vector of claim 9.

11. A lentiviral vector particle constructed from a pSMPUW lentiviral expression plasmid comprising (a) a modified 5' long terminal repeat in LTR, wherein the promoter of the modified 5' LTR is a cytomegalovirus promoter, (b) a functional *COL7A1* gene, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted.

12. A stable virus packaging cell line producing the lentiviral vector particle of claim 10 or claim 11.

13. A pharmaceutical composition comprising a fibroblast obtained from a patient suffering from RDEB or DDEB transduced with the lentiviral vector of claim 10 or claim 11.

14. A cell transduced *in vitro* or *ex vivo* with the vector of claim 10 or claim 11.

15. An autologous population of cells for use in treating a patient suffering from pseudosyndactyly, wherein said autologous population of cells is to be obtained from said patient and transduced with a lentiviral vector particle comprising a functional *COL7A1* gene or a functional variant thereof, and expressing type VII collagen, wherein said lentiviral vector particle has a transducing vector copy number in the range of 0.1 to 5.0 copies per cell, wherein said transducing lentiviral vector particle is constructed from a transfer lentiviral vector comprising (a) a modified 5' long terminal repeat in LTR, wherein the promoter of the modified 5' LTR is a cytomegalovirus promoter, (b) a functional *COL7A1* gene, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted, wherein the *COL7A1* gene or the functional variant thereof is incorporated into the cells to form genetically modified cells having a functional *COL7A1* gene.

16. A method of making an isolated population of Type VII collagen (C7) expressing genetically modified cells autologous to a patient suffering from C7 deficiency comprising: contacting cells, obtained from the dermis or epidermis of the C7-deficient patient, with a transducing lentiviral vector particle comprising the *COL7A1* gene or a functional variant thereof to form autologous genetically modified cells comprising the *COL7A1* gene having a vector copy number of wherein said lentiviral vector particle has a transducing vector copy number in the range of 0.1 to 5.0 copies per cell, and culturing said autologous genetically modified cells to obtain an isolated population of C7 expressing genetically modified cells, wherein said transducing lentiviral vector particle is constructed from a transfer lentiviral vector comprising (a) a modified 5' long terminal repeat in LTR, wherein the promoter of the modified 5' LTR is a cytomegalovirus promoter, (b) a functional *COL7A1* gene, (c) at least one lentiviral central polypurine tract, and (d) a modified 3' LTR, wherein the modified 3' LTR comprises a deletion relative to the wild-type 3' LTR, wherein a hepatitis virus post-transcriptional regulatory element (PRE) has been deleted, wherein the *COL7A1* gene or the functional variant thereof is incorporated into the cells to form genetically modified cells having a functional *COL7A1* gene.

17. An isolated population of Type VII collagen (C7)-expressing genetically modified cells produced by the method of claim 16.

18. A pharmaceutical formulation comprising the isolated population of claim 17.

19. A method of increasing the integrated transgene copy number per cell in genetically modified human dermal fibroblasts or keratinocytes comprising
(a) contacting a transducing lentiviral vector comprising a nucleotide sequence encoding a *COL7A1* gene or a functional variant thereof with a human dermal fibroblast or keratinocyte obtained from a C7-deficient patient to form a first transduction composition;
(b) subjecting said first transduction composition to spinoculation to form transduced human dermal fibroblast or keratinocyte;
(c) contacting the transduced human dermal fibroblasts or keratinocytes of step (b) with a second transducing lentiviral vector to form a second transduction composition; and
(d) subjecting said first transduction composition to spinoculation to form transduced human dermal fibroblast or keratinocyte,
wherein the integrated transgene copy number of the transduced human dermal fibroblasts or keratinocytes is at least 5, 10, 15, 20, 25, 27, 28, 29, 30, 35, 40, 45, or 50- fold higher relative to transduced human dermal fibroblasts or keratinocytes not subjected to spinoculation or a second transduction step, and wherein the transducing lentiviral vector further comprises (a) a modified 5' long terminal repeat (LTR), wherein the promoter of the modified 5' LTR is a cytomegalovirus promoter, (b) at least one lentiviral central polypurine tract element, and (c) a modified 3' LTR, wherein the modified 3' LTR comprises a deletion relative to the wild-type 3' LTR, wherein a hepatitis virus post- transcriptional regulatory element (PRE) has been deleted.

## Patentansprüche

1. Autologe genetisch modifizierte Zellen zur Verwendung in einem Verfahren zur Behandlung eines Patienten, der an einem Mangel von Kollagen Typ VII (C7) leidet, wobei das Verfahren umfasst:
(i) Inkontaktbringen von Zellen, erhalten aus der Dermis oder Epidermis des Patienten mit C7-Mangel, mit einem transduzierenden lentiviralen Vektor, umfassend eine Nukleotidsequenz, die *COL7A1* oder eine funktionelle Variante davon kodiert, um die autologen genetisch modifizierten Zellen zu bilden, die eine Vektorkopienzahl aufweisen, wobei der lentivirale Vektor eine transduzierende Vektorkopienzahl im Bereich von 0,1 bis 5,0 Kopien pro Zelle aufweist;
(ii) Kultivieren der autologen genetisch modifizierten Zellen; und
(iii) Verabreichen einer wirksamen Menge der genetisch modifizierten Zellen an den Patienten mit C7-Mangel,
wobei der transduzierende lentivirale Vektor ferner (a) einen modifizierten 5'-Long Terminal Repeat (LTR), wobei der Promotor des modifizierten 5'-LTR ein Cytomegalovirus-Promotor ist, (b) mindestens ein lentivirales zentrales Polypurintraktelement und (c) einen modifizierten 3'-LTR umfasst, wobei der modifizierte 3'-LTR eine Deletion bezüglich dem Wildtyp-3'-LTR umfasst, wobei ein posttranskriptionelles regulatorisches Element (PRE) des Hepatitis-Virus deletiert wurde.

2. Autologe genetisch modifizierte Zellen für die Verwendung nach Anspruch 1, wobei die Zellen ausgewählt sind aus Fibroblasten und Keratinozyten.

3. Autologe genetisch modifizierte Zellen für die Verwendung nach Anspruch 1, wobei der C7-Mangel dystrophische Epidermolysis bullosa ist.

4. Autologe genetisch modifizierte Zellen für die Verwendung nach Anspruch 1, wobei der C7-Mangel dystrophische Epidermolysis bullosa, rezessive dystrophische Epidermolysis bullosa (RDEB), dominante dystrophische Epidermolysis bullosa (DDEB), Hallopeau-Siemens, Nicht-Hallopeau-Siemens RDEB, RDEB inversa, prätibiale RDEB, acrale RDEB oder RDEB centripetalis ist.

5. Autologe genetisch modifizierte Zellen für die Verwendung nach Anspruch 1, wobei der transduzierende lentivirale Vektor in Form eines lentiviralen Vektorteilchens vorliegt.

6. Autologe genetisch modifizierte Zellen für die Verwendung nach Anspruch 1 oder Anspruch 2, wobei die genetisch modifizierten Zellen dem Patienten durch Injektion, topisch, oral oder in eine biokompatible Matrix eingebettet zu verabreichen sind.

7. Autologer genetisch modifizierter Fibroblast von einem Patienten, der an einem Mangel von Kollagen Typ VII leidet, der mit einem lentiviralen Vektorteilchen, umfassend ein funktionelles *COL7A1*-Gen oder eine funktionelle Variante davon, und das Kollagen Typ VII exprimiert, transduziert ist, wobei das lentivirale Vektorteilchen eine transduzierende Vektorkopienzahl im Bereich von 0,1 bis 5,0 Kopien pro Zelle aufweist, wobei das transduzierende lentivirale Vektorteilchen aus einem lentiviralen Transfervektor konstruiert ist, umfassend (a) einen modifizierten 5'-Long Terminal Repeat in LTR, wobei der Promotor des modifizierten 5'-LTR ein Cytomegalovirus-Promotor ist, (b) ein funktionelles *COL7A1*-Gen, (c) mindestens einen lentiviralen zentralen Polypurintrakt und (d) einen modifizierten 3'-LTR, wobei der modifizierte 3'-LTR eine Deletion bezüglich dem Wildtyp-3'-LTR umfasst, wobei ein posttranskriptionelles regulatorisches Element (PRE) des Hepatitis-Virus deletiert wurde, wobei das *COL7A1*-Gen oder die funktionelle Variante davon in die Zellen eingebaut wird, um genetisch modifizierte Zellen zu bilden, die ein funktionelles *COL7A*1-Gen aufweisen.

8. Selbstinaktivierender lentiviraler Vektor, gebildet aus einem Transfervektor, umfassend (a) einen modifizierten 5'-Long Terminal Repeat in LTR, wobei der Promotor des modifizierten 5'-LTR ein Cytomegalovirus-Promotor ist, (b) das *COL7A1*-Gen oder eine funktionelle Variante davon, (c) mindestens ein lentivirales zentrales Polypurintraktelement und (d) einen modifizierten 3'-LTR, wobei der modifizierte 3'-LTR eine Deletion bezüglich dem Wildtyp-3'-LTR umfasst, und wobei ein posttranskriptionelles regulatorisches Element (PRE) des Hepatitis-Virus deletiert wurde, wobei das deletierte PRE ein posttranskriptionelles regulatorisches Element des Murmeltiers (WPRE) ist.

9. Transfervektor, umfassend die Aminosäuresequenz, dargelegt in SEQ ID NO: 34.

10. Lentivirales Vektorteilchen, umfassend den selbstinaktivierenden lentiviralen Vektor nach Anspruch 8 oder den Transfervektor nach Anspruch 9.

11. Lentivirales Vektorteilchen, konstruiert aus einem pSMPUW-lentiviralen Expressionsplasmid, umfassend (a) einen modifizierten 5'-Long Terminal Repeat in LTR, wobei der Promotor des modifizierten 5'-LTR ein Cytomegalovirus-Promotor ist, (b) ein funktionelles *COL7A1*-Gen, (c) mindestens einen lentiviralen zentralen Polypurintrakt und (d) einen modifizierten 3'-LTR, wobei der modifizierte 3'-LTR eine Deletion bezüglich dem Wildtyp-3'-LTR umfasst, wobei ein posttranskriptionelles regulatorisches Element (PRE) des Hepatitis-Virus deletiert wurde.

12. Stabile Virusverpackungszelllinie, die das lentivirale Vektorteilchen nach Anspruch 10 oder Anspruch 11 herstellt.

13. Pharmazeutische Zusammensetzung, umfassend einen Fibroblasten, erhalten von einem Patienten, der an RDEB oder DDEB leidet, der mit dem lentiviralen Vektor nach Anspruch 10 oder Anspruch 11 transduziert ist.

14. Zelle, transduziert *in vitro* oder *ex vivo* mit dem Vektor nach Anspruch 10 oder Anspruch 11.

15. Autologe Population von Zellen zur Verwendung bei der Behandlung eines Patienten, der an Pseudosyndatylie leidet, wobei die autologe Population von Zellen von dem Patienten zu erhalten und mit einem lentiviralen Vektorteilchen zu transduzieren ist, das ein funktionelles *COL7A1*-Gen oder eine funktionelle Variante davon umfasst, und das Kollagen Typ VII exprimiert, wobei das lentivirale Vektorteilchen eine transduzierende Vektorkopienzahl im Bereich von 0,1 bis 5,0 Kopien pro Zelle aufweist, wobei das transduzierende lentivirale Vektorteilchen aus einem lentiviralen Transfervektor konstruiert ist, umfassend (a) einen modifizierten 5'-Long Terminal Repeat in LTR, wobei der Promotor des modifizierten 5'-LTR ein Cytomegalovirus-Promotor ist, (b) ein funktionelles *COL7A1*-Gen, (c) mindestens einen lentiviralen zentralen Polypurintrakt und (d) einen modifizierten 3'-LTR, wobei der modifizierte 3'-LTR eine Deletion bezüglich dem Wildtyp-3'-LTR umfasst, wobei ein posttranskriptionelles regulatorisches Element (PRE) des Hepatitis-Virus deletiert wurde, wobei das COL7A1-Gen oder die funktionelle Variante davon in die Zellen eingebaut wird, um genetisch modifizierte Zellen zu bilden, die ein funktionelles *COL7A1*-Gen aufweisen.

16. Verfahren zur Herstellung einer isolierten Population von genetisch modifizierte Zellen, die Kollagen Typ VII (C7) exprimieren, und die zu einem Patienten, der an einem C7-Mangel leidet, autolog sind, umfassend: Inkontaktbringen von Zellen, erhalten aus der Dermis oder Epidermis des Patienten mit C7-Mangel, mit einem transduzierenden lentiviralen Vektorteilchen, umfassend das *COL7A1*-Gen oder eine funktionelle Variante davon, um autologe genetisch modifizierte Zellen zu bilden, die das *COL7A1*-Gen umfassen, das eine Vektorkopienzahl von aufweist, wobei das lentivirale Vektorteilchen eine transduzierende Vektorkopienzahl im Bereich von 0,1 bis 5,0 Kopien pro Zelle aufweist, und Kultivieren der autologen genetisch modifizierten Zellen, um eine isolierte Population von C7-exprimierenden genetisch modifizierten Zellen zu erhalten, wobei das transduzierende lentivirale Vektorteilchen aus einem lentiviralen Transfervektor konstruiert ist, umfassend (a) einen modifizierten 5'-Long Terminal Repeat in LTR, wobei der Promotor des modifizierten 5'-LTR ein Cytomegalovirus-Promotor ist, (b) ein funktionelles *COL7A*1-Gen, (c) mindestens einen lentiviralen zentralen Polypurintrakt und (d) einen modifizierten 3'-LTR, wobei der modifizierte 3'-LTR eine Deletion bezüglich dem Wildtyp-3'-LTR umfasst, wobei ein posttranskriptionelles regulatorisches Element (PRE) des Hepatitis-Virus deletiert wurde, wobei das COL7A1-Gen oder die funktionelle Variante davon in die Zellen eingebaut wird, um genetisch modifizierte Zellen zu bilden, die ein funktionelles *COL7A*1-Gen aufweisen.

17. Isolierte Population von Kollagen Typ VII(C7)-exprimierenden genetisch modifizierten Zellen, hergestellt durch das Verfahren nach Anspruch 16.

18. Pharmazeutische Formulierung, umfassend die isolierte Population nach Anspruch 17.

19. Verfahren zum Erhöhen der integrierten Transgen-Kopienzahl pro Zelle in genetisch modifizierten humanen dermalen Fibroblasten oder Keratinozyten, umfassend
(a) Inkontaktbringen eines transduzierenden lentiviralen Vektors, umfassend eine Nukleotidsequenz, die ein *COL7A*1-Gen oder eine funktionelle Variante davon kodiert, mit einem humanen dermalen Fibroblasten oder Keratinozyten, erhalten aus einem Patienten mit C7-Mangel, um eine erste Transduktionszusammensetzung zu bilden;
(b) Unterziehen der ersten Transduktionszusammensetzung einer Spinokulation, um transduzierten humanen dermalen Fibroblasten oder Keratinozyten zu bilden;
(c) Inkontaktbringen des transduzierten humanen dermalen Fibroblasten oder Keratinozyten von Schritt (b) mit einem zweiten transduzierenden lentiviralen Vektor, um eine zweite Transduktionszusammensetzung zu bilden; und
(d) Unterziehen der ersten Transduktionszusammensetzung einer Spinokulation, um transduzierten humanen dermalen Fibroblasten oder Keratinozyten zu bilden,
wobei die integrierte Transgen-Kopienzahl der transduzierten humanen dermalen Fibroblasten oder Keratinozyten mindestens 5-, 10-, 15-, 20-, 25-, 27-, 28-, 29-, 30-, 35-, 40-, 45- oder-50-fach höher ist, bezüglich transduzierter humaner dermaler Fibroblasten oder Keratinozyten, die nicht einer Spinokulation oder einem zweiten Transduktionsschritt unterzogen werden, und wobei der transduzierende lentivirale Vektor ferner (a) einen modifizierten 5'-Long Terminal Repeat (LTR), wobei der Promotor des modifizierten 5'-LTR ein Cytomegalovirus-Promotor ist, (b) mindestens ein lentivirales zentrales Polypurintraktelement und (c) einen modifizierten 3'-LTR umfasst, wobei der modifizierte 3'-LTR eine Deletion bezüglich dem Wildtyp-3'-LTR umfasst, wobei ein posttranskriptionelles regulatorisches Element (PRE) des Hepatitis-Virus deletiert wurde.

## Revendications

1. Cellules autologues génétiquement modifiées pour utilisation dans un procédé de traitement d'un patient souffrant d'un déficit en collagène de type VII (C7), dans lequel ledit procédé comprend :
(i) la mise en contact de cellules, obtenues à partir du derme ou de l'épiderme du patient déficitaire en C7, avec un vecteur lentiviral de transduction comprenant une séquence de nucléotides codant pour COL7A1 ou un variant fonctionnel de celui-ci pour former les cellules autologues génétiquement modifiées ayant un nombre de copies de vecteur dans lequel ledit vecteur lentiviral a un nombre de copies de vecteur de transduction dans la plage de 0,1 à 5,0 copies par cellule ;
(ii) la culture desdites cellules autologues génétiquement modifiées ; et
(iii) l'administration d'une quantité efficace des cellules génétiquement modifiées au patient déficitaire en C7,
dans lesquelles le vecteur lentiviral de transduction comprend en outre (a) une longue répétition terminale (LTR) 5' modifiée, dans lesquelles le promoteur de la LTR 5' modifiée est un promoteur de cytomégalovirus, (b) au moins un élément de tractus de polypurine central lentiviral, et (c) une LTR 3' modifiée, dans lesquelles la LTR 3' modifiée comprend une délétion par rapport à la LTR 3' de type sauvage, dans lesquelles un élément régulateur post-transcriptionnel (PRE) de virus d'hépatite a été délété.

2. Cellules autologues génétiquement modifiées pour l'utilisation selon la revendication 1, dans lesquelles les cellules sont sélectionnées parmi des fibroblastes et des kératinocytes.

3. Cellules autologues génétiquement modifiées pour l'utilisation selon la revendication 1, dans lesquelles le déficit en C7 est une épidermolyse bulleuse dystrophique.

4. Cellules autologues génétiquement modifiées pour l'utilisation selon la revendication 1, dans lesquelles le déficit en C7 est une épidermolyse bulleuse dystrophique, une épidermolyse bulleuse dystrophique récessive (RDEB), une épidermolyse bulleuse dystrophique dominante (DDEB), une RDEB de Hallopeau-Siemens, une RDEB non Hallopeau-Siemens, une RDEB inversa, une RDEB prétibiale, une RDEB acrale ou une RDEB centripète.

5. Cellules autologues génétiquement modifiées pour l'utilisation selon la revendication 1, dans lesquelles ledit vecteur lentiviral de transduction est sous la forme d'une particule de vecteur lentiviral.

6. Cellules autologues génétiquement modifiées pour l'utilisation selon la revendication 1 ou la revendication 2, dans lesquelles les cellules génétiquement modifiées doivent être administrées au patient par injection, par voie topique, par voie orale, ou intégrées dans une matrice biocompatible.

7. Fibroblaste autologue génétiquement modifié provenant d'un patient souffrant d'un déficit en collagène de type VII transduit avec une particule de vecteur lentiviral comprenant un gène *COL7A1* fonctionnel ou un variant fonctionnel de celui-ci, et exprimant du collagène de type VII, dans lequel ladite particule de vecteur lentiviral a un nombre de copies de vecteur de transduction dans la plage de 0,1 à 5,0 copies par cellule, dans lequel ladite particule de vecteur lentiviral de transduction est construite à partir d'un vecteur lentiviral de transfert comprenant (a) une longue répétition terminale 5' modifiée dans la LTR, dans lequel le promoteur de la LTR 5' modifiée est un promoteur de cytomégalovirus, (b) un gène *COL7A1* fonctionnel, (c) au moins un tractus de polypurine lentiviral central, et (d) une LTR 3' modifiée, dans lequel la LTR 3' modifiée comprend une délétion par rapport à la LTR 3' de type sauvage, dans lequel un élément régulateur post-transcriptionnel (PRE) de virus d'hépatite a été délété, dans lequel le gène *COL7A1* ou le variant fonctionnel de celui-ci est incorporé dans les cellules pour former des cellules génétiquement modifiées ayant un gène *COL7A1* fonctionnel.

8. Vecteur lentiviral à auto-inactivation formé à partir d'un vecteur de transfert comprenant (a) une longue terminaison 5' modifiée dans LTR, dans lequel le promoteur de la LTR 5' modifiée est un promoteur de cytomégalovirus, (b) le gène *COL7A1* ou un variant fonctionnel de celui-ci, (c) au moins un élément de tractus de polypurine central lentiviral, et (d) une LTR 3' modifiée, dans lequel la LTR 3' modifiée comprend une délétion par rapport à la LTR 3' de type sauvage, et, dans lequel un élément régulateur post-transcriptionnel (PRE) de virus d'hépatite a été délété, dans lequel le PRE délété est un élément régulateur post-transcriptionnel PRE de marmotte (WPRE).

9. Vecteur de transfert comprenant la séquence d'acides aminés présentée dans SEQ ID NO: 34.

10. Particule de vecteur lentiviral comprenant le vecteur lentiviral à auto-inactivation selon la revendication 8 ou le vecteur de transfert selon la revendication 9.

11. Particule de vecteur lentiviral construite à partir d'un plasmide d'expression lentivirale pSMPUW comprenant (a) une longue répétition terminale 5' modifiée dans la LTR, dans laquelle le promoteur de la LTR 5' modifiée est un promoteur de cytomégalovirus, (b) un gène *COL7A1* fonctionnel, (c) au moins un tractus de polypurine lentiviral central, et (d) une LTR 3' modifiée, dans laquelle la LTR 3' modifiée comprend une délétion par rapport à la LTR 3' de type sauvage, dans laquelle un élément régulateur post-transcriptionnel (PRE) de virus d'hépatite a été délété.

12. Lignée cellulaire d'encapsidation stable produisant la particule de vecteur lentiviral selon la revendication 10 ou la revendication 11.

13. Composition pharmaceutique comprenant un fibroblaste obtenu auprès d'un patient souffrant de RDEB ou de DDEB transduit avec le vecteur lentiviral selon la revendication 10 ou la revendication 11.

14. Cellule transduite *in vitro* ou *ex vivo* avec le vecteur selon la revendication 10 ou la revendication 11.

15. Population autologue de cellules pour une utilisation dans le traitement d'un patient souffrant de pseudosyndactylie, dans laquelle ladite population de cellules doit être obtenue auprès dudit patient et transduite avec une particule de vecteur lentiviral comprenant un gène COL7A1 fonctionnel ou un variant fonctionnel de celui-ci, et exprimant du collagène de type VII, dans laquelle ladite particule de vecteur lentiviral a un nombre de copies de vecteur de transduction dans la plage de 0,1 à 5,0 copies par cellule, dans laquelle ladite particule de vecteur lentiviral de transduction est construite à partir d'un vecteur lentiviral de transfert comprenant (a) une longue répétition terminale 5' modifiée dans la LTR, dans laquelle le promoteur de la LTR 5' modifiée est un promoteur de cytomégalovirus, (b) un gène *COL7A1* fonctionnel, (c) au moins un tractus de polypurine lentiviral central, et (d) une LTR 3' modifiée, dans laquelle la LTR 3' modifiée comprend une délétion par rapport à la LTR 3' de type sauvage, dans laquelle un élément régulateur post-transcriptionnel (PRE) de virus d'hépatite a été délété, dans laquelle le gène *COL7A1* ou le variant fonctionnel de celui-ci est incorporé dans les cellules pour former des cellules génétiquement modifiées ayant un gène *COL7A1* fonctionnel.

16. Procédé de fabrication d'une population isolée de cellules génétiquement modifiées exprimant du collagène de type VII (C7) autologues à un patient souffrant d'un déficit en C7 comprenant : la mise en contact de cellules, obtenues à partir du derme ou de l'épiderme du patient déficitaire en C7, avec une particule de vecteur lentiviral de transduction comprenant le gène *COL7A1* ou un variant fonctionnel de celui-ci pour former des cellules autologues génétiquement modifiées comprenant le gène *COL7A1* ayant un nombre de copies de vecteur de dans lequel ladite particule de vecteur lentiviral a un nombre de copies de vecteur de transduction dans la plage de 0,1 à 5,0 copies par cellule, et la culture desdites cellules autologues génétiquement modifiées pour obtenir une population isolée de cellules génétiquement modifiées exprimant C7, dans lequel ladite particule de vecteur lentiviral de transduction est construite à partir d'un vecteur lentiviral de transfert comprenant (a) une longue répétition terminale 5' modifiée dans LTR, dans lequel le promoteur de la LTR 5' modifiée est un promoteur de cytomégalovirus, (b) un gène *COL7A1* fonctionnel, (c) au moins un tractus de polypurine lentiviral central, et (d) une LTR 3' modifiée, dans lequel la LTR 3' modifiée comprend une délétion par rapport à la LTR 3' de type sauvage, dans lequel un élément régulateur post-transcriptionnel (PRE) de virus d'hépatite a été délété, dans lequel le gène *COL7A1* ou le variant fonctionnel de celui-ci est incorporé dans les cellules pour former des cellules génétiquement modifiées ayant un gène *COL7A1* fonctionnel.

17. Population isolée de cellules génétiquement modifiées exprimant du collagène de type VII (C7) produite par le procédé selon la revendication 16.

18. Formulation pharmaceutique comprenant la population isolée selon la revendication 17.

19. Procédé d'augmentation du nombre de copies de transgènes intégrés par cellule dans des fibroblastes dermiques ou kératinocytes humains génétiquement modifiés comprenant
(a) la mise en contact d'un vecteur lentiviral de transduction comprenant une séquence de nucléotides codant pour un gène *COL7A1* ou un variant fonctionnel de celui-ci avec un fibroblaste dermique ou kératinocyte humain obtenu auprès d'un patient déficitaire en C7 pour former une première composition de transduction ;
(b) la soumission de ladite première composition de transduction à une spinoculation pour former un fibroblaste ou kératinocyte dermique humain transduit ;
(c) la mise en contact des fibroblastes dermiques ou kératinocytes humains transduits de l'étape (b) avec un deuxième vecteur lentiviral de transduction pour former une deuxième composition de transduction ; et
(d) la soumission de ladite première composition de transduction à une spinoculation pour former un fibroblaste ou kératinocyte dermique humain transduit,
dans lequel le nombre de copies de transgènes intégrés des fibroblastes ou kératinocytes dermiques humains transduits est au moins 5, 10, 15, 20, 25, 27, 28, 29, 30, 35, 40, 45, ou 50 fois plus élevé par rapport à des fibroblastes dermiques ou kératinocytes humains transduits non soumis à une spinoculation ou à une deuxième étape de transduction, et dans lequel le vecteur lentiviral de transduction comprend en outre (a) une longue répétition terminale (LTR) 5' modifiée, dans lequel le promoteur de la LTR 5' modifiée est un promoteur de cytomégalovirus, (b) au moins un élément central lentiviral de tractus de polypurine, et (c) une LTR 3' modifiée, dans lequel la LTR 3' modifiée comprend une délétion par rapport à la LTR 3' de type sauvage, dans lequel un élément régulateur post-transcriptionnel (PRE) de virus d'hépatite a été délété.
